(19) Europäisches Patentamt
European Patent Office
Office européen des brevets

(11) EP 4 215 534 B1

(12) **EUROPEAN PATENT SPECIFICATION**

(45) Date of publication and mention of the grant of the patent:
**24.06.2026 Bulletin 2026/26**

(21) Application number: **21869461.0**

(22) Date of filing: **17.09.2021**

(51) International Patent Classification (IPC):
***C07D 513/04*** *(2006.01)* ***A61K 31/429*** *(2006.01)*
***A61K 31/437*** *(2006.01)* ***A61K 31/5377*** *(2006.01)*
***A61K 31/55*** *(2006.01)* ***A61P 7/06*** *(2006.01)*
***A61P 9/10*** *(2006.01)* ***A61P 13/12*** *(2006.01)*
***A61P 19/08*** *(2006.01)* ***A61P 19/10*** *(2006.01)*
***A61P 21/00*** *(2006.01)* ***A61P 25/00*** *(2006.01)*
***A61P 25/16*** *(2006.01)* ***A61P 25/24*** *(2006.01)*
***A61P 25/28*** *(2006.01)* ***A61P 35/00*** *(2006.01)*
***A61P 43/00*** *(2006.01)* ***C07D 519/00*** *(2006.01)*

(52) Cooperative Patent Classification (CPC):
**A61P 7/06; C07D 513/04; C07D 519/00**

(86) International application number:
**PCT/JP2021/034327**

(87) International publication number:
**WO 2022/059779 (24.03.2022 Gazette 2022/12)**

(54) **AMINE DERIVATIVE**

AMINDERIVAT

DÉRIVÉ D’AMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.09.2020 JP 2020157398**
**28.07.2021 JP 2021122893**

(43) Date of publication of application:
**26.07.2023 Bulletin 2023/30**

(73) Proprietor: **Carna Biosciences, Inc.**
**Kobe-shi, Hyogo 650-0047 (JP)**

(72) Inventors:
- **TOJO, Shingo**
**Osaka-shi, Osaka 541-0045 (JP)**
- **URABE, Daisuke**
**Osaka-shi, Osaka 554-0022 (JP)**
- **WATANABE, Hitoshi**
**Osaka-shi, Osaka 554-0022 (JP)**
- **KAWAHATA, Wataru**
**Kobe-shi, Hyogo 650-0047 (JP)**
- **MORIYAMA, Hideki**
**Kobe-shi, Hyogo 650-0047 (JP)**
- **ASAMITSU, Yuko**
**Kobe-shi, Hyogo 650-0047 (JP)**

(74) Representative: **Vossius & Partner**
**Patentanwälte Rechtsanwälte mbB**
**Siebertstraße 3**
**81675 München (DE)**

(56) References cited:
**WO-A1-2021/153665**
**JP-A- 2003 521 543**
**JP-A- 2013 510 824**
**JP-A- 2014 525 928**
**US-A1- 2017 007 583**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present invention relates to a medicament, particularly a novel amine derivative having a DYRK inhibitory effect or a pharmaceutically acceptable salt thereof.

## Background Art

DYRK (dual-specificity tyrosine-phosphorylation regulated kinase) is one of the bispecific protein kinases that phosphorylate tyrosine, serine, and threonine. DYRK functions as a tyrosine kinase only in the case of autophosphorylation and catalyzes the phosphorylation of serine or threonine residues on exogenous substrates. Five members of the DYRK family are known in humans: DYRK1A, DYRK1B, DYRK2, DYRK3, and DYRK4 (Non Patent Literature 1).

It has been widely reported that DYRK1A is associated with neuropsychiatric diseases. For example, in patients with Alzheimer's disease, the expression of β-amyloid is significantly consistent with that of DYRK1A (Non Patent Literature 2), and it is speculated that DYRK1A is involved in abnormal phosphorylation of a tau protein (Tau), which is considered to contribute to the onset of Alzheimer's disease (Non Patent Literature 3).

In addition, Parkinson's disease is a neurodegenerative disease caused by the degeneration of dopamine neurons, which are important for motor function, but one of the causes is considered to be mitochondrial dysfunction (Non Patent Literature 4). An enzyme involved in protein degradation called Parkin is known to metabolize abnormal mitochondria and suppress abnormal accumulation, but DYRK1A has been reported to suppress the activity of this parkin protein (Non Patent Literature 5).

The gene for DYRK1A is located in the Down's syndrome critical region, and it has been reported that mice overexpressing DYRK1A exhibit neuropsychiatric dysfunction and appear like Down's syndrome (Non Patent Literature 6). It has also been reported that DYRK1A expression is increased in the brain of patients with Down's syndrome and Down's syndrome-like model mice (Non Patent Literature 7). These reports suggest that DYRK1A is involved in the onset of neurological symptoms in the patients with Down's syndrome (Non Patent Literature 8).

In addition, it has been reported that early-onset Alzheimer's disease occurs frequently in patients with Down's syndrome, thus indicating that DYRK1A is closely related to Alzheimer's disease (Non Patent Literature 8).

Therefore, compounds inhibiting DYRK1A are considered useful for treating neuropsychiatric diseases such as Alzheimer's disease, Down's syndrome, mental retardation, memory impairment, memory loss, and Parkinson's disease.

Recently, it has been reported that DYRK1A is highly expressed in brain tumors such as glioblastoma and regulates the expression of an epidermal growth factor receptor (EGFR) (Non Patent Literature 9). Therefore, compounds inhibiting DYRK1A are considered useful for treating EGFR-dependent cancers by suppressing the proliferation of cancer cells in EGFR-dependent brain tumors and other tumors.

Compounds inhibiting the family enzymes DYRK1B, DYRK2, and DYRK3 are also considered to have various pharmaceutical applications. For example, it has been reported that DYRK1B is highly expressed in quiescent (GO-phase) cancer cells and contributes to resistance to various chemotherapeutic agents (Non Patent Literature 10). It has also been reported that inhibition of DYRK1B promotes withdrawal from the G0 phase and enhances sensitivity to chemotherapeutic agents (Non Patent Literature 11). Therefore, compounds inhibiting DYRK1B are considered useful for treating pancreatic cancer, ovarian cancer, osteosarcoma, colorectal cancer, and lung cancer (Non Patent Literatures 11, 12, 13, 14, and 15).

It is suggested that DYRK2 controls p53 to induce apoptosis in response to DNA damages (Non Patent Literature 16). Furthermore, it has been reported that compounds inhibiting DYRK3 are useful for treating sickle cell anemia and chronic kidney disease (Non Patent Literature 17).

In addition to Patent Literature 1 for compounds inhibiting DYRK, Patent Literature 2 has been reported for DYRK1A and DYRK1B inhibitors. However, the alkyne derivative of the present invention is not disclosed therein.

## PRIOR ART DOCUMENT (S)

## PATENT DOCUMENT (S)

[Patent Literature 1] WO2010/10797
[Patent Literature 2] WO2013/26806

## NON-PATENT DOCUMENT(S)

[Non-Patent Literature 1] Becker W. et al., J.Biol.Chem., 1998, 273, 25893-25902
[Non-Patent Literature 2] Kimura R. et al., Hum.Mol.Genet., 2007, 16, 15-23
[Non-Patent Literature 3] Ryoo SR. et al., J.Biol.Chem., 2007, 282, 34850-34857
[Non-Patent Literature 4] Narendra D. et al., J.Cell.Biol., 2008, 183, 795-803
[Non-Patent Literature 5] Im E., J.Neurochem., 2015, 134, 756-768
[Non-Patent Literature 6] Branchi I. et al., J. Neuropathol. Exp. Neurol., 2004, 63, 429-440
[Non-Patent Literature 7] Dowjat WK. et al., Neurosci.Lett., 2007, 413, 77-81
[Non-Patent Literature 8] Wegiel J. et al., FEBS J., 2011, 278, 236-245
[Non-Patent Literature 9] Pozo N. et al., J.Clin.Invest., 2013, 123, 2475-2487.
[Non-Patent Literature 10] Deng X. et al., Cancer Res., 2006, 66, 4149-4158.
[Non-Patent Literature 11] Ewton DZ. et al., Mol.Cancer Ther., 2011, 10, 2104-2114.
[Non-Patent Literature 12] Deng X. et al., Genes Cancer., 2014, 5, 201-211.
[Non-Patent Literature 13] Yang C. et al., Carcinogenesis., 2010, 31, 522-528.
[Non-Patent Literature 14] Jin K. et al., J.Biol.Chem., 2009, 284, 22916-22925.
[Non-Patent Literature 15] Gao J et al., Cancer Cell Int. 2013, 13, 2
[Non-Patent Literature 16] Taira N. et al., Mol.Cell., 2007, 25, 725-738.
[Non-Patent Literature 17] Bogacheva O. et al., J.Biol.Chem., 2008, 283, 36665-36675.

## DISCLOSURE OF INVENTION

## PROBLEM TO BE SOLVED BY THE INVENTION

An object of the present invention is to provide a medicament, particularly a novel compound having a DYRK inhibitory effect Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

## MEANS FOR SOLVING PROBLEM

That is, the present invention is as follows.

[Item 1]

A compound represented by the following formula (1):

[Formula 1]

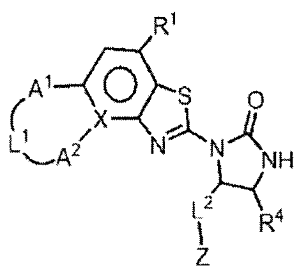

(1)

wherein

- $A^1$ represents an oxygen atom or a nitrogen atom (=N-),
- $A^2$ represents $CR^B$, $CR^CR^D$, an oxygen atom, or $NR^{A1}$,
- $L^1$ represents optionally substituted methylene, optionally substituted ethylene, optionally substituted methine, optionally substituted ethanediylidene, =N-, or $NR^{A2}$,
- $R^{A1}$, $R^{A2}$, $R^B$, $R^C$, and $R^D$ each independently represent a hydrogen atom or optionally substituted $C_{1-6}$ alkyl,
- $R^1$ represents a hydrogen atom, a halogen atom, or optionally substituted $C_{1-6}$ alkyl,

X represents a carbon atom or a nitrogen atom,
$L^2$ represents optionally substituted $C_{1-4}$ alkylene,
$R^E$ represents optionally substituted $C_{1-6}$ alkyl,
Z represents $-NR^2R^3$ or $-OR^7$,
$R^7$ represents optionally substituted $C_{1-6}$ alkyl, or optionally substituted $C_{1-7}$ alkylene formed together with $R^4$, wherein $R^4$ and $R^7$, together with the carbon atom and the oxygen atom to which they, respectively, are attached, form an optionally substituted 5- to 11-membered saturated heterocycle,
$R^2$ represents a hydrogen atom, optionally substituted $C_{1-6}$ alkyl, C(O)-$R^E$, $C_{3-10}$ cycloalkyl, $C_{2-6}$ alkynyl, or a cyclic group of a 4- to 11-membered saturated heterocycle,
$R^3$ represents a hydrogen atom, optionally substituted $C_{1-6}$ alkyl, or C(O)-$R^E$, and
$R^4$ represents optionally substituted $C_{1-6}$ alkyl,
wherein $R^2$ and $R^3$, together with the nitrogen atom to which they are attached, may form an optionally substituted 4- to 11-membered saturated heterocycle, or $R^3$ and $R^4$, together with the nitrogen atom and the carbon atom to which they, respectively, are attached, may form an optionally substituted 4- to 11-membered saturated heterocycle, or any carbon atom on the saturated heterocycle constituted by $R^2$, $R^3$, and the nitrogen atom, and $R^4$ together may form a 4- to 11-membered saturated heterocycle (as used herein, may also be referred to as "compound (1)" or the "compound represented by formula (1)"),
or a pharmaceutically acceptable salt thereof.

[Item 2]

The compound according to item 1 or a pharmaceutically acceptable salt thereof, wherein Z represents $-NR^2R^3$, and $R^2$ and $R^3$ each independently represent a hydrogen atom, optionally substituted $C_{1-6}$ alkyl, or C(O)-$R^E$, wherein $R^2$ and $R^3$, together with the nitrogen atom to which they are attached, may form an optionally substituted 4- to 8-membered saturated heterocycle.

[Item 3]

The compound according to item 1 or a pharmaceutically acceptable salt thereof, wherein Z represents $-NR^2R^3$, and $R^3$ and $R^4$, together with the nitrogen atom and the carbon atom to which they, respectively, are attached, form an optionally substituted 4- to 8-membered saturated heterocycle.

[Item 4]

The compound according to any one of items 1 to 3 or a pharmaceutically acceptable salt thereof, wherein $R^1$ is a hydrogen atom.

[Item 5]

The compound according to any one of items 1 to 4 or a pharmaceutically acceptable salt thereof, wherein X is a carbon atom.

[Item 6]

The compound according to item 5 or a pharmaceutically acceptable salt thereof, wherein $A^1$ is an oxygen atom, $A^2$ is methylene, and $L^1$ is methylene.

[Item 7]

The compound according to item 1, 2, 4, 5, or 6 or a pharmaceutically acceptable salt thereof, wherein formula (1) is represented by the following formula (1a):

[Formula 2]

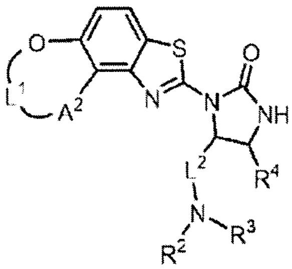

(1a)

wherein

$A^2$ represents optionally substituted methylene or an oxygen atom,
$L^1$ represents optionally substituted methylene or optionally substituted ethylene,
$L^2$ represents optionally substituted $C_{1-4}$ alkylene,
$R^2$ and $R^3$ each independently represent a hydrogen atom, optionally substituted $C_{1-6}$ alkyl, or C(O)-$R^E$, wherein $R^2$ and $R^3$, together with the nitrogen atom to which they are attached, may form an optionally substituted 4- to 8-membered saturated heterocycle,
$R^E$ represents optionally substituted $C_{1-6}$ alkyl, and
$R^4$ is optionally substituted $C_{1-6}$ alkyl.

[Item 8] The compound according to item 7, wherein $R^2$ and $R^3$ each independently are optionally substituted $C_{1-6}$ alkyl, or a pharmaceutically acceptable salt thereof.

[Item 9]

The compound according to item 7 or a pharmaceutically acceptable salt thereof, wherein $R^2$ and $R^3$, together with the nitrogen atom to which they are attached, form an optionally substituted 4- to 8-membered saturated heterocycle.

[Item 10]

The compound according to any one of items 7 to 9 or a pharmaceutically acceptable salt thereof, wherein $L^2$ is $C_{1-3}$ alkylene.

[Item 11]

The compound according to item 1, 3, 4, 5, or 6 or a pharmaceutically acceptable salt thereof, wherein formula (1) is represented by the following formula (1b):

[Formula 3]

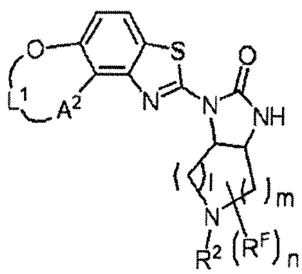

(1b)

wherein

$A^2$ represents optionally substituted methylene or an oxygen atom,
$L^1$ represents optionally substituted methylene or optionally substituted ethylene,
l and m each independently represent 1, 2, or 3, wherein a sum of l and m is 5 or less,
n represents 1, 2, 3, or 4,

$R^2$ represents a hydrogen atom, optionally substituted $C_{1-6}$ alkyl, $C_{3-10}$ cycloalkyl, or C(O)-$R^E$,
$R^E$ represents optionally substituted $C_{1-6}$ alkyl,
$R^F$ represents a hydrogen atom, a halogen atom, or optionally substituted $C_{1-6}$ alkyl,
when n is 2, 3, or 4, each $R^F$ may be the same or different, and two $R^F$ on the same carbon atom, together with the carbon atom to which they are each attached, may form a spiro ring consisting of a 4- to 8-membered saturated heterocycle or a 3-to 8-membered saturated carbocycle, or two $R^F$ on different carbon atoms may bond together to form a crosslink.

[Item 12]

The compound according to item 11 or a pharmaceutically acceptable salt thereof, wherein $R^2$ is a hydrogen atom, optionally substituted $C_{1-6}$ alkyl, or $C_{3-10}$ cycloalkyl.

[Item 13]

The compound according to item 11 or a pharmaceutically acceptable salt thereof, wherein $R^2$ is optionally substituted $C_{1-6}$ alkyl.

[Item 14]

The compound according to any one of items 11 to 13 or a pharmaceutically acceptable salt thereof, wherein l and m are each independently 1 or 2.

[Item 15]

The compound according to item 1, wherein Z is -$OR^7$, and $R^4$ and $R^7$, together with the carbon atom and the oxygen atom to which they, respectively, are attached, form an optionally substituted 5- to 8- membered saturated heterocycle, or a pharmaceutically acceptable salt thereof.

[Item 16]

The compound according to item 1 or 15 or a pharmaceutically acceptable salt thereof, wherein formula (1) is represented by the following formula (1c):

[Formula 4]

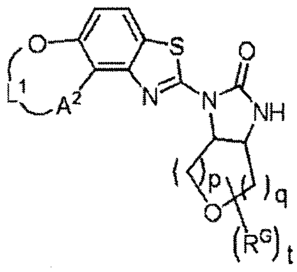

(1c)

wherein

$A^2$ represents optionally substituted methylene or an oxygen atom,
$L^1$ represents optionally substituted methylene or optionally substituted ethylene,
p and q each independently represent 1, 2, or 3, wherein a sum of p and q is 5 or less,
t represents 1, 2, 3, or 4,
$R^G$ represents a hydrogen atom, a halogen atom, optionally substituted $C_{1-6}$ alkyl, optionally substituted $C_{1-6}$ alkoxy, or a CN group,
when t is 2, 3, or 4, each $R^G$ may be the same or different, and two $R^G$ on the same carbon atom, together with the carbon atom to which they are each attached, may form a spiro ring consisting of a 4- to 8-membered saturated heterocycle or a 3-to 8-membered saturated carbocycle, or two $R^G$ on different carbon atoms may bond together to form a cross-link.

[Item 17]

The compound according to any one of items 1 to 16 or a pharmaceutically acceptable salt thereof, wherein the compound is selected from the group of the following compounds:

cis-3-(7,8-dihydrofuro[3,2-e][1,3]benzothiazol-2-yl)-5-methyloctahydro-2H-imidazo[4,5-c]pyridin-2-one (Example 7);
cis-3-(7,8-dihydrofuro[3,2-e][1,3]benzothiazol-2-yl)-5-(2-methoxyethyl)octahydro-2H-imidazo[4,5-c]pyridin-2-one (Example 8);
cis-1-(7,8-dihydrofuro[3,2-e] [1,3]benzothiazol-2-yl)-5-methyloctahydro-2H-imidazo[4,5-c]pyridin-2-one (Example 9);
cis-3-(7,8-dihydrofuro[3,2-e][1,3]benzothiazol-2-yl)-5-ethyloctahydro-2H-imidazo[4,5-c]pyridin-2-one (Example 11);
cis-1-(7,8-dihydrofuro[3,2-e][1,3]benzothiazol-2-yl)-5-methylhexahydropyrrolo[3,4-d]imidazol-2(1H)-one (Example 14);
cis-3-(7,8-dihydrofuro[3,2-e][1,3]benzothiazol-2-yl)-5-(propan-2-yl)octahydro-2H-imidazo[4,5-c]pyridin-2-one (Example 15);
cis-3-(7,8-dihydrofuro[3,2-e][1,3]benzothiazol-2-yl)-5-methyloctahydroimidazo[4,5-c]azepin-2(1H)-one (Example 17);
(3aS,7aS)-3-(7,8-dihydrofuro[3,2-e][1,3]benzothiazol-2-yl)-5-(oxetan-3-yl)octahydro-2H-imidazo[4,5-c]pyridin-2-one (Example 19);
(3aR,7aR)-1-(2H-[1,3]dioxolo[4,5-e][1,3]benzothiazol-7-yl)hexahydropyrano[3,4-d]imidazol-2(3H)-one (Example 21);
(4S,5R)-1-(7,8-dihydrofuro[3,2-e][1,3]benzothiazol-2-yl)-4-methyl-5-[(morpholin-4-yl)methyl]imidazolidin-2-one (Example 23);
(4R,5R)-1-(7,8-dihydrofuro[3,2-e][1,3]benzothiazol-2-yl)-4-methyl-5-[(morpholin-4-yl)methyl]imidazolidin-2-one (Example 24);
(3aR,7aS)-3-(7,8-dihydrofuro[3,2-e][1,3]benzothiazol-2-yl)-5-methyloctahydro-2H-imidazo[4,5-c]pyridin-2-one (Example 28);
(3aR,7aS)-5-cyclopropyl-3-(7,8-dihydro[1,4]dioxino[2,3-e][1,3]benzothiazol-2-yl)octahydro-2H-imidazo[4,5-c]pyridin-2-one (Example 34);
(3aR,7aS)-5-cyclopropyl-3-(2H-[1,3]dioxolo[4,5-e][1,3]benzothiazol-7-yl)octahydro-2H-imidazo[4,5-c]pyridin-2-one (Example 36);
(3aR,7aR)-1-(7,8-dihydro[1,4]dioxino[2,3-e][1,3]benzothiazol-2-yl)hexahydropyrano[3,4-d]imidazol-2(3H)-one (Example 37);
(3aR,6S,7aR)-1-(7,8-dihydrofuro[3,2-e][1,3]benzothiazol-2-yl)-6-methylhexahydropyrano[3,4-d]imidazol-2(3H)-one (Example 38); (3aR,6S,7aR)-1-(7,8-dihydro[1,4]dioxino[2,3-e][1,3]benzothiazol-2-yl)-6-methylhexahydropyrano[3,4-d]imidazol-2(3H)-one (Example 39);
(3aR,6R,7aR)-1-(7,8-dihydrofuro[3,2-e][1,3]benzothiazol-2-yl)-6-(fluoromethyl)hexahydropyrano[3,4-d]imidazol-2(3H)-one (Example 40);
(3aR,6R,7aR)-1-(7,8-dihydro[1,4]dioxino[2,3-e][1,3]benzothiazol-2-yl)-6-(fluoromethyl)hexahydropyrano[3,4-d]imidazol-2(3H)-one (Example 41);
(3aR,6S,7aR)-1-(7,8-dihydrofuro[3,2-e][1,3]benzothiazol-2-yl)-6-ethylhexahydropyrano[3,4-d]imidazol-2(3H)-one (Example 42);
(3aR,6S,7aR)-1-(7,8-dihydro[1,4]dioxino[2,3-e][1,3]benzothiazol-2-yl)-6-ethylhexahydropyrano[3,4-d]imidazol-2(3H)-one (Example 43);
(4S,5S)-1-(7,8-dihydrofuro[3,2-e][1,3]benzothiazol-2-yl)-5-(methoxymethyl)-4-methylimidazolidin-2-one (Example 44);
(3aR,6aS)-1-(7,8-dihydrofuro[3,2-e][1,3]benzothiazol-2-yl)tetrahydro-1H-furo[3,4-d]imidazol-2(3H)-one (Example 65);
rac-(3aR,7aS)-3-(7,8-dihydrofuro[3,2-e][1,3]benzothiazol-2-yl)-5-(oxetan-3-yl)octahydro-2H-imidazo[4,5-c]pyridin-2-one (Example 71);
rac-(3aR,7aS)-5-cyclobutyl-3-(7,8-dihydrofuro[3,2-e][1,3]benzothiazol-2-yl)octahydro-2H-imidazo[4,5-c]pyridin-2-one (Example 72);
rac-(3aR,8aS)-3-(7,8-dihydrofuro[3,2-e][1,3]benzothiazol-2-yl)-5-(oxetan-3-yl)octahydroimidazo[4,5-c]azepin-2(1H)-one (Example 73);
rac-(3aR,7aS)-3-(7,8-dihydro[1,4]dioxino[2,3-e][1,3]benzothiazol-2-yl)-5-methyloctahydro-2H-imidazo[4,5-c]pyridin-2-one (Example 76);
rac-(3aR,7aS)-1-(7,8-dihydro[1,4]dioxino[2,3-e][1,3]benzothiazol-2-yl)-5-methyloctahydro-2H-imidazo[4,5-c]pyridin-2-one (Example 77);
rac-(3aR,7aS)-1-(2H-[1,3]dioxolo[4,5-e][1,3]benzothiazol-7-yl)-5-methyloctahydro-2H-imidazo[4,5-c]pyridin-2-one (Example 78);
rac-(3aR,7aS)-3-(2H-[1,3]dioxolo[4,5-e][1,3]benzothiazol-7-yl)-5-methyloctahydro-2H-imidazo[4,5-c]pyridin-2-one (Example 79);
rac-(3aR,7aS)-3-(7,8-dihydro[1,4]dioxino[2,3-e][1,3]benzothiazol-2-yl)-5-(oxetan-3-yl)octahydro-2H-imidazo[4,5-c]pyridin-2-one (Example 80);
rac-(3aR,7aS)-3-(2H-[1,3]dioxolo[4,5-e][1,3]benzothiazol-7-yl)-5-(oxetan-3-yl)octahydro-2H-imidazo[4,5-c]pyridin-2-one (Example 81);
rac-(3aR,8aS)-3-(2H-[1,3]dioxolo[4,5-e][1,3]benzothiazol-7-yl)-5-methyloctahydroimidazo[4,5-c]azepin-2(1H)-one (Example 85);
rac-(3aR,8aS)-3-(2H-[1,3]dioxolo[4,5-e][1,3]benzothiazol-7-yl)-5-(oxetan-3-yl)octahydroimidazo[4,5-c]azepin-2(1H)-one (Example 86);
(3aS,6S,7aR)-1-(7,8-dihydrofuro[3,2-e][1,3]benzothiazol-2-yl)-5,6-dimethyloctahydro-2H-imidazo[4,5-c]pyridin-2-one (Example 93);
rac-(3aR,7aS)-5-cyclopropyl-3-(7,8-dihydrofuro[3,2-e][1,3]benzothiazol-2-yl)octahydro-2H-imidazo[4,5-c]pyridin-2-one (Example 98);
rac-(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Example 99);
rac-[(3aR,7aS)-3-(7,8-dihydrofuro[3,2-e][1,3]benzothiazol-2-yl)-2-oxooctahydro-5H-imidazo[4,5-c]pyridin-5-yl] acetonitrile (Example 100);
rac-(3aR,6aS)-1-(7,8-dihydrofuro[3,2-e][1,3]benzothiazol-2-yl)-5-(2-propyn-1-yl)hexahydropyrrolo[3,4-d]imidazol-2(1H)-one (Example 102);
rac-[(3aR,6aS)-1-(7,8-dihydrofuro[3,2-e][1,3]benzothiazol-2-yl)-2-oxohexahydropyrrolo[3,4-d]imidazol-5(1H)-yl] acetonitrile (Example 103);
rac-(3aR,8aS)-5-cyclopropyl-3-(7,8-dihydrofuro[3,2-e][1,3]benzothiazol-2-yl)octahydroimidazo[4,5-c]azepin-2(1H)-one (Example 104);
rac-(3aR,8aS)-3-(7,8-dihydrofuro[3,2-e][1,3]benzothiazol-2-yl)-5-(2-propyn-1-yl)octahydroimidazo[4,5-c]azepin-2(1H)-one (Example 105);
rac-[(3aR,8aS)-3-(7,8-dihydrofuro[3,2-e][1,3]benzothiazol-2-yl)-2-oxooctahydroimidazo[4,5-c]azepin-5(1H)-yl] acetonitrile (Example 106);
rac-(3aR,7aS)-3-(7,8-dihydrofuro[3,2-e][1,3]benzothiazol-2-yl)-5-(2,2,2-trifluoroethyl)octahydro-2H-imidazo[4,5-c] pyridin-2-one (Example 107);
rac-(3aR,7aS)-5-(2,2-difluoroethyl)-3-(7,8-dihydrofuro[3,2-e][1,3]benzothiazol-2-yl)octahydro-2H-imidazo[4,5-c] pyridin-2-one (Example 108);
(3aR,7aS)-5-cyclopropyl-3-(7,8-dihydrofuro[3,2-e][1,3]benzothiazol-2-yl)octahydro-2H-imidazo[4,5-c]pyridin-2-one (Example 110);
[(3aR,7aS)-3-(7,8-dihydrofuro[3,2-e][1,3]benzothiazol-2-yl)-2-oxooctahydro-5H-imidazo[4,5-c]pyridin-5-yl]acetonitrile (Example 112);
(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Example 114);
(4S,5R)-1-(7,8-dihydrofuro[3,2-e][1,3]benzothiazol-2-yl)-5-{[(3S)-3-fluoropyrrolidin-1-yl]methyl}-4-methylimidazolidin-2-one (Example 115);
(4S,5R)-5-[(3,3-difluoroazetidin-1-yl)methyl]-1-(7,8-dihydrofuro[3,2-e][1,3]benzothiazol-2-yl)-4-methylimidazolidin-2-one (Example 116);
(4S,5R)-5-[(3,3-difluoropyrrolidin-1-yl)methyl]-1-(7,8-dihydrofuro[3,2-e][1,3]benzothiazol-2-yl)-4-methylimidazolidin-2-one (Example 117);
(4S,5R)-1-(7,8-dihydrofuro[3,2-e][1,3]benzothiazol-2-yl)-5-[(3-fluoroazetidin-1-yl)methyl]-4-methylimidazolidin-2-one (Example 118);
rac-(3aR,7aR)-3-(7,8-dihydrofuro[3,2-e][1,3]benzothiazol-2-yl)hexahydropyrano[3,4-d]imidazol-2(3H)-one (Example 120);
rac-(3aR,7aR)-1-(7,8-dihydrofuro[3,2-e][1,3]benzothiazol-2-yl)hexahydropyrano[3,4-d]imidazol-2(3H)-one (Example 121);
(3aR,7aR)-1-(7,8-dihydrofuro[3,2-e][1,3]benzothiazol-2-yl)hexahydropyrano[3,4-d]imidazol-2(3H)-one (Example 122);
(3aR,7aR)-1-(7,8-dihydrofuro[3,2-e][1,3]benzothiazol-2-yl)-6,6-dimethylhexahydropyrano[3,4-d]imidazol-2(3H)-one (Example 124);
(3aR,6R,7aR)-1-(7,8-dihydrofuro[3,2-e][1,3]benzothiazol-2-yl)-2-oxooctahydropyrano[3,4-d]imidazole-6-carbonitrile (Example 127);
(3aR,6R,7aR)-1-(7,8-dihydrofuro[3,2-e][1,3]benzothiazol-2-yl)-6-methoxyhexahydropyrano[3,4-d]imidazol-2(3H)-one (Example 129);
(3aR,6R,7aR)-1-(2H-[1,3]dioxolo[4,5-e][1,3]benzothiazol-7-yl)-2-oxooctahydropyrano[3,4-d]imidazole-6-carbonitrile (Example 131);
(3aR,6R,7aR)-1-(7,8-dihydro[1,4]dioxino[2,3-e][1,3]benzothiazol-2-yl)-2-oxooctahydropyrano[3,4-d]imidazole-6-carbonitrile (Example 133);
(3aR,8aR)-1-(7,8-dihydro[1,4]dioxino[2,3-e][1,3]benzothiazol-2-yl)hexahydro-1H-oxepino[3,4-d]imidazol-2(3H)-one (Example 135);
(3aR,6S,7aR)-1-(2H-[1,3]dioxolo[4,5-e][1,3]benzothiazol-7-yl)-6-ethylhexahydropyrano[3,4-d]imidazol-2(3H)-one (Example 138);
(3aR,6S,7aR)-1-(2H-[1,3]dioxolo[4,5-e][1,3]benzothiazol-7-yl]-6-methylhexahydropyrano[3,4-d]imidazol-2(3H)-one (Example 139);
(3aR,6R,7aR)-1-(2H-[1,3]dioxolo[4,5-e][1,3]benzothiazol-7-yl]-6-(fluoromethyl)hexahydropyrano[3,4-d]imidazol-2(3H)-one (Example 140);
(3aR,6R,7aR)-1-(7,8-dihydrofuro[3,2-e][1,3]benzothiazol-2-yl)-6-(hydroxymethyl)hexahydropyrano[3,4-d]imidazol-2(3H)-one (Example 144);
(3aR,6R,7aR)-1-(7,8-dihydro[1,4]dioxino[2,3-e][1,3]benzothiazol-2-yl)-6-(hydroxymethyl)hexahydropyrano[3,4-d]imidazol-2(3H)-one (Example 146);
(3aR,6R,7aR)-1-(2H-[1,3]dioxolo[4,5-e][1,3]benzothiazol-7-yl)-6-(hydroxymethyl)hexahydropyrano[3,4-d]imidazol-2(3H)-one (Example 147);
(3aR,6R,7aR)-1-(7,8-dihydrofuro[3,2-e][1,3]benzothiazol-2-yl)-6-ethynylhexahydropyrano[3,4-d]imidazol-2(3H)-one (Example 149);
(3aR,7aR)-1-(7,8-dihydrofuro[3,2-e][1,3]benzothiazol-2-yl)-6-fluorohexahydropyrano[3,4-d]imidazol-2(3H)-one (Example 151);
(3aR,6S,6aS)-1-(7,8-dihydrofuro[3,2-e][1,3]benzothiazol-2-yl)-6-(hydroxymethyl)tetrahydro-1H-furo[3,4-d]imidazol-2(3H)-one (Example 157);
(3aR,6S,6aS)-1-(7,8-dihydrofuro[3,2-e][1,3]benzothiazol-2-yl)-6-(fluoromethyl)tetrahydro-1H-furo[3,4-d]imidazol-2(3H)-one (Example 159);
(3aR,6R,6aS)-1-(7,8-dihydrofuro[3,2-e][1,3]benzothiazol-2-yl)-6-methyltetrahydro-1H-furo[3,4-d]imidazol-2(3H)-one (Example 161); and
[(3aS,4R,6aR)-3-(7,8-dihydrofuro[3,2-e][1,3]benzothiazol-2-yl)-2-oxohexahydro-1H-furo[3,4-d]imidazol-4-yl]acetonitrile (Example 162).

[Item 18]

A medicament comprising the compound according to any one of items 1 to 17 or a pharmaceutically acceptable salt thereof as an active ingredient.

[Item 19]

A pharmaceutical composition comprising the compound according to any one of items 1 to 17 or a pharmaceutically acceptable salt thereof as an active ingredient.

[Item 20]

A therapeutic agent and/or a prophylactic agent for a disease involving DYRK, comprising the compound according to any one of items 1 to 17 or a pharmaceutically acceptable salt thereof as an active ingredient.

[Item 21]

The therapeutic agent and/or the prophylactic agent according to item 20, wherein the disease involving DYRK is frontotemporal dementia, progressive supranuclear palsy, corticobasal degeneration, Lewy body dementia, vascular dementia, traumatic brain injury, chronic traumatic encephalopathy, stroke, Alzheimer’s disease, Parkinson’s disease, Down’s disease, or depression, and mental retardation, memory impairment, memory loss, learning disability, intellectual disability, cognitive dysfunction, mild cognitive impairment, or dementia symptom associated therewith, or brain tumor, pancreatic cancer, ovarian cancer, osteosarcoma, large intestine cancer, lung cancer, bone resorption disease, osteoporosis, sickle cell anemia, chronic renal disease, or bone resorption disease.

[Item 22]

A method for treating and/or preventing a disease involving DYRK, comprising administration of a therapeutically effective amount of the compound according to any one of items 1 to 17 or a pharmaceutically acceptable salt thereof to a patient in need of treatment.

[Item 23]

Use of the compound according to any one of items 1 to 17 or a pharmaceutically acceptable salt thereof, for producing a therapeutic agent and/or a prophylactic agent for a disease involving DYRK.

[Item 24]

The compound according to any one of items 1 to 17 or a pharmaceutically acceptable salt thereof, for use in treatment and/or prevention of a disease involving DYRK.

[Item 25]

A medicament obtained by combining the medicament according to item 18 and at least one or more agents selected from agents classified into an anticancer agent, an antipsychotic drug, an antidementia drug, an antiepileptic drug, an antidepressant drug, a gastrointestinal drug, a thyroid hormone drug, or an antithyroid drug.

[Item 26]

The medicament according to item 18, for treating frontotemporal dementia, progressive supranuclear palsy, corticobasal degeneration, Levy body dementia, vascular dementia, traumatic brain injury, chronic traumatic encephalopathy, stroke, Alzheimer's disease, Parkinson's disease, Down syndrome, or depression, and complication, mental retardation, memory impairment, memory loss, learning disability, intellectual disability, cognitive dysfunction, mild cognitive impairment, or treating dementia symptom progression or preventing dementia onset associated therewith, or treating brain tumor, pancreatic cancer, ovarian cancer, osteosarcoma, large intestine cancer, lung cancer, bone resorption disease, osteoporosis, sickle cell anemia, chronic renal disease, or bone resorption disease, in combination with at least one or more agents selected from agents classified into an anticancer agent, an antipsychotic drug, an antidementia drug, an antiepileptic drug, an antidepressant drug, a gastrointestinal drug, a thyroid hormone drug, or an antithyroid drug.

## EFFECT OF THE INVENTION

The present inventors have carried out various studies in order to solve the above problems and as a result, have found that the amine derivative represented by the above formula (1) and a pharmaceutically acceptable salt thereof are an excellent group of drugs having an excellent DYRK inhibitory action, and have completed the present invention. The compound provided by the present invention is useful as a pharmaceutical (pharmaceutical composition) for prevention or treatment of a disease known to be associated with a DYRK1A-mediated abnormal cellular response, such as a psychiatric or neurologic disease such as Alzheimer's disease, Parkinson's disease, Down's disease, or depression, and mental retardation, memory impairment, memory loss, learning disability, intellectual disability, cognitive dysfunction, mild cognitive impairment, or a therapeutic drug for dementia symptom progression or a prophylactic drug for dementia onset associated therewith, or further a tumor such as brain tumor. The compound provided by the present invention is, as an inhibitor of DYRK1B, useful as a pharmaceutical (pharmaceutical composition) for prevention or treatment of a tumor such as pancreatic cancer, ovarian cancer, osteosarcoma, large intestine cancer, or lung cancer. Further, the compound provided by the present invention is useful as a pharmaceutical (pharmaceutical composition) for prevention or treatment of bone resorption disease and osteoporosis because DYRK2 controls p53 in response to DNA damage to induce apoptosis. In addition, the compound provided by the present invention is, as an inhibitor of DYRK3, useful as a pharmaceutical (pharmaceutical composition) for prevention or treatment of sickle cell anemia, chronic renal disease, bone resorption disease, and osteoporosis. In addition, the compound provided by the present invention is, as a compound that inhibits DYRK, useful as a reagent for pathological imaging related to the above diseases or a reagent for a basic experiment or for research.

## BESTMODE TO CARRY OUT THE INVENTION

The terms used herein will be described below.

"DYRK" stands for Dual-specificity tYrosine-phosphorylation Regulated protein Kinase, and means one or two or more of the DYRK family (DYRK1A, DYRK1B, DYRK2, DYRK3, and DYRK4) .

Examples of a "halogen atom" include a fluorine atom, a chlorine atom, a bromine atom, or an iodine atom. The halogen atom is preferably a fluorine atom.

"$C_{1-6}$ alkyl" means a linear or branched saturated hydrocarbon group having 1 to 6 carbon atoms, and "$C_6$ alkyl" means a linear or branched saturated hydrocarbon group having 6 carbon atoms. The same also applies to other numbers. The $C_{1-6}$ alkyl is preferably "$C_{1-4}$ alkyl" and more preferably "$C_{1-3}$ alkyl." Specific examples of the "$C_{1-3}$ alkyl" include methyl, ethyl, propyl, and 1-methylethyl. Specific examples of the "$C_{1-4}$ alkyl" include butyl, 1,1-dimethylethyl, 1-methylpropyl, and 2-methylpropyl, in addition to those given as specific examples of the "$C_{1-3}$ alkyl." Specific examples of the "$C_{1-6}$ alkyl" include pentyl, 1,1-dimethylpropyl, 1,2-dimethylpropyl, 1-methylbutyl, 2-methylbutyl, 4-methylpentyl, 3-methylpentyl, 2-methylpentyl, 1-methylpentyl, and hexyl, in addition to those given as specific examples of the "$C_{1-4}$ alkyl."

"$C_{1-6}$ alkoxy" means "$C_{1-6}$ alkyloxy," and the "$C_{1-6}$ alkyl" moiety is defined as the "$C_{1-6}$ alkyl." The "$C_{1-6}$ alkoxy" is preferably "$C_{1-4}$ alkoxy" and more preferably "$C_{1-3}$ alkoxy." Specific examples of the "$C_{1-3}$ alkoxy" include methoxy, ethoxy, propoxy, and 1-methylethoxy. Specific examples of the "$C_{1-4}$ alkoxy" include butoxy, 1,1-dimethylethoxy, 1-methylpropoxy, and 2-methylpropoxy, in addition to those given as specific examples of the "$C_{1-3}$ alkoxy." Specific examples of the "$C_{1-6}$ alkoxy" include pentyloxy, 1,1-dimethylpropoxy, 1,2-dimethylpropoxy, 1-methylbutoxy, 2-methylbutoxy, 4-methylpentyloxy, 3-methylpentyloxy, 2-methylpentyloxy, 1-methylpentyloxy, and hexyloxy, in addition to those given as specific examples of the "$C_{1-4}$ alkoxy."

"$C_{1-6}$ alkoxycarbonyl" refers to "carbonyl" substituted with the "$C_{1-6}$ alkoxy." The "$C_{1-6}$ alkoxycarbonyl" is preferably "$C_{1-4}$ alkoxycarbonyl" and more preferably "$C_{1-3}$ alkoxycarbonyl." Specific examples of the "$C_{1-3}$ alkoxycarbonyl" include methoxycarbonyl, ethoxycarbonyl, propoxycarbonyl, and 1-methylethoxycarbonyl. Specific examples of the "$C_{1-4}$ alkoxycarbonyl" include butoxycarbonyl, 1,1-dimethylethoxycarbonyl, 1-methylpropoxycarbonyl, and 2-methylpropoxycarbonyl, in addition to those given as specific examples of the "$C_{1-3}$ alkoxycarbonyl." Specific examples of the "$C_{1-6}$ alkoxycarbonyl" include pentyloxycarbonyl, 1,1-dimethylpropoxycarbonyl, 1,2-dimethylpropoxycarbonyl, 1-methylbutoxycarbonyl, 2-methylbutoxycarbonyl, 4-methylpentyloxycarbonyl, 3-methylpentyloxycarbonyl, 2-methylpentyloxycarbonyl, 1-methylpentyloxycarbonyl, and hexyloxycarbonyl, in addition to those given as specific examples of the "$C_{1-4}$ alkoxycarbonyl."

"$C_{1-7}$ alkylene," "$C_{1-4}$ alkylene," or "$C_{1-3}$ alkylene" means a linear or branched divalent saturated hydrocarbon group having 1 to 7, 1 to 4, or 1 to 3 carbon atoms, respectively. The "$C_{1-7}$ alkylene" is preferably "$C_{1-4}$ alkylene," the "$C_{1-4}$ alkylene" is preferably "$C_{1-3}$ alkylene," and the "$C_{1-3}$ alkylene" is preferably "$C_{1-2}$ alkylene." Specific examples of the "$C_{1-2}$ alkylene" include methylene and ethylene. Specific examples of the "$C_{1-3}$ alkylene" include propylene and 1-methylethylene, in addition to those given as specific examples of the "$C_{1-2}$ alkylene." Specific examples of the "$C_{1-4}$ alkylene" include butylene, 1,1-dimethylethylene, 1,2-dimethylethylene, 1-methylpropylene, and 2-methylpropylene, in addition to those given as specific examples of the "$C_{1-3}$ alkylene." Specific examples of the "$C_{1-7}$ alkylene" include pentylene, 1,1-dimethylpropylene, 2,2-dimethylpropylene, 1,2-dimethylpropylene, 1,3-dimethylpropylene, 1-methylbutylene, 2-methylbutylene, hexylene, 1,1-dimethylbutylene, 2,2-dimethylbutylene, 1,2-dimethylbutylene, 1,3-dimethylbutylene, 1,4-dimethylbutylene, 2,3-dimethylbutylene, 1-methylpentylene, 2-methylpentylene, 3-methylpentylene, heptylene, 1,1-dimethylpentylene, 2,2-dimethylpentylene, 3,3-dimethylpentylene, 1,2-dimethylpentylene, 1,3-dimethylpentylene, 1,4-dimethylpentylene, 1,5-dimethylpentylene, 2,3-dimethylpentylene, 2,4-dimethylpentylene, 2,5-dimethylpentylene, 1-methylhexylene, 2-methylhexylene, and 3-methylhexylene, in addition to those given as specific examples of the "$C_{1-4}$ alkylene."

"$C_{2-6}$ alkynyl" means a linear or branched saturated hydrocarbon group having 2 to 6 carbon atoms and having 1 to 3 triple bonds. The "alkynyl" is preferably "$C_{2-4}$ alkynyl" and more preferably "$C_{2-3}$ alkynyl." Specific examples of the "alkynyl" include ethynyl, propargyl, and 2-butynyl.

"$C_{3-10}$ cycloalkyl" means a cyclic saturated hydrocarbon group having 3 to 10 carbon atoms, and also includes one having partially an unsaturated bond and one having a crosslinked structure. The "$C_{3-10}$ cycloalkyl" is preferably "$C_{3-7}$ cycloalkyl." Specific examples of the "$C_{3-7}$ cycloalkyl" include cyclopropyl, cyclobutyl, cyclopentyl, cyclohexyl, and cycloheptyl. Specific examples of the "$C_{3-10}$ cycloalkyl" include cyclooctyl, cyclononyl, cyclodecyl, and adamantyl, in addition to those given as specific examples of the "$C_{3-7}$ cycloalkyl."

A "4- to 11-membered saturated heterocycle" means a monocyclic or bicyclic saturated heterocycle containing one or more heteroatoms selected from the group of a nitrogen atom, an oxygen atom, and a sulfur atom, and also includes one having partially an unsaturated bond and one having a crosslinked structure. The "4- to 11-membered saturated heterocycle" is preferably a "4- to 8-membered saturated heterocycle," more preferably a "5- to 8-membered saturated heterocycle," and further preferably "5- to 7-membered saturated heterocycle." Specific examples of the "4- to 8-membered saturated heterocycle" include an azetidine ring, a pyrrolidine ring, a piperidine ring, an azepane ring, an azocane ring, a morpholine ring, a piperazine ring, an oxazocane ring, an azabicycloheptane ring, an azabicyclooctane

ring, an oxetane ring, a thietane ring, a tetrahydrofuran ring, a tetrahydrothiophene ring, a tetrahydropyran ring, a thiomorpholine ring, and a 1,4-dioxane ring. Specific examples of the "4- to 11-membered saturated heterocycle" include an azonane ring, an oxazonane ring, an azecane ring, an oxazecane ring, an azacycloundecane ring, an azabicyclononane ring, and an azabicyclodecane ring, in addition to those given as specific examples of the "4- to 8-membered saturated heterocycle."

A "3- to 8-membered saturated carbocycle" means a monocyclic or bicyclic saturated aliphatic carbocycle, and also includes one having partially an unsaturated bond and one having a crosslinked structure. The "3- to 8-membered saturated carbocycle" is preferably a "4- to 8-membered saturated carbocycle," more preferably a "5- to 8-membered saturated carbocycle," and further preferably "5- to 7-membered saturated carbocycle." Specific examples of the "3-to 8-membered saturated heterocycle" include cyclopropane, cyclobutane, cyclopentane, cyclohexane, cycloheptane, cyclooctane, and bicycloheptane.

In the structural formula represented by formula (1), the tricyclic heterocycle formed by including $A^1$, $A^2$, and $L^1$ represents a chemically stable heterocycle and also includes one having partially an unsaturated bond. The tricyclic heterocycle preferably has the structure shown below.

[Formula 5]

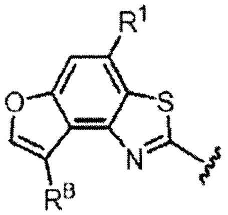
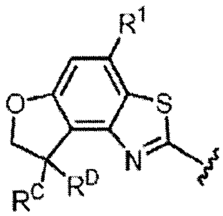
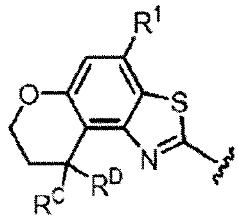
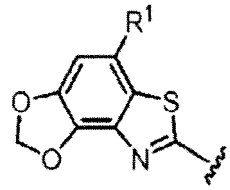
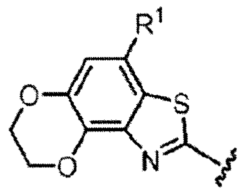
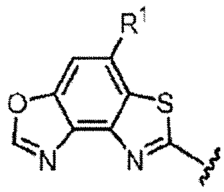
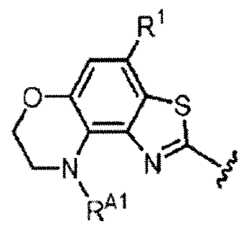
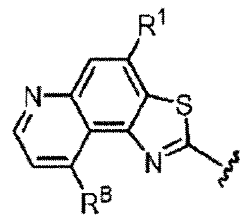
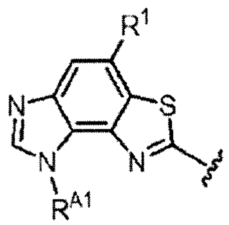
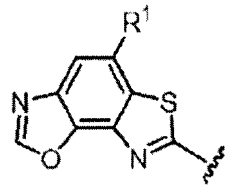
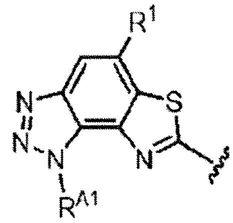
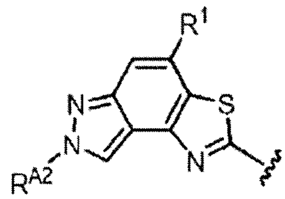
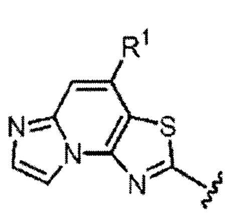
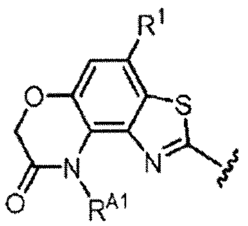

A "4- to 11-membered saturated heterocycle formed by $R^2$ and $R^3$ together with the nitrogen atom to which they are attached," a "4- to 11-membered saturated heterocycle formed by $R^3$ and $R^4$ together with the nitrogen atom to which they are attached," and a "4- to 11-membered saturated heterocycle formed by any carbon atom on a saturated heterocycle constituted by $R^2$, $R^3$, and a nitrogen atom, and $R^4$ together" mean a monocyclic or bicyclic saturated heterocycle containing one or two or more heteroatoms other than the nitrogen atom as an atom constituting the ring, and also includes one having partially an unsaturated bond and one having a crosslinked structure. The "4- to 11-membered saturated heterocycle formed by $R^2$ and $R^3$ together with the nitrogen atom to which they are attached," the "4- to 11-membered saturated heterocycle formed by $R^3$ and $R^4$ together with the nitrogen atom to which they are attached," and the "4- to 11-membered saturated heterocycle formed by any carbon atom on a saturated heterocycle constituted by $R^2$, $R^3$, and a nitrogen atom, and $R^4$ together" is each preferably a "4- to 8-membered saturated heterocycle." Specific examples of the "4- to 8-membered saturated heterocycle" include an azetidine ring, a pyrrolidine ring, a piperidine ring, an azepane ring, an azocane ring, a morpholine ring, a piperazine ring, an oxazocane ring, an azabicycloheptane ring, and an azabicyclooctane ring. Specific examples of the "4- to 11-membered saturated heterocycle" include an azonane ring, an oxazonane ring, an azecane ring, an oxazecane ring, an azacycloundecane ring, an azabicyclononane ring, and an azabicyclodecane ring, in addition to those given as specific examples of the "4- to 8-membered saturated heterocycle."

When Z is $-OR^7$, a "5- to 11-membered saturated heterocycle formed by $R^4$ and $R^7$ together with the carbon atom and the oxygen atom to which they, respectively, are attached" means a monocyclic or bicyclic saturated heterocycle containing one or two or more heteroatoms other than the oxygen atom as an atom constituting the ring, and also includes one having partially an unsaturated bond and one having a crosslinked structure. The "5- to 11-membered saturated heterocycle formed by $R^4$ and $R^7$ together with the carbon atom and the oxygen atom to which they, respectively, are attached" is preferably a "5- to 8-membered saturated heterocycle." Specific examples of the "5-to 8-membered saturated heterocycle" include a tetrahydrofuran ring, a tetrahydropyran ring, an oxepane ring, and an oxocane ring. Specific examples of the "5- to 11-membered saturated heterocycle" include an oxonane ring, an oxecane ring, and an oxacycloundecane ring, in addition to those given as specific examples of the "5- to 8-membered saturated heterocycle."

As used herein, for example, the bond of a substituent drawn in such a way as to cross a pyrrolidine ring as represented by the following formula (W) means substitution with one substituent at any substitutable position on the pyrrolidine ring, and specifically, the compound represented by the following formula (W-1) or (W-2) is included in the compound represented by the following formula (W).

[Formula 6]

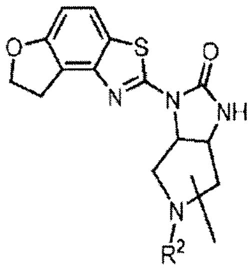

(W)

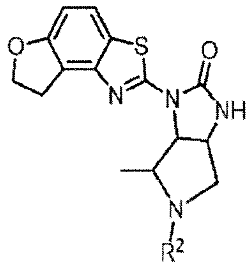

(W-1)

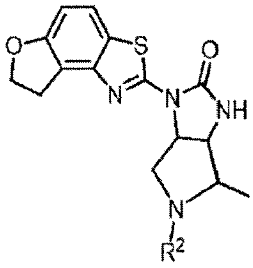

(W-2)

In the compound of the present invention represented by formula (1), the definitions and preferred ranges of $A^1$, $A^2$, $L^1$, $L^2$, $R^{A1}$, $R^{A2}$, $R^B$, $R^C$, $R^D$, $R^E$, $R^F$, $R^1$, $R^2$, $R^3$, $R^4$, X, 1, m, n, Z, $R^7$, p, q, t, and $R^G$ are as follows, and the technical scope of the present invention is not limited to the scope of compounds listed below.

$A^1$ is an oxygen atom or a nitrogen atom (=N-) and preferably an oxygen atom.

$A^2$ is $CR^B$, $CR^CR^D$, an oxygen atom, or $NR^{A1}$, preferably $CR^CR^D$ or an oxygen atom, and more preferably methylene or an oxygen atom.

$L^1$ is optionally substituted methylene, optionally substituted ethylene, optionally substituted methine, optionally substituted ethanediylidene, or =N-, and preferably methylene.

$L^2$ is optionally substituted $C_{1-4}$ alkylene and preferably methylene, ethylene, or propylene. $L^2$ is more preferably methylene or ethylene.

$R^{A1}$, $R^{A2}$, $R^B$, $R^C$, and $R^D$ are each a hydrogen atom or optionally substituted $C_{1-6}$ alkyl and preferably a hydrogen atom.

$R^E$ and $R^G$ are each optionally substituted $C_{1-6}$ alkyl and preferably methyl.

$R^F$ is a hydrogen atom, a halogen atom, or optionally substituted $C_{1-6}$ alkyl and preferably a hydrogen atom or optionally substituted $C_{1-6}$ alkyl.

$R^1$ is a hydrogen atom, a halogen atom, or optionally substituted $C_{1-6}$ alkyl, preferably a hydrogen atom or a halogen atom, and more preferably a hydrogen atom.

$R^2$ is a hydrogen atom, optionally substituted $C_{1-6}$ alkyl, $C(O)-R^E$, $C_{3-10}$ cycloalkyl, $C_{2-6}$ alkynyl, or a cyclic group of a 4- to 11-membered saturated heterocycle, preferably a hydrogen atom, optionally substituted $C_{1-6}$ alkyl, or $C_{3-10}$ cycloalkyl, and more preferably a hydrogen atom or optionally substituted $C_{1-6}$ alkyl.

$R^3$ is a hydrogen atom, optionally substituted $C_{1-6}$ alkyl, or $C(O)-R^E$ and preferably a hydrogen atom or optionally substituted $C_{1-6}$ alkyl.

$R^4$ is optionally substituted $C_{1-6}$ alkyl, preferably optionally substituted $C_{1-4}$ alkyl, and more preferably methyl or ethyl.

X is a carbon atom or a nitrogen atom and preferably a carbon atom.

l, m, p, and q are each 1, 2, or 3 and preferably 1 or 2.

n and t are each 1, 2, 3, or 4 and preferably 1 or 2.

Z is $-NR^2R^3$ or $-OR^7$, and when Z is $-NR^2R^3$, preferably $R^2$ and $R^3$, together with the nitrogen atom to which they are attached, form an optionally substituted 4- to 8-membered saturated heterocycle, or $R^3$ and $R^4$, together with the nitrogen atom and the carbon atom to which they, respectively, are attached, form an optionally substituted 4- to 8-membered saturated heterocycle; when Z is $-OR^7$, $R^7$ is optionally substituted $C_{1-6}$ alkyl, or optionally substituted $C_{1-7}$ alkylene formed together with $R^4$, preferably optionally substituted methylene or optionally substituted ethylene, and $R^4$ and $R^7$, together with the carbon atom and the oxygen atom to which they, respectively, are attached, form an optionally substituted 5- to 8-membered saturated heterocycle.

As used herein, the substituent when the "optionally substituted $C_{1-6}$ alkyl" is substituted is one or more substituents selected from the group consisting of a halogen atom, hydroxy (wherein the hydroxy is optionally substituted with a protective group for hydroxy), optionally substituted $C_{3-10}$ cycloalkyl and optionally substituted $C_{1-6}$ alkoxy, a $C_{2-6}$ alkynyl group, nitrile, $C_{1-6}$ alkoxycarbonyl, formyl, and a 4-to 11-membered saturated heterocycle (wherein the saturated heterocycle is optionally substituted with optionally substituted $C_{1-6}$ alkyl, a halogen atom, and a protective group for a nitrogen atom), and substitution with such a substituent occurs at any substitutable position. The number of the substituents is preferably 1 to 5, and more preferably 1 to 3. When substitution with two or more substituents occurs, these substituents may be the same or different.

As used herein, the substituent when the "optionally substituted $C_{3-10}$ cycloalkyl" is substituted is one or more substituents selected from the group consisting of a halogen atom, hydroxy, $C_{1-6}$ alkyl, $C_{1-6}$ alkoxy, and a $C_{3-10}$ cycloalkyl group, and substitution with such a substituent occurs at any substitutable position. The number of the substituents is preferably 1 to 5, and more preferably 1 to 3. When substitution with two or more substituents occurs, these substituents may be the same or different.

As used herein, the substituent when the "optionally substituted $C_{1-6}$ alkoxy" is substituted is one or more substituents selected from the group consisting of a halogen atom, hydroxy, $C_{1-6}$ alkyl, $C_{1-6}$ alkoxy, and a $C_{3-8}$ cycloalkyl group, and substitution with such a substituent occurs at any substitutable position. The number of the substituents is preferably 1 to 5, and more preferably 1 to 3. When substitution with two or more substituents occurs, these substituents may be the same or different.

As used herein, the substituent when each of the "optionally substituted $C_{1-7}$ alkylene," the "optionally substituted $C_{1-4}$ alkylene," and the "optionally substituted $C_{1-3}$ alkylene" is substituted is one or more substituents selected from the group consisting of a halogen atom, hydroxy, $C_{1-6}$ alkyl optionally substituted with a halogen atom or hydroxy (wherein the hydroxy is optionally substituted with a protective group for hydroxy), $C_{1-6}$ alkoxy, a $C_{3-8}$ cycloalkyl group, nitrile, $C_{1-6}$ alkoxycarbonyl, formyl, a $C_{2-6}$ alkynyl group, an oxo group, and a 4- to 11-membered saturated heterocycle (wherein the saturated heterocycle is optionally substituted with optionally substituted $C_{1-6}$ alkyl, a halogen atom, and a protective group for a nitrogen atom), and substitution with such a substituent occurs at any substitutable position. The number of the substituents is preferably 1 to 5, and more preferably 1 to 3. When substitution with two or more substituents occurs, these substituents may be the same or different, and two substituents on the same carbon atom, together with the carbon atom to which they are attached, may form a spiro ring consisting of a 4- to 8-membered saturated heterocycle or a 3-to 8-membered saturated carbocycle.

As used herein, the substituent when the "optionally substituted ethylene" is substituted is one or more substituents selected from the group consisting of $C_{1-6}$ alkyl optionally substituted with a halogen atom or hydroxy (wherein the hydroxy is optionally substituted with a protective group for hydroxy), nitrile, $C_{1-6}$ alkoxycarbonyl, formyl, a $C_{2-6}$ alkynyl group, a 4- to 11-membered saturated heterocycle (wherein the saturated heterocycle is optionally substituted with optionally substituted $C_{1-6}$ alkyl, a halogen atom, and a protective group for a nitrogen atom), and an oxo group, and substitution with such a substituent occurs at any substitutable position. The number of the substituents is preferably 1 to 4. When substitution with two or more substituents occurs, these substituents may be the same or different, and two substituents on the same carbon atom, together with the carbon atom to which they are attached, may form a spiro ring consisting of a 4- to 8-membered saturated heterocycle or a 3- to 8-membered saturated carbocycle.

As used herein, the substituent when each of "optionally substituted methylene," "optionally substituted methine," and "optionally substituted ethanediylidene" is substituted is one or more substituents selected from the group consisting of $C_{1-6}$ alkyl optionally substituted with a halogen atom or hydroxy (wherein the hydroxy is optionally substituted with a protective group for hydroxy), nitrile, $C_{1-6}$ alkoxycarbonyl, formyl, a $C_{2-6}$ alkynyl group, an oxo group, and a 4- to 11-membered saturated heterocycle (wherein the saturated heterocycle is optionally substituted with optionally substituted $C_{1-6}$ alkyl, a halogen atom, and a protective group for a nitrogen atom), and substitution with such a substituent occurs at any substitutable position. The number of the substituents is preferably 1 to 4. When substitution with two or more substituents occurs, these substituents may be the same or different, and two substituents on the same carbon atom,

together with the carbon atom to which they are attached, may form a spiro ring consisting of a 4- to 8-membered saturated heterocycle or a 3- to 8-membered saturated carbocycle.

As used herein, the substituent that each of the "optionally substituted 4- to 11-membered saturated heterocycle," the "optionally substituted 4- to 8-membered saturated heterocycle," the "optionally substituted 5- to 8-membered saturated heterocycle," and the "optionally substituted 5- to 11-membered saturated heterocycle" optionally has is one or more substituents selected from the group consisting of a halogen atom, hydroxy, $C_{1-6}$ alkyl optionally substituted with a halogen atom or hydroxy (wherein the hydroxy is optionally substituted with a protective group for hydroxy), $C_{1-6}$ alkoxy, $C_{3-8}$ cycloalkyl, nitrile, $C_{1-6}$ alkoxycarbonyl, formyl, a $C_{2-6}$ alkynyl group, an oxo group, and a 4- to 11-membered saturated heterocycle (wherein the saturated heterocycle is optionally substituted with optionally substituted $C_{1-6}$ alkyl, a halogen atom, and a protective group for a nitrogen atom), and substitution with such a substituent occurs at any substitutable position. The number of the substituents is preferably 1 to 5, and more preferably 1 to 3. When substitution with two or more substituents occurs, these substituents may be the same or different, and two substituents on the same carbon atom on the ring, together with the carbon atom to which they are attached, may form a spiro ring consisting of a 4- to 8-membered saturated heterocycle or a 3- to 8-membered saturated carbocycle, or two substituents on different carbon atoms on the ring may combine to form a crosslink.

Among the compounds of the present invention represented by formula (1), examples of a preferred compound include the following compounds or pharmaceutically acceptable salts thereof.

A compound wherein $A^1$ is an oxygen atom, $A^2$ is methylene, $L^1$ is methylene, $L^2$ is optionally substituted $C_{1-4}$ alkylene, X is a carbon atom, $R^1$ is a hydrogen atom, and $R^2$, $R^3$, and $R^4$ are each optionally substituted $C_{1-6}$ alkyl.

A compound wherein $A^1$ is an oxygen atom, $A^2$ is methylene, $L^1$ is methylene, $L^2$ is methylene or ethylene, X is a carbon atom, $R^1$ is a hydrogen atom, $R^2$ and $R^3$ are each optionally substituted $C_{1-6}$ alkyl, and $R^4$ is methyl or ethyl.

A compound wherein $A^1$ is an oxygen atom, $A^2$ is methylene, $L^1$ is methylene, $L^2$ is optionally substituted $C_{1-4}$ alkylene, X is a carbon atom, $R^1$ is a hydrogen atom, $R^2$ and $R^3$, together with the nitrogen atom to which they are attached, form an optionally substituted 4- to 11-membered saturated heterocycle, and $R^4$ is optionally substituted $C_{1-6}$ alkyl.

A compound wherein $A^1$ is an oxygen atom, $A^2$ is methylene, $L^1$ is methylene, $L^2$ is methylene or ethylene, X is a carbon atom, $R^1$ is a hydrogen atom, $R^2$ and $R^3$, together with the nitrogen atom to which they are attached, form an optionally substituted 4- to 8-membered saturated heterocycle, and $R^4$ is methyl or ethyl.

A compound wherein $A^1$ is an oxygen atom, $A^2$ is methylene, $L^1$ is methylene, X is a carbon atom, $R^1$ is a hydrogen atom, $R^2$ is a hydrogen atom or optionally substituted $C_{1-6}$ alkyl, $R^3$ and $R^4$, together with the nitrogen atom and the carbon atom to which they, respectively, are attached, form an optionally substituted 4- to 11-membered saturated heterocycle.

A compound wherein $A^1$ is an oxygen atom, $A^2$ is methylene, $L^1$ is methylene, X is a carbon atom, $R^1$ is a hydrogen atom, $R^2$ is optionally substituted $C_{1-6}$ alkyl, $R^3$ and $R^4$, together with the nitrogen atom and the carbon atom to which they, respectively, are attached, form an optionally substituted 4- to 8-membered saturated heterocycle.

A compound wherein $A^1$ and $A^2$ are each an oxygen atom, $L^1$ is methylene or ethylene, X is a carbon atom, $R^1$ is a hydrogen atom, $L^2$ is optionally substituted $C_{1-4}$ alkylene, and $R^2$, $R^3$, and $R^4$ are each optionally substituted $C_{1-6}$ alkyl.

A compound wherein $A^1$ and $A^2$ are each an oxygen atom, $L^1$ is methylene or ethylene, X is a carbon atom, $L^2$ is optionally substituted $C_{1-4}$ alkylene, $R^1$ is a hydrogen atom, $R^2$ and $R^3$, together with the nitrogen atom to which they are attached, form an optionally substituted 4- to 11-membered saturated heterocycle, and $R^4$ is optionally substituted $C_{1-6}$ alkyl.

A compound wherein $A^1$ and $A^2$ are each an oxygen atom, $L^1$ is methylene or ethylene, X is a carbon atom, $R^1$ is a hydrogen atom, $R^2$ is a hydrogen atom or optionally substituted $C_{1-6}$ alkyl, $R^3$ and $R^4$, together with the nitrogen atom and the carbon atom to which they, respectively, are attached, form an optionally substituted 4- to 11-membered saturated heterocycle.

A compound wherein $A^1$ is an oxygen atom, $A^2$ is methylene, $L^1$ is methylene or ethylene, $L^2$ is optionally substituted $C_{1-4}$ alkylene, X is a carbon atom, $R^1$ is a hydrogen atom, Z is $-OR^7$, $R^7$ is optionally substituted $C_{1-7}$ alkylene formed together with $R^4$, and $R^4$ and $R^7$, together with the carbon atom and the oxygen atom to which they, respectively, are attached, form an optionally substituted 5- to 11-membered saturated heterocycle.

A compound wherein $A^1$ is an oxygen atom, $A^2$ is methylene, $L^1$ is methylene or ethylene, $L^2$ is methylene or ethylene, X is a carbon atom, $R^1$ is a hydrogen atom, Z is $-OR^7$, $R^7$ is optionally substituted $C_{1-7}$ alkylene formed together with $R^4$, and $R^4$ and $R^7$, together with the carbon atom and the oxygen atom to which they, respectively, are attached, form an optionally substituted 5- to 11-membered saturated heterocycle.

A compound wherein $A^1$ and $A^2$ are each an oxygen atom, $L^1$ is methylene or ethylene, $L^2$ is optionally substituted $C_{1-4}$ alkylene, X is a carbon atom, $R^1$ is a hydrogen atom, Z is $-OR^7$, $R^7$ is optionally substituted $C_{1-7}$ alkylene formed together with $R^4$, and $R^4$ and $R^7$, together with the carbon atom and the oxygen atom to which they, respectively, are attached, form an optionally substituted 5- to 11-membered saturated heterocycle.

A compound wherein $A^1$ and $A^2$ are each an oxygen atom, $L^1$ is methylene or ethylene, $L^2$ is methylene or ethylene, X is a carbon atom, $R^1$ is a hydrogen atom, Z is $-OR^7$, $R^7$ is optionally substituted $C_{1-7}$ alkylene formed together

with $R^4$, and $R^4$ and $R^7$, together with the carbon atom and the oxygen atom to which they, respectively, are attached, form an optionally substituted 5- to 11-membered saturated heterocycle.

Among the compounds of the present invention represented by formula (1), specific examples of a preferred compound include the following compounds or pharmaceutically acceptable salts thereof.

cis-3-(7,8-Dihydrofuro[3,2-e][1,3]benzothiazol-2-yl)-5-methyloctahydro-2H-imidazo[4,5-c]pyridin-2-one (Example 7);

cis-3-(7,8-dihydrofuro[3,2-e][1,3]benzothiazol-2-yl)-5-(2-methoxyethyl)octahydro-2H-imidazo[4,5-c]pyridin-2-one (Example 8);
cis-1-(7,8-dihydrofuro[3,2-e][1,3]benzothiazol-2-yl)-5-methyloctahydro-2H-imidazo[4,5-c]pyridin-2-one (Example 9);
cis-3-(7,8-dihydrofuro[3,2-e][1,3]benzothiazol-2-yl)-5-ethyloctahydro-2H-imidazo[4,5-c]pyridin-2-one (Example 11);
cis-1-(7,8-dihydrofuro[3,2-e][1,3]benzothiazol-2-yl)-5-methylhexahydropyrrolo[3,4-d]imidazol-2(1H)-one (Example 14);
cis-3-(7,8-dihydrofuro[3,2-e][1,3]benzothiazol-2-yl)-5-(propan-2-yl)octahydro-2H-imidazo[4,5-c]pyridin-2-one (Example 15);
cis-3-(7,8-dihydrofuro[3,2-e][1,3]benzothiazol-2-yl)-5-methyloctahydroimidazo[4,5-c]azepin-2(1H)-one (Example 17);
(3aS,7aS)-3-(7,8-dihydrofuro[3,2-e][1,3]benzothiazol-2-yl)-5-(oxetan-3-yl)octahydro-2H-imidazo[4,5-c]pyridin-2-one (Example 19);
(3aR,7aR)-1-(2H-[1,3]dioxolo[4,5-e][1,3]benzothiazol-7-yl)hexahydropyrano[3,4-d]imidazol-2(3H)-one (Example 21);
(4S,5R)-1-(7,8-dihydrofuro[3,2-e][1,3]benzothiazol-2-yl)-4-methyl-5-[(morpholin-4-yl)methyl]imidazolidin-2-one (Example 23);
(4R,5R)-1-(7,8-dihydrofuro[3,2-e][1,3]benzothiazol-2-yl)-4-methyl-5-[(morpholin-4-yl)methyl]imidazolidin-2-one (Example 24);
(3aR,7aS)-3-(7,8-dihydrofuro[3,2-e][1,3]benzothiazol-2-yl)-5-methyloctahydro-2H-imidazo[4,5-c]pyridin-2-one (Example 28);
(3aR,7aS)-5-cyclopropyl-3-(7,8-dihydro[1,4]dioxino[2,3-e][1,3]benzothiazol-2-yl)octahydro-2H-imidazo[4,5-c]pyridin-2-one (Example 34);
(3aR,7aS)-5-cyclopropyl-3-(2H-[1,3]dioxolo[4,5-e][1,3]benzothiazol-7-yl)octahydro-2H-imidazo[4,5-c]pyridin-2-one (Example 36);
(3aR,7aR)-1-(7,8-dihydro[1,4]dioxino[2,3-e][1,3]benzothiazol-2-yl)hexahydropyrano[3,4-d]imidazol-2(3H)-one (Example 37);
(3aR,6S,7aR)-1-(7,8-dihydrofuro[3,2-e][1,3]benzothiazol-2-yl)-6-methylhexahydropyrano[3,4-d]imidazol-2(3H)-one (Example 38);
(3aR,6S,7aR)-1-(7,8-dihydro[1,4]dioxino[2,3-e][1,3]benzothiazol-2-yl)-6-methylhexahydropyrano[3,4-d]imidazol-2(3H)-one (Example 39);
(3aR,6R,7aR)-1-(7,8-dihydrofuro[3,2-e][1,3]benzothiazol-2-yl)-6-(fluoromethyl)hexahydropyrano[3,4-d]imidazol-2(3H)-one (Example 40);
(3aR,6R,7aR)-1-(7,8-dihydro[1,4]dioxino[2,3-e][1,3]benzothiazol-2-yl)-6-(fluoromethyl)hexahydropyrano[3,4-d]imidazol-2(3H)-one (Example 41);
(3aR,6S,7aR)-1-(7,8-dihydrofuro[3,2-e][1,3]benzothiazol-2-yl)-6-ethylhexahydropyrano[3,4-d]imidazol-2(3H)-one (Example 42);
(3aR,6S,7aR)-1-(7,8-dihydro[1,4]dioxino[2,3-e][1,3]benzothiazol-2-yl)-6-ethylhexahydropyrano[3,4-d]imidazol-2(3H)-one (Example 43);
(4S,5S)-1-(7,8-dihydrofuro[3,2-e][1,3]benzothiazol-2-yl)-5-(methoxymethyl)-4-methylimidazolidin-2-one (Example 44);
(3aR,6aS)-1-(7,8-dihydrofuro[3,2-e][1,3]benzothiazol-2-yl)tetrahydro-1H-furo[3,4-d]imidazol-2(3H)-one (Example 65);
rac-(3aR,7aS)-3-(7,8-dihydrofuro[3,2-e][1,3]benzothiazol-2-yl)-5-(oxetan-3-yl)octahydro-2H-imidazo[4,5-c]pyridin-2-one (Example 71);
rac-(3aR,7aS)-5-cyclobutyl-3-(7,8-dihydrofuro[3,2-e][1,3]benzothiazol-2-yl)octahydro-2H-imidazo[4,5-c]pyridin-2-one (Example 72);
rac-(3aR,8aS)-3-(7,8-dihydrofuro[3,2-e][1,3]benzothiazol-2-yl)-5-(oxetan-3-yl)octahydroimidazo[4,5-c]azepin-2(1H)-one (Example 73);
rac-(3aR,7aS)-3-(7,8-dihydro[1,4]dioxino[2,3-e][1,3]benzothiazol-2-yl)-5-methyloctahydro-2H-imidazo[4,5-c]pyridin-2-one (Example 76);

rac-(3aR,7aS)-1-(7,8-dihydro[1,4]dioxino[2,3-e][1,3]benzothiazol-2-yl)-5-methyloctahydro-2H-imidazo[4,5-c]pyridin-2-one (Example 77);
rac-(3aR,7aS)-1-(2H-[1,3]dioxolo[4,5-e][1,3]benzothiazol-7-yl)-5-methyloctahydro-2H-imidazo[4,5-c]pyridin-2-one (Example 78);
rac-(3aR,7aS)-3-(2H-[1,3]dioxolo[4,5-e][1,3]benzothiazol-7-yl)-5-methyloctahydro-2H-imidazo[4,5-c]pyridin-2-one (Example 79);
rac-(3aR,7aS)-3-(7,8-dihydro[1,4]dioxino[2,3-e][1,3]benzothiazol-2-yl)-5-(oxetan-3-yl)octahydro-2H-imidazo[4,5-c]pyridin-2-one (Example 80);
rac-(3aR,7aS)-3-(2H-[1,3]dioxolo[4,5-e][1,3]benzothiazol-7-yl)-5-(oxetan-3-yl)octahydro-2H-imidazo[4,5-c]pyridin-2-one (Example 81);
rac-(3aR,8aS)-3-(2H-[1,3]dioxolo[4,5-e][1,3]benzothiazol-7-yl)-5-methyloctahydroimidazo[4,5-c]azepin-2(1H)-one (Example 85);
rac-(3aR,8aS)-3-(2H-[1,3]dioxolo[4,5-e][1,3]benzothiazol-7-yl)-5-(oxetan-3-yl)octahydroimidazo[4,5-c]azepin-2(1H)-one (Example 86);
(3aS,6S,7aR)-1-(7,8-dihydrofuro[3,2-e][1,3]benzothiazol-2-yl)-5,6-dimethyloctahydro-2H-imidazo[4,5-c]pyridin-2-one (Example 93);
rac-(3aR,7aS)-5-cyclopropyl-3-(7,8-dihydrofuro[3,2-e][1,3]benzothiazol-2-yl)octahydro-2H-imidazo[4,5-c]pyridin-2-one (Example 98);
rac-(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Example 99);
rac-[(3aR,7aS)-3-(7,8-dihydrofuro[3,2-e][1,3]benzothiazol-2-yl)-2-oxooctahydro-5H-imidazo[4,5-c]pyridin-5-yl]acetonitrile (Example 100);
rac-(3aR,6aS)-1-(7,8-dihydrofuro[3,2-e][1,3]benzothiazol-2-yl)-5-(2-propyn-1-yl)hexahydropyrrolo[3,4-d]imidazol-2(1H)-one (Example 102);
rac-[(3aR,6aS)-1-(7,8-dihydrofuro[3,2-e][1,3]benzothiazol-2-yl)-2-oxohexahydropyrrolo[3,4-d]imidazol-5(1H)-yl]acetonitrile (Example 103);
rac-(3aR,8aS)-5-cyclopropyl-3-(7,8-dihydrofuro[3,2-e][1,3]benzothiazol-2-yl)octahydroimidazo[4,5-c]azepin-2(1H)-one (Example 104);
rac-(3aR,8aS)-3-(7,8-dihydrofuro[3,2-e][1,3]benzothiazol-2-yl)-5-(2-propyn-1-yl)octahydroimidazo[4,5-c]azepin-2(1H)-one (Example 105);
rac-[(3aR,8aS)-3-(7,8-dihydrofuro[3,2-e][1,3]benzothiazol-2-yl)-2-oxooctahydroimidazo[4,5-c]azepin-5(1H)-yl]acetonitrile (Example 106);
rac-(3aR,7aS)-3-(7,8-dihydrofuro[3,2-e][1,3]benzothiazol-2-yl)-5-(2,2,2-trifluoroethyl)octahydro-2H-imidazo[4,5-c]pyridin-2-one (Example 107);
rac-(3aR,7aS)-5-(2,2-difluoroethyl)-3-(7,8-dihydrofuro[3,2-e][1,3]benzothiazol-2-yl)octahydro-2H-imidazo[4,5-c]pyridin-2-one (Example 108);
(3aR,7aS)-5-cyclopropyl-3-(7,8-dihydrofuro[3,2-e][1,3]benzothiazol-2-yl)octahydro-2H-imidazo[4,5-c]pyridin-2-one (Example 110);
[(3aR,7aS)-3-(7,8-dihydrofuro[3,2-e][1,3]benzothiazol-2-yl)-2-oxooctahydro-5H-imidazo[4,5-c]pyridin-5-yl]acetonitrile (Example 112);
(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Example 114);
(4S,5R)-1-(7,8-dihydrofuro[3,2-e][1,3]benzothiazol-2-yl)-5-{[(3S)-3-fluoropyrrolidin-1-yl]methyl}-4-methylimidazolidin-2-one (Example 115);
(4S,5R)-5-[(3,3-difluoroazetidin-1-yl)methyl]-1-(7,8-dihydrofuro[3,2-e][1,3]benzothiazol-2-yl)-4-methylimidazolidin-2-one (Example 116);
(4S,5R)-5-[(3,3-difluoropyrrolidin-1-yl)methyl]-1-(7,8-dihydrofuro[3,2-e][1,3]benzothiazol-2-yl)-4-methylimidazolidin-2-one (Example 117);
(4S,5R)-1-(7,8-dihydrofuro[3,2-e][1,3]benzothiazol-2-yl)-5-[(3-fluoroazetidin-1-yl)methyl]-4-methylimidazolidin-2-one (Example 118);
rac-(3aR,7aR)-3-(7,8-dihydrofuro[3,2-e][1,3]benzothiazol-2-yl)hexahydropyrano[3,4-d]imidazol-2(3H)-one (Example 120);
rac-(3aR,7aR)-1-(7,8-dihydrofuro[3,2-e][1,3]benzothiazol-2-yl)hexahydropyrano[3,4-d]imidazol-2(3H)-one (Example 121);
(3aR,7aR)-1-(7,8-dihydrofuro[3,2-e][1,3]benzothiazol-2-yl)hexahydropyrano[3,4-d]imidazol-2(3H)-one (Example 122);
(3aR,7aR)-1-(7,8-dihydrofuro[3,2-e][1,3]benzothiazol-2-yl)-6,6-dimethylhexahydropyrano[3,4-d]imidazol-2(3H)-one (Example 124);

(3aR,6R,7aR)-1-(7,8-dihydrofuro[3,2-e][1,3]benzothiazol-2-yl)-2-oxooctahydropyrano[3,4-d]imidazole-6-carbonitrile (Example 127);
(3aR,6R,7aR)-1-(7,8-dihydrofuro[3,2-e][1,3]benzothiazol-2-yl)-6-methoxyhexahydropyrano[3,4-d]imidazol-2(3H)-one (Example 129);
(3aR,6R,7aR)-1-(2H-[1,3]dioxolo[4,5-e][1,3]benzothiazol-7-yl)-2-oxooctahydropyrano[3,4-d]imidazole-6-carbonitrile (Example 131);
(3aR,6R,7aR)-1-(7,8-dihydro[1,4]dioxino[2,3-e][1,3]benzothiazol-2-yl)-2-oxooctahydropyrano[3,4-d]imidazole-6-carbonitrile (Example 133);
(3aR,8aR)-1-(7,8-dihydro[1,4]dioxino[2,3-e][1,3]benzothiazol-2-yl)hexahydro-1H-oxepino[3,4-d]imidazol-2(3H)-one (Example 135);
(3aR,6S,7aR)-1-(2H-[1,3]dioxolo[4,5-e][1,3]benzothiazol-7-yl)-6-ethylhexahydropyrano[3,4-d]imidazol-2(3H)-one (Example 138);
(3aR,6S,7aR)-1-(2H-[1,3]dioxolo[4,5-e][1,3]benzothiazol-7-yl]-6-methylhexahydropyrano[3,4-d]imidazol-2(3H)-one (Example 139);
(3aR,6R,7aR)-1-(2H-[1,3]dioxolo[4,5-e][1,3]benzothiazol-7-yl]-6-(fluoromethyl)hexahydropyrano[3,4-d]imidazol-2(3H)-one (Example 140);
(3aR,6R,7aR)-1-(7,8-dihydrofuro[3,2-e][1,3]benzothiazol-2-yl)-6-(hydroxymethyl)hexahydropyrano[3,4-d]imidazol-2(3H)-one (Example 144);
(3aR,6R,7aR)-1-(7,8-dihydro[1,4]dioxino[2,3-e][1,3]benzothiazol-2-yl)-6-(hydroxymethyl)hexahydropyrano[3,4-d] imidazol-2(3H)-one (Example 146);
(3aR,6R,7aR)-1-(2H-[1,3]dioxolo[4,5-e][1,3]benzothiazol-7-yl)-6-(hydroxymethyl)hexahydropyrano[3,4-d]imidazol-2(3H)-one (Example 147);
(3aR,6R,7aR)-1-(7,8-dihydrofuro[3,2-e][1,3]benzothiazol-2-yl)-6-ethynylhexahydropyrano[3,4-d]imidazol-2(3H)-one (Example 149);
(3aR,7aR)-1-(7,8-dihydrofuro[3,2-e][1,3]benzothiazol-2-yl)-6-fluorohexahydropyrano[3,4-d]imidazol-2(3H)-one (Example 151);
(3aR,6S,6aS)-1-(7,8-dihydrofuro[3,2-e][1,3]benzothiazol-2-yl)-6-(hydroxymethyl)tetrahydro-1H-furo[3,4-d]imidazol-2(3H)-one (Example 157);
(3aR,6S,6aS)-1-(7,8-dihydrofuro[3,2-e][1,3]benzothiazol-2-yl)-6-(fluoromethyl)tetrahydro-1H-furo[3,4-d]imidazol-2(3H)-one (Example 159);
(3aR,6R,6aS)-1-(7,8-dihydrofuro[3,2-e][1,3]benzothiazol-2-yl)-6-methyltetrahydro-1H-furo[3,4-d]imidazol-2(3H)-one (Example 161); and
[(3aS,4R,6aR)-3-(7,8-dihydrofuro[3,2-e][1,3]benzothiazol-2-yl)-2-oxohexahydro-1H-furo[3,4-d]imidazol-4-yl]acetonitrile (Example 162).

Among the compounds of the present invention represented by formula (1), specific examples of a more preferable compound include the following compounds or pharmaceutically acceptable salts thereof.

(3aR,7aR)-1-(2H-[1,3]Dioxolo[4,5-e][1,3]benzothiazol-7-yl)hexahydropyrano[3,4-d]imidazol-2(3H)-one (Example 21);
(4S,5R)-1-(7,8-dihydrofuro[3,2-e][1,3]benzothiazol-2-yl)-4-methyl-5-[(morpholin-4-yl)methyl]imidazolidin-2-one (Example 23);
(3aR,7aS)-5-cyclopropyl-3-(7,8-dihydro[1,4]dioxino[2,3-e][1,3]benzothiazol-2-yl)octahydro-2H-imidazo[4,5-c]pyridin-2-one (Example 34);
(3aR,7aS)-5-cyclopropyl-3-(2H-[1,3]dioxolo[4,5-e][1,3]benzothiazol-7-yl)octahydro-2H-imidazo[4,5-c]pyridin-2-one (Example 36);
(3aR,7aR)-1-(7,8-dihydro[1,4]dioxino[2,3-e][1,3]benzothiazol-2-yl)hexahydropyrano[3,4-d]imidazol-2(3H)-one (Example 37);
(3aR,6S,7aR)-1-(7,8-dihydrofuro[3,2-e][1,3]benzothiazol-2-yl)-6-methylhexahydropyrano[3,4-d]imidazol-2(3H)-one (Example 38); (3aR,6S,7aR)-1-(7,8-dihydro[1,4]dioxino[2,3-e][1,3]benzothiazol-2-yl)-6-methylhexahydropyrano[3,4-d]imidazol-2(3H)-one (Example 39);
(3aR,6R,7aR)-1-(7,8-dihydrofuro[3,2-e][1,3]benzothiazol-2-yl)-6-(fluoromethyl)hexahydropyrano[3,4-d]imidazol-2(3H)-one (Example 40);
(3aR,6R,7aR)-1-(7,8-dihydro[1,4]dioxino[2,3-e][1,3]benzothiazol-2-yl)-6-(fluoromethyl)hexahydropyrano[3,4-d]imidazol-2(3H)-one (Example 41);
(3aR,6S,7aR)-1-(7,8-dihydrofuro[3,2-e][1,3]benzothiazol-2-yl)-6-ethylhexahydropyrano[3,4-d]imidazol-2(3H)-one (Example 42);
(3aR,6S,7aR)-1-(7,8-dihydro[1,4]dioxino[2,3-e][1,3]benzothiazol-2-yl)-6-ethylhexahydropyrano[3,4-d]imidazol-2(3H)-one (Example 43);
(4S,5S)-1-(7,8-dihydrofuro[3,2-e][1,3]benzothiazol-2-yl)-5-(methoxymethyl)-4-methylimidazolidin-2-one (Example 44);
rac-(3aR,8aS)-3-(7,8-dihydrofuro[3,2-e][1,3]benzothiazol-2-yl)-5-(oxetan-3-yl)octahydroimidazo[4,5-c]azepin-2(1H)-one (Example 73);
rac-(3aR,7aS)-5-cyclopropyl-3-(7,8-dihydrofuro[3,2-e][1,3]benzothiazol-2-yl)octahydro-2H-imidazo[4,5-c]pyridin-2-one (Example 98);
rac-(3aR,8aS)-5-cyclopropyl-3-(7,8-dihydrofuro[3,2-e][1,3]benzothiazol-2-yl)octahydroimidazo[4,5-c]azepin-2(1H)-one (Example 104);
(3aR,7aS)-5-cyclopropyl-3-(7,8-dihydrofuro[3,2-e][1,3]benzothiazol-2-yl)octahydro-2H-imidazo[4,5-c]pyridin-2-one (Example 110);
[(3aR,7aS)-3-(7,8-dihydrofuro[3,2-e][1,3]benzothiazol-2-yl)-2-oxooctahydro-5H-imidazo[4,5-c]pyridin-5-yl]acetonitrile (Example 112);
(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Example 114);
(3aR,7aR)-1-(7,8-dihydrofuro[3,2-e][1,3]benzothiazol-2-yl)hexahydropyrano[3,4-d]imidazol-2(3H)-one (Example 122);
(3aR,7aR)-1-(7,8-dihydrofuro[3,2-e][1,3]benzothiazol-2-yl)-6,6-dimethylhexahydropyrano[3,4-d]imidazol-2(3H)-one (Example 124);
(3aR,6R,7aR)-1-(7,8-dihydrofuro[3,2-e][1,3]benzothiazol-2-yl)-2-oxooctahydropyrano[3,4-d]imidazole-6-carbonitrile (Example 127);
(3aR,6R,7aR)-1-(2H-[1,3]dioxolo[4,5-e][1,3]benzothiazol-7-yl)-2-oxooctahydropyrano[3,4-d]imidazole-6-carbonitrile (Example 131);
(3aR,6R,7aR)-1-(7,8-dihydro[1,4]dioxino[2,3-e][1,3]benzothiazol-2-yl)-2-oxooctahydropyrano[3,4-d]imidazole-6-carbonitrile (Example 133);
(3aR,6S,7aR)-1-(2H-[1,3]dioxolo[4,5-e][1,3]benzothiazol-7-yl)-6-ethylhexahydropyrano[3,4-d]imidazol-2(3H)-one (Example 138);
(3aR,6S,7aR)-1-(2H-[1,3]dioxolo[4,5-e][1,3]benzothiazol-7-yl]-6-methylhexahydropyrano[3,4-d]imidazol-2(3H)-one (Example 139);
(3aR,6R,7aR)-1-(2H-[1,3]dioxolo[4,5-e][1,3]benzothiazol-7-yl]-6-(fluoromethyl)hexahydropyrano[3,4-d]imidazol-2(3H)-one (Example 140);
(3aR,6R,7aR)-1-(7,8-dihydrofuro[3,2-e][1,3]benzothiazol-2-yl)-6-ethynylhexahydropyrano[3,4-d]imidazol-2(3H)-one (Example 149); and
(3aR,7aR)-1-(7,8-dihydrofuro[3,2-e][1,3]benzothiazol-2-yl)-6-fluorohexahydropyrano[3,4-d]imidazol-2(3H)-one (Example 151).

Hereinafter, the method for producing the compound represented by formula (1) in the present invention will be described with reference to examples, but the present invention is not limited thereto.

The compound of the present invention is synthesized by a production method shown below and a method combining a known compound and a known synthesis method.

Each of the compounds in a reaction scheme also includes a salt thereof, and examples of the salt include the same as a salt of compound (1). These reactions are merely examples, and the compound of the present invention can also be appropriately produced by other methods based on the knowledge of a person who is familiar organic synthesis.

In each of the production methods described below, even if the use of a protective group is not specifically specified, when a functional group that requires protection is present, the target product may be obtained by, if necessary, protecting the functional group and deprotecting the same after completion of the reaction or after carrying out a series of reactions.

As the protective group, a usual protective group described in, for example, reference (T.W. Greene and P.G.M. Wuts, "Protective Groups in Organic Synthesis," 3rd Ed., John Wiley and Sons, inc., New York (1999)) can be used, and more specifically, examples of a protective group for an amino group include tert-butoxycarbonyl, benzyloxycarbonyl, dimethylformamide, p-toluenesulfonyl, o-nitrobenzenesulfonyl, and tetrahydropyranyl, examples of a protective group for a hydroxy group include trialkylsilyl, acetyl, benzyl, tetrahydropyranyl, and methoxymethyl, examples of a protective group for an aldehyde group include dialkyl acetal, and cyclic alkyl acetal, and examples of a protective group for a carboxyl group include a tert-butyl ester, an ortho ester, and an acid amide.

The compounds wherein these functional groups are protected are also encompassed by the compound of the above formula (1).

The introduction and elimination of a protective group can be carried out by a method commonly used in organic synthetic chemistry (for example, a method described in T.W. Greene and P.G.M. Wuts, "Protective Groups in Organic

Synthesis," 3rd Ed., John Wiley and Sons, inc., New York (1999)) or a method similar thereto.

Production method 1

The compound represented by formula (1-9) is produced, for example, by the method shown below.

[Formula 7]

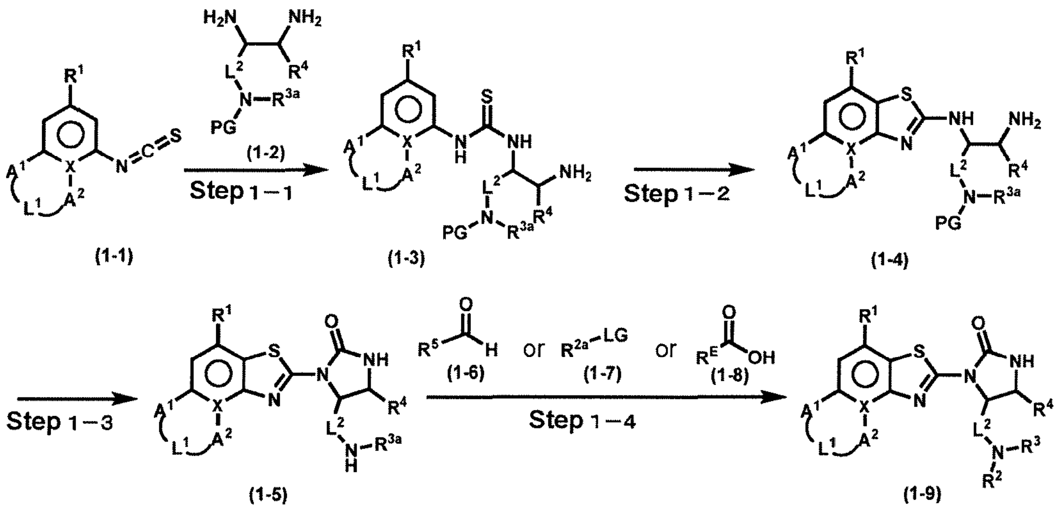


wherein $R^1$, $R^2$, $R^3$, $R^4$, $A^1$, $A^2$, $L^1$, $L^2$, $R^E$, and X are defined as described in item 1 above; $R^{2a}$ and $R^{3a}$ each represent optionally substituted $C_{1-6}$ alkyl; $R^5$ represents optionally substituted $C_{1-5}$ alkyl; LG represents a leaving group (for example, an iodine atom, a bromine atom, a chlorine atom, or -O-substituted sulfonyl group (for example, a methanesulfonyl group or a p-toluenesulfonyl group)); and PG represents a protective group (for example, a tert-butoxycarbonyl group, a benzyloxycarbonyl group, or a benzyl group).

Step 1-1: Production step of compound (1-3)

Compound (1-3) can be obtained by reacting compound (1-1) with compound (1-2) by a method similar to a known synthesis method (for example, Chemical & Pharmaceutical Bulletin, 1406, (2007), or Advanced Synthesis & Catalysis, 1643, (2005)). As compound (1-1), a compound produced by a known synthesis method (for example, Bioorganic & Medicinal Chemistry Letters, 28, (2007), or J. Org. Chem. 2613, (1986)) or a synthesis method similar thereto can be used. As compound (1-2), a commercially available product or a compound produced by a known synthesis method (for example, Tetrahedron Letters, 946, (2015), or Synlett, 426, (1999)) or a synthesis method similar thereto can be used.

Step 1-2: Production step of compound (1-4)

Compound (1-4) is produced by cyclizing compound (1-3) by a method similar to a known synthesis method (for example, Journal of Organic Chemistry, 8693, (2003) or WO2013043001).

Step 1-3: Production step of compound (1-5)

Compound (1-5) is produced by cyclizing compound (1-4) by a method similar to a known synthesis method (for example, Organic Letters, 5136, (2015), or Bioorganic & Medicinal Chemistry, 822, (2008)).

Step 1-4: Production step of compound (1-9)

Compound (1-9) can be obtained by reacting compound (1-5) with compound (1-6) in an inert solvent in the presence of a borohydride compound and, if necessary, an acid. As compound (1-6), a commercially available product or a compound produced by a known synthesis method (for example, US200619965, or Journal of Medicinal Chemistry, 3680, (2003)) or a synthesis method similar thereto can be used.

Specific examples of the inert solvent include an ether-based solvent such as tetrahydrofuran, tetrahydropyran,

1,4-dioxane, or 1,2-dimethoxyethane; a halogenated hydrocarbon such as chloroform, dichloromethane, or 1,2-dichloroethane; a protic polar solvent such as methanol, ethanol, 1-propanol, 2-propanol, or water; and mixed solvents thereof. The inert solvent is preferably tetrahydrofuran, dichloromethane, chloroform, or methanol.

Specific examples of the acid include a carboxylic acid such as formic acid, propionic acid, acetic acid, or trifluoroacetic acid; and a mineral acid such as hydrochloric acid.

Specific examples of the borohydride compound include sodium triacetoxyborohydride, sodium cyanoborohydride, and sodium borohydride. The borohydride compound is preferably sodium triacetoxyborohydride or sodium cyanoborohydride.

The reaction temperature is not particularly limited, and is usually selected from the range from 0°C to the boiling point of the solvent used. The reaction temperature is preferably 0°C to 20°C. The reaction time is usually 30 minutes to 72 hours.

Compound (1-9) is also produced by using compound (1-7) and a base and reacting the same with compound (1-5) in an inert solvent, in the presence of a halogenating agent, if necessary. As compound (1-7), a commercially available product or a compound produced by a known synthesis method (for example, US2013/1503254 or EP1679308, (2006)) or a synthesis method similar thereto can be used.

Specific examples of the inert solvent include an ether-based solvent such as tetrahydrofuran, tetrahydropyran, 1,4-dioxane, or 1,2-dimethoxyethane; an aromatic hydrocarbon such as toluene, xylene, or pyridine; an aprotic polar solvent such as acetonitrile, propionitrile, N,N-dimethylformamide, N,N-dimethylacetamide, N-methyl-2-pyrrolidinone, or dimethyl sulfoxide; a halogenated hydrocarbon such as chloroform, dichloromethane, or 1,2-dichloroethane, and mixed solvents thereof. The inert solvent is preferably acetonitrile, N,N-dimethylacetamide, or dimethyl sulfoxide.

Specific examples of the base include an organic base such as triethylamine, diisopropylethylamine, or pyridine; and an inorganic base such as potassium carbonate, sodium carbonate, cesium carbonate, potassium hydroxide, or sodium hydroxide. The base is preferably diisopropylethylamine, potassium carbonate, or cesium carbonate.

Specific examples of the halogenating agent include an inorganic halide such as potassium iodide or sodium iodide; and an organic halide such as tetrabutylammonium iodide.

The reaction temperature is not particularly limited, and is usually selected from the range from 0°C to the boiling point of the solvent used. The reaction temperature is preferably 0°C to 100°C. The reaction time is usually 30 minutes to 72 hours.

Compound (1-9) is also produced by using a carboxylic acid (1-8) and a condensing agent or an acid anhydride corresponding to the carboxylic acid (1-8) and reacting the same with compound (1-5) in an inert solvent, in the presence of a base and an additive, if necessary. As the carboxylic acid (1-8) or the acid anhydride corresponding thereto, a commercially available product or a compound produced by a known synthesis method (for example, Journal of Organic Chemistry 2564, (1999), or Organic Letters 4739, (2004)) or a synthesis method similar thereto can be used.

Specific examples of the inert solvent include an ether-based solvent such as tetrahydrofuran, tetrahydropyran, 1,4-dioxane, or 1,2-dimethoxyethane; an aromatic hydrocarbon such as toluene, xylene, or pyridine; an aprotic polar solvent such as acetonitrile, propionitrile, N,N-dimethylformamide, N,N-dimethylacetamide, N-methyl-2-pyrrolidinone, or dimethyl sulfoxide; a halogenated hydrocarbon such as chloroform, dichloromethane, or 1,2-dichloroethane, and mixed solvents thereof. The inert solvent is preferably N,N-dimethylacetamide, N-methyl-2-pyrrolidinone, chloroform, or dichloromethane.

Specific examples of the condensing agent include dicyclohexylcarbodiimide, diisopropylcarbodiimide, 1-ethyl-3-(3-dimethylaminopropyl)-carbodiimide, benzotriazol-1-yl-tris(dimethylamino)phosphonium hexafluorophosphate, diphenylphosphonyldiamide, N,N-carbonyldimidazole, O-(benzotriazol-1-yl)-N,N,N',N'-tetramethyluronium hexafluorophosphate, and O-(7-azabenzotriazol-1-yl)-N,N,N',N'-tetramethyluronium hexafluorophosphate.

Specific examples of the additive include N-hydroxysuccinimide, 1-hydroxybenzotriazole, and 3-hydroxy-4-oxo-3,4-dihydro-1,2,3-benzotriazine, and the reaction can be carried out by adding such an additive.

Specific examples of the base include an organic base such as triethylamine, diisopropylethylamine, or pyridine.

The reaction temperature is not particularly limited, and is usually selected from the range from about -20°C to the boiling point of the solvent used. The reaction temperature is preferably 0°C to 20°C. The reaction time is usually 10 minutes to 48 hours.

Production method 2

The compound represented by formula (2-7) is produced, for example, by the method shown below.

[Formula 8]

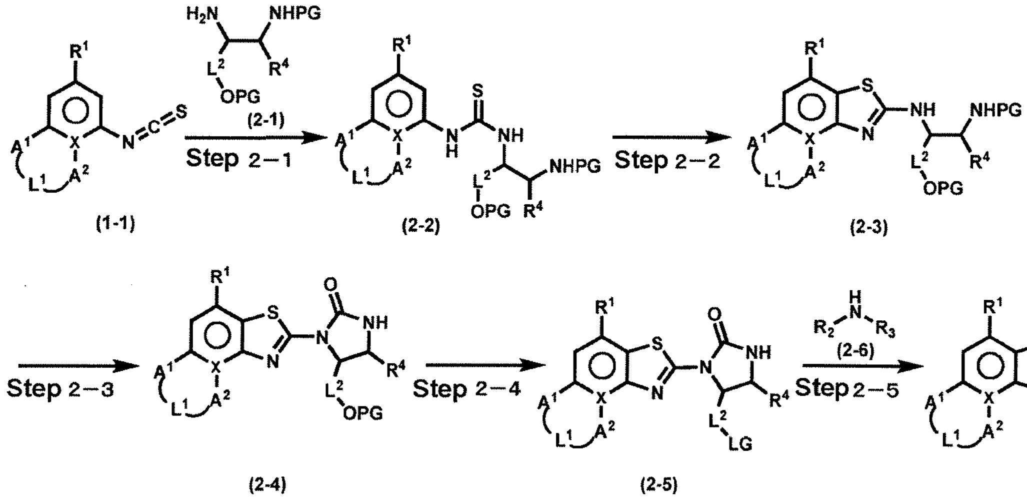


(1-1) (2-2) (2-3) (2-4) (2-5) (2-7)

wherein $R^1$, $R^2$, $R^3$, $R^4$, $A^1$, $A^2$, $L^1$, $L^2$, and X are defined as described in item 1 above; LG represents a leaving group (for example, an iodine atom, a bromine atom, a chlorine atom, or an -O-substituted sulfonyl group (for example, a methanesulfonyl group or a p-toluenesulfonyl group); and PG represents a protective group (for example, a tert-butoxycarbonyl group, a benzyloxycarbonyl group, or a benzyl group).

Step 2-1: Production step of compound (2-2)

Compound (2-2) is produced according to the method described in step 1-1 by using compound (1-1) and compound (2-1). As compound (2-1), a compound produced by a known synthesis method (for example, Chemical Communications 7693, (2015) or WO2015061572) or a synthesis method similar thereto can be used.

Step 2-2: Production step of compound (2-3)

Compound (2-3) is produced according to the method described in step 1-2 by using compound (2-2).

Step 2-3: Production step of compound (2-4)

Compound (2-4) is produced by using compound (2-3) and according to the method described in step 1-3 after deprotection of the protective group.

Step 2-4: Production step of compound (2-5)

When LG is a substituted sulfonyl group, compound (2-5) can be obtained by deprotecting the protective group and then reacting compound (2-4) with a sulfonyl chloride in an inert solvent in the presence of a base. When LG is a halogen, compound (2-5) can be obtained by deprotecting the protective group and then reacting compound (2-4) with a halogenating agent in an inert solvent.

Specific examples of the inert solvent include an ether-based solvent such as tetrahydrofuran, tetrahydropyran, 1,4-dioxane, or 1,2-dimethoxyethane; an aromatic hydrocarbon such as toluene or xylene; a halogenated hydrocarbon such as chloroform, dichloromethane, or 1,2-dichloroethane; an aprotic polar solvent such as acetonitrile, N,N-dimethylformamide, N,N-dimethylacetamide, or N-methyl-2-pyrrolidinone; and mixed solvents thereof. The inert solvent is preferably tetrahydrofuran, chloroform, or dichloromethane.

Specific examples of the base include an organic base such as triethylamine, diisopropylethylamine, pyridine, 2,4,6-trimethylpyridine, or 4-dimethylaminopyridine. The base is preferably triethylamine or diisopropylamine.

Specific examples of the sulfonyl chloride include methanesulfonyl chloride, monochloromethanesulfonyl chloride, benzenesulfonyl chloride, p-toluenesulfonyl chloride, o-nitrobenzenesulfonyl chloride, and p-nitrobenzenesulfonyl chloride. The sulfonyl chloride is preferably methanesulfonyl chloride.

Specific examples of the halogenating agent include thionyl chloride, oxalyl dichloride, and phosphorus tribromide. The halogenating agent is preferably thionyl chloride or phosphorus tribromide.

The reaction temperature is not particularly limited, and is usually selected from the range from -20°C to the

boiling point of the solvent used. The reaction temperature is preferably 0°C to 60°C. The reaction time is usually 5 minutes to 24 hours.

Step 2-5: Production step of compound (2-7)

Compound (2-7) can be obtained by reacting compound (2-5) with (2-6) in an inert solvent in the presence of a base and, if necessary, a halide. As compound (2-6), a commercially available product or a compound produced by a known synthesis method (for example, WO20073965 or US2010216812) or a synthesis method similar thereto can be used.

Specific examples of the inert solvent include an ether-based solvent such as tetrahydrofuran, tetrahydropyran, 1,4-dioxane, or 1,2-dimethoxyethane; a halogenated hydrocarbon such as chloroform, dichloromethane, or 1,2-dichloroethane; an aprotic polar solvent such as acetonitrile, N,N-dimethylformamide, N,N-dimethylacetamide, N-methyl-2-pyrrolidinone, or dimethyl sulfoxide; and mixed solvents thereof. The inert solvent is preferably acetonitrile, tetrahydrofuran, dichloromethane, or N,N-dimethylformamide.

Specific examples of the base include an organic base such as triethylamine, diisopropylethylamine, pyridine, 2,4,6-trimethylpyridine, or 4-dimethylaminopyridine; and an inorganic base such as potassium carbonate, sodium carbonate, or cesium carbonate.

Specific examples of the halide include an organic halide such as tetrabutylammonium iodide or tetrabutylammonium bromide; and an inorganic halide such as potassium iodide, potassium bromide, sodium iodide, or sodium bromide.

The reaction temperature is not particularly limited, and is usually selected from the range from -20°C to the boiling point of the solvent used or compound (2-6). The reaction temperature is preferably 0°C to 120°C. The reaction time is usually 30 minutes to 72 hours.

Production method 3

The compound represented by formula (1-4) is also produced, for example, by the method shown below.

[Formula 9]

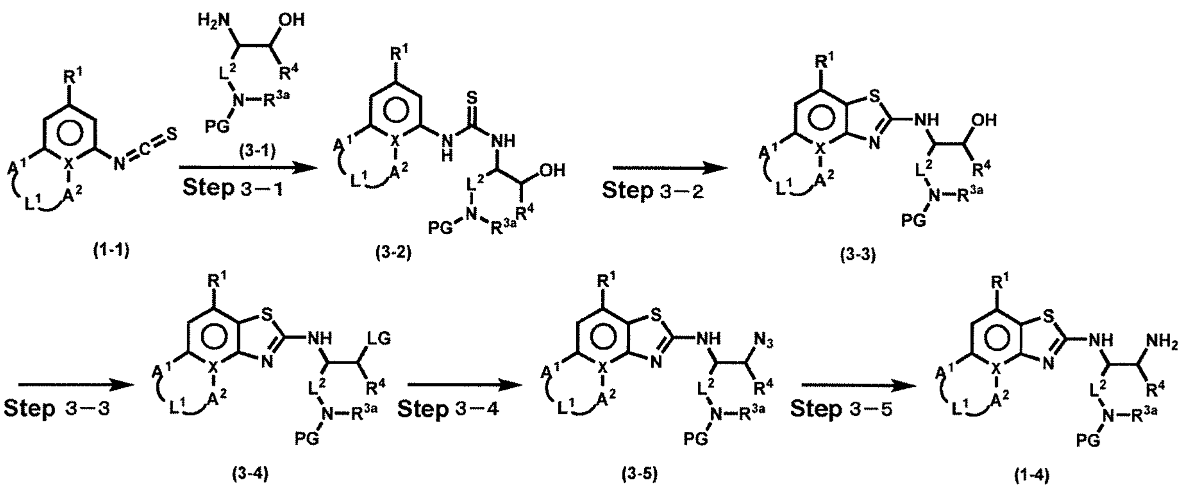


(1-1) (3-2) (3-3) (3-4) (3-5) (1-4)

wherein $R^1$, $R^2$, $R^3$, $R^4$, $A^1$, $A^2$, $L^1$, $L^2$, and X are defined as described in item 1 above; $R^{3a}$ each represent optionally substituted $C_{1-6}$ alkyl;; LG represents a leaving group (for example, an iodine atom, a bromine atom, a chlorine atom, or a -O-substituted sulfonyl group (for example, a methanesulfonyl group or a p-toluenesulfonyl group)); and PG represents a protective group (for example, a tert-butoxycarbonyl group, a benzyloxycarbonyl group, or a benzyl group).

Step 3-1: Production step of compound (3-2)

Compound (3-2) is produced according to the method described in step 1-1 by using compound (1-1) and compound (3-1). As compound (3-1), a commercially available product or a compound produced by a known synthesis method (for example, WO2008136457 or WO2014015905) or a synthesis method similar thereto can be used.

Step 3-2: Production step of compound (3-3)

Compound (3-3) is produced according to the method described in step 1-2 by using compound (3-2).

Step 3-3: Production step of compound (3-4)

Compound (3-4) is produced according to the method described in step 2-4 by using compound (3-3).

Step 3-4: Production step of compound (3-5)

Compound (3-5) is produced by synthesizing the same by a method similar to a known synthesis method (for example, Journal of Medicinal Chemistry 3918, (1995) or WO2015177326) by using compound (3-4).

Step 3-5: Production step of compound (1-4)

Compound (1-4) is also produced by synthesizing the same by a method similar to a known synthesis method (for example, Bioorganic & Medicinal Chemistry Letters 5227 (2007) or WO2016044641) by using compound (3-5).

Production method 4

The compound represented by formula (4-5) is produced, for example, by the method shown below.

[Formula 10]

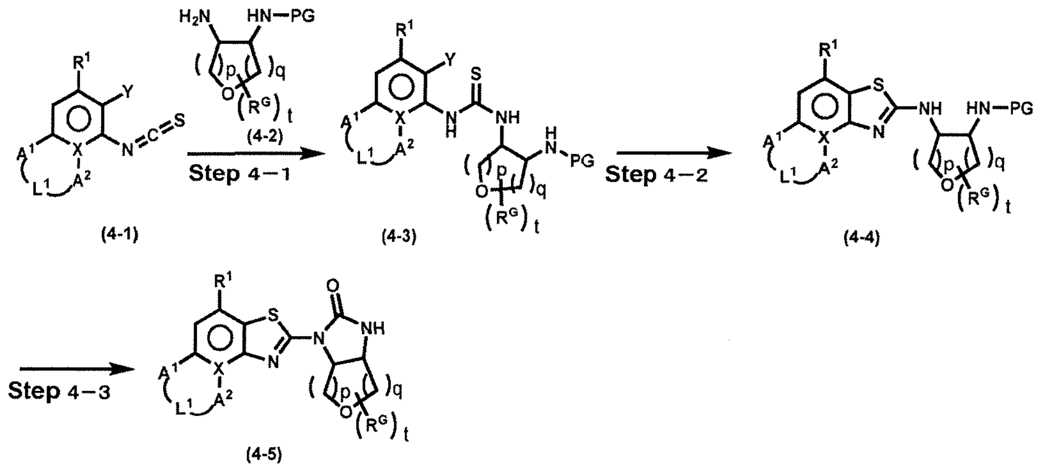


(4-1) (4-2) (4-3) (4-4) (4-5)

wherein $R^1$, $A^1$, $A^2$, $L^1$, and X are defined as described in item 1 above, and $R^G$, p, q, and t are defined as described in item 16 above; Y Represents a halogen atom (for example, an iodine atom, a bromine atom, or a chlorine atom); and PG represents a protective group (for example, a tert-butoxycarbonyl group or a benzyloxycarbonyl group).

Step 4-1: Production step of compound (4-3)

Compound (4-3) is produced according to the method described in step 1-1 by using compound (4-1) and compound (4-2). As compound (4-1), a compound produced by a known synthesis method (for example, Bioorganic & Medicinal Chemistry Letters, 28, (2007) or Journal of Organic Chemistry 2613, (1986)) or a synthesis method similar thereto can be used. As compound (4-2), a commercially available product or a compound produced by a known synthesis method (for example, WO2012061418 or WO2010097248) or a synthesis method similar thereto can be used.

Step 4-2: Production step of compound (4-4)

Compound (4-4) is produced by using compound (4-3) and cyclizing the same by a method similar to a known synthesis method (for example, Chemical Communications 446, (2004) or Journal of Organic Chemistry 8719, (2009)).

Step 4-3: Production step of compound (4-5)

Compound (4-5) is produced by using compound (4-4) and according to the method described in step 1-3 after deprotection of the protective group.

Production method 5

The compound represented by formula (5-4) is produced, for example, by the method shown below.

[Formula 11]

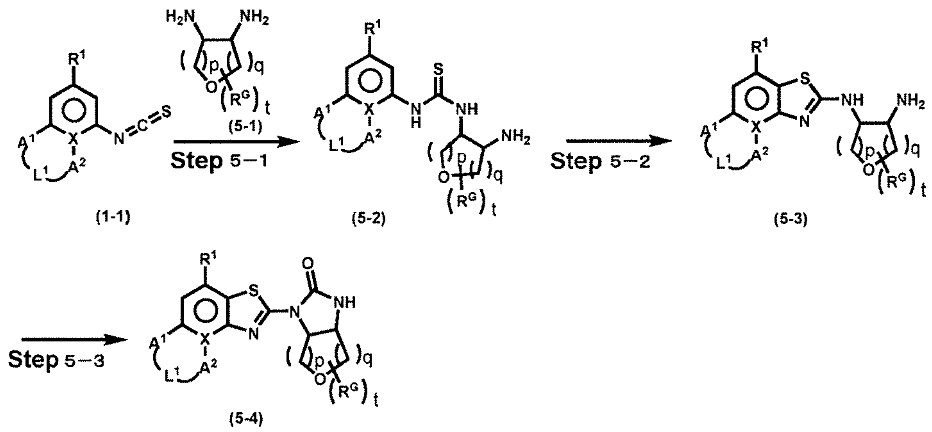


wherein $R^1$, $A^1$, $A^2$, $L^1$, and X are defined as described in item 1 above, and $R^G$, p, q, and t are defined as described in item 16 above.

Step 5-1: Production step of compound (5-2)

Compound (5-2) is produced according to the method described in step 1-1 by using compound (1-1) and compound (5-1). As compound (5-1), a commercially available product or a compound produced by a known synthesis method (for example, Journal of the American Chemical Society, 12521, (1996) or Green Chemistry 451, (2005)) or a synthesis method similar thereto can be used.

Step 5-2: Production step of compound (5-3)

Compound (5-3) is produced according to the method described in step 1-2 by using compound (5-2).

Step 5-3: Production step of compound (5-4)

Compound (5-4) is produced according to the method described in step 1-3 by using compound (5-3).

By carrying out the above production methods in an appropriate combination, the compound of the present invention having a desired functional group at a desired position can be obtained. Isolation and purification of intermediates and products in the above production methods can be carried out by appropriately combining methods used in ordinary organic synthesis, such as filtration, extraction, washing, drying, concentration, crystallization, and various chromatography. In addition, such an intermediate can also be subjected to the next reaction without any particular purification.

Examples of the "pharmaceutically acceptable salt" include an acid addition salt and a base addition salt. Examples of the acid addition salt include an inorganic acid salt such as a hydrochloride, a hydrobromide, a sulfate, a hydroiodide, a nitrate, or a phosphate, or an organic acid salt such as a citrate, an oxalate, a phthalate, a fumarate, a maleate, a succinate, a malate, an acetate, a formate, a propionate, a benzoate, a trifluoroacetate, a methanesulfonate, a benzenesulfonate, a para-toluenesulfonate, or a camphorsulfonate. In addition, examples of the base addition salt include an inorganic base salt such as a sodium salt, a potassium salt, a calcium salt, a magnesium salt, a barium salt, or an aluminum salt, or an organic base salt such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, or N,N-dibenzylethylamine. Further, examples of the "pharmaceutically acceptable salt" also include

an amino acid salt with a basic amino acid or an acidic amino acid, such as arginine, lysine, ornithine, aspartic acid, or glutamic acid.

Suitable salts of a raw material compound and an intermediate and a salt acceptable as a raw material for a pharmaceutical are conventional nontoxic salts, and examples thereof include an acid addition salt such as an organic acid salt (for example, an acetate, a trifluoroacetate, a maleate, a fumarate, a citrate, a tartrate, a methanesulfonate, a benzenesulfonate, a formate, or a p-toluenesulfonate) and an inorganic acid salt (for example, a hydrochloride, a hydrobromide, a hydroiodide, a sulfate, a nitrate, or a phosphate), a salt with an amino acid (for example, arginine, aspartic acid, or glutamic acid), a metal salt such as an alkali metal salt (for example, a sodium salt or a potassium salt) and an alkaline earth metal salt (for example, a calcium salt or a magnesium salt), an ammonium salt, or an organic base salt (for example, a trimethylamine salt, a triethylamine salt, a pyridine salt, a picoline salt, a dicyclohexylamine salt, or an N,N'-dibenzylethylenediamine salt), and such a nontoxic salt can be appropriately selected by those skilled in the art.

Some of the raw material compounds or intermediates in the above production methods can exist in the form of a salt such as a hydrochloride depending on the reaction conditions and the like, and can be used as they are or in free form. When a raw material compound or an intermediate is obtained in the form of a salt and the raw material compound or intermediate is to be used or obtained in free form, this salt can be converted to a free form by dissolving or suspending the salt in a suitable solvent and neutralizing the same with, for example, a base such as a sodium hydrogen carbonate aqueous solution.

For some compounds (1) or pharmaceutically acceptable salts thereof, an isomer such as a tautomer such as a ketoenol form, a regioisomer, a geometric isomer, or an optical isomer can exist, and all possible isomers, including these, and mixtures of the isomers at any ratio are also encompassed by the present invention.

In addition, the optical isomer can be separated by carrying out a known separation step such as a method using an optically active column or a fractional crystallization method in an appropriate step of the above production methods. In addition, an optically active substance can also be used as a starting material.

As used herein, a compound with stereochemistry (S, R) notation in its chemical structural formula means an optically active form, and when stereochemistry is not particularly indicated, the compound means a racemic form. Regarding the cyclic urea moiety of the compound of the present invention, a cis form or a trans form exists, and in particular, when stereochemistry (S, R) is not indicated, the moiety means a racemic form.

When a salt of compound (1) is to be obtained, if the salt of compound (1) can be obtained, the salt may be purified as it is, and if compound (1) is obtained in free form, the salt thereof may be formed by dissolving or suspending compound (1) in a suitable solvent and adding an acid or a base. In addition, compound (1) or a pharmaceutically acceptable salt thereof may exist in the form of a solvate with water or any of various solvents, and such a solvate is also encompassed by the present invention.

As used herein, the "hydrogen atom" includes $^1H$ and $^2H$ (D), and a deuterium conversion form obtained by converting any one or two or more $^1H$ in the compound represented by formula (1) into $^2H$ (D) is also encompassed by the compound represented by formula (1).

The compound of the present invention can be administered, directly or by being formulated into an appropriate dosage form, by oral administration or parenteral administration. Examples of the dosage form include, but are not limited to, a tablet, a capsule, a powder, a granule, a liquid, a suspension, an injection, a patch, and a cataplasm. The formulation is produced by a known method by using a pharmaceutically acceptable additive. As an additive, an excipient, a disintegrant, a binding agent, a plasticizer, a lubricant, a coating agent, a solubilizing agent, a dissolution aid, a thickening agent, a dispersing agent, a stabilizing agent, a sweetening agent, a flavoring agent, or the like can be used depending on the purpose. Specifically, examples thereof include lactose, mannitol, crystalline cellulose, low substituted hydroxypropylcellulose, corn starch, partially pregelatinized starch, carmellose calcium, croscarmellose sodium, hydroxypropylcellulose, hydroxypropylmethylcellulose, polyvinyl alcohol, magnesium stearate, sodium stearyl fumarate, polyethylene glycol, propylene glycol, titanium oxide, and talc.

The administration route of the compound of the present invention may be oral administration, parenteral administration, or rectal administration, and the daily dosage thereof varies depending on the type of the compound, the administration method, the symptom/age of the patient, and the like. For example, in the case of oral administration, usually about 0.01 to 1000 mg, further preferably about 0.1 to 500 mg, per kg of human or mammal body weight can be administered in one to several divided doses. In the case of parenteral administration such as intravenous injection, usually, for example, about 0.01 to 300 mg, further preferably about 1 to 100 mg, per kg of human or mammal body weight can be administered.

In addition, compound (1) of the present invention or a pharmaceutically acceptable salt thereof can be used, as a DYRK inhibitor, as a reagent for pathological imagery related to the above diseases or a reagent for a basic experiment or for research.

## Examples

Hereinafter, the present invention will be described more specifically with reference to Examples, Reference Examples, and Test Examples, but the present invention is not limited thereto at all. The compound names shown in the following Examples and Reference Examples do not necessarily follow the IUPAC nomenclature.

The following abbreviations may be used herein.

$(Boc)_2O$: di-tert-butyl dicarbonate
Cbz: benzyloxycarbonyl
DIAD: diisopropyl azodicarboxylate
Boc: tert-butoxycarbonyl
Bn: benzyl
TBDMS: tert-butyldimethylsilyl
Ac: acetyl
Ms: methanesulfonyl
DMSO: dimethyl sulfoxide
Ts: p-toluenesulfonyl
Tf: trifluoromethanesulfonyl
Rt: retention time

Physical property data of each compound of the Examples and the Reference Examples were measured under the following conditions.
Nuclear magnetic resonance spectrum ($^1$H-NMR):
Device used: JEOL JNM-AL400; Brucker AVANCE 400 Spectrometer

Liquid chromatography/mass spectrometry (LC/MS):

(1) Method A and Method B

Detection device: ACQUITY (R) SQ deteceter (Waters Corporation)
HPLC: ACQUITY UPLC (R)
SYSTEM Column: Waters ACQUITY UPLC (R) BEH C18 (1.7 um, 2.1 mm × 30 mm)
Flow rate: 0.8 mL/min; Detection UV: 220 nm and 254 nm;
Temperature: 40°C

[Table 1]

| Method | Solvent | Gradient condition |
|---|---|---|
| **Method A** | **Solvent A: 0.05% formic acid/water Solvent B : $CH_3CN$** | **0.0 - 1.3 min Linear gradient from B 2% to 96%** |
| **Method B** | **Solvent A : 0.05%formic acid/water Solvent B : $CH_3CN$** | **0.0 - 1.3 min Linear gradient from B 10% to 95%** |

(2) Method C

Detection device: ACQUITY (R) QDa deteceter (Waters Corporation)
HPLC: ACQUITY UPLC (R)
SYSTEM Column: Waters ACQUITY UPLC (R) BEH C18 (1.7 um, 2.1 mm × 30 mm)

Flow rate: 0.6 mL/min; Detection UV: 20 to 400 nm;
Temperature: 40°C

[Table 2]

| Method | Solvent | Gradient condition |
|---|---|---|
| **Method C** | **Solvent A :** **0.1%formic acid/water** **Solvent B :** **0.1%formic acid/$CH_3CN$** | **0.5 - 2.5 min Linear gradient from B 3% to 95%** |

(3) Method D

Detection device: ACQUITY (R) SQ deteceter 2 (Waters Corporation)
HPLC: ACQUITY UPLC (R)
SYSTEM Column: Waters ACQUITY UPLC (R) BEH C18 (1.7 um, 2.1 mm × 50 mm)
Flow rate: 0.6 mL/min; Detection UV: 210 nm to 400 nm;
Temperature: 35°C

[Table 3]

| Method | Solvent | Gradient condition |
|---|---|---|
| **Method D** | **Solvent A:** **0.07%formic acid/water** **Solvent B:** **0.07%formic acid/$CH_3CN$** | **0.3 - 2.7 min Linear gradient from B 3% to 98%** |

(4) Method E

Detection device: LCMS-2010A (Shimadzu Corporation)
HPLC: Prominence (R)
SYSTEM Column: Cadenza CD-C18 (3.0 um, 2.0 mm × 50 mm)
Flow rate: 0.5 mL/min; Detection UV: 215 and 254 nm;
Temperature: 40°C

[Table 4]

| Method | Solvent | Gradient condition |
|---|---|---|
| **Method E** | **Solvent A:** **0.1%formic acid/water** **Solvent B:** **0.1%formic acid/$CH_3CN$** | **0.5 - 2.5 min Linear gradient from B 5% to 95%** |

The compound names in the Reference Examples and the Examples were named by using ACD/Name (ACD/Labs 12.0, Advanced Chemistry Development Inc.).

Reference Example 1 and Reference Example 2

Reference Example 1: tert-butyl cis-3-amino-4-{[(2,3-dihydro-1-benzofuran-4-yl)carbamothioyl]amino}piperidine-1-carboxylate
Reference Example 2: tert-butyl cis-4-amino-3-{[(2,3-dihydro-1-benzofuran-4-yl)carbamothioyl]amino}piperidine-1-carboxylate

[Formula 12]

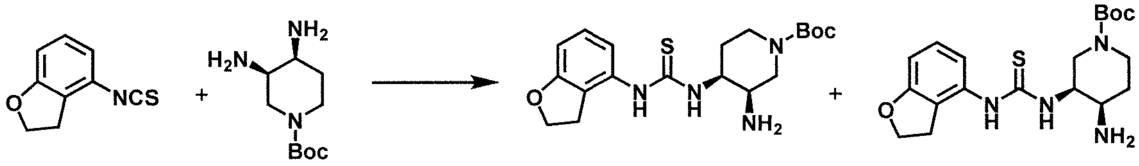

4-

Isothiocyanato-2,3-dihydrobenzofuran (886 mg) was added to a chloroform solution (20 mL) of cis-1-Boc-3,4-diaminopiperidine (1.08 g) and N,N-diisopropylethylamine (647 mg) under ice cooling, and the resulting mixture was stirred under ice cooling for 2 hours. The reaction mixture was directly purified by silica gel column chromatography (chloroform/methanol) to obtain the title compounds (Reference Example 1: 1.04 g, Reference Example 2: 680 mg).
Reference Example 1: LC-MS $[M+H]^+$/Rt (min): 393.2/0.562 (Method B)
Reference Example 2: LC-MS $[M+H]^+$/Rt (min): 393.2/0.559 (Method B)

Reference Examples 3 to 7

The compounds of Reference Examples 3 to 7 were obtained by using the corresponding raw material compounds according to the method described in Reference Example 1 and Reference Example 2.

[Table 5]

| Reference Example | Chemical structure | Physical property data |
|---|---|---|
| 3 | 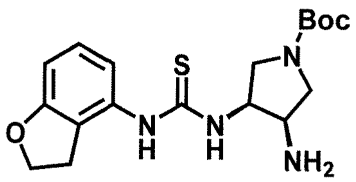 | **LC-MS $[M+H]^+$/Rt (min): 378.2/0.630 (Method A).** |
| 4 | 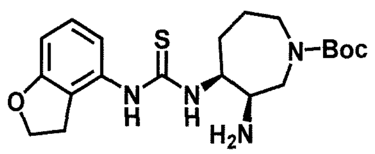 | **LC-MS $[M+H]^+$/Rt (min): 407.2/0.698 (Method B).** |
| 5 | 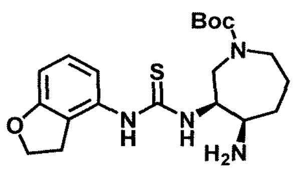 | **LC-MS $[M+H]^+$/Rt (min): 407.2/0.682 (Method B).** |
| 6 | 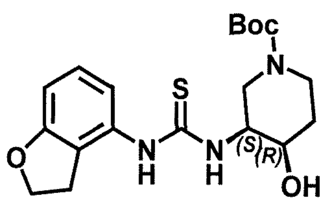 | **$^1$H-NMR ($CDCl_3$) $\delta$: 7.58 (1H, brs), 7.16 (1H, dd, $J$ = 7.9, 8.0 Hz), 6.73 (1H, d, $J$ = 8.0 Hz), 6.73 (1H, d, $J$ = 7.9 Hz), 6.45 (1H, brs), 4.69-4.56 (3H, m), 4.17-4.05 (2H, m), 3.60-3.46 (4H, m), 3.19 (2H, t, $J$ = 8. 5 Hz), 1.86-1.77 (1H, m), 1.53-1.46 (1H, m), 2.40 (9H, s).** |

(continued)

| Reference Example | Chemical structure | Physical property data |
|---|---|---|
| 7 | 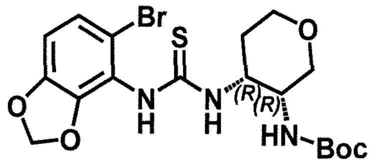 | **$^1$H-NMR ($CDCl_3$) $\delta$: 7.58 (1H, brs), 7.16 (1H, dd, $J$ = 7.9, 8.0 Hz), 6.73 (1H, d, $J$ = 8.0 Hz), 6.73 (1H, d, $J$ = 7.9 Hz), 6.45 (1H, brs), 4.69-4.56 (3H, m), 4.17-4.05 (2H, m), 3.60-3.46 (4H, m), 3.19 (2H, t, $J$ = 8. 5 Hz), 1.86-1.77 (1H, m), 1.53-1.46 (1H, m), 2.40 (9H, s).** |

Reference Example 8

tert-Butyl cis-4-amino-3-[(7,8-dihydrofuro[3,2-e] [1,3]benzothiazol-2-yl)amino]piperidine-1-carboxylate

[Formula 13]

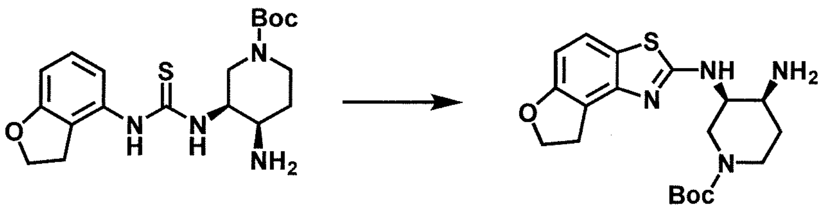

Sodium hydrogen carbonate (580 mg) and benzyltrimethylammonium tribromide (2.69 g) were added to a chloroform solution (70 mL) of the compound of Reference Example 2 (2.71 g), and the resulting mixture was stirred for 1 hour. Saturated aqueous sodium bicarbonate was added to the reaction mixture, and the resulting mixture was extracted with chloroform. The organic layer was dried over sodium sulfate, filtered, and then concentrated under reduced pressure to obtain the title compound (2.64 g).
LC-MS $[M+H]^+$/Rt (min): 391.2/0.600 (Method B)

Reference Examples 9 to 13

The compounds of Reference Examples 9 to 13 were obtained by using the corresponding raw material compounds according to the method described in Reference Example 8.

[Table 6]

| Reference Example | Chemical structure | Physical property data |
|---|---|---|
| 9 | 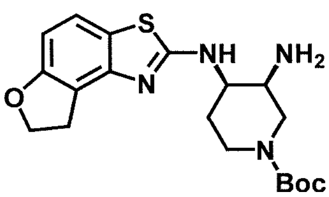 | **LC-MS $[M+H]^+$/Rt (min): 391. 2/0.568 (Method B).** |
| 10 | 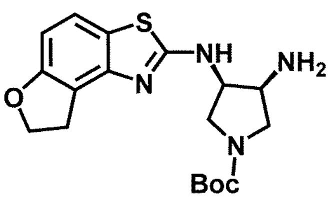 | **LC-MS $[M+H]^+$/Rt (min): 377. 2/0. 689 (Method A).** |

(continued)

| Reference Example | Chemical structure | Physical property data |
|---|---|---|
| **11** | 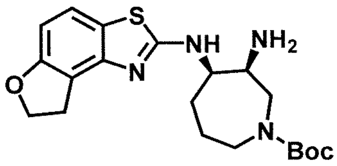 | **LC-MS $[M+H]^+$/Rt (min): 405.2/0.700 (Method B).** |
| **12** | 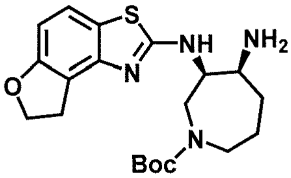 | **LC-MS $[M+H]^+$/Rt (min): 405.2/0.676 (Method B).** |
| **13** | 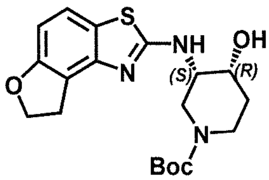 | **LC-MS $[M+H]^+$/Rt (min): 392. 5/1. 792 (Method D).** |

Reference Example 14

tert-Butyl cis-3-(7,8-dihydrobenzofuro[3,2-e][1,3]benzothiazol-2-yl)-2-oxooctahydro-5H-imidazo[4,5-c]pyridine-5-carboxylate

[Formula 14]

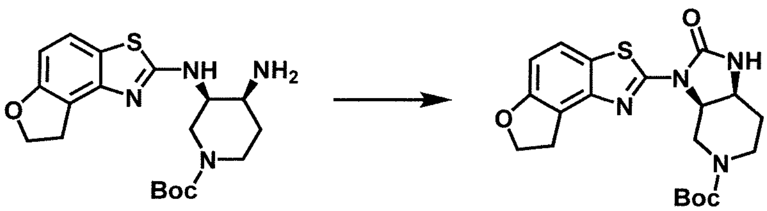

Triethylamine (0.162 mL) and di(N-succinimidyl) carbonate (99 mg) were added to a chloroform solution (4 mL) of the compound of Reference Example 8 (151 mg), and the resulting mixture was stirred for 0.5 hours. Saturated aqueous sodium bicarbonate was added to the reaction mixture, and the resulting mixture was extracted with chloroform. The organic layer was dried over sodium sulfate, filtered, and then concentrated under reduced pressure to obtain the title compound (114 mg).
LC-MS $[M+H]^+$/Rt (min): 417.2/0.803 (Method B)

Reference Examples 15 to 19

The compounds of Reference Examples 15 to 19 were obtained by using the corresponding raw material compounds according to the method described in Reference Example 14.

[Table 7]

| Reference Example | Chemical structure | Physical property data |
|---|---|---|
| 15 | 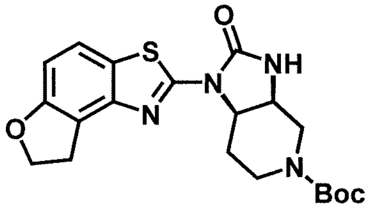 | LC-MS [M+H]$^{+}$/Rt (min): 417.3/0.898 (Method B) |
| 16 | 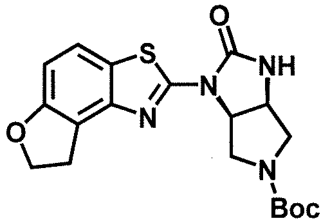 | LC-MS [M+H] $^{+}$/Rt (min): 403.2/0.907 (Method A). |
| 17 | 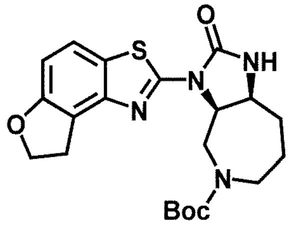 | LC-MS [M+H] $^{+}$/Rt (min): 431.3/0.906 (Method B) |
| 18 | 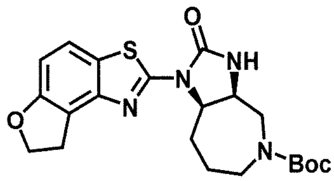 | LC-MS [M+H]$^{+}$/Rt (min): 431.3/1.000 (Method B) |
| 19 | 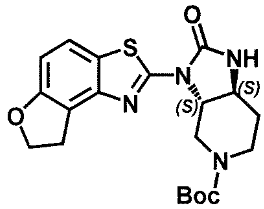 | LC-MS [M+H]$^{+}$/Rt (min): 417.4/2.180 (Method D) |

Reference Example 20

(2S,3R)-3-(Benzylamino)-4-{ [tert-butyl(dimethyl)silyl]oxy}butan-2-ol

[Formula 15]

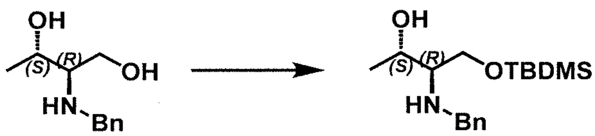

Triethylamine (2.4 mL), N,N-dimethylaminopyridine (1.3 mL), and TBDMS chloride were added to a chloroform solution (65 mL) of (2R,3S)-2-(benzylamino)butane-1,3-diol (4.46 g) under ice cooling, and the resulting mixture was stirred under ice cooling for 4 hours. Saturated aqueous sodium bicarbonate was added to the reaction mixture, and the resulting mixture was extracted with chloroform. The organic layer was dried over sodium sulfate, filtered, and then

concentrated under reduced pressure. The residue was purified by silica gel column chromatography (chloroform/methanol) to obtain the title compound (5.42 g).
LC-MS $[M+H]^+$/Rt (min): 310.3/0.872 (Method A)

Reference Example 21

The compound of Reference Example 21 was obtained by using the corresponding raw material compounds according to the method described in Reference Example 20.

[Table 8]

| Reference Example | Chemical structure | Physical property data |
|---|---|---|
| 21 | 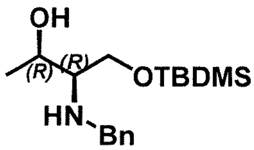 | LC-MS $[M+H]^+$/Rt (min): 310.2/0.820 (Method B) |

Reference Example 22

Benzyl benzyl[(2R,3S)-1-{[tert-butyl(dimethyl)silyl]oxy}-3-hydroxybutan-2-yl]carbamate

[Formula 16]

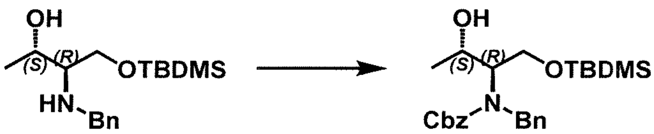

Sodium carbonate (5.57 g) and benzyl chloroformate (2.96 mL) were added to a mixed solution of the compound of Reference Example 20 (5.42 g) in tetrahydrofuran (45 mL) and water (30 mL), and the resulting mixture was stirred at room temperature for 14 hours. Water was added to the reaction mixture, and the resulting mixture was extracted with ethyl acetate. The organic layer was dried over sodium sulfate, filtered, and then concentrated under reduced pressure. The residue was purified by silica gel column chromatography (hexane/ethyl acetate) to obtain the title compound (6.61 g). LC-MS $[M+H]^+$/Rt (min): 444.4/1.389 (Method A)

Reference Example 23

The compound of Reference Example 23 was obtained by using the corresponding raw material compounds according to the method described in Reference Example 22.

[Table 9]

| Reference Example | Chemical structure | Physical property data |
|---|---|---|
| 23 | 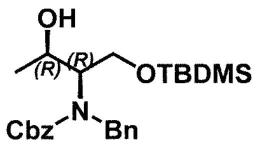 | LC-MS $[M+H]^+$/Rt (min): 444.3/1.367 (Method B). |

Reference Example 24

Benzyl benzyl[(2S,3R)-1-{[tert-butyl(dimethyl)silyl]oxy}-3-(1,3-dioxo-1,3-dihydro-2H-isoindol-2-yl)butan-2-yl]carbamate

[Formula 17]

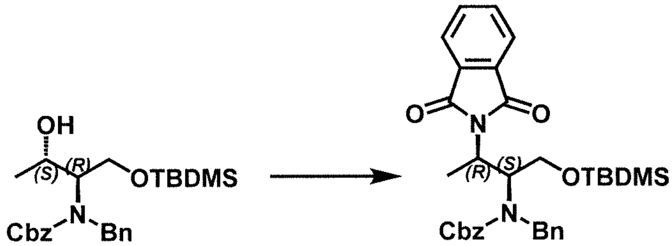

DIAD (3.81 mL) was added dropwise to a toluene solution (60 mL) of the compound of Reference Example 22 (6.61 g) and phthalimide (2.74 g) and triphenylphosphine (4.89 g) under ice cooling, and then the resulting mixture was stirred at room temperature for 7 hours. The reaction mixture was concentrated under reduced pressure, and the residue was purified by silica gel column chromatography (hexane/ethyl acetate) to obtain the title compound (7.03 g).
LC-MS $[M+H]^+$/Rt (min): 573.4/1.488 (Method A).

Reference Example 25

The compound of Reference Example 25 was obtained by using the corresponding raw material compounds according to the method described in Reference Example 24.

[Table 10]

| Reference Example | Chemical structure | Physical property data |
|---|---|---|
| 25 | 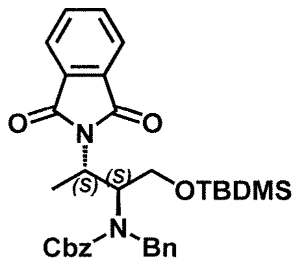 | LC-MS $[M+H]^+$/Rt (min): 573.3/1.465 (Method B). |

Reference Example 26

2-[(2R,3S)-3-amino-4-{[tert-butyl(dimethyl)silyl]oxy}butan-2-yl]-1H-isoindole-1,3(2H)-dione

[Formula 18]

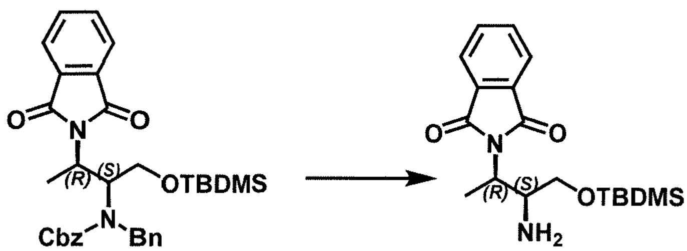

Palladium hydroxide carbon (1.63 g) was added to an ethanol solution (150 mL) of the compound of Reference Example 24 (7.03 g), and the resulting mixture was stirred at room temperature for 35 hours in a hydrogen atmosphere. The reaction mixture was filtered through cerite and then concentrated under reduced pressure. The residue was purified by amino silica gel column chromatography (hexane/ethyl acetate, chloroform/methanol) to obtain the title compound (816 mg).
LC-MS $[M+H]^+$/Rt (min): 349.3/0.906 (Method A).

Reference Example 27

The compound of Reference Example 27 was obtained by using the corresponding raw material compounds according to the method described in Reference Example 26.

[Table 11]

| Reference Example | Chemical structure | Physical property data |
|---|---|---|
| 27 | 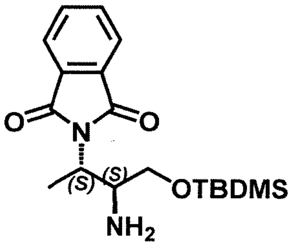 | LC-MS $[M+H]^+$/Rt (min): 349.1/0.797 (Method B). |

Reference Example 28

N-[(2S,3S)-1-{[tert-butyl(dimethyl)silyl]oxy}-3-(1,3-dioxo-1,3-dihydro-2H-isoindol-2-yl)butan-2-yl]-N'-(2,3-dihydro-1-benzofuran-7-yl)thiourea

[Formula 19]

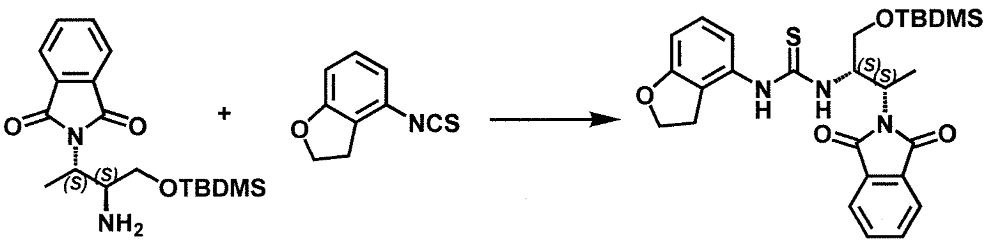

Diisopropylethylamine (0.65 mL) and 4-isothiocyanato-2,3-dihydrobenzofuran (226 mg) were added to a chloroform solution (5 mL) of the compound of Reference Example 27 (444 mg) under ice cooling, and the resulting mixture was stirred at normal temperature for 3 hours. 4-Isothiocyanato-2,3-dihydrobenzofuran (22.6 mg) was added, and the resulting mixture was stirred overnight. Saturated aqueous sodium bicarbonate and water were added to the reaction mixture, and the resulting mixture was extracted with chloroform. The organic layer was dried over sodium sulfate, filtered, and then concentrated under reduced pressure. The residue was purified by silica gel column chromatography (hexane/ethyl acetate) to obtain the title compound (660 mg).
LC-MS $[M+H]^+$/Rt (min): 526.3/1.251 (Method B).

Reference Example 29

The compound of Reference Example 29 was obtained by using the corresponding raw material compounds according to the method described in Reference Example 28.

[Table 12]

| Reference Example | Chemical structure | Physical property data |
|---|---|---|
| 29 | 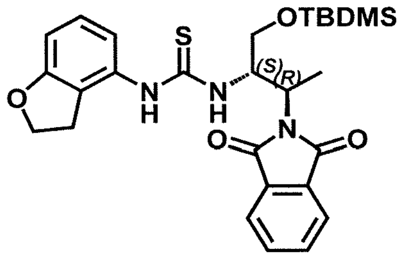 | LC-MS [M+H]+/Rt (min): 526.4/1.327 (Method A). |

Reference Example 30

2-{ (2S,3S)-4-{[tert-Butyl(dimethyl)silyl]oxy}-3-[(6,7-dihydrofuro[2,3-e][1,3]benzothiazol-2-yl)amino]butan-2-yl}-1H-isoindole-1,3(2H)-dione

[Formula 20]

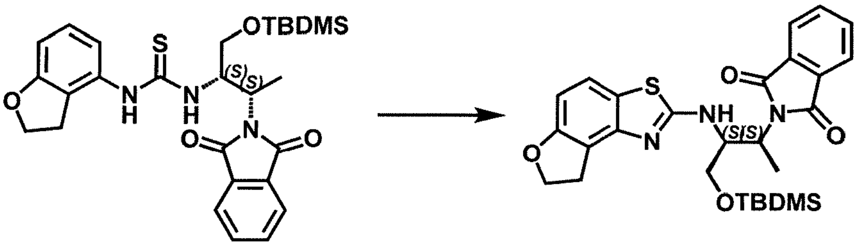

Sodium hydrogen carbonate (105 mg) was added to a chloroform solution (7 mL) of the compound of Reference Example 28 (660 mg) at normal temperature. Benzyltrimethylammonium tribromide (441 mg) was added under ice cooling, and the resulting mixture was stirred under ice cooling for 2 hours. Saturated aqueous sodium bicarbonate and water were added to the reaction mixture, and the resulting mixture was extracted with chloroform. The organic layer was dried over sodium sulfate, filtered, and then concentrated under reduced pressure. Imidazole (129 mg) and TBDMS chloride (189 mg) were added to a chloroform/tetrahydrofuran solution (4 mL/8 mL) of the residue, and the resulting mixture was stirred for 4 hours. Imidazole (200 mg) and TBDMS chloride (190 mg) were added, and the resulting mixture was stirred for 1 hour. Water was added to the reaction mixture, and the resulting mixture was extracted with ethyl acetate. The organic layer was dried over sodium sulfate, filtered, and then concentrated under reduced pressure. The residue was purified by silica gel column chromatography (hexane/ethyl acetate) to obtain the title compound (596 mg).
LC-MS [M+H]+/Rt (min): 524.4/1.353 (Method A).

Reference Example 31

The compound of Reference Example 31 was obtained by using the corresponding raw material compounds according to the method described in Reference Example 30.

[Table 13]

| Reference Example | Chemical structure | Physical property data |
|---|---|---|
| 31 | 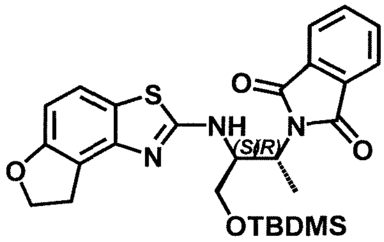 | LC-MS $[M+H]^+$/Rt (min): 524.4/1.407 (Method A). |

Reference Example 32

(2S,3S)-1-{[tert-Butyl(dimethyl)silyl]oxy}-$N^2$-(6,7-dihydrofuro[2,3-e][1,3]benzothiazol-2-yl)butane-2,3-diamine

[Formula 21]

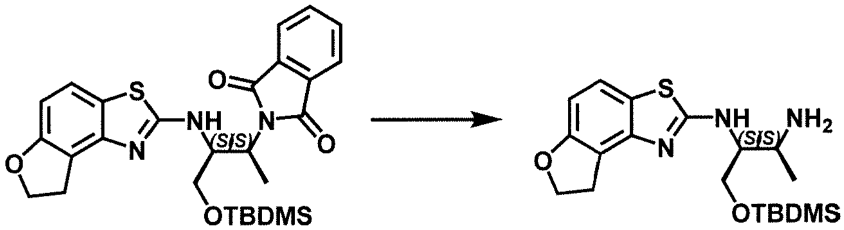

Hydrazine monohydrate (0.554 mL) was added to a methanol/tetrahydrofuran solution (10 mL/10 mL) of the compound of Reference Example 30 (596 mg), and the resulting mixture was stirred at 70°C for 4 hours. After cooling to normal temperature, ethyl acetate (20 mL) was added to the reaction mixture, and the resulting mixture was stirred for 20 minutes. The reaction mixture was filtered through cerite and then concentrated under reduced pressure to obtain the title compound (490 mg).
LC-MS $[M+H]^+$/Rt (min): 394.3/0.948 (Method A).

Reference Example 33

The compound of Reference Example 33 was obtained by using the corresponding raw material compounds according to the method described in Reference Example 32.

[Table 14]

| Reference Example | Chemical structure | Physical property data |
|---|---|---|
| 33 | 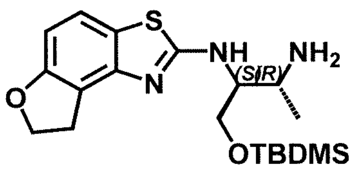 | LC-MS $[M+H]^+$/Rt (min): 394.3/1.017 (Method A). |

Reference Example 34

(4S,5S)-5-({[tert-Butyl(dimethyl)silyl]oxy}methyl)-1-(6,7-dihydrofuro[2,3-e][1,3]benzothiazol-2-yl)-4-methylimidazolidin-2-one

[Formula 22]

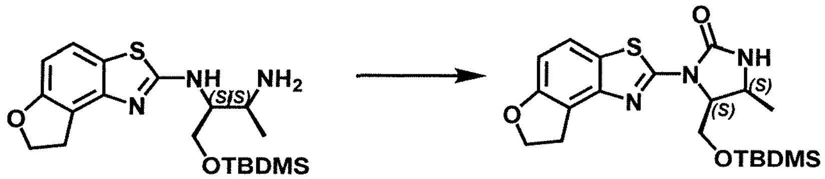

Diisopropylethylamine (0.582 mL) and di(N-succinimidyl) carbonate (307 mg) were added to a chloroform solution (10 mL) of the compound of Reference Example 32 (490 mg), and the resulting mixture was stirred for 2 hours. Saturated aqueous sodium bicarbonate was added to the reaction mixture, and the resulting mixture was extracted with chloroform. The organic layer was dried over sodium sulfate, filtered, and then concentrated under reduced pressure. The residue was purified by silica gel column chromatography (chloroform/methanol) to obtain the title compound (295 mg). LC-MS $[M+H]^+$/Rt (min): 420.3/1.241 (Method A).

Reference Example 35

The compound of Reference Example 35 was obtained by using the corresponding raw material compounds according to the method described in Reference Example 34.

[Table 15]

| Reference Example | Chemical structure | Physical property data |
|---|---|---|
| 35 | 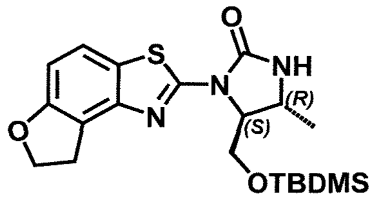 | LC-MS $[M+H]^+$/Rt (min): 420.4/1.279 (Method A). |

Reference Example 36

(4S,5S)-1-(6,7-dihydrofuro[2,3-e][1,3]benzothiazol-2-yl)-5-(hydroxymethyl)-4-methylimidazolidin-2-one

[Formula 23]

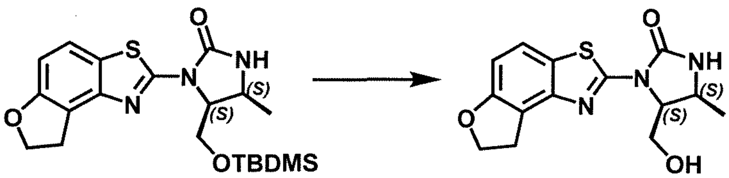

A 2 M hydrogen chloride ethanol solution (1 mL) was added to a methanol suspension (5 mL) of the compound of Reference Example 34 (295 mg), and the resulting mixture was stirred for 1 hour. A 2 M hydrogen chloride ethanol solution (4 mL) was added, and the resulting mixture was stirred for 5 hours. A 2 M hydrogen chloride ethanol solution (4 mL) was further added, and the resulting mixture was stirred overnight. The reaction mixture was concentrated under reduced pressure, saturated aqueous sodium bicarbonate was added, and the resulting mixture was extracted with chloroform/ethanol (3/1). The organic layer was dried over sodium sulfate, filtered, and then concentrated under reduced pressure. The residue was purified by silica gel column chromatography (chloroform/methanol) to obtain the title compound (235 mg). LC-MS $[M+H]^+$/Rt (min): 306.1/0.600 (Method B).

Reference Example 37

The compound of Reference Example 37 was obtained by using the corresponding raw material compounds according to the method described in Reference Example 36.

[Table 16]

| Reference Example | Chemical structure | Physical property data |
|---|---|---|
| 37 | 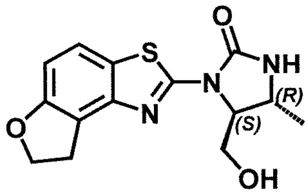 | LC-MS [M+H] +/Rt (min): 306. 10/0. 700 (Method A). |

Reference Example 38

[(4S,5S)-3-(6,7-Dihydrofuro[2,3-e][1,3]benzothiazol-2-yl)-5-methyl-2-oxoimidazolidin-4-yl]methyl methanesulfonate

[Formula 24]

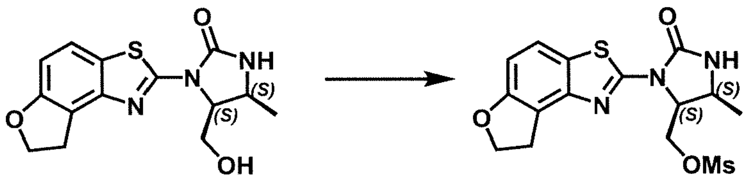

Triethylamine (0.063 mL) and methanesulfonyl chloride (0.017 mL) were added to a tetrahydrofuran solution (4 mL) of the compound of Reference Example 36 (46 mg) under ice cooling, and the resulting mixture was stirred at room temperature for 40 minutes. Triethylamine (0.063 mL) and methanesulfonyl chloride (0.023 mL) were added to the reaction mixture under ice cooling, and the resulting mixture was stirred at room temperature for 3 hours. Saturated aqueous sodium bicarbonate and water were added to the reaction mixture, and the resulting mixture was extracted with chloroform. The organic layer was dried over sodium sulfate, filtered, and then concentrated under reduced pressure to obtain the title compound (122 mg). The next reaction was allowed to proceed without further purification.
LC-MS [M+H]+/Rt (min): 306.1/0.600 (Method B).

Reference Examples 39 to 41

The compounds of Reference Examples 39 to 41 were obtained by using the corresponding raw material compounds according to the method described in Reference Example 38.

[Table 17]

| Reference Example | Chemical structure | Physical property data |
|---|---|---|
| 39 | 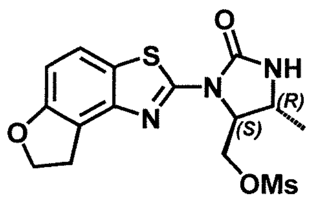 | LC-MS [M+H]+/Rt (min): 384.1/0.785 (Method A). |

(continued)

| Reference Example | Chemical structure | Physical property data |
|---|---|---|
| 40 | 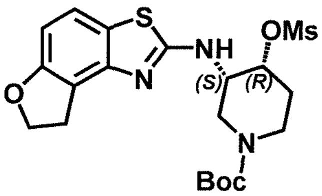 | LC-MS [M+H]$^{+}$/Rt (min): 470.25/2. 437 (Method D). |
| 41 | 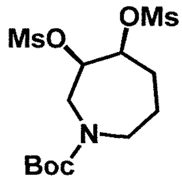 | LC-MS [M+H]$^{+}$/Rt (min): 388.1/0.782 (Method B). |

Reference Example 42

tert-Butyl cis-3,4-diazidoazepane-1-carboxylate

[Formula 25]

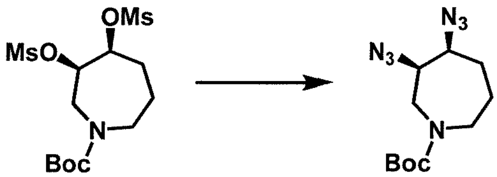

Sodium azide (1.41 g) was added to a dimethylformamide solution (5 mL) of the compound of Reference Example 41 (1.25 g), and the resulting mixture was stirred at 65°C for 4 hours and at 70°C for 8 hours. Dimethyl sulfoxide (3 mL) was added, and the resulting mixture was stirred at 90°C for 24 hours. Water was added to the reaction mixture, and the resulting mixture was extracted with ethyl acetate. The organic layer was washed with saturated brine, then dried over sodium sulfate, filtered, and then concentrated under reduced pressure. The residue was purified by silica gel column chromatography (hexane/ethyl acetate) to obtain the title compound (639 mg).
LC-MS [M+H]$^{+}$/Rt (min): 282.2/1.045 (Method B).

Reference Example 43

The compound of Reference Example 43 was obtained by using the corresponding raw material compounds according to the method described in Reference Example 42.

[Table 18]

| Reference Example | Chemical structure | Physical property data |
|---|---|---|
| 43 | 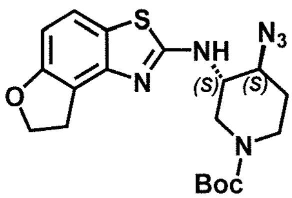 | LC-MS [M+H] $^{+}$/Rt (min): 417. 47/2. 232 (Method D). |

Reference Example 44

tert-Butyl cis-3,4-diaminoazepane-1-carboxylate

[Formula 26]

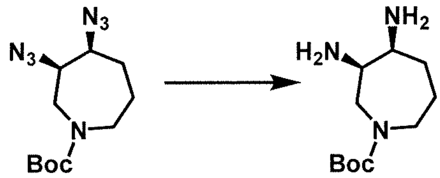

Palladium hydroxide carbon (130 mg) was added to an ethanol solution (15 mL) of the compound of Reference Example 42 (639 mg), and the resulting mixture was stirred for 8 hours in a hydrogen atmosphere. The reaction mixture was filtered through cerite and then concentrated under reduced pressure. The residue was purified by amino silica gel column chromatography (chloroform/methanol) to obtain the title compound (380 mg).
LC-MS $[M+H]^+$/Rt (min): 230.2/0.276 (Method B).

Reference Example 45

The compound of Reference Example 45 was obtained by using the corresponding raw material compounds according to the method described in Reference Example 44.

[Table 19]

| Reference Example | Chemical structure | Physical property data |
|---|---|---|
| 45 | 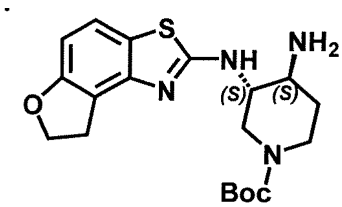 | LC-MS $[M+H]^+$/Rt (min): 391. 45/1. 586 (Method D). |

Reference Example 46

5-Bromo-4-isothiocyanato-2H-1,3-benzodioxole

[Formula 27]

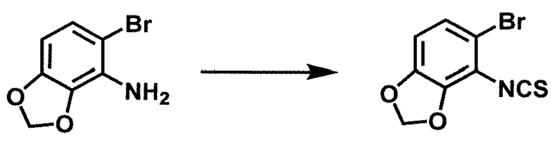

Thiophosgene (17.8 mL) was added to a solution of 5-bromo-1,3-benzodioxol-4-amine (20 g) in water (40 mL) under ice cooling, and the resulting mixture was stirred at room temperature for 4 hours. Water was added to the reaction mixture, and the resulting mixture was extracted with ethyl acetate. The organic layer was dried over sodium sulfate, filtered, and then concentrated under reduced pressure. The residue was purified by silica gel column chromatography (petroleum ether) to obtain the title compound (10 g).
$^1$H-NMR (DMSO-$D_6$) $\delta$: 7.18 (1H, d, J = 8.4 Hz), 6.91 (1H, d, J = 8.4 Hz), 6.22 (2H, s).

Reference Example 47

tert-Butyl {(3R,4R)-4-[(2H-[1,3]dioxolo[4,5-e][1,3]benzothiazol-7-yl)amino]oxan-3-yl}carbamate

[Formula 28]

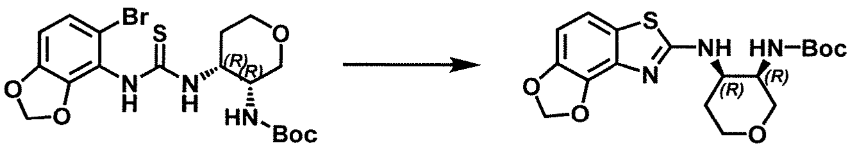

1,10-Phenanthroline (84 mg), cesium carbonate (1.51 g), and copper(I) iodide (22 mg) were added to an acetonitrile solution (23 mL) of the compound of Reference Example 7 (1.1 g), and the resulting mixture was stirred at 95°C for 4 hours. The reaction mixture was filtered through cerite and then concentrated under reduced pressure. The residue was purified by silica gel column chromatography (hexane/ethyl acetate) to obtain the title compound (759 mg).
$^1$H-NMR ($CDCl_3$) $\delta$: 7.00 (1H, d, J = 8.2 Hz), 6.68 (1H, d, J = 8.2 Hz), 6.16 (1H, s), 6.07-5.99 (2H, m), 5.27 (1H, s), 4.21 (1H, d, J = 8.2 Hz), 4.05 (1H, s), 3.97 (1H, dd, J = 4.5, 11.9 Hz), 3.90-3.83 (1H, m), 3.69-3.61 (1H, m), 3.61-3.51 (1H, m), 2.38-2.25 (1H, m), 1.73 ((1H, dtd, J = 4.9, 12.0, 13.5 Hz), 1.47 (9H, s).

Reference Example 48

5-Isothiocyanato-2,3-dihydro-1,4-benzodioxin

[Formula 29]

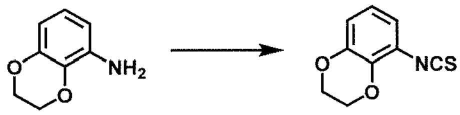

5-Amino-1,4-benzodioxane (2.75 g) was added to a chloroform solution (20 mL) of 1,1'-thiocarbonyldi-2(1H)-pyridone (4.43 g) at room temperature, and the resulting mixture was stirred at room temperature overnight. Water was added to the reaction mixture, and the resulting mixture was extracted with chloroform. The organic layer was dried over sodium sulfate, filtered, and then concentrated under reduced pressure. The residue was purified by silica gel column chromatography (hexane/ethyl acetate) to obtain the title compound (3.38 g).
$^1$H-NMR ($CDCl_3$) $\delta$: 6.80-6.67 (3H, m), 4.37-4.33 (2H, m), 4.28-4.24 (2H, m).

Reference Examples 49 to 57

The compounds of Reference Examples 49 to 57 were obtained by using the corresponding raw material compounds according to the method described in Reference Example 1 and Reference Example 2.

[Table 20]

| Reference Example | Chemical structure | Physical property data |
|---|---|---|
| 49 | 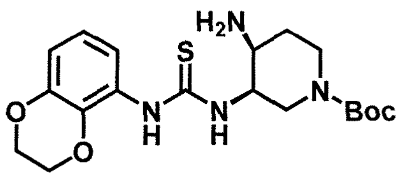 | LC-MS [M+H] $^+$/Rt (m i n) : 409.2/0.594 (Method B). |

(continued)

| Reference Example | Chemical structure | Physical property data |
|---|---|---|
| 50 | 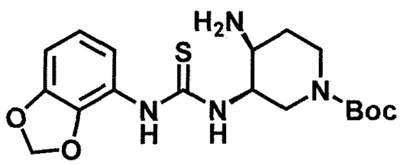 | LC-MS [M+H]$^+$/Rt (m i n) : 395. 2/0.675 (Method A). |
| 51 | 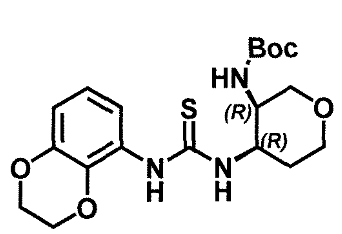 | $^1$H-NMR ($CDCl_3$) $\delta$: 7.42 (1H, s), 6. 87-6. 70 (3H, m), 5.15-5.07 (1H, m), 4.68-4.56 (1H, m), 4.39-4.18 (4H, m), 3.98-3.89 (2H, m), 3.85-3. 76 (1H, m), 3. 62 (1H, dd, $J$ = 1.6, 11.9 Hz), 3. 57-3. 50 (1H, m), 2.29 (1H, s), 1.60 (2H, s), 1. 35 (9H, s). |
| 52 | 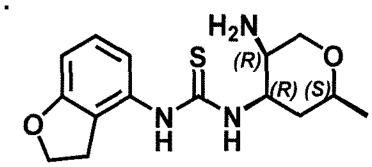 | LC-MS [M+H]$^+$/Rt (min): 308.1/1.624 (Method C). |
| 53 | 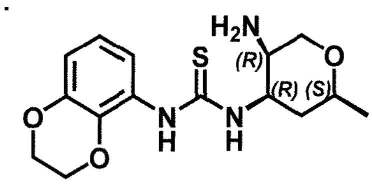 | LC-MS [M+H]$^+$/Rt (m i n) : 324. 1/1. 623 (Method C). |
| 54 | 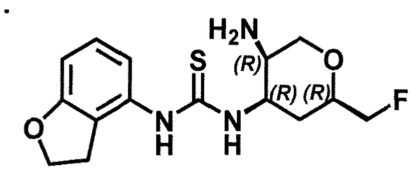 | LC-MS [M+H]$^+$/Rt (m i n) : 326.1/1.490 (Method C). |
| 55 | 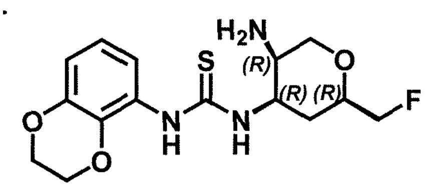 | LC-MS [M+H] $^+$/Rt (m i n) : 342.1/1.474 (Method C). |
| 56 | 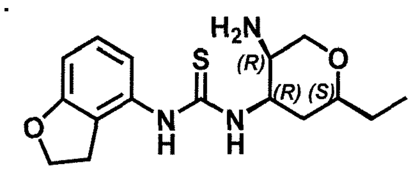 | LC-MS [M+H]$^+$/Rt (min): 322.2/1.757 (Method C). |
| 57 | 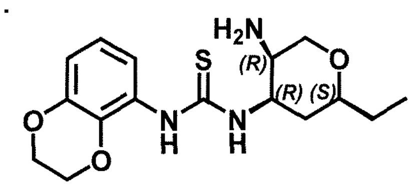 | LC-MS [M+H]$^+$/Rt (min): 338.2/1.707 (Method C). |

Reference Examples 58 to 66

The compounds of Reference Examples 58 to 66 were obtained by using the corresponding raw material compounds according to the method described in Reference Example 8.

[Table 21]

| Ref. Example | Chemical structure | Physical property data |
|---|---|---|
| 58 | 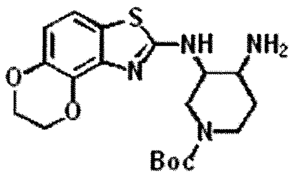 | LC-MS $[M+H]^+$/Rt (min): 407.2/0.609 (Method B). |
| 59 | 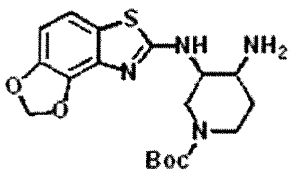 | $^1$H-NMR ($CDCl_3$) $\delta$: 6.99 (1H, d, $J$ = 5.8 Hz), 6.67 (1H, d, $J$ = 7.9 Hz), 6.01 (2H, s), 5.84 (1H. d, $J$ = 6.1 Hz), 4.01 (2H, bs), 3.19 (1H, bs), 3.27 (1H, d, $J$ = 12.8 Hz), 3.22-3.1 8 (IH, m), 3.09 (1H, t, $J$ = 10.1 Hz ), 1.77-1.71 (1H, m), 1.56-1.49 (1H , m), 1.35 (9H, s). |
| 60 | 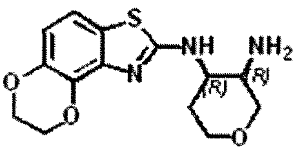 | LC-MS $[M+H]^+$/Rt (min): 308.1/2.024 (Method C). |
| 61 | 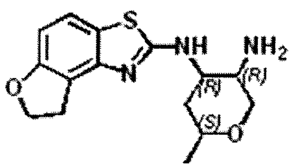 | LC-MS $[M+H]^+$/Rt (min): 306.1/1.657 (Method C). |
| 62 | 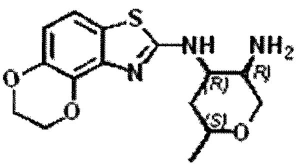 | LC-MS $[M+H]^+$/Rt (min): 322.2/1.657 (Method C). |
| 63 | 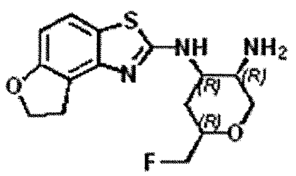 | LC-MS $[M+H]^+$/Rt (min): 324.1/1.574 (Method C). |
| 64 | 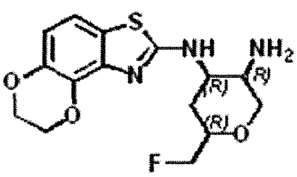 | LC-MS $[M+H]^+$/Rt (min): 340.1/1.657 (Method C). |
| 65 | 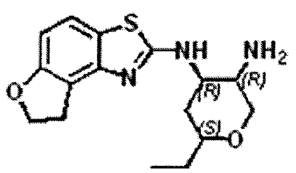 | LC-MS $[M+H]^+$/Rt (min): 320.2/1.757 (Method C) |

(continued)

| Ref. Example | Chemical structure | Physical property data |
|---|---|---|
| 66 | 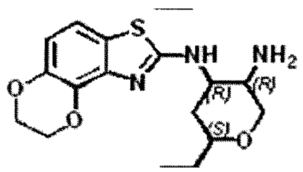 | LC-MS [M+H]+/Rt (min): 336.2/1.740 (Method C) |

Reference Examples 67 and 68

The compounds of Reference Examples 67 and 68 were obtained by using the corresponding raw material compounds according to the method described in Reference Example 14.

[Table 22]

| Reference Example | Chemical structure | Physical property data |
|---|---|---|
| 67 | 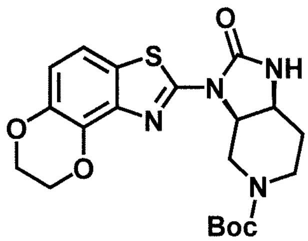 | LC-MS [M+H]+/Rt (min): 433.2/0.764 (Method B) |
| 68 | 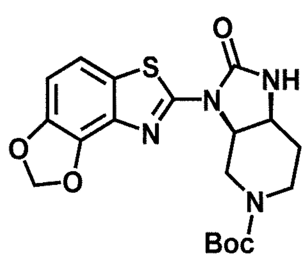 | LC-MS [M+H]+/Rt (min): 419.2/0.819 (Method B) |

Reference Example 69

(3R,4S,6R)-6-cyanooxane-3,4-diyl diacetate

[Formula 30]

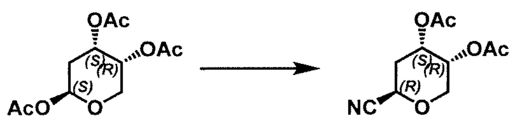

Trimethylsilyl cyanide (1.93 mL) and a boron trifluoride diethyl ether complex (4.56 mL) were added to a dichloromethane solution (40 mL) of (2S,4S,5R)-tetrahydro-2H-pyran-2,4,5-triyltriacetate (3.12 g) under ice cooling, and the resulting mixture was stirred under ice cooling for 1 hour. Saturated aqueous sodium bicarbonate was added to the reaction mixture, and the resulting mixture was extracted with chloroform. The organic layer was dried over sodium sulfate, filtered, and then concentrated under reduced pressure to obtain the title compound (2.94 g).
$^1$H-NMR ($CDCl_3$) δ: 5.38-5.28 (1H, m), 5.21-5.13 (1H, m), 4.84 (1H, t, J = 4.6 Hz), 4.01 (1H, dd, J = 2.5, 12.9 Hz), 3.93 (1H, dd, J = 4.5, 12.9 Hz), 2.42-2.29 (1H, m), 2.14-2.08 (4H, m), 2.07 (3H, s).

Reference Example 70

Methyl 2,6-anhydro-3-deoxy-D-ribo-hexonate

[Formula 31]

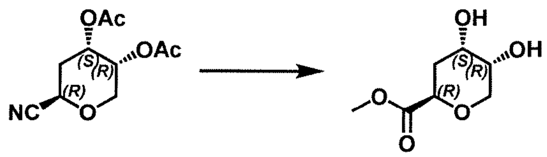

Reference Example 69 was added to a 5% hydrogen chloride methanol solution (30 mL) at room temperature, and the resulting mixture was stirred under reflux overnight. The reaction mixture was cooled to 0°C, and then the resulting insoluble matter was filtered. The filtrate was concentrated under reduced pressure, and then the residue was purified by silica gel column chromatography (chloroform/methanol) to obtain the title compound (2.82 g).
$^1$H-NMR ($CDCl_3$) δ: 4.39 (1H, ddd, J = 0.7, 3.0, 10.8 Hz), 4.19-4.10 (1H, m), 3.94-3.87 (1H, m), 3.86-3.79 (1H, m), 3.77 (3H, s), 3.66 (1H, dd, J = 9.4, 10.8 Hz), 2.41 (1H, dd, J = 1.1, 2.9 Hz), 2.28-2.18 (2H, m), 1.97-1.86 (1H, m).

Reference Example 71

Methyl 2,6-anhydro-4,5-O-benzylidene-3-deoxy-D-ribohexonate

[Formula 32]

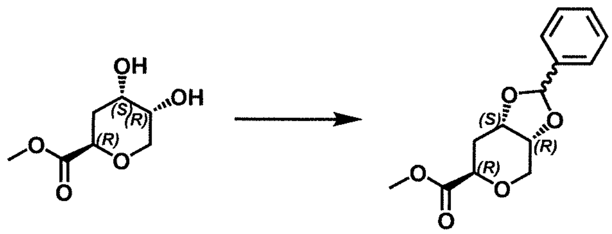

Benzaldehyde dimethyl acetal (5.55 mL) and para-toluenesulfonic acid hydrate (0.424 g) were added to a dimethylformamide solution (30 mL) of Reference Example 70 (2.67 g) at room temperature, and the resulting mixture was stirred at 60 to 70°C overnight. The reaction mixture was cooled to room temperature, then water was added, and the resulting mixture was extracted with hexane/ethyl acetate (1:1). The organic layer was dried over sodium sulfate, filtered, and then concentrated under reduced pressure. The residue was purified by silica gel column chromatography (hexane/ethyl acetate) to obtain the title compound (4.3 g) as a mixture at about 3:2.
$^1$H-NMR ($CDCl_3$) δ: 7.60-7.31 (5H, m), 6.35 (0.4H, s), 5.79 (0.6H, s), 4.58-3.98 (4H, m), 3.83-3.72 (3H, m), 3.72-3.47 (1H, m), 2.60-2.40 (1H, m), 2.15-1.96 (1H, m).

Reference Example 72

2,6-Anhydro-4,5-O-benzylidene-3-deoxy-D-ribo-hexitol

[Formula 33]

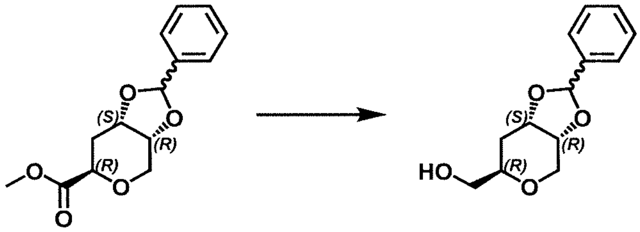

Sodium borohydride (2.25 g) was added to a methanol solution (40 mL) of Reference Example 71 (3.93 g) under ice cooling, and the resulting mixture was stirred under ice cooling for 1 hour. A saturated ammonium chloride aqueous solution was added to the reaction mixture, and the resulting mixture was extracted with ethyl acetate. The organic layer was dried over sodium sulfate, filtered, and then concentrated under reduced pressure. The residue was purified by silica gel column chromatography (hexane/ethyl acetate) to obtain the title compound (3.08 g) as a mixture at about 3:2.
$^1$H-NMR ($CDCl_3$) δ: 7.59-7.31 (5H, m), 6.34 (0.4H, s), 5.83 (0.6H, s), 4.54-4.20 (2H, m), 4.11-3.92 (2H, m), 3.84-3.62 (2H, m), 2.24-1.73 (3H, m).

Reference Example 73

2,6-Anhydro-4,5-O-benzylidene-1,3-dideoxy-1-iodo-D-ribohexitol

[Formula 34]

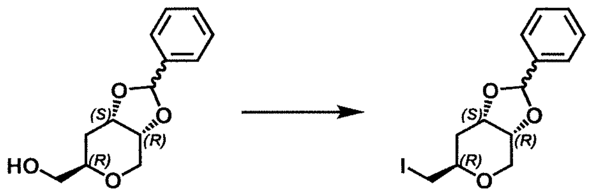

Triphenylphosphine (13.8 g), imidazole (8.96 g), and iodine (13.4) were added to a tetrahydrofuran solution (210 mL) of Reference Example 72 (10.4 g) under ice cooling, and the resulting mixture was stirred under ice cooling for 30 minutes and at room temperature for 3.5 hours. Ice water was added to the reaction mixture, and the resulting mixture was extracted with ethyl acetate. The organic layer was washed with a 5% sodium thiosulfate aqueous solution, then dried over sodium sulfate, filtered, and then concentrated under reduced pressure. The residue was purified by silica gel column chromatography (hexane/ethyl acetate) to obtain the title compound (14.0 g) as a mixture at about 3:2.
$^1$H-NMR ($CDCl_3$) δ: 7.61-7.31 (5H, m), 6.34 (0.4H, s), 5.75 (0.6H, s), 4.55-4.23 (2H, m), 4.14-3.94 (1H, m), 3.73-3.47 (2H, m), 3.34-3.10 (2H, m), 2.41-2.30 (1H, m), 1.94-1.67 (1H, m).

Reference Example 74

2,6-Anhydro-4,5-O-benzylidene-1,3-dideoxy-1-fluoro-D-ribohexitol

[Formula 35]

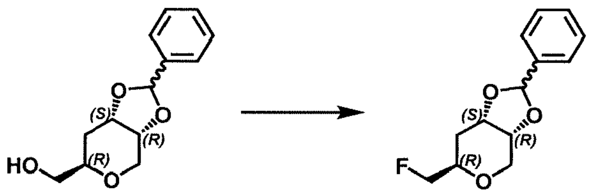

Diisopropylethylamine (3.44 mL) and diethylaminosulfur trifluoride (1.04 mL) were added to a dichloromethane solution (33 mL) of Reference Example 72 (10.4 g) under ice cooling, and the resulting mixture was stirred under reflux for 19 hours. Diisopropylethylamine (1.72 mL) and diethylaminosulfur trifluoride (0.52 mL) were further added at room

temperature, and the resulting mixture was stirred under reflux for 2 hours. Saturated aqueous sodium bicarbonate was added to the reaction mixture under ice cooling, and the resulting mixture was extracted with chloroform. The organic layer was dried over sodium sulfate, filtered, and then concentrated under reduced pressure. The residue was purified by silica gel column chromatography (hexane/ethyl acetate) to obtain the title compound (1.25 g) as a mixture at about 3:2.
$^1$H-NMR ($CDCl_3$) δ: 7.57-7.32 (5H, m), 6.38 (0.4H, s), 5.90 (0.6H, s), 4.57-4.26 (4H, m), 4.15-3.85 (2H, m), 3.63-3.49 (1H, m), 2.22-1.84 (2H, m).

Reference Example 75

2,6-Anhydro-4,5-O-benzylidene-3-deoxy-D-ribo-hexose

[Formula 36]

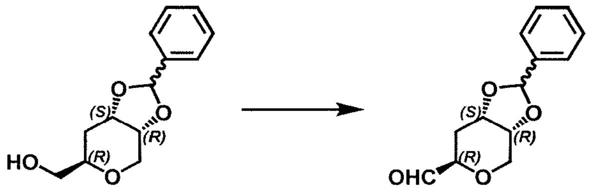

The Dess-Martin reagent (4.31 g) was added to a dichloromethane solution (85 mL) of Reference Example 72 (2.00 g) under ice cooling, and the resulting mixture was stirred at room temperature overnight. Saturated aqueous sodium bicarbonate and a saturated sodium thiosulfate aqueous solution were added to the reaction mixture under ice cooling, and the resulting mixture was extracted with chloroform. The organic layer was washed with saturated brine, then dried over sodium sulfate, filtered, and then concentrated under reduced pressure. The residue was purified by silica gel column chromatography (hexane/ethyl acetate) to obtain the title compound (0.83 g).
$^1$H-NMR ($CDCl_3$) δ: 9.70 (1H, s), 7.58-7.45 (2H, m), 7.43-7.37 (1H, m), 5.92 (1H, s), 4.57-4.43 (1H, s), 4.35-4.14 (2H, m), 4.01 (1H, dd, J = 5.3, 12.1 Hz), 3.64 (1H, dd, J = 7.2, 12.1 Hz), 2.44 (1H,ddd, J = 2.6, 3.8, 15.1 Hz), 1.92 (1H, ddd, J = 3.8, 11.9, 15.1 Hz).

Reference Example 76

3,7-Anhydro-5,6-O-benzylidene-1,2,4-trideoxy-D-ribo-hept-1-ynitol

[Formula 37]

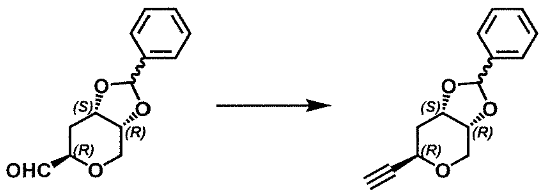

Potassium carbonate (0.979 mg) and dimethyl (1-diazo-2-oxopropyl)phosphonate (0.638 mL) were added to a methanol solution (35 mL) of Reference Example 75 (0.83 g) at room temperature, and the resulting mixture was stirred at room temperature overnight. Water was added to the reaction mixture, and the resulting mixture was extracted with ethyl acetate. The organic layer was washed with water and saturated brine, then dried over sodium sulfate, filtered, and then concentrated under reduced pressure. The residue was purified by silica gel column chromatography (hexane/ethyl acetate) to obtain the title compound (0.415 mg).
$^1$H-NMR ($CDCl_3$) δ: 7.57-7.46 (2H, m), 7.45-7.34 (3H, m), 5.91 (1H, s), 4.56-4.45 (2H, m), 4.27-4.23 (1H, m), 4.10 (1H, dd, J = 4.9, 12.5 Hz), 3.71 (1H, dd, J = 6.1, 12.5 Hz), 2.50 (1H, d, J = 2.2 Hz), 2.38-2.32 (1H, m), 2.20-2.13 (1H, m).

Reference Example 77

2,6-Anhydro-4,5-O-benzylidene-1,3-dideoxy-D-ribo-hexitol

[Formula 38]

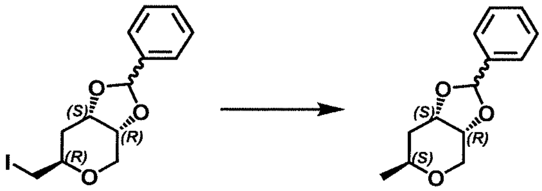

Sodium borohydride (7.64 g) was added to a dimethyl sulfoxide solution (200 mL) of Reference Example 73 (10.4 g) under ice cooling, and the resulting mixture was stirred at room temperature overnight. The reaction mixture was poured into ice water and then extracted with hexane/ethyl acetate (1:1). The organic layer was washed with water, then dried over sodium sulfate, filtered, and then concentrated under reduced pressure. The residue was purified by silica gel column chromatography (hexane/ethyl acetate) to obtain the title compound (7.34 g) as a mixture at about 3:2.
$^1$H-NMR ($CDCl_3$) $\delta$: 7.60-7.32 (5H, m), 6.37 (0.4H, s), 5.78 (0.6H, s), 4.09-3.91 (1H, m), 3.82-3.64 (1H, m), 3.56-3.32 (1H, m), 2.32-2.11 (1H, m), 1.85-1.62 (1H, m), 1.24-1.14 (3H, m).

Reference Example 78

2,6-Anhydro-1,3-dideoxy-D-ribo-hexitol

[Formula 39]

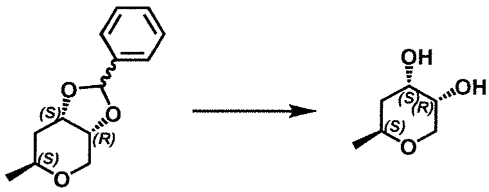

Para-toluenesulfonic acid hydrate (0.951 g) was added to a methanol solution (160 mL) of Reference Example 77 (7.34 g) at room temperature, and the resulting mixture was stirred at room temperature overnight. Diisopropylethylamine (1.16 mL) was added to the reaction mixture at room temperature, and the resulting mixture was stirred at room temperature for 15 minutes, and then concentrated under reduced pressure. The residue was purified by silica gel column chromatography (chloroform/methanol) to obtain the title compound (3.83 g). $^1$H-NMR ($CDCl_3$) $\delta$: 4.18-4.02 (1H, m), 3.91-3.66 (3H, m), 3.64-3.44 (1H, m), 2.18 (1H, d, J = 3.1 Hz), 2.04-1.94 (1H, m), 1.90 (1H, ddd, J = 2.2, 3.8, 14.3 Hz), 1.54 (1H, dddd, J = 1.2, 2.7, 11.1, 14.1 Hz), 1.16 (3H, d, J = 6.3 Hz).

Reference Example 79

The compound of Reference Example 79 was obtained by using the corresponding raw material compounds according to the method described in Reference Example 78.

[Table 23]

| Reference Example | Chemical structure | Physical property data |
|---|---|---|
| 79 | 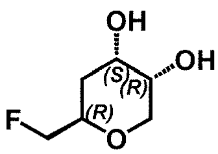 | $^1$H-NMR ($CDCl_3$) $\delta$: 4. 53-4. 26 (2H, m), 4.22-4.17 (1H, m), 4.03-3.90 (1H, m), 3. 85-3. 75 (2H, m), 3. 66-3. 56 (1H, m), 2. 27-2. 22 (1H, m), 1. 98-1.91 (1H, m), 1.90-1.82 (1H, m), 1. 77-1. 68 (1H, m). |

Reference Example 80

3,7-Anhydro-1,2,4-trideoxy-D-ribo-heptitol

[Formula 40]

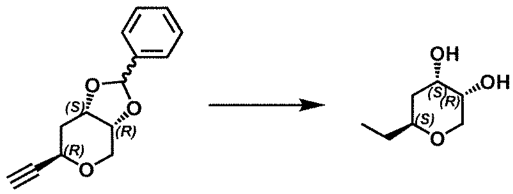

10% Palladium carbon (95 mg) was added to a methanol solution (18 mL) of Reference Example 76 (410 mg) at room temperature, and the resulting mixture was stirred overnight in a room temperature hydrogen atmosphere. The reaction mixture was filtered through cerite, and then the filtrate was concentrated under reduced pressure. The residue was purified by silica gel column chromatography (hexane/ethyl acetate) to obtain the title compound (240 mg).
$^1$H-NMR ($CDCl_3$) $\delta$: 4.16-4.07 (1H, m), 3.81-3.69 (2H, m), 3.62-3.49 (2H, m), 2.33-2.27 (1H, m), 2.16 (1H, d, J = 6.3 Hz), 1.91 (1H, ddd, J = 2.2, 3.8, 14.3 Hz), 1.59-1.34 (3H, m), 0.93 (3H, t, J = 7.5 Hz).

Reference Examples 81 to 83

The compounds of Reference Examples 81 to 83 were obtained by using the corresponding raw material compounds according to the method described in Reference Example 38.

[Table 24]

| Reference Example | Chemical structure | Physical property data |
|---|---|---|
| 81 | 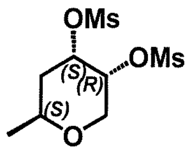 | $^1$H-NMR ($CDCl_3$) $\delta$: 5.24-5.11 (1H, m), 4. 75 (1H, ddd, *J* = 3.0, 5. 5, 10.7 Hz), 3. 96-3. 84 (2H, m), 3.80 (1H, dd, *J* = 10.7, 10.9Hz),3.13 (3H, s), 3.10 (3H, s), 2.21 (1H, ddd, *J* = 2.1, 4. 0, 14. 9 Hz), 1. 74 (1H, ddd, *J* = 2. 3, 11. 2, 14.9 Hz), 1.20 (3H, d, *J* = 6. 2 Hz). |
| 82 | 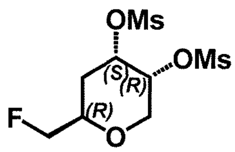 | $^1$H-NMR ($CDCl_3$) $\delta$: 5.31-5.23 (1H, m), 4.83-4.72 (1H, m), 4.59-4.27 (2H, m), 4.07-3.92 (2H, m), 3.84 (1H, t, J = 10.9 Hz), 3.15 (3H, s), 3.11 (3H, s), 2.27-2.16 (1H, m), 2.04-1.96 (1H, m). |
| 83 | 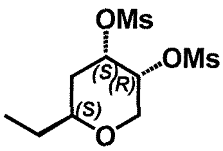 | $^1$H-NMR ($CDCl_3$) $\delta$: 5. 23-5. 17 (1H, m), 4. 78-4. 70 (1H, m), 3. 98-3. 91 (1H , m) , 3.78 (dd, *J* = 10.9, 10.9 Hz), 3. 68-3. 59 (1H, m), 3.13 (3H, s), 3.10 (3H, s), 2.24-2.17 (1H, m), 1.79-1.68 (1H, m), 1.57-1. 41 (2H, m), 0.94 (3H, t, *J* = 7.5 Hz). |

Reference Examples 84 to 86

The compounds of Reference Examples 84 to 86 were obtained by using the corresponding raw material compounds according to the method described in Reference Example 42.

[Table 25]

| Reference Example | Chemical structure | Physical property data |
|---|---|---|
| 84 | 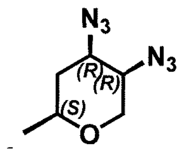 | $^1$H-NMR ($CDCl_3$) $\delta$: 4.12 (1H, dd, $J$ = 2.0, 12. 7 Hz), 3. 67 (1H, dd, $J$ = 1.4, 2.8 Hz), 3. 63-3. 43 (3H, m), 1.89-1.76 (3H, d, $J$ = 6. 2 Hz). |
| 85 | 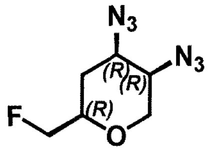 | $^1$H-NMR ($CDCl_3$) $\delta$: 4.55-4.32 (2H, m), 4.19 (1H, dd, $J$ = 2.0, 12. 8 Hz), 3. 76-3. 64 (3H, m), 3.61 (1H, dd, $J$ = 1. 5, 12. 8 Hz), 1. 97-1. 81 (2H, m). |
| 86 | 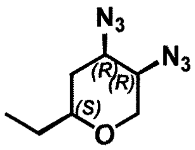 | $^1$H-NMR ($CDCl_3$) $\delta$: 4.13 (1H, dd, $J$ = 2.0, 12.7 Hz), 3.70-3.64 (1H, m), 3.63-3.57 (1H, m), 3.55 (1H, dd, $J$ = 1.5, 12.7 Hz), 3.31-3.22 (1H, m), 1.89-1.48 (4H, m), 0.96 (3H, t, $J$ = 7.5 Hz). |

Reference Examples 87 to 89

The compounds of Reference Examples 87 to 89 were obtained by using the corresponding raw material compounds according to the method described in Reference Example 44.

[Table 26]

| Reference Example | Chemical structure | Physical property data |
|---|---|---|
| 87 | 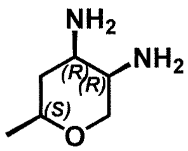 | $^1$H-NMR ($CDCl_3$) $\delta$: 3.87 (1H, dd, $J$ = 2.0, 11.7 Hz), 3.54 (1H, dd, $J$ = 1. 7, 11.7 Hz), 3.41 (1H, dqd, $J$ = 2. 2, 6.2, 11.1 Hz), 2.90 (1H, ddd, $J$ = 3. 6, 4. 5, 12.0 Hz), 2.76-2.64 (1H, m), 1.57-1.52 (1H, m), 1.47-1.32 (4H, m), 1.31-1.21 (1H, m), 1.20 (3H, d, $J$ = 6. 2 Hz). |
| 88 | 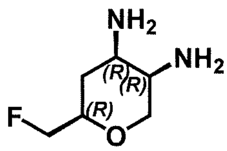 | LC-MS [M+H]$^+$/Rt (min): 149.1/0.224 (Method C) |
| 89 | 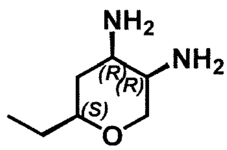 | LC-MS [M+H] $^+$/Rt (m i n) : 145.1/0.224 (Method C). |

Reference Example 90

tert-Butyl 3-buten-1-yl(1-(hydroxymethyl)cyclopropyl)carbamate

[Formula 41]

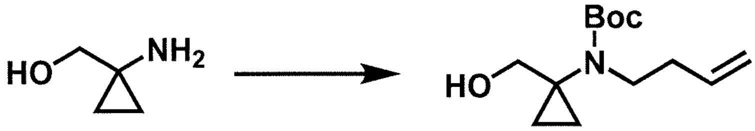

Potassium carbonate (4.56 g), sodium iodide (4.95 g), and 4-bromo-1-butene (3.02 mL) were added to an acetonitrile solution (50 mL) of (1-aminocyclopropyl)methanol at room temperature, and the resulting mixture was stirred at 85°C for 12.5 hours. The reaction mixture was cooled to room temperature, and then filtered and concentrated. Tetrahydrofuran (50 mL), triethylamine (8.36 mL), and $(Boc)_2O$ (7.20 g) were added to the residue, and the resulting mixture was stirred at room temperature for 1.5 hours. Saturated aqueous sodium bicarbonate was added to the reaction mixture, and the resulting mixture was extracted with ethyl acetate. The organic layer was dried over sodium sulfate, filtered, and then concentrated under reduced pressure. The residue was purified by silica gel chromatography (hexane/ethyl acetate) to obtain the title compound (3.52 g).
LC-MS $[M+H]^+$/Rt (min): 242.5/0.936 (Method B)

Reference Example 91

tert-Butyl 3-buten-1-yl(1-formylcyclopropyl)carbamate

[Formula 42]

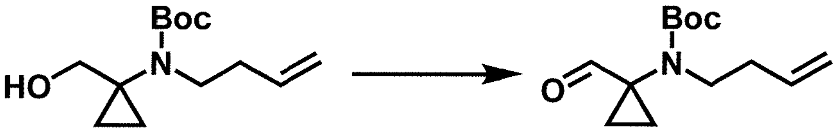

A chloroform suspension (20 mL) of the Dess-Martin reagent (2.80 g) was added to a chloroform solution (30 mL) of the compound of Reference Example 90 (1.52 g) at room temperature, and the resulting mixture was stirred at room temperature for 1 hour. The Dess-Martin reagent (900 mg) was added to the reaction mixture at room temperature, and the resulting mixture was stirred for 40 minutes. Sodium hydrogen carbonate (2.80 g) and diethyl ether were added to the reaction mixture, and the resulting mixture was stirred at room temperature, then filtered through cerite, and concentrated under reduced pressure. The residue was purified by column chromatography (hexane/ethyl acetate) to obtain the title compound (1.35 g). LC-MS $[M-56+H]^+$/Rt (min): 184.3/1.029 (Method B)

Reference Example 92

tert-Butyl 3-buten-1-yl(1-vinylcyclopropyl)carbamate

[Formula 43]

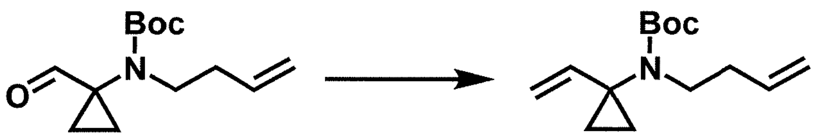

Potassium tert-butoxide (1.27 g) was added to a tetrahydrofuran suspension (19 mL) of methyltriphenylphosphonium bromide (4.03 g) at normal temperature, and the resulting mixture was stirred for 1 hour. A tetrahydrofuran solution (9 mL) of the compound of Reference Example 91 (1.35 g) was added to the reaction mixture at room temperature, and the resulting mixture was stirred for 1 hour. Water was added to the reaction mixture, and the resulting mixture was extracted with ethyl acetate. The organic layer was dried over sodium sulfate, then filtered, and concentrated under reduced pressure. The residue was purified by column chromatography (hexane/ethyl acetate) to obtain the title compound (1.23 g).
LC-MS $[M-56+H]^+$/Rt (min): 182.3/1.282 (Method B)

Reference Example 93

tert-Butyl 2-propen-1-yl[1-(2-propen-1-yl)cyclopropyl]carbamate

[Formula 44]

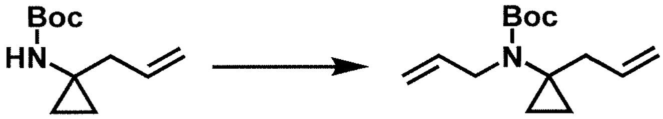

Sodium hydride (60% in oil; 150 mg) and allyl bromide (0.318 mL) were added to a dimethylformamide solution (10 mL) of tert-butyl (1-allylcyclopropyl)carbamate (2.90 mL) at 0°C, and the resulting mixture was stirred at room temperature for 4 hours. Water was added to the reaction mixture, and the resulting mixture was extracted with ethyl acetate. The organic layer was dried over sodium sulfate, then filtered, and concentrated under reduced pressure. The residue was purified by silica gel column chromatography (hexane/ethyl acetate) to obtain the title compound (0.68 g). $^{1}$H-NMR (DMSO-$d_6$) $\delta$: 5.82-5.72 (2H, m), 5.21-4.80 (4H, m), 3.79 (2H, brs), 2.29 (2H, brs), 1.42 (9H, s), 0.85 (2H, brs), 0.64 (2H, brs).

Reference Example 94

The compound of Reference Example 94 was obtained by using the corresponding raw material compounds according to the method described in Reference Example 93.

[Table 27]

| Reference Example | Chemical structure | Physical property data |
|---|---|---|
| 94 | 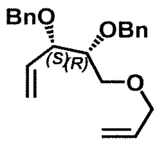 | $^{1}$H-NMR ($CDCl_3$) $\delta$: 7.39-7. 20 (10H, m), 5.96-5.81 (2H, m), 5.38-5.11 (4H, m), 4.71 (2H, s), 4.63 (1H, d, $J$ = 11. 9 Hz), 4.40 (1H, d, $J$ = 11. 9 Hz), 4.04-3.92 (3H, m), 3.72 (1H, dt, $J$ = 4.5, 5.9 Hz), 3.66-3. 52 (2H, m). |

Reference Example 95

tert-Butyl 4-azaspiro[2.5]-7-octene-4-carboxylate

[Formula 45]

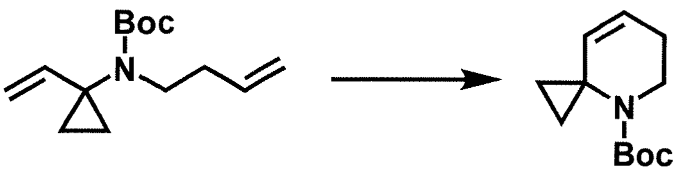

The Grubbs second generation catalyst (220 mg) was added to a solution of the compound of Reference Example 93 (1.23 g) in toluene (52 mL) at room temperature, and the resulting mixture was stirred at 50°C for 7.5 hours. The reaction mixture was concentrated under reduced pressure, and then the residue was purified by column chromatography (hexane/ethyl acetate) to obtain the title compound (1.04 g).
LC-MS $[M-56+H]^+$/Rt (min): 154.1/1.151 (Method B)

Reference Examples 96 and 97

The compounds of Reference Examples 96 and 97 were obtained by using the corresponding raw material compounds according to the method described in Reference Example 95.

[Table 28]

| Reference Example | Chemical structure | Physical property data |
|---|---|---|
| 96 | 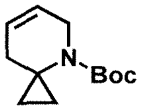 | $^1$H-NMR (DMSO-$d_6$) $\delta$: 5.80-5.68 (2H, m), 3.93 (2H, brs), 2.03 (2H, brs), 1.43 (9H, s), 0.88-0.85 (2H, m), 0.61-0.58 (2H, m). |
| 97 | 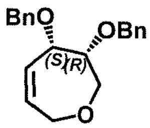 | $^1$H-NMR ($CDCl_3$) $\delta$: 7. 39-7. 26 (10H, m), 5.84-5.69 (2H, m), 4.81-4.52 (5H, m), 4.31-4.15 (2H, m), 4.07-4.00 (1H, m), 3. 98-3. 90 (1H, m), 3. 78 (1H, dd, $J$ = 3.8, 12. 6 Hz). |

Reference Example 100

tert-Butyl (2S)-2-methyl-4-[(trifluoromethanesulfonyl)oxy]-3,6-dihydropyridine-1(2H)-carboxylate

Reference Example 101

tert-Butyl (6S)-6-methyl-4-[(trifluoromethanesulfonyl)oxy]-3,6-dihydropyridine-1(2H)-carboxylate

[Formula 46]

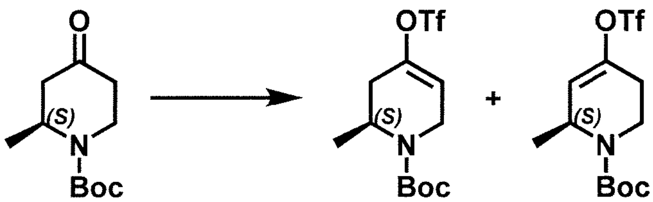

A tetrahydrofuran solution (7 mL) of tert-butyl (S)-2-methyl-4-oxopiperidine-1-carboxylate (1.20 g) was added to a tetrahydrofuran solution (7 mL) of lithium diisopropylamide (1.1 M tetrahydrofuran solution, 6.6 mL) at 0°C, and the resulting mixture was stirred at 0°C for 10 minutes. A tetrahydrofuran solution (14 mL) of N-phenylbis(trifluoromethanesulfonimide) (2.60 g) was added dropwise at 0°C over 5 minutes, and the resulting mixture was stirred at room temperature for 2 hours. A saturated ammonium chloride aqueous solution was added to the reaction mixture, and the resulting mixture was extracted with ethyl acetate. The organic layer was dried over sodium sulfate, then filtered, and concentrated under reduced pressure. The residue was purified by silica gel column chromatography (hexane/ethyl acetate) to obtain the title compound (0.70 g) as a mixture with N-phenylbis(trifluoromethanesulfonimide).
$^1$H-NMR (DMSO-$d_6$) $\delta$: 5.73-5.69 (1H, m), 4.64-3.60 (2H, m), 2.97-2.51 (1H, m), 2.23-2.02 (1H, m), 1.46 (s, 4.5H), 1.45 (s, 4.5H), 1.21 (1.5H, d, J = 6.8 Hz), 1.16 (1.5H, d, J = 6.8 Hz).

Reference Examples 102 and 103

The compounds of Reference Examples 102 and 103 were obtained by using the corresponding raw material compounds according to the method described in Reference Examples 100 and 101.

[Table 29]

| Reference Example | Chemical structure | Physical property data |
|---|---|---|
| 102 | 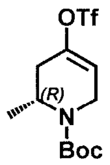 | Obtained as a mixture. $^1$H-NMR (DMSO-$d_6$) $\delta$: 5. 74-5. 69 (1H, m), 4. 64-3. 59 (2H, m), 2. 97-2. 51 (1H, m), 2.21-2.02 (1H, m), 1.45 (s, 4.5H), 1.45 (s, 4.5H), 1. 21 (1. 5H, d, $J$ = 6. 8 Hz), 1. 15 (1. 5H, d, $J$ = 6. 8 Hz). |
| 103 | 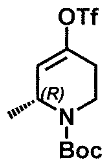 | |

Reference Example 104

tert-Butyl (2S)-2-methyl-3,6-dihydropyridine-1(2H)-carboxylate

Reference Example 105

tert-Butyl (6S)-6-methyl-3,6-dihydropyridine-1(2H)-carboxylate

[Formula 47]

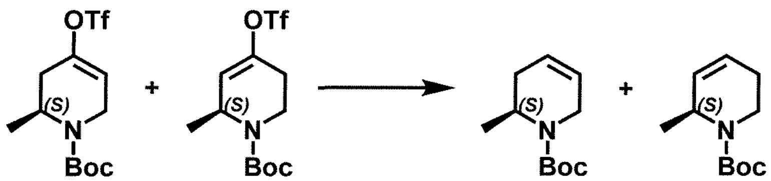

Triphenylphosphine (52.0 mg) and palladium acetate (22.0 mg) were added to a tetrahydrofuran solution (2 mL) of a mixture (345 mg) of Reference Example 100, Reference Example 101, and N-phenylbis(trifluoromethanesulfonimide) at room temperature, and the resulting mixture was stirred at room temperature for 5 minutes. A tetrahydrofuran solution (4 mL) of formic acid (51.0 mg) and diisopropylethylamine (116 mg) was added dropwise at room temperature over 3 minutes, and then the resulting mixture was stirred under reflux for 1 hour. Water was added to the reaction mixture, and the resulting mixture was extracted with ethyl acetate. The organic layer was dried over sodium sulfate, then filtered, and concentrated under reduced pressure. The residue was purified by silica gel column chromatography (hexane/ethyl acetate) to obtain the title compounds (45.0 mg) as a mixture. $^1$H-NMR (DMSO-$d_6$) $\delta$: 5.78-5.55 (2H, m), 4.46-3.47 (2H, m), 2.80-1.78 (2H, m), 1.45 (s, 4.5H), 1.44 (s, 4.5H), 1.14 (1.5H, d, J = 6.8 Hz), 1.08 (1.5H, d, J = 6.8 Hz).

Reference Examples 106 and 107

The compounds of Reference Examples 106 and 107 were obtained by using the corresponding raw material compounds according to the method described in Reference Examples 104 and 105.

[Table 30]

| Reference Example | Chemical structure | Physical property data |
|---|---|---|
| 106 | 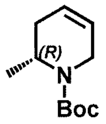 | Obained as a mixture. $^1$H-NMR (DMSO-$d_6$) δ : 5. 78-5. 55 (2H, m), 4. 46-3. 47 (2H, m), 2.80-1.79 (2H, m), 1.44 (s, 4. 5H), 1.44 (s, 4.5H), 1.14 (1. 5H, d, *J* = 6.8 Hz), 1.08 (1.5H, d, *J* = 6.8 Hz). |
| 107 | 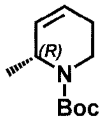 | |

Reference Example 108

The compound of Reference Example 108 was obtained by using the corresponding raw material compounds according to the method described in Reference Example 20.

[Table 31]

| Reference Example | Chemical structure | Physical property data |
|---|---|---|
| 108 | 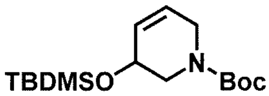 | LC-MS [M+H] $^+$ /Rt (min): 314. 2/1. 423 (Method B) |

Reference Example 109

tert-Butyl rac-(3R,4R,5R)-3-{[tert-butyl(dimethyl)silyl]oxy}-4,5-dihydroxypiperidine-1-carboxylate

[Formula 48]

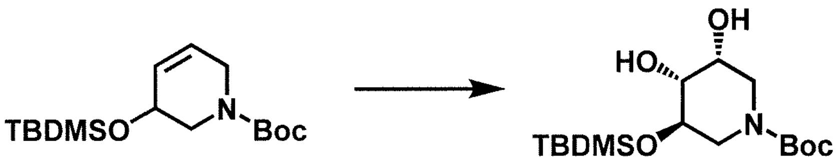

4-Methylmorpholine-4-oxide monohydrate (2.68 g) and osmium tetroxide (immobilized catalyst I) (2.06 g) were added to a water/acetone/acetonitrile solution (15 mL/15 mL/15 mL) of the compound of Reference Example 108 (4.76 g) at room temperature, and the resulting mixture was stirred at room temperature for 16 hours. 4-Methylmorpholine 4-oxide monohydrate (3.48 g) and osmium tetroxide (immobilized catalyst I) (6.18 g) were added at room temperature, and the resulting mixture was stirred at room temperature for 25 hours. The reaction mixture was filtered and concentrated under reduced pressure. The residue was purified by column chromatography (hexane/ethyl acetate) to obtain the title compound (4.80 g).
LC-MS [M+H]$^+$/Rt (min): 348.2/1.066 (Method B)

Reference Example 110

1,6-Anhydro-2,3-dideoxy-D-erythro-2-hexenitol

[Formula 49]

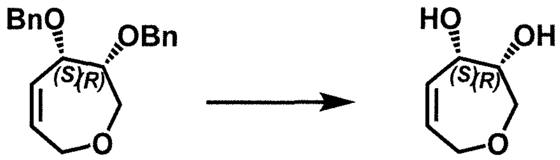

A catalytic amount of 10% palladium carbon was added to a methanol solution (50 mL) of the compound of Reference Example 97 (2.36 g) at room temperature, and the resulting mixture was stirred at room temperature overnight in a normal pressure hydrogen atmosphere. The reaction mixture was filtered and concentrated under reduced pressure to obtain the title compound (1.00 g).
$^1$H-NMR ($CDCl_3$) δ: 3.87-3.81 (1H, m), 3.79-3.71 (4H, m), 3.63-3.56 (1H, m), 1.95-1.81 (2H, m), 1.81-1.71 (1H, m), 1.72-1.56 (1H, m).

Reference Examples 111 to 118

The compounds of Reference Examples 111 to 118 were obtained by using the corresponding raw material compounds according to the method described in Reference Example 38.

[Table 32]

| Reference Example | Chemical structure | Physical property data |
|---|---|---|
| 111 | 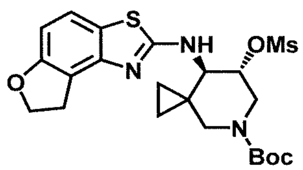 | LC-MS [M+H] $^+$ /Rt (min): 496.3/1.035 (Method B) |
| 112 | 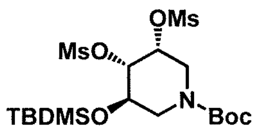 | LC-MS [M+H] $^+$ /Rt (min): 504.3/1.224 (Method B) |
| 113 | 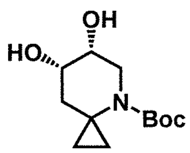 | $^1$H-NMR (DMSO-$d_6$) δ : 4. 00-3. 83 (3H, m), 3.14-3.11 (1H, m), 2.32-2.08 (2H, m), 1. 49-1. 39 (1H, m), 1. 44 (s, 9H), 1. 28-1.00 (2H, m), 0.84-0.78 (1H, m), 0.66 (brs, 1H), 0.48-0.43 (1H, m). |
| 114 | 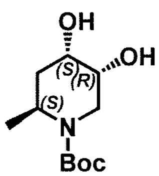 | Obtained as a mixture. LC-MS [M+H] $^+$ /Rt (min): 232.1/0.496, 232.1/0.496, 232.1/0.496 (Method B) |
| 115 | 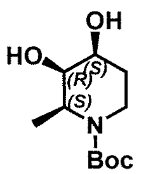 | |
| 116 | 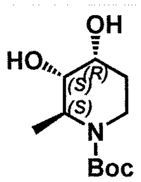 | |

(continued)

| Reference Example | Chemical structure | Physical property data |
|---|---|---|
| 117 | 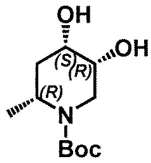 | Obtained as a mixture. |
| 118 | 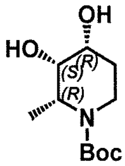 | LC-MS [M+H] + /Rt (min): 232.2/0.502, 232.2/0.502 (Method B) |

Reference Example 119

(2R,4S,5R)-2-Cyano-5-[(4-methylbenzene-1-sulfonyl)oxy]oxane-4-yl 4-methylbenzene-1-sulfonate

[Formula 50]

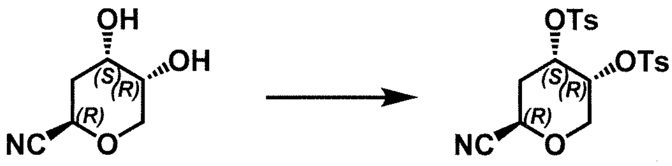

p-Toluenesulfonyl chloride (3.53 g) was added to a pyridine solution (15 mL) of (2R,4S,5R)-4,5-dihydroxytetrahydro-2H-pyran-2-carbonitrile (1.06 g) at 0°C, and the resulting mixture was stirred at 50°C overnight. The reaction mixture was cooled to room temperature, then ice water was added, and the resulting mixture was extracted with ethyl acetate. The organic layer was washed with 1 N hydrochloric acid and saturated aqueous sodium bicarbonate, then filtered, and concentrated under reduced pressure. Diethyl ether was added to the residue, the resulting mixture was stirred, and then the resulting solid was collected by filtration to obtain the title compound (2.55 g).
$^1$H-NMR ($CDCl_3$) δ: 7.74 (2H, d, J = 6.4 Hz), 7.71 (2H, d, J = 6.4 Hz), 7.39-7.32(4H, m), 4.78-4.68 (2H, m), 4.51 (1H, dt, J = 2.9, 5.6 Hz), 4.01 (1H, dd, J = 5.6, 12.9 Hz), 3.80 (1H, dd, J = 2.7, 12.9 Hz), 2.49-2.42 (1H, m), 2.47 (6H, s), 2.01-1.87 (1H, m).

Reference Example 120

The compound of Reference Example 120 was obtained by using the corresponding raw material compounds according to the method described in Reference Example 119.

[Table 33]

| Reference Example | Chemical structure | Physical property data |
|---|---|---|
| 120 | 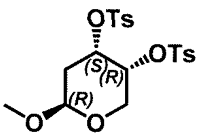 | $^1$H-NMR ($CDCl_3$) δ : 7.80 (2H, d, *J* = 8.3 Hz), 7.67 (2H, d, *J* = 8. 3 Hz), 7. 39-7. 28 (4H, m), 4. 85-4. 67 (2H, m), 4. 61 (1H, td, *J* = 1. 8, 3. 3 Hz), 3. 88 (1H, dd, *J* = 3.0, 13.1 Hz), 3.72 (1H, dd, *J* = 1.4, 13.1 Hz), 3.28 (3H, s), 2.45 (6H, s), 2.14 (1H, ddd, *J* = 3. 4, 11. 6, 12. 8 Hz), 1. 86-1.70 (1H, m). |

Reference Examples 121 to 132

The compounds of Reference Examples 121 to 132 were obtained by using the corresponding raw material compounds according to the method described in Reference Example 38.

[Table 34-1]

| Reference Example | Chemical structure | Physical property data |
|---|---|---|
| 121 | 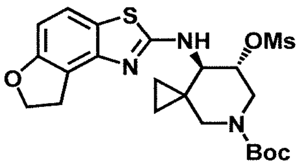 | LC-MS [M+H] + /Rt (min): 496.3/1.035 (Method B) |
| 122 | 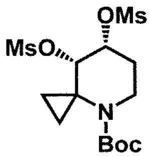 | LC-MS [M+H] + /Rt (min) : 347.2/0.945 (Method B) |
| 123 | 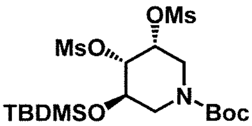 | LC-MS [M+H] + /Rt (min): 504.3/1.224 (Method B) |
| 124 | 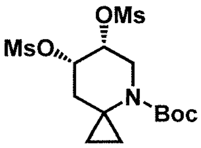 | $^1$H-NMR ($CDCl_3$) $\delta$: 4. 97 (2H, brs), 3.12-3.08 (8H, m), 1. 47-1. 15 (2H, m), 1.46 (s, 9H), 1.28-1.00 (2H, m), 0. 93 (2H, brs), 0.57 (brs, 2H). |
| 125 | 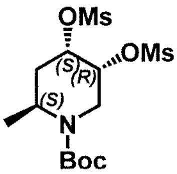 | Obtained as a mixture.<br>LC-MS [M+H] + /Rt (min): 388. 1/0. 775, 388.1/0.775, 388.1/0.775 (Method B) |
| 126 | 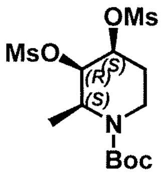 | |
| 127 | 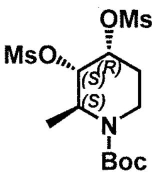 | |
| 128 | 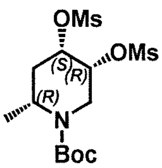 | Obtained as a mixture. |

(continued)

| Reference Example | Chemical structure | Physical property data |
|---|---|---|
| 129 | 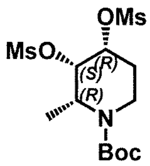 | LC-MS [M+H] $^{+}$ /Rt (min): 388.2/0.773, 388.2/0.773 (Method B) |
| 130 | 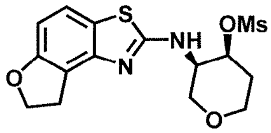 | LC-MS [M+H] $^{+}$ /Rt (min): 371.2/2.851 (Method C) |

[Table 34-2]

| | | |
|---|---|---|
| 131 | 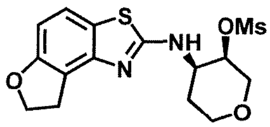 | LC-MS [M+H] $^{+}$/Rt (m i n) : 371. 2/2. 825 (Method C) |
| 132 | 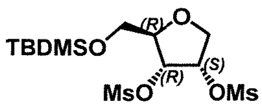 | $^{1}$H-NMR ($CDCl_3$) $\delta$: 5. 25-5. 19 (1H, m), 5.19-5.14 (1H, m), 4. 24-4. 19 (1H, m), 4. 19-4. 16 (1H, m), 4.06-4.00 (1H, m), 3.83 (2H, dd, *J* = 2. 5, 11.7 Hz), 3. 77 (1H, dd, *J* = 2. 4, 11.7 Hz), 3.14 (3H, s), 3. 13 (3H, s), 0.91 (9H, s), 0.09 (3H, s), 0.08 (3H, s). |

Reference Examples 133 to 146

The compounds of Reference Examples 133 to 146 were obtained by using the corresponding raw material compounds according to the method described in Reference Example 42.

[Table 35-1]

| Reference Example | Chemical structure | Physical property data |
|---|---|---|
| 133 | 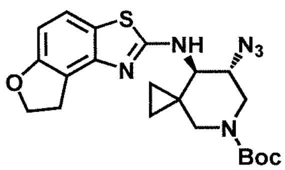 | LC-MS [M+H] $^{+}$ /Rt (min): 443.3/1.091 (Method B) |
| 134 | 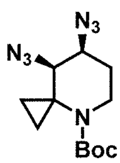 | LC-MS [M+H] $^{+}$ /Rt (min): 294.2/1.030 (Method B) |
| 135 | 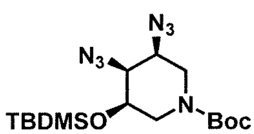 | LC-MS [M+H] $^{+}$ /Rt (min): 398. 4/1. 327 (Method B) |
| 136 | 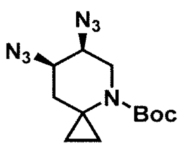 | LC-MS [M+H] $^{+}$ /Rt (min): 294.2/1.031 (Method B) |

(continued)

| Reference Example | Chemical structure | Physical property data |
|---|---|---|
| 137 | 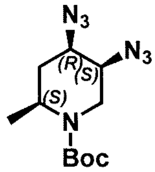 | Obtained as a mixture. |
| 138 | 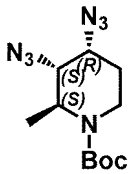 | LC-MS [M+H] + /Rt (min): 282.2/1.053, 282.2/1.053 (Method B) |
| 139 | 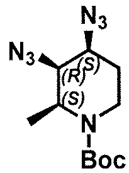 | LC-MS [M+H] + /Rt (min): 282.2/1.053 (Method B) |
| 140 | 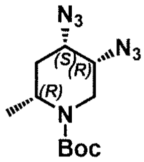 | Obtained as a mixture. |
| 141 | 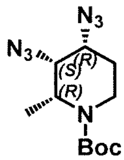 | LC-MS [M+H] + /Rt (min): 282.2/1.065, 282.2/1.065 (Method B) |
| 142 | 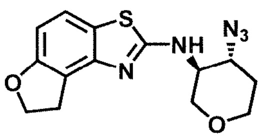 | LC-MS [M+H] + /Rt (min): 318.1/3.218 (Method C) |

[Table 35-2]

| | | |
|---|---|---|
| 143 | 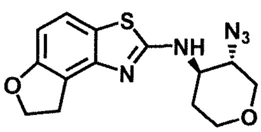 | LC-MS [M+H] +/Rt (m i n) : 318. 1/3. 192 (Method C) |
| 144 | 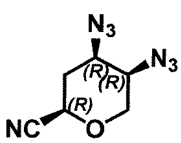 | $^1$H-NMR ($CDCl_3$) $\delta$: 4.45 (1H, dd, *J* = 4.1, 7.5 Hz), 4. 16-4. 12 (1H, m), 3. 89-3. 83 (1H, m), 3. 76-3. 66 (2H, m), 2.34 (1H, ddd, *J* = 7.5, 8.4, 13.8 Hz), 2.19 (1H, dtd, *J* = 0.7, 4.1, 13.8 Hz). |

(continued)

| | | |
|---|---|---|
| 145 | 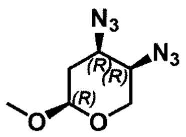 | $^1$H-NMR ($CDCl_3$) $\delta$: 4.53-4.41 (1H, m), 4.14-4.02 (1H, m), 3.79 (1H, ddd, $J$ = 3.4, 5.2, 8.1 Hz), 3.65 (1H, td, $J$ = 3.2, 6.0 Hz), 3. 63-3. 52 (1H, m), 3.45 (3H, s), 2.10-1.98 (2H, m). |
| 146 | 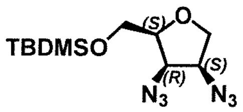 | $^1$H-NMR ($CDCl_3$) $\delta$: 4.26-4.12 (2H, m), 4.10-3.99 (2H, m), 3. 89-3. 72 (3H, m), 0.90 (9H, s), 0.10 (3H, s), 0.08 (3H, s). |

Reference Example 147

tert-Butyl rac-(3R,4R,5S)-3,4-diazido-5-hydroxypiperidine-1-carboxylate

[Formula 51]

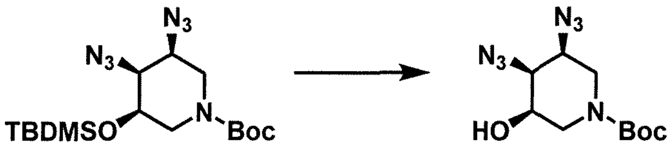

Tetrabutylammonium fluoride (1 M tetrahydrofuran solution, 3.01 mL) was added to a tetrahydrofuran solution (10 mL) of the compound of Reference Example 135 (798 mg) at room temperature, and the resulting mixture was stirred for 1 hour. Methanol was added to the reaction mixture, and the resulting mixture was concentrated under reduced pressure. The residue was purified by column chromatography (hexane/ethyl acetate) to obtain the title compound (542 mg).
LC-MS $[M+H]^+$/Rt (min): 284.2/0.829 (Method B)

Reference Example 148

tert-Butyl rac-(3R,4R,5R)-3,4-diazido-5-fluoropiperidine-1-carboxylate

[Formula 52]

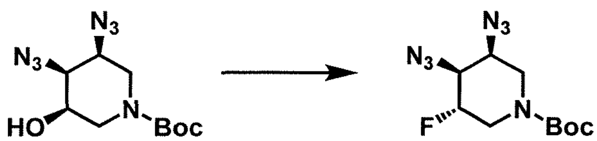

Bis(2-methoxyethyl)aminosulfur-trifluoride (0.548 mL) was added to a chloroform solution (10 mL) of the compound of Reference Example 147 (442 mg) at room temperature, and the resulting mixture was stirred at room temperature overnight. The reaction mixture was directly purified by column chromatography (hexane/ethyl acetate) to obtain the title compound (343 mg).
LC-MS $[M+H]^+$/Rt (min): 286.1/0.944 (Method B)

Reference Example 149

tert-Butyl 7-oxa-3-azaspiro[bicyclo[4.1.0]heptane-2,1'-cyclopropane]-3-carboxylate

[Formula 53]

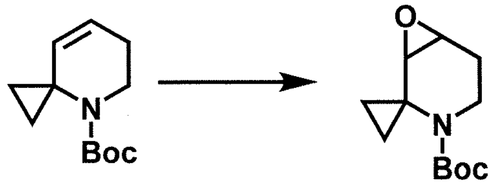

m-Chloroperbenzoic acid (592 mg) was added to a chloroform solution (10 mL) of the compound of Reference Example 95 (418 mg) at 0°C, and the resulting mixture was stirred at 0°C for 2 hours and at room temperature for 3 hours. Saturated aqueous sodium bicarbonate was added to the reaction mixture, and the resulting mixture was extracted with chloroform. The organic layer was washed with a mixed solution of saturated aqueous sodium bicarbonate and a saturated sodium thiosulfate aqueous solution, dried over sodium sulfate, then filtered, and concentrated under reduced pressure. The residue was purified by column chromatography (hexane/ethyl acetate) to obtain the title compound (333 mg).
LC-MS $[M+H]^+$/Rt (min): 226.1/0.979 (Method B)

Reference Example 150

tert-Butyl rac-(7R,8R)-8-azido-7-hydroxy-4-azaspiro[2.5]octane-4-carboxylate

[Formula 54]

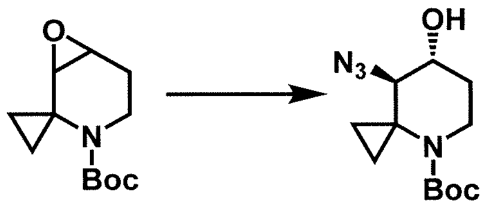

Sodium azide (115 mg) and ammonium chloride (95 mg) were added to a methanol/water solution (6 mL/4 mL) of the compound of Reference Example 149 (333 mg) at room temperature, and the resulting mixture was stirred at 65°C for 24 hours and at 100°C for 11 hours. Saturated aqueous sodium bicarbonate was added to the reaction mixture, and the resulting mixture was extracted with ethyl acetate. The organic layer was dried over sodium sulfate, then filtered, and concentrated under reduced pressure. The residue was purified by column chromatography (hexane/ethyl acetate) to obtain the title compound (100 mg). LC-MS $[M+H]^+$/Rt (min): 269.3/0.892 (Method A)

Reference Example 151

tert-Butyl rac-(6R,7S)-6,7-diamino-4-azaspiro[2.5]octane-4-carboxylate

[Formula 55]

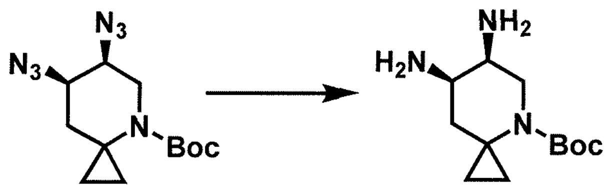

Triphenylphosphine (1.41 g) was added to a tetrahydrofuran-water solution (10:1) (18 mL) of the compound of Reference Example 136 (530 mg) at room temperature, and the resulting mixture was stirred at 60°C for 4 hours. The reaction mixture was concentrated under reduced pressure, and then the residue was purified by silica gel column chromatography (ethyl acetate/methanol) to obtain the title compound (380 mg).
LC-MS $[M+H]^+$/Rt (min): 242.2/0.241 (Method B)

Reference Examples 152 to 166

The compounds of Reference Examples 152 to 166 were obtained by using the corresponding raw material compounds according to the method described in Reference Example 44 or Reference Example 151.

[Table 36-1]

| Reference Example | Chemical structure | Physical property data |
|---|---|---|
| 152 | 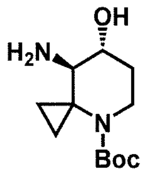 | LC-MS [M+H] + /Rt (min): 243.2/0.392 (Method B) |
| 153 | 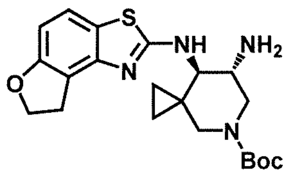 | LC-MS [M+H] + /Rt (min) : 417.3/0.766 (Method B) |
| 154 | 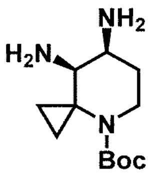 | LC-MS [M+H] + /Rt (min): 242.2/0.329 (Method B) |
| 155 | 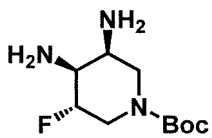 | LC-MS [M+H] + /Rt (min): 234.1/0.194 (Method B) |
| 156 | 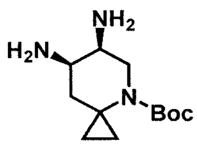 | LC-MS [M+H] + /Rt (min): 242.2/0.251 (Method B) |
| 157 | 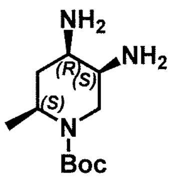 | Obtained as a mixture. |
| 158 | 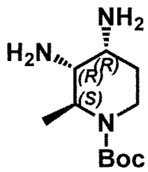 | LC-MS [M+H] + /Rt (min): 230.2/0.287, 230.2/0.287 (Method B) |
| 159 | 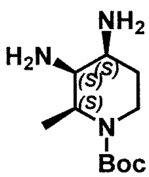 | LC-MS [M+H] + /Rt (min): 230.2/0.221 (Method B) |

(continued)

| Reference Example | Chemical structure | Physical property data |
|---|---|---|
| 160 | 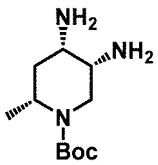 | Obtained as a mixture. |
| 161 | 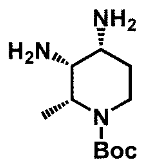 | LC-MS [M+H] + /Rt (min): 230.2/0.198, 230.2/0.198 (Method B) |

[Table 36-2]

| | | |
|---|---|---|
| 162 | 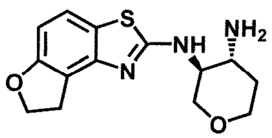 | LC-MS [M+H] +/Rt (min): 292.2/2.089 (Method C) |
| 163 | 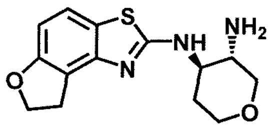 | LC-MS [M+H] +/Rt (min) : 292. 2/2.050 (Method C) |
| 164 | 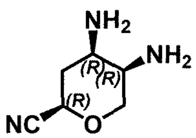 | $^1$H-NMR ($CDCl_3$) δ: 4.29-4.19 (1H, m), 3.93 (1H, dd, $J$ = 3.7, 11.9 Hz), 3. 56 (1H, dd, $J$ = 2.3, 11.9 Hz), 3.04-2.92 (1H, m), 2.83 (1H, dt, $J$ = 2. 4, 3.8 Hz), 2. 02-1.82 (2H, m), 1. 69-1. 38 (4H, m). |
| 165 | 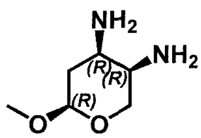 | LC-MS [M+H]+/Rt (min): 147.1/0.331 (Method C) |
| 166 | 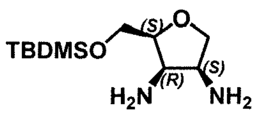 | LC-MS [M+H]+/Rt (min): 247. 2/1. 690 (Method C) |

Reference Example 167

Di-tert-butyl (3R,4R)-2,3,4,7-tetrahydrooxepin-3,4-diylbis rac-carbamate

[Formula 56]

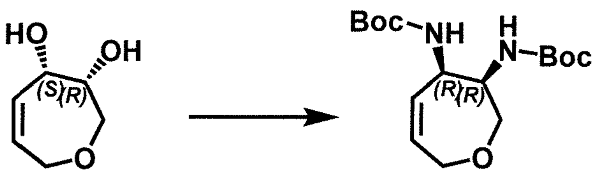

Triethylamine (6.33 mL) and methanesulfonyl chloride (1.77 mL) were added to a dichloromethane solution (8 mL) of the compound of Reference Example 110 (1.00 g) under ice cooling, and the resulting mixture was stirred under ice cooling for 2 hours. Water was added to the reaction mixture, and the resulting mixture was extracted with dichloromethane. The organic layer was washed with saturated aqueous sodium bicarbonate and saturated brine, then dried over sodium hydrogen sulfate, filtered, and then concentrated under reduced pressure. Sodium azide (1.94 g) was added to a dimethylformamide solution (12 mL) of the resulting crude product at room temperature, and the resulting mixture was stirred at 65°C for 18 hours. Water was added to the reaction mixture, and the resulting mixture was extracted with ethyl acetate. The organic layer was washed with water and saturated brine, then dried over sodium hydrogen sulfate, filtered, and then concentrated under reduced pressure. 10% Palladium carbon (0.10 g) was added to an ethanol solution (15 mL) of the resulting crude product at room temperature, and the resulting mixture was stirred at room temperature for 3 days in a 35 bar hydrogen atmosphere. The reaction mixture was filtered and then concentrated under reduced pressure. Triethylamine (1.59 mL) and $(Boc)_2O$ (2.64 mL) were added to a methanol solution (28 mL) of the resulting crude product at room temperature, and the resulting mixture was stirred at room temperature overnight. The reaction mixture was concentrated under reduced pressure and then purified by silica gel chromatography (hexane/ethyl acetate) to obtain the title compound (314 mg). $^1H$-NMR ($CDCl_3$) δ: 5.26 (2H, s), 4.08-3.90 (1H, m), 3.89-3.71 (2H, m), 3.72-3.50 (3H, m), 1.92-1.62 (4H, m), 1.46 (9H, s), 1.45 (9H, s).

Reference Examples 168 to 206

The compounds of Reference Examples 168 to 206 were obtained by using the corresponding raw material compounds according to the method described in Reference Example 1 and Reference Example 2 or Reference Example 24.

[Table 37-1]

| Reference Example | Chemical structure | Physical property data |
|---|---|---|
| 168 | 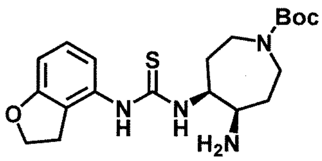 | LC-MS [M+H] + /Rt (min): 407.5/0.655 (Method B) |
| 169 | 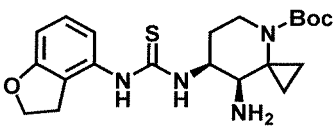 | LC-MS [M+H] + /Rt (min): 419. 3/0.711 (Method B) |
| 170 | 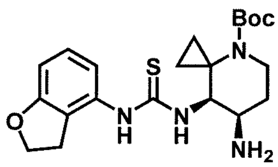 | LC-MS [M+H] + /Rt (min): 419. 3/0.693 (Method B) |
| 171 | 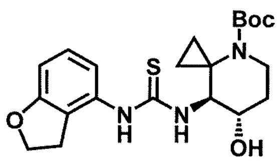 | LC-MS [M+H] + /Rt (min): 420. 3/0.838 (Method B) |
| 172 | 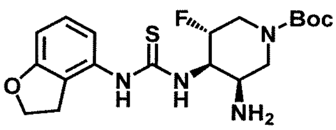 | LC-MS [M+H] + /Rt (min): 411.2/0.586 (Method B) |
| 173 | 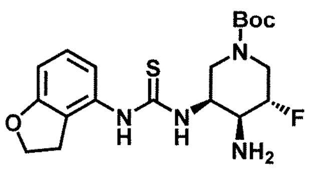 | LC-MS [M+H] + /Rt (min) : 411. 3/0.600 (Method B) |

(continued)

| Reference Example | Chemical structure | Physical property data |
|---|---|---|
| 174 | 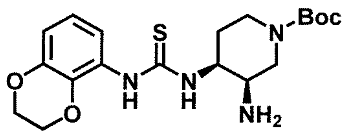 | LC-MS [M+H] + /Rt (min): 409.2/0.576 (Method B) |
| 175 | 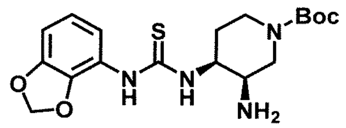 | LC-MS [M+H] + /Rt (min): 395. 2/0.659 (Method A) |
| 176 | 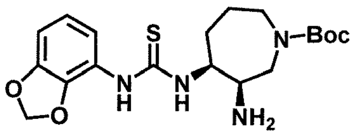 | LC-MS [M+H] + /Rt (min): 409.4/0.786 (Method A) |
| 177 | 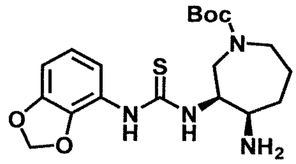 | LC-MS [M+H] + /Rt (min): 409.4/0.769 (Method A). |

[Table 37-2]

| | | |
|---|---|---|
| 178 | 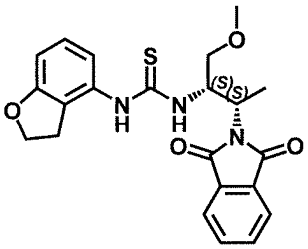 | LC-MS $[M+H]^+$/Rt (min): 426.2/0.846 (Method B) |
| 179 | 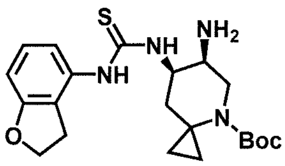 | LC-MS $[M+H]^+$/Rt (min): 419.2/0.682 (Method B) |
| 180 | 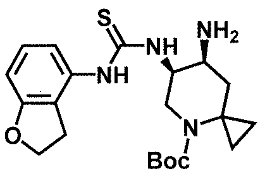 | LC-MS $[M+H]^+$/Rt (min): 419.2/0.682 (Method B) |
| 181 | 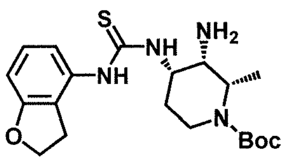 | LC-MS $[M+H]^+$/Rt (min): 407.2/0.591 (Method B) |
| 182 | 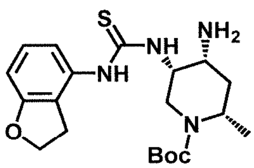 | LC-MS $[M+H]^+$/Rt (min): 407. 3/0. 675 (Method B) |

(continued)

| | | |
|---|---|---|
| 183 | 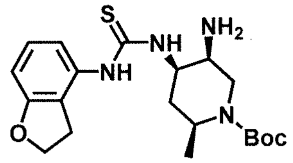 | Obtained as a mixture. |
| 184 | 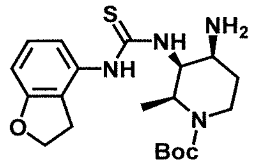 | LC-MS [M+H] $^{+}$ /Rt (min): 407. 3/0. 641, 407.3/0.678 (Method B) |
| 185 | 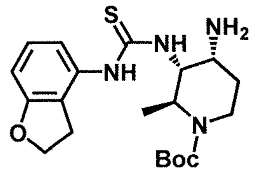 | LC-MS [M+H]$^{+}$/Rt (min): 407.2/0.627 (Method B) |
| 186 | 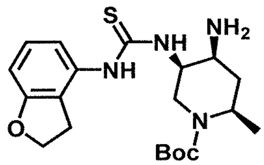 | LC-MS [M+H]$^{+}$/Rt (min): 407. 3/0. 624 (Method B) |
| 187 | 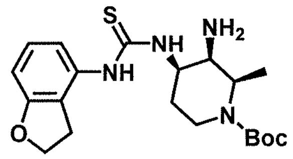 | LC-MS [M+H]$^{+}$/Rt (min): 407. 3/0.558 (Method B) |

[Table 37-3]

| | | |
|---|---|---|
| 188 | 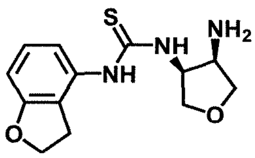 | LC-MS [M+H]$^{+}$/Rt (min): 280.1/1.653 (Method C) |
| 189 | 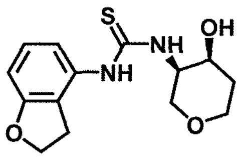 | LC-MS [M+H]$^{+}$/Rt (min): 295.2/2.502 (Method C) |
| 190 | 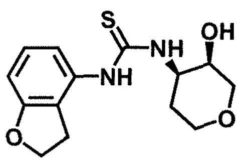 | LC-MS [M+H]$^{+}$/Rt (min): 295.2/2.436 (Method C) |
| 191 | 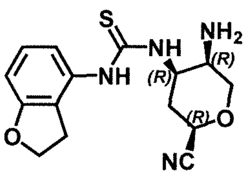 | Obtained as a mixture. |

(continued)

| | | |
|---|---|---|
| 192 | 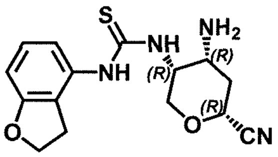 | LC-MS [M+H] + /Rt (min) : 319. 1/1. 507, 319. 1/1. 540 (Method C) |
| 193 | 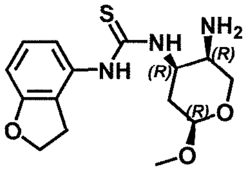 | Obtained as a mixture. |
| 194 | 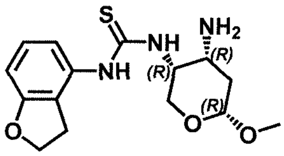 | LC-MS [M+H] + /Rt (min): 324.1/1.907. 324.1/1.857 (Method C) |
| 195 | 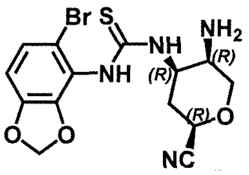 | LC-MS [M+2H] + /Rt (min): 401. 0/1. 623 (Method C) |
| 196 | 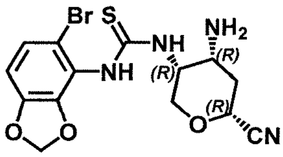 | LC-MS [M+2H] + /Rt (min): 401.0/1.623 (Method C) |
| 197 | 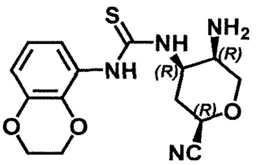 | LC-MS [M+H] + /Rt (min): 335.0/1.573 (Method C) |

[Table 37-4]

| | | |
|---|---|---|
| 198 | 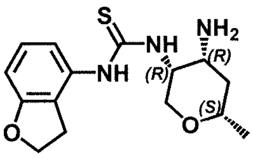 | LC-MS [M+H] + /Rt (min): 308.1/1.590 (Method C) |
| 199 | 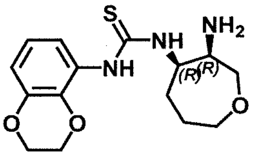 | $^1$H-NMR (DMSO-$d_6$) δ: 9. 17 (1H, s), 7.09 (1H, dd, *J* = 1. 6, 8. 0 Hz), 6. 76 (1H, t, *J* = 8.0 Hz), 6.69 (1H, dd, *J* = 1. 6, 8.0 Hz), 4.36-4.13 (6H, m), 3.76-3.60 (2H, m), 3.60-3.48 (1H, m), 3.37-3.27 (1H, m), 2.95-2.85 (1H, m), 1. 84-1. 49 (5H, m). |
| 200 | 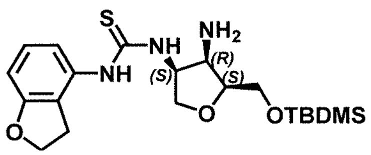 | LC-MS [M+H] + /Rt (min): 424.3/2.090 (Method C) |

(continued)

| | | |
|---|---|---|
| 201 | 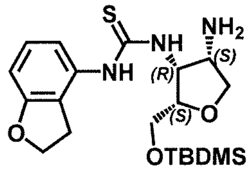 | LC-MS [M+H] + /Rt (min): 424.3/2.090 (Method C) |
| 202 | 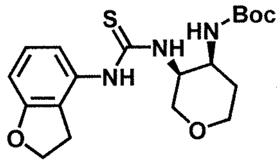 | LC-MS [M+H]+/Rt (min): 394.1/3.063 (Method C) |
| 203 | 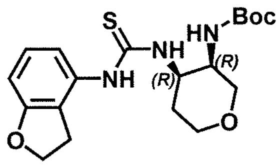 | $^1$H-NMR ($CDCl_3$) δ: 7.47 (1H, s), 7. 12 (1H, t, *J* = 8.0 Hz), 6. 73-6. 65 (2H, m), 5. 09 (1H, d, *J* = 8. 6 Hz), 4. 72-4. 52 (3H, m), 3.99-3.87 (2H, m), 3.87-3.76 (1H, m), 3.66-3.47 (2H, m), 3.30-3.06 (2H, m), 2.32-2.23 (1H ,m), 1.64-1.50 (1H, m), 1.31 (9H, s). |
| 204 | 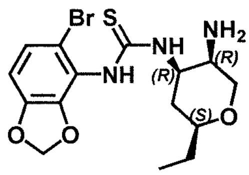 | LC-MS [M+2H] + /Rt (min): 404.0/1.790 (Method C) |
| 205 | 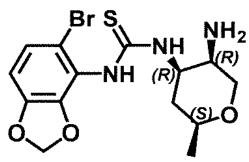 | LC-MS [M+2H] + /Rt (min): 390.1/1.673 (method C) |
| 206 | 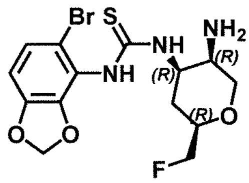 | LC-MS [M+2H] + /Rt (min): 408.1/1.673 (method C) |

Reference Example 207

tert-Butyl rac-{(3R,4S)-4-[(7,8-dihydrofuro[3,2-e][1,3]benzothiazol-2-yl)amino]oxolan-3-yl}carbamate

[Formula 57]

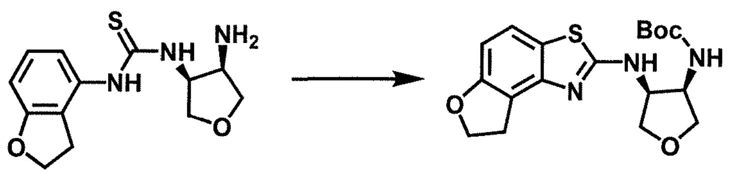

Benzyltrimethylammonium tribromide (1.30 g) was added to a chloroform suspension (30 mL) of the compound of Reference Example 188 (947 mg) at 0°C, and the resulting mixture was stirred at 0°C for 15 minutes. Triethylamine (0.945 mL) and $(Boc)_2O$ (814 mg) were added at 0°C, and the resulting mixture was stirred at room temperature for 3 hours. Saturated aqueous sodium bicarbonate was added to the reaction mixture, and the resulting mixture was extracted with chloroform. The organic layer was dried over sodium sulfate, filtered, and then concentrated under reduced pressure. The

residue was purified by silica gel chromatography (hexane/ethyl acetate) to obtain the title compound (688 mg). LC-MS $[M+H]^+$/Rt (min): 378.1/3.197 (Method C)

Reference Examples 208 to 248

The compounds of Reference Examples 208 to 248 were obtained by using the corresponding raw material compounds according to the methods described in Reference Example 8, Reference Example 26, and Reference Example 47.

[Table 38-1]

| Reference Example | Chemical structure | Physical property data |
|---|---|---|
| 208 | 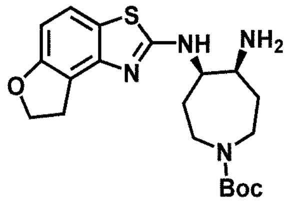 | LC-MS $[M+H]^+$/Rt (min): 405. 3/0. 770 (Method B) |
| 209 | 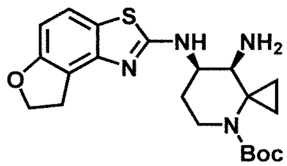 | LC-MS $[M+H]^+$ /Rt (min): 417.3/0.673 (Method B) |
| 210 | 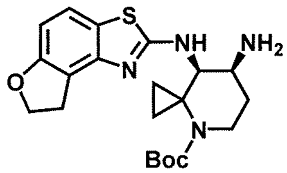 | LC-MS $[M+H]^+$ /Rt (min): 417.3/0.717 (Method B) |
| 211 | 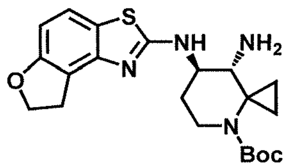 | LC-MS $[M+H]^+$ /Rt (min): 417.5/0.694 (Method B) |
| 212 | 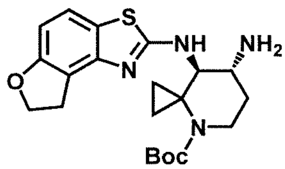 | LC-MS $[M+H]^+$ /Rt (min): 417.3/0.766 (Method B) |
| 213 | 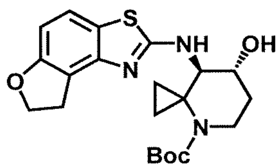 | LC-MS $[M+H]^+$ /Rt (min): 418.2/0.879 (Method B) |
| 214 | 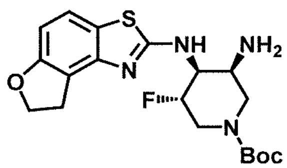 | LC-MS $[M+H]^+$ /Rt (min): 409.2/0.571 (Method B) |

(continued)

| Reference Example | Chemical structure | Physical property data |
|---|---|---|
| 215 | 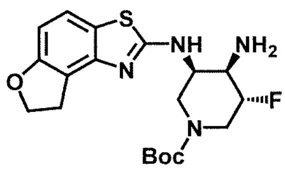 | LC-MS [M+H] + /Rt (min) : 409. 2/0.600 (Method B) |
| 216 | 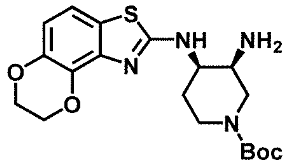 | LC-MS [M+H] + /Rt (min): 407. 2/0. 547 (Method B). |

[Table 38-2]

| | | |
|---|---|---|
| 217 | 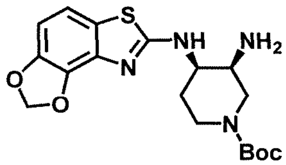 | $^{1}$H-NMR ($CDCl_3$) δ: 6.97 (1H, d, *J* = 7. 9 Hz), 6.64 (1H, d, *J* = 7.9 Hz), 6. 02-6.00 (3H, m), 4.03-4.00 (3H, m), 3.13 (1H, br s), 3.06-3.02 (1H, m), 2.87 (1H, t, *J* = 12.4 Hz), 2.02-1.98 (1H, m), 1.64-1.54 (3H, m), 1. 45 (9H, s). |
| 218 | 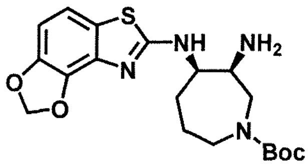 | LC-MS [M+H] + /Rt (min): 407.3/0.723 (Method A) |
| 219 | 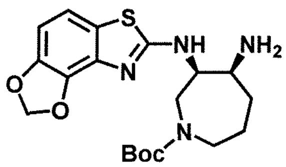 | LC-MS [M+H] + /Rt (min) : 407. 3/0. 678 (Method A) |
| 220 | 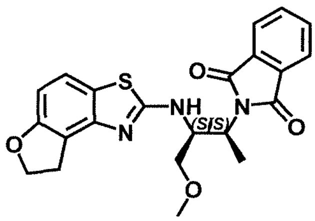 | LC-MS [M+H] + /Rt (min): 424.2/0.907 (Method B) |
| 221 | 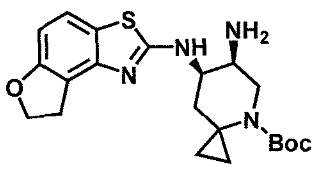 | LC-MS [M+H] + /Rt (min): 417. 3/0. 643 (Method B) |
| 222 | 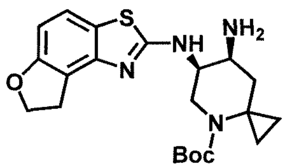 | LC-MS [M+H] + /Rt (min): 417. 3/0. 663 (Method B) |

(continued)

| | | |
|---|---|---|
| 223 | 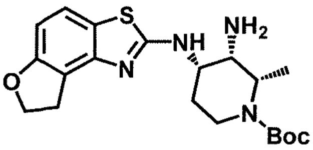 | LC-MS [M+H] + /Rt (min): 405. 3/0. 600 (Method B) |
| 224 | 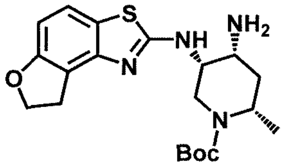 | LC-MS [M+H] + /Rt (min) : 405.2/0.663 (Method B) |
| 225 | 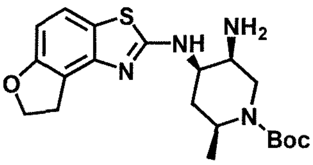 | Obtained as a mixture. |
| 226 | 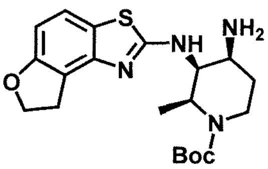 | LC-MS [M+H] + /Rt (min): 405. 3/0. 662, 405.3/0.689 (Method B) |

[Table 38-3]

| | | |
|---|---|---|
| 227 | 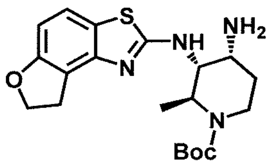 | LC-MS [M+H] + /Rt (min): 405. 3/0. 642 (Method B) |
| 228 | 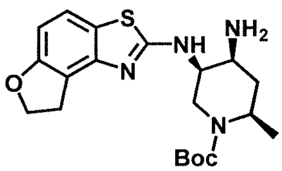 | LC-MS [M+H] + /Rt (min): 405. 3/0. 653 (Method B) |
| 229 | 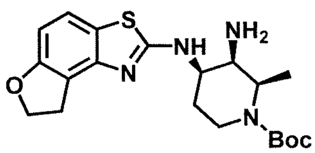 | LC-MS [M+H] + /Rt (min): 405. 3/0. 623 (Method B) |
| 230 | 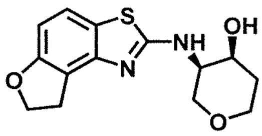 | LC-MS [M+H] + /Rt (min): 293. 2/2. 575 (Method C) |
| 231 | 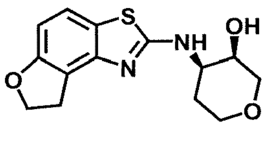 | LC-MS [M+H] + /Rt (min): 293. 2/2. 649 (Method C) |

(continued)

| | | |
|---|---|---|
| 232 | 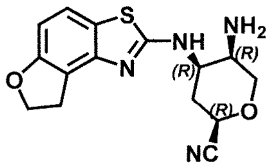 | LC-MS [M+H] + /Rt (min): 317.1/1.624 (Method C) |
| 233 | 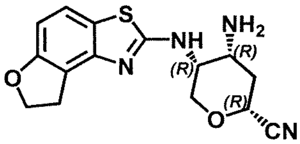 | LC-MS [M+H] + /Rt (min): 317.2/1.673 (Method C) |
| 234 | 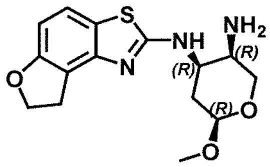 | LC-MS [M+H] + /Rt (min): 322. 1/2. 179 (Method C) |
| 235 | 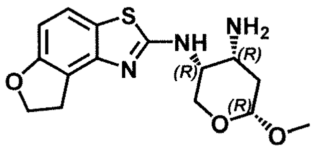 | LC-MS [M+H] + Rt (min): 322.2/2.169 (Method C) |
| 236 | 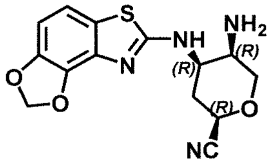 | LC-MS [M+H] + /Rt (m in) : 319. 1/1. 607 (Method C) |
| 237 | 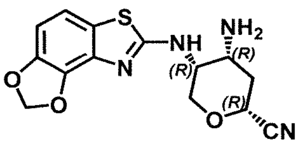 | LC-MS [M+H] + /Rt (min) : 319. 1/1. 673 (Method C) |

[Table 38-4]

| | | |
|---|---|---|
| 238 | 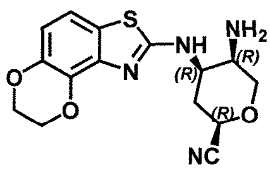 | LC-MS [M+H] + /Rt (min): 333.1/1.640 (Method C) |
| 239 | 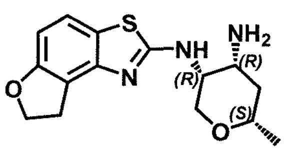 | LC-MS [M+H] + /Rt (min): 306.1/1.624 (Method C) |
| 240 | 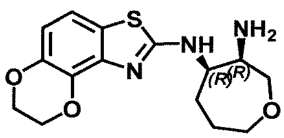 | LC-MS [M+H] + /Rt (min): 322.1/1.656 (Method C) |

(continued)

| | | |
|---|---|---|
| 241 | 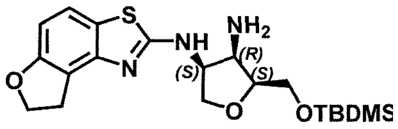 | LC-MS [M+H] + Rt (min): 422.3/2.290 (Method C) |
| 242 | 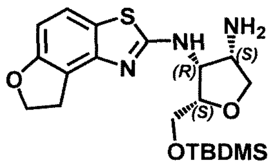 | LC-MS [M+H] + /Rt (min): 422. 3/2. 107 (Method C) |
| 243 | 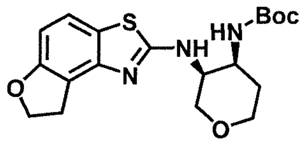 | LC-MS [M+H] + /Rt (min): 392.3/3.259 (Method C) |
| 244 | 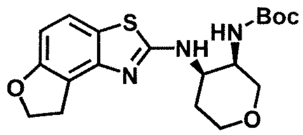 | $^1$H-NMR ($CDCl_3$) δ : 7.28 (1H, dd, *J* = 0.9, 8.4 Hz), 6. 62 (1H, d, *J* = 8. 4 Hz), 6.00 (1H, brs), 5. 24 (1H, brs), 4.64 (2H, t, *J* = 8. 7 Hz), 4. 08-3. 96 (3H, m), 3. 89-3. 84 (1H, m), 3. 69-3. 52 (2H, m), 3. 46-3. 33 (2H, m), 2. 28-2. 23 (1H, m), 1. 80-1. 65 (1H, m), 1. 47 (9H, s). |
| 245 | 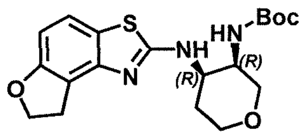 | $^1$H-NMR ($CDCl_3$) δ: 7.28 (1H, dd, *J* = 0.8, 8.3 Hz), 6. 62 (1H, d, *J* = 8. 3 Hz), 6.00 (1H, brs), 5.24 (1H, brs), 4.64 (2H, t, *J* = 8. 7 Hz), 4. 11-3.94 (3H, m), 3. 88-3. 86 (1H, m), 3. 70-3. 52 (2H, m), 3. 45-3. 35 (2H, m), 2. 28-2. 23 (1H, m), 1. 80-1. 63 (1H, m), 1. 47 (9H, s). |
| 246 | 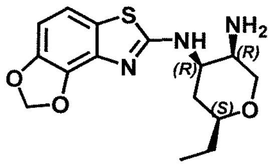 | LC-MS [M+H] + /Rt (min) : 322.2/1.740 (Method C) |

[Table 38-5]

| | | |
|---|---|---|
| 247 | 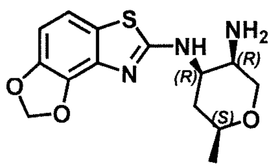 | LC-MS [M+H] + /Rt (min): 308.1/1.690 (Method C) |
| 248 | 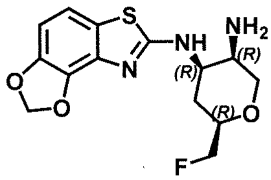 | LC-MS [M+H] + /Rt (min): 326.1/1.673 (Method C) |

Reference Examples 249 to 268

The compounds of Reference Examples 249 to 268 were obtained by using the corresponding raw material compounds according to the method described in Reference Example 14.

[Table 39-1]

| Reference Example | Chemical structure | Physical property data |
|---|---|---|
| 249 | 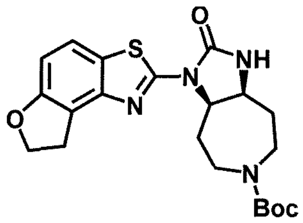 | LC-MS [M+H] $^+$/Rt (min): 431.2/1.097 (Method B) |
| 250 | 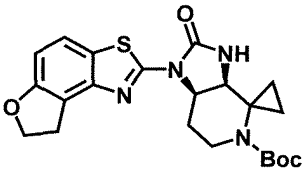 | LC-MS [M+H]$^+$/Rt (min): 443.2/0.975 (Method B) |
| 251 | 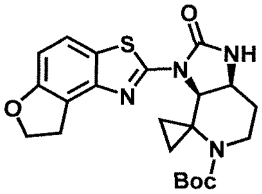 | LC-MS [M+H]$^+$/Rt (min): 443.2/0.904 (Method B) |
| 252 | 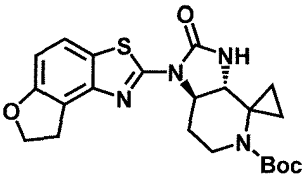 | LC-MS [M+H]$^+$/Rt (min): 443.3/1.089 (Method B) |
| 253 | 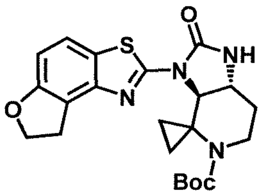 | LC-MS [M+H]$^+$/Rt (min): 443.3/1.047 (Method B) |
| 254 | 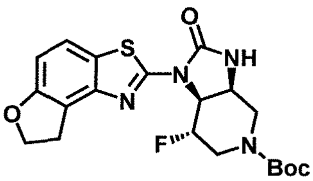 | LC-MS [M+H] $^+$ /Rt (min) : 435.2/0.912 (Method B) |
| 255 | 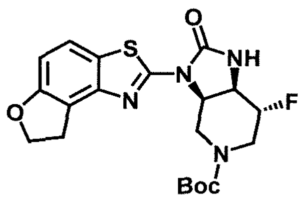 | LC-MS [M+H] $^+$ /Rt (min): 435.2/0.899 (Method B) |
| 256 | 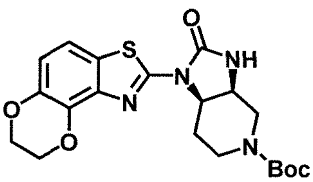 | LC-MS [M+H] $^+$/Rt (min): 433.2/0.854 (Method B) |

(continued)

| Reference Example | Chemical structure | Physical property data |
|---|---|---|
| 257 | 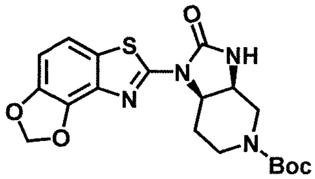 | LC-MS [M+H]$^+$/Rt (min): 419.2/0.917 (Method B) |

[Table 39-2]

| | | |
|---|---|---|
| 258 | 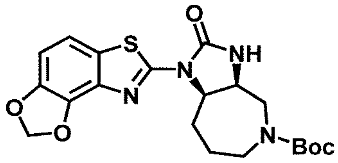 | LC-MS [M+H]$^+$/Rt (min): 433.3/1.000 (Method A |
| 259 | 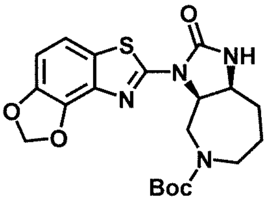 | LC-MS [M+H]$^+$/Rt (min): 433.3/0.915 (Method A) |
| 260 | 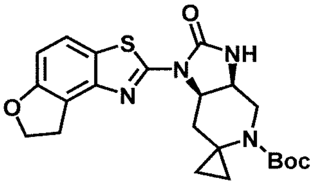 | LC-MS [M+H] $^+$/Rt (min): 443.3/0.976 (Method B) |
| 261 | 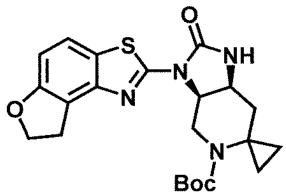 | LC-MS [M+H] $^+$/Rt (min): 443.3/0.999 (Method B) |
| 262 | 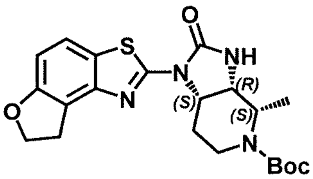 | LC-MS [M+H]$^+$/Rt (min): 431.2/0.982 (Method B) |
| 263 | 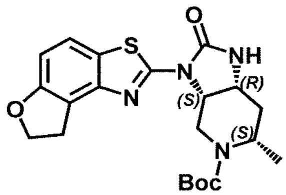 | LC-MS [M+H]$^+$/Rt (min): 431.2/0.999 (Method B) |

(continued)

| | | |
|---|---|---|
| 264 | 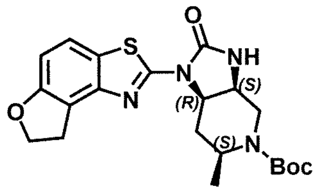 | LC-MS [M+H] $^+$/Rt (min): 431.3/0.997 (Method B) |
| 265 | 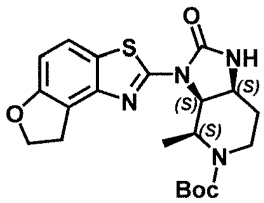 | LC-MS [M+H] $^+$/Rt (min): 431.3/0.963 (Method B) |
| 266 | 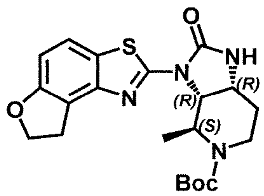 | LC-MS [M+H]$^+$/Rt (min): 431.2/1.011 (Method B) |

[Table 39-3]

| | | |
|---|---|---|
| 267 | 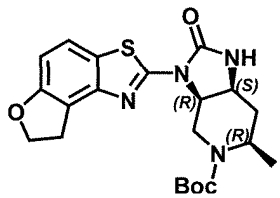 | LC-MS [M+H]$^+$/Rt (min): 431.2/0.997 (Method B) |
| 268 | 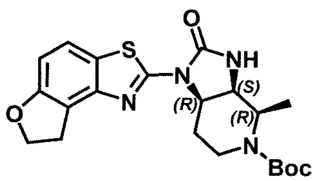 | LC-MS [M+H]$^+$/Rt (min): 431.2/0.981 (Method B) |

Reference Example 269

The compound of Reference Example 269 was obtained by using the corresponding raw material compounds according to the method described in Reference Example 28.

[Table 40]

| Reference Example | Chemical structure | Physical property data |
|---|---|---|
| 269 | 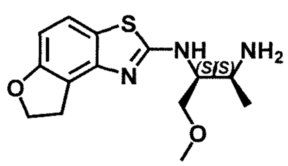 | LC-MS [M+H] $^+$ /Rt (min): 294.2/0.419 (Method B) |

Reference Example 270

tert-Butyl rac-(3aR,6aS)-3-(7,8-dihydrofuro[3,2-e][1,3]benzothiazol-2-yl)-2-oxohexahydro-1H-furo[3,4-d]imidazole-1-carboxylate

[Formula 58]

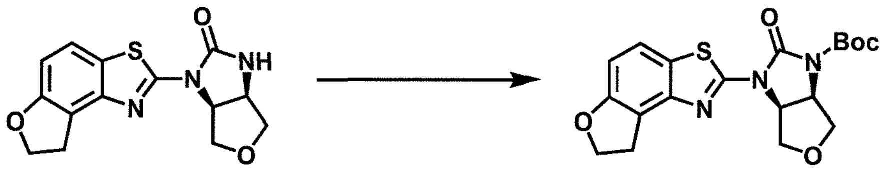

Sodium hydride (60% in oil, 130 mg) and $(Boc)_2O$ (0.33 mL) were added to a tetrahydrofuran solution (10 mL) of the compound of Example 119 (380 mg) at 0°C, and the resulting mixture was stirred at room temperature for 16 hours. Water was added to the reaction mixture, and the resulting mixture was extracted with ethyl acetate. The organic layer was dried over sodium sulfate, filtered, and then concentrated under reduced pressure. The residue was purified by silica gel chromatography (petroleum ether/ethyl acetate) to obtain the title compound (465 mg).
LC-MS $[M+H]^+$/Rt (min): 404.6/2.568 (Method D)

Reference Example 271

tert-Butyl (3aS,6aR)-3-(7,8-dihydrofuro[3,2-e][1,3]benzothiazol-2-yl)-2-oxohexahydro-1H-furo[3,4-d]imidazole-1-carboxylate

Reference Example 272

tert-Butyl (3aR,6aS)-3-(7,8-dihydrofuro[3,2-e][1,3]benzothiazol-2-yl)-2-oxohexahydro-1H-furo[3,4-d]imidazole-1-carboxylate

[Formula 59]

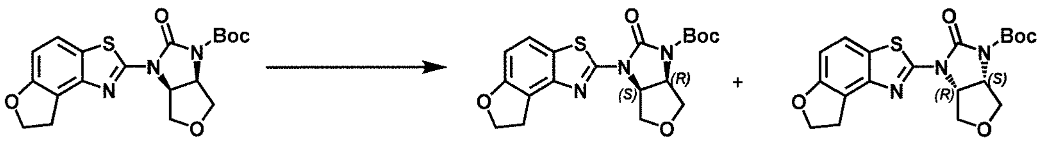

Ref.Example 270 Ref. Example 271 Ref.Example 272

The compound of Reference Example 270 (465 mg) was optically fractionated under the following conditions of supercritical fluid chromatography to obtain the title compounds (Reference Example 271: 160 mg-first peak: 2.01 min, Reference Example 272: 160 mg-second peak: 4.03 min).
Column: CHIRALCEL (R) OJ-H; Solvents: liquid A: carbon dioxide, liquid B: methanol; Mobile phase condition: A/B (%) = 55/45; Flow rate: 90 mL/min (during analysis: 3 mL/min); Detection UV: 214 nm; Column temperature: 30°C

[Table 41]

| Reference Example | Physical property data |
|---|---|
| 271 | LC-MS $[M+H]^+$/Rt (min): 404.5/2.542 (Method D) |
| 272 | LC-MS $[M+H]^+$/Rt (min): 404.5/2.542 (Method D) |

Reference Example 273

tert-Butyl { (3R,4R)-4-[(7,8-dihydrofuro[3,2-e][1,3]benzothiazol-2-yl)amino]-6,6-dimethyloxan-3-yl}carbamate

[Formula 60]

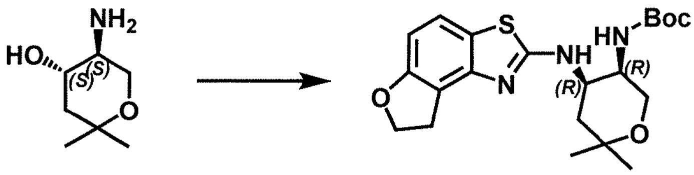

Triethylamine (0.065 mL) and $(Boc)_2O$ (0.107 mL) were added to a dichloromethane solution (4 mL) of (4S,5S)-5-amino-2,2-dimethyltetrahydro-2H-pyran-4-ol) (56.0 mg) at room temperature, and the resulting mixture was stirred at room temperature overnight. The reaction mixture was concentrated under reduced pressure and then purified by silica gel chromatography (hexane/ethyl acetate) to obtain a Boc form (70.0 mg). Triethylamine (0.048 mL) and methanesulfonyl chloride (0.024 mL) were added to a dichloromethane solution (6 mL) of the obtained Boc form (70.0 mg) under ice cooling, and the resulting mixture was stirred at room temperature for 2 hours. Water was added to the reaction mixture, and the resulting mixture was extracted with ethyl acetate. The organic layer was dried over sodium sulfate, then filtered, and concentrated under reduced pressure to obtain a crude product (106 mg). Sodium azide (63.9 mg) and sodium acetate (53.8 mg) were added to a dimethylformamide solution (5 mL) of the obtained crude product (106 mg) at room temperature, and the resulting mixture was stirred at 80°C for 3 days. Water was added to the reaction mixture, and the resulting mixture was extracted with ethyl acetate. The organic layer was washed with saturated brine, then dried over sodium sulfate, filtered, and concentrated under reduced pressure to obtain a crude product. A catalytic amount of 10% palladium carbon was added to a methanol solution (10 mL) of the obtained crude product at room temperature, and the resulting mixture was stirred at room temperature overnight in a normal pressure hydrogen atmosphere. The reaction mixture was filtered through cerite and then concentrated under reduced pressure to obtain a crude product (84.0 mg). 4-Isothiocyanato-2,3-dihydrobenzofuran (67.0 mg) was added to a tetrahydrofuran solution (3 mL) of the obtained crude product (84.0 mg) at room temperature, and the resulting mixture was stirred at room temperature overnight. The reaction mixture was concentrated under reduced pressure and then purified by silica gel chromatography (hexane/ethyl acetate) to obtain the title compound (18.0 mg).
LC-MS $[M+H]^+$/Rt (min): 420.2/2.273 (Method C)

Reference Example 274

The compound of Reference Example 274 was obtained by using the corresponding raw material compounds according to the method described in Reference Example 273.

[Table 42]

| Reference Example | Chemical structure | Physical property data |
|---|---|---|
| **274** | 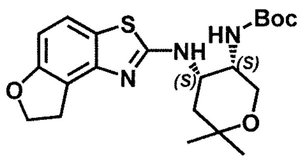 | **LC-MS $[M+H]^+$ /Rt (min) : 420.2/2.273 (Method C)** |

Reference Example 275

1,4-Anhydro-5-O-[tert-butyl(dimethyl)silyl]-2,3-bis-O-(trifluoromethanesulfonyl)-D-ribitol

[Formula 61]

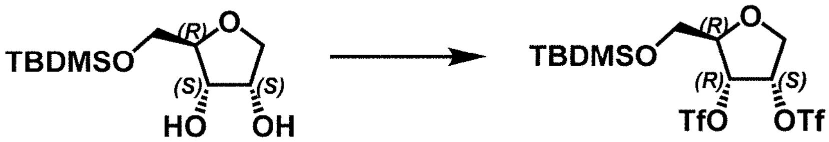

Pyridine (7.81 mL) and trifluoromethanesulfonic anhydride (8.16 mL) were added to a dichloromethane solution (322 mL) of (2R,3S,4S)-2-[{(tertbutyldimethylsilyl)oxy}methyl]tetrahydrofuran-3,4-diol (4.00 g) under ice cooling, and the resulting mixture was stirred under ice cooling for 1 hour. Ice water was added to the reaction mixture, and the resulting mixture was extracted with chloroform. The organic layer was dried over sodium hydrogen sulfate, filtered, and then concentrated under reduced pressure. The residue was purified by silica gel chromatography (hexane/ethyl acetate) to obtain the title compound (7.04 g).
$^1$H-NMR ($CDCl_3$) δ: 5.47-5.36 (2H, s), 4.34-4.25 (2H, m), 4.19-4.00 (1H, m), 3.87-3.70 (2H, m), 0.90 (9H, s), 0.09 (3H, s), 0.08 (3H, s).

Reference Example 276

2,5-Anhydro-1-O-[tert-butyl(dimethyl)silyl]-D-arabinitol

[Formula 62]

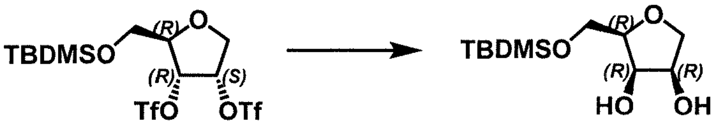

18-Crown-6 (7.82 g) and cesium acetate (7.91 g) were added to a dimethylformamide solution (92 mL) of the compound of Reference Example 275 (7.04 g) at room temperature, and the resulting mixture was stirred at 40°C for 13 hours. Cesium acetate (7.91 g) was added, and the resulting mixture was further stirred at 40°C for 3 hours. The reaction mixture was concentrated under reduced pressure, then ethyl acetate and water were added to the residue, and the resulting mixture was extracted with ethyl acetate. The organic layer was washed with saturated brine, then dried over sodium hydrogen sulfate, filtered, and concentrated under reduced pressure. The residue was purified by silica gel chromatography (hexane/ethyl acetate) to obtain a monoacetate form (2.78 g) as a mixture. The obtained monoacetate form (2.78 g) was dissolved in a 7 N ammonia methanol solution (70 mL), and the resulting solution was stirred at room temperature for 10 hours. The reaction mixture was concentrated under reduced pressure, and then the residue was purified by silica gel chromatography (hexane/ethyl acetate) to obtain the title compound (2.15 g). $^1$H-NMR (DMSO-$d_6$) δ: 4.80 (1H, d, J = 6.1 Hz), 4.58 (1H, d, J = 4.6 Hz), 4.17-4.07 (1H, m), 3.97-3.90 (1H, m), 3.82-3.74 (2H, m), 3.30 (1H, dd, J = 6.7, 8.2 Hz), 3.66-3.57 (1H, m), 3.45 (1H, dd, J = 6.8, 8.2 Hz), 0.86 (9H, s), 0.03 (3H, s), 0.03 (3H, s).

Reference Example 277

The compound of Reference Example 277 was obtained by using the corresponding raw material compounds according to the method described in Reference Example 38.

[Table 43]

| Reference Example | Chemical structure | Physical property data |
|---|---|---|
| 277 | 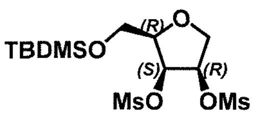 | **$^1$H-NMR ($CDCl_3$) δ: 5.32-5.22 (2H, m), 4.19-4. 06 (2H, m), 3.97 (1H, dd, *J* = 6.8, 9.5 Hz), 3.90-3.76 (2H, m), 3.15 (6H, s), 0.89 (9H, s), 0.09 (3H, s), 0.08 (3H, s).** |

Reference Example 278

The compound of Reference Example 278 was obtained by using the corresponding raw material compounds according to the method described in Reference Example 42.

[Table 44]

| Reference Example | Chemical structure | Physical property data |
|---|---|---|
| 278 | 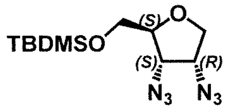 | $^1$H-NMR ($CDCl_3$) $\delta$: 4. 19-4. 04 (3H, m), 3.94-3.89 (1H, m), 3.85-3.71 (3H, m), 0.90 (9H, s), 0.09 (3H, s), 0.08 (3H, s). |

Reference Example 279

The compound of Reference Example 279 was obtained by using the corresponding raw material compounds according to the method described in Reference Example 44.

[Table 45]

| Reference Example | Chemical structure | Physical property data |
|---|---|---|
| 279 | 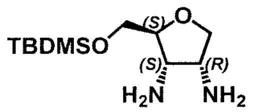 | LC-MS [M+H] $^+$ /Rt (min): 247.2/1.723 (Method C) |

Reference Examples 280 and 281

The compounds of Reference Examples 280 and 281 were obtained by using the corresponding raw material compounds according to the method described in Reference Example 1 and Reference Example 2.

[Table 46]

| Reference Example | Chemical structure | Physical property data |
|---|---|---|
| 280 | 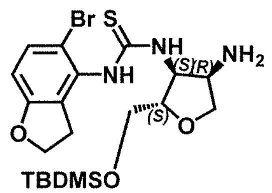 | LC-MS [M+2H] $^+$ /Rt (min): 504.2/1.907 (Method C) |
| 281 | 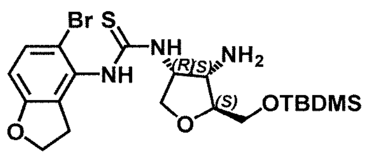 | LC-MS [M+2H] $^+$ /Rt (min): 504.2/1.973 (Method C) |

Reference Examples 282 and 283

The compounds of Reference Examples 282 and 283 were obtained by using the corresponding raw material compounds according to the method described in Reference Example 47.

[Table 47]

| Reference Example | Chemical structure | Physical property data |
|---|---|---|
| 282 | 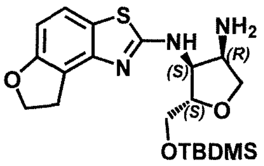 | LC-MS [M+H] + /Rt (min): 422. 2/2. 223 (Method C) |
| 283 | 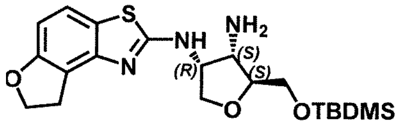 | LC-MS [M+H] +/Rt (min): 422. 2/2. 223 (Method C) |

Reference Examples 284 and 285

The compounds of Reference Examples 284 and 285 were obtained by using the corresponding raw material compounds according to the method described in Reference Example 38.

[Table 48]

| Reference Example | Chemical structure | Physical property data |
|---|---|---|
| 284 | 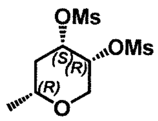 | $^1$H-NMR ($CDCl_3$) $\delta$: 4. 96-4. 91 (1H, m), 4.91-4.83 (1H, m), 4.24 (1H, dd, $J$ = 2. 3, 13.6 Hz), 3.62 (1H, dd, $J$ = 1. 0, 13.6 Hz), 3.59-3.51 (1H, m), 3.14 (3H, s), 3.12 (3H, s), 2.07-1.86 (2H, m), 1.29 (3H, d, $J$ = 6. 2 Hz). |
| 285 | 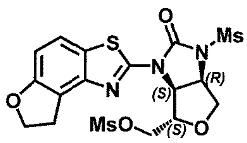 | LC-MS [M+H] + /Rt (min): 490.1/2.290 (Method C) |

Reference Example 286

The compound of Reference Example 286 was obtained by using the corresponding raw material compounds according to the method described in Reference Example 42.

[Table 49]

| Reference Example | Chemical structure | Physical property data |
|---|---|---|
| 286 | 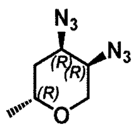 | $^1$H-NMR ($CDCl_3$) $\delta$ : 4.08 (1H, dd, $J$ = 2. 8, 4. 1 Hz), 3.84 (1H, ddd, $J$ = 1. 4, 4.6, 10.9 Hz), 3. 77-3. 65 (2H, m), 3.60 (1H, ddd, $J$ = 3. 2, 4. 6, 10.7 Hz), 1. 91 (1H, ddd, $J$ = 2.1, 3.6, 14.3 Hz), 1.65-1.55 (1H, m), 1. 17 (3H, d, $J$ = 6. 2 Hz). |

Reference Example 287

The compound of Reference Example 287 was obtained by using the corresponding raw material compounds according to the method described in Reference Example 44.

[Table 50]

| Reference Example | Chemical structure | Physical property data |
|---|---|---|
| 287 | 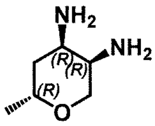 | $^1$H-NMR ($CDCl_3$) δ : 3. 84-3. 72 (1H, m), 3. 64-3. 55 (1H, m), 3.45 (1H, dd, *J* = 10. 5, 11. 2 Hz), 3. 25-3. 22 (1H, m), 2. 92 (1H, ddd, *J* = 3. 7, 4. 8, 10.5 Hz), 1. 73-1. 56 (6H, m), 1. 16 (3H, d, *J* = 6. 3 Hz). |

Reference Example 288

The compound of Reference Example 288 was obtained by using the corresponding raw material compounds according to the method described in Reference Example 1 and Reference Example 2.

[Table 51]

| Reference Example | Chemical structure | Physical property data |
|---|---|---|
| 288 | 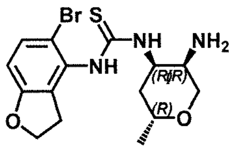 | LC-MS [M+2H] $^+$ /Rt (min): 388.1/1.740 (Method C) |

Reference Example 289

The compound of Reference Example 289 was obtained by using the corresponding raw material compounds according to the method described in Reference Example 47.

[Table 52]

| Reference Example | Chemical structure | Physical property data |
|---|---|---|
| 289 | 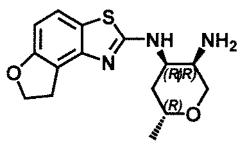 | LC-MS [M+H] $^+$ /Rt (min): 306.1/1.673 (Method C) |

Reference Example 290

The compound of Reference Example 290 was obtained by using the corresponding raw material compounds according to the method described in Reference Example 77.

[Table 53]

| Reference Example | Chemical structure | Physical property data |
|---|---|---|
| 290 | 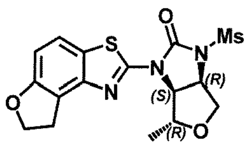 | LC-MS [M+H] $^+$ /Rt (min): 396.1/2.340 (Method C) |

Example 1

cis-3-(7,8-Dihydrofuro[3,2-e][1,3]benzothiazol-2-yl)octahydro-2H-imidazo[4,5-c]pyridin-2-one

[Formula 63]

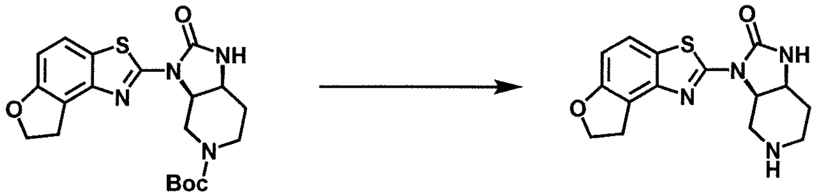

Trifluoroacetic acid (3 mL) was added to the compound of Reference Example 14 (114 mg), and the resulting mixture was stirred for 11 minutes. The reaction mixture was concentrated under reduced pressure, and the residue was purified by amino silica gel column chromatography (chloroform/methanol) to obtain the title compound (62 mg).
LC-MS $[M+H]^+$/Rt (min): 317.1/0.399 (Method B) ; $^1$H-NMR (DMSO-$d_6$) $\delta$: 7.73 (1H, s), 7.57 (1H, d, J = 8.5 Hz), 6.72 (1H, d, J = 8.5 Hz), 4.64-4.56 (2H, m), 4.52-4.44 (1H, m), 4.04-3.99 (1H, m), 3.53-3.46 (1H, m), 3.39-3.33 (2H, m), 2.77-2.69 (1H, m), 2.62-2.51 (2H, m), 2.30-2.19 (1H, m), 1.81-1.69 (2H, m).

Examples 2 to 6

The compounds of Examples 2 to 6 were obtained by using the corresponding raw material compounds according to the method described in Example 1.

[Table 54]

| Example | Chemical structure | Physical property data |
|---|---|---|
| 2 | 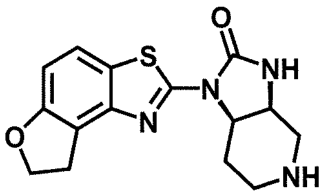 | LC-MS $[M+H]^+$/Rt (min): 317.1/0.432 (Method B); $^1$H-NMR (DMSO-$d_6$) $\delta$ : 7.73 (1H, s), 7. 57 (1H, d, $J$ = 7.9 Hz), 6.72 (1H, d, $J$ = 7.9 Hz), 4.66-4.55 (3H, m), 4.11-4.05 (1H, m), 3. 74-3. 70 (1H, m), 3. 35 (2H, t, $J$ = 8. 9 Hz), 2.98-2.92 (1H, m), 2.84-2.77 (2H, m), 2. 50-2. 41 (1H, m), 2.33-2.25 (1H, m), 1.58-1.47 (1H, m). |
| 3 | 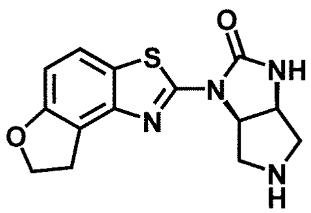 | LC-MS $[M+H]^+$/Rt (min): 303.1/0.533 (Method A); $^1$H-NMR (400 MHz, DMSO-$d_6$) $\delta$: 8.01 (1H, s), 7.57 (1H, d, $J$ = 8.5 Hz), 6.72 (1H, d, $J$ = 8.5 Hz), 5.36 (1H, brs), 4.94 (1H, dd, $J$ = 4. 9, 7.9 Hz), 4.60 (2H, t, $J$ = 9. 2 Hz), 4.28 (1H, dd, $J$ = 5. 2, 8.2 Hz), 3.42-3.31 (5H, m), 2.99-2.93 (2H, m), 2.81 (1H, dd, $J$ = 5. 2, 12.5 Hz). |
| 4 | 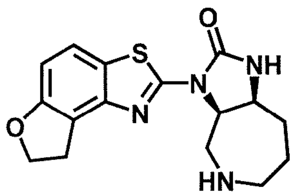 | LC-MS $[M+H]^+$/Rt (min): 331.2/0.454 (Method B); $^1$H-NMR ($CDCl_3$, 50°C) $\delta$: 7.44 (1H, d, $J$ = 8. 6 Hz), 6.75 (1H, d, $J$ = 8.6 Hz), 4. 87-4.77 (2H, m), 4.68-4.60 (2H, m), 4.29-4.21 (1H, m), 3.55-3.39 (4H, m), 2.91-2.84 (2H, m), 2.02-1.83 (3H, m), 1.62-1.51 (2H, m). |
| 5 | 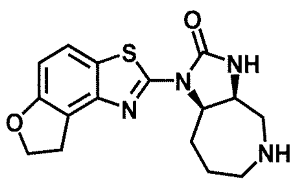 | LC-MS [M+H] $^+$/Rt (min): 331.2/0.523 (Method B); $^1$H-NMR ($CDCl_3$, 50°C) $\delta$ : 7.45 (1H, d, $J$ = 8. 6 Hz), 6. 75 (1H, d, $J$ = 8. 6 Hz), 4. 94-4.88 (1H, m), 4.83 (1H, s), 4.70-4.60 (2H, m), 4. 18-4. 10 (1H, m), 3.45 (2H, t, $J$ = 8.9 Hz), 3.15-3.08 (1H, m), 2.97-2.81 (3H, m), 2.42-2.27 (2H, m), 1.76-1.53 (3H, m). |

(continued)

| Example | Chemical structure | Physical property data |
|---|---|---|
| 6 | 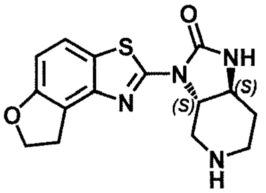 | LC-MS $[M+H]^+$/Rt (min): 317.2/1.275 (Method D); $^1$H-NMR (DMSO-$d_6$) $\delta$ : 7.94 (1H, s), 7.60 (1H, d, $J$ = 8. 3 Hz), 6. 76 (1H, d, $J$ = 8. 3 Hz), 4.61 (2H, dt, $J$ = 2. 4, 8. 7 Hz), 4. 25 (1H, dd, $J$ = 3. 5, 11. 8 Hz), 4. 12 (1H, brs), 3.67-3.57 (1H, m), 3.40-3.34 (2H, m), 3.17 (1H, d, $J$ = 2.8 Hz), 3.01 (1H, dd, $J$ = 2. 4, 13. 4 Hz), 2. 80-2. 71 (1H, m), 2.62-2.53 (1H, m), 1. 94-1. 83 (1H, m), 1.59-1.49 (1H, m). |

Example 7

cis-3-(7,8-Dihydrofuro[3,2-e][1,3]benzothiazol-2-yl)-5-methyloctahydro-2H-imidazo[4,5-c]pyridin-2-one

[Formula 64]

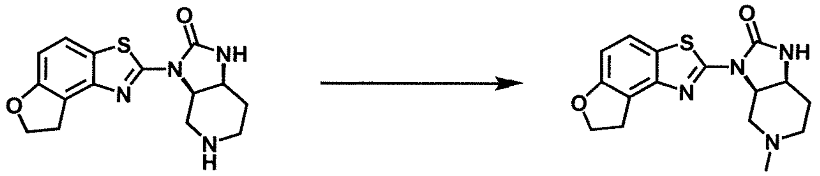

A 37% formaldehyde solution (0.02 mL) and methanol (1 mL) were added to a methanol (2 mL) solution of the compound of Example 1 (21.8 mg), and the resulting mixture was stirred for 10 minutes. Sodium triacetoxyborohydride (50 mg) was added, and the resulting mixture was stirred for 20 minutes. Saturated aqueous sodium bicarbonate was added to the reaction mixture, and the resulting mixture was extracted with chloroform. The organic layer was dried over sodium sulfate, filtered, and then concentrated under reduced pressure. The residue was purified by silica gel column chromatography (chloroform/methanol). Methanol was added to the resulting solid, the resulting mixture was stirred, and the solid was collected by filtration and then dried to obtain the title compound (12 mg).
LC-MS $[M+H]^+$/Rt (min): 331.1/0.412 (Method B); $^1$H-NMR (DMSO-$d_6$) $\delta$: 7.73 (1H, s), 7.58 (1H, d, J = 7.9 Hz), 6.73 (1H, d, J = 7.9 Hz), 4.69-4.56 (3H, m), 4.00-3.94 (1H, m), 3.43-3.27 (3H, m), 2.56-2.51 (1H, m), 2.16 (3H, s), 2.12-2.04 (1H, m), 1.96-1.75 (3H, m).

Example 8

cis-3-(7,8-Dihydrofuro[3,2-e][1,3]benzothiazol-2-yl)-5-(2-methoxyethyl)octahydro-2H-imidazo[4,5-c]pyridin-2-one

[Formula 65]

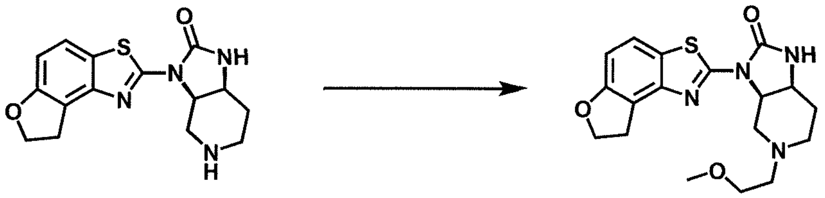

2-Chloroethylmethyl ether (0.036 mL), potassium iodide (66 mg), and potassium carbonate (111 mg) were added to a suspension of the compound of Example 1 (32 mg) in acetonitrile (1 mL), and the resulting mixture was stirred at 80°C for 15 hours. Saturated aqueous sodium bicarbonate was added to the reaction mixture, and the resulting mixture was extracted with chloroform. The organic layer was dried over sodium sulfate, filtered, and then concentrated under reduced pressure. The residue was purified by reverse phase column chromatography (0.035% trifluoroacetic acid in acetonitrile/0.05% trifluoroacetic acid in water) and amino silica gel column chromatography (chloroform/methanol). Ethyl acetate

was added to the resulting solid, the resulting mixture was stirred, and the solid was collected by filtration and then dried to obtain the title compound (19 mg).
LC-MS [M+H]+/Rt (min): 375.1/0.463 (Method B); $^1$H-NMR ($CDCl_3$) δ: 7.45 (1H, d, J = 8.2 Hz), 6.76 (1H, d, J = 8.2 Hz), 4.92-4.85 (1H, m), 4.77 (1H, s), 4.70-4.61 (2H, m), 4.09-4.05 (1H, m), 3.69-3.63 (1H, m), 3.51-3.38 (4H, m), 3.34 (3H, s), 2.82-2.76 (1H, m), 2.65-2.54 (2H, m), 2.34-2.26 (1H, m), 2.20-2.02 (2H, m), 1.91-1.87 (1H, m).

Examples 9 to 19

The compounds of Examples 9 to 19 were obtained by using the corresponding raw material compounds according to the method described in Example 7 or Example 8.

[Table 55-1]

| Example | Chemical structure | Physical property data |
|---|---|---|
| 9 | 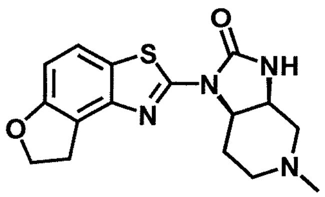 | LC-MS [M+H]+/Rt (min): 331.1/0.432 (Method B); $^1$H-NMR (DMSO-$d_6$) δ: 7.84 (1H, s), 7.57 (1H, d, *J* = 8.2 Hz), 6.72 (1H, d, *J* = 8.2 Hz), 4. 64-4. 49 (3H, m), 3.90-3.87 (1H, m), 3.35 (2H, t, *J* = 8. 9 Hz), 2. 79-2. 73 (1H, m), 2.59-2.52 (1H, m), 2.41-2.24 (2H, m), 2.17 (3H, s), 2.03-1.95 (1H, m), 1. 84-1. 73 (1H, m). |
| 10 | 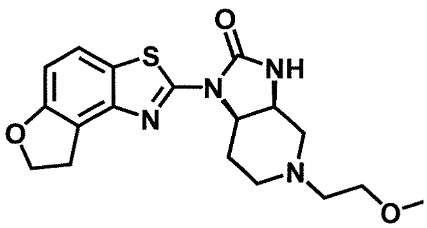 | LC-MS [M+H]+/Rt (min): 375.1/0.444 (Method B) ; $^1$H-NMR (DMSO-$d_6$) δ: 7.83 (1H, s), 7. 57 (1H, d, *J* = 8. 2 Hz), 6.72 (1H, d, *J* = 8.2 Hz), 4. 64-4. 51 (3H, m), 3.89-3.86 (1H, m), 3.42 (2H, t, *J* = 5. 8 Hz), 3. 35 (2H, t, *J* = 8.9 Hz), 3.23 (3H, s), 2.87-2.82 (1H, m), 2.69-2.63 (1H, m), 2.55-2.49 (2H, m), 2.47-2.42 (1H, m), 2. 37-2. 32 (1H, m), 2.18-2.11 (1H, m), 1. 87-1. 77 (1H, m). |
| 11 | 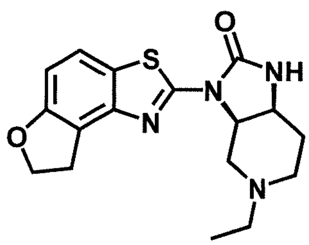 | LC-MS [M+H]+/Rt (min): 345.2/0.449 (Method B) ; $^1$H-NMR ($CDCl_3$) δ: 7.47 (1H, d, *J* = 8. 5 Hz), 6. 78 (1H, d, *J* = 8.5 Hz), 4. 92-4. 82 (2H, m), 4.73-4.64 (2H, m), 4.13-4.07 (1H, m), 3. 66-3. 60 (1H, m), 3. 49-3. 42 (2H, m), 2.79-2.71 (1H, m), 2.51-2.42 (2H, m), 2.31-2.23 (1H, m), 2.20-2.01 (2H, m), 2.00-1.89 (1H, m), 1. 08 (3H, t, *J* = 7. 1 Hz). |
| 12 | 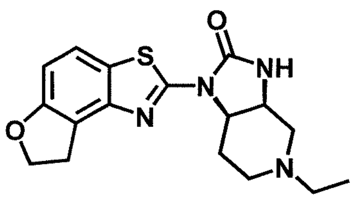 | LC-MS [M+H]+/Rt (min): 345.1/0.448 (Method B) ; $^1$H-NMR ($CDCl_3$) δ: 7.47 (1H, d, *J* = 8. 5 Hz), 6. 77 (1H, d, *J* = 8.5 Hz), 5.36 (1H, s), 4. 78-4. 63 (3H, m), 4. 04-3. 98 (1H, m), 3. 50-3.42 (2H, m), 3.02-2.95 (1H, m), 2.81-2.74 (1H, m), 2.65-2.40 (4H, m), 2.18-1.99 (2H, m), 1.11 (3H, t, *J* = 7. 1 Hz). |

[Table 55-2]

| | | |
|---|---|---|
| 13 | 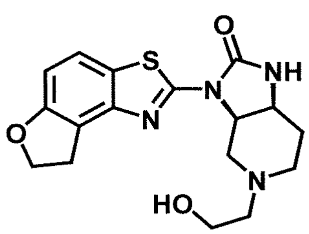 | LC-MS [M+H]+/Rt (min): 361.2/0.428 (Method B) ; $^1$H-NMR ($CDCl_3$) δ: 7.47 (1H, d, *J* = 8. 5 Hz), 6. 79 (1H, d, *J* = 8.5 Hz), 4.87-4.82 (2H, m), 4.73-4.64 (2H, m), 4.13-4.09 (1H, m), 3.67-3.59 (3H, m), 3.53-3.45 (2H, m), 2.78-2.74 (1H, m), 2. 65-2. 54 (3H, m), 2.47-2.35 (2H, m), 2.12-2.03 (1H, m), 1.98-1.93 (1H, m) . |

(continued)

| | | |
|---|---|---|
| 14 | 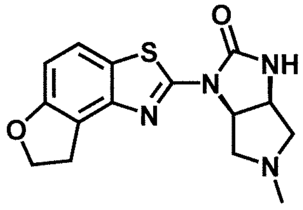 | LC-MS [M+H]$^+$/Rt (min): 317.1/0.532 (Method A); $^1$H-NMR (400 MHz, DMSO-$d_6$) $\delta$: 8.03 (1H, s), 7.57 (1H, d, *J* = 8.5 Hz), 6.72 (1H, d, *J* = 8.5 Hz), 4. 92 (1H, dd, *J* = 5. 2, 8. 2 Hz), 4. 59 (2H, t, *J* = 8.5 Hz), 4. 24 (1H, dd, *J* = 5. 2, 8. 2 Hz), 3. 35 (2H, t, *J* = 8. 5 Hz), 3.29-3.23 (1H, m), 2.82 (1H, d, *J* = 9. 8 Hz), 2. 33-2. 27 (1H, m), 2.24 (3H, s), 2.18 (1H, dd, *J* = 4. 9, 9.8 Hz). |
| 15 | 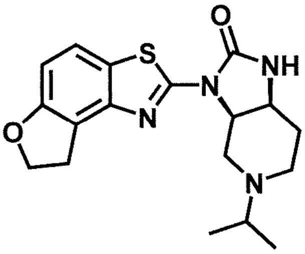 | LC-MS [M+H]$^+$/Rt (min): 359.1/0.491 (Method B) ; $^1$H-NMR ($CDCl_3$) $\delta$ : 7.47 (1H, d, *J* = 8. 2 Hz), 6. 77 (1H, d, *J* = 8.2 Hz), 4.96 (1H, s), 4.88-4.80 (1H, m), 4.73-4.64 (2H, m), 4.12-4.08 (1H, m), 3.59-3.53 (1H, m), 3.48-3.42 (2H, m), 2.88-2.79 (1H, m), 2.69-2.65 (1H, m), 2.50-2.42 (1H, m), 2.39-2.32 (1H, m), 2.08-1.91 (2H, m), 1.04-0.98 (6H, m). |
| 16 | 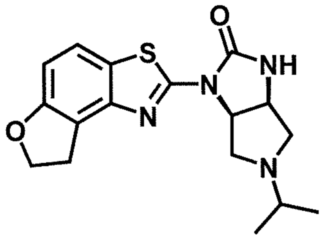 | LC-MS [M+H]$^+$/Rt (min): 345.2/0.593 (Method A); $^1$H-NMR (400 MHz, $CDCl_3$) $\delta$ : 7.40 (1H, d, *J* = 8. 2 Hz), 6.71 (1H, d, *J* = 8. 2 Hz), 5. 14 (1H, s), 5.02-4.96 (1H, m), 4.61 (1H, t, *J* = 8.7 Hz), 4.28-4.22 (1H, m), 3.41 (2H, t, *J* = 8.7), 3. 28 (1H, d, *J* = 10. 1 Hz), 2.88 (1H, d, *J* = 10.1 Hz), 2.63 (1H, dd, *J* = 5. 7, 10.1 Hz), 2. 50-2. 37 (2H, m), 1. 01 (3H, d, *J* = 6. 4 Hz), 0.99 (3H, d, J = 6.4 Hz). |

[Table 55-3]

| | | |
|---|---|---|
| **17** | 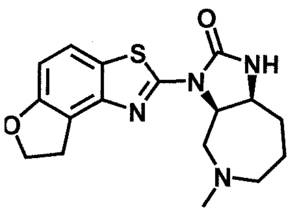 | **LC-MS [M+H]$^+$/Rt (min): 345.2/0.484 (Method B) ; $^1$H-NMR ($CDCl_3$) $\delta$: 7.45 (1H, d, *J* = 8. 5 Hz), 6. 75 (1H, d, *J* = 8.5 Hz), 5.02 (1H, s), 4.94-4.86 (1H, m), 4.71-4.60 (2H, m), 4.25-4.17 (1H, m), 3.51-3.28 (3H, m), 2.97-2.87 (1H, m), 2.79-2.71 (1H, m), 2.42-2.36 (1H, m), 2.39 (3H, s), 2.06-1.99 (1H, m), 1.92-1.76 (2H, m), 1.66-1.56 (1H, m).** |
| **18** | 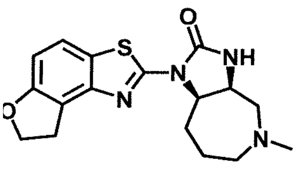 | **LC-MS [M+H]$^+$/Rt (min): 345. 2/0. 525 (Method B); ’H-NMR ($CDCl_3$) $\delta$: 7.45 (1H, d, *J* = 8.5 Hz), 6.75 (1H, d, *J* = 8.5 Hz), 5.05-4.99 (1H, m), 4.88-4.80 (1H, m), 4.69-4.59 (2H, m), 4.25-4.16 (1H, m), 3.50-3.40 (2H, m), 2.78-2.64 (3H, m), 2. 58-2. 48 (1H, m), 2. 46-2. 38 (1H, m), 2. 41 (3H, s), 2. 09-1. 96 (1H, m), 1. 82-1. 76 (1H, m), 1. 70-1. 63 (1H, m).** |
| **19** | 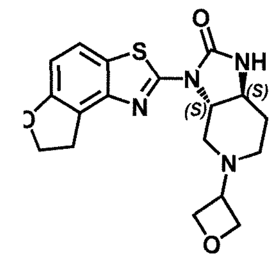 | **LC-MS [M+H]$^+$/Rt (min): 373.11/1.404 (Method D); $^1$H-NMR (DMSO-$d_6$) $\delta$ : 8. 01 (1H, s), 7. 61 (1H, d, J = 8.3 Hz), 6. 76 (1H, d, *J* = 8.3 Hz), 4. 67-4. 56 (4H, m), 4.55-4.45 (2H, m), 4.28-4.20 (1H m), 3.82-3.68 (2H, m), 3.43-3.35 (3H, m), 2.85 (1H, d, *J* = 10.8 Hz), 2.25 (1H, t, *J* = 10. 0 Hz), 2. 16-2. 08 (1H, m), 2.00-1. 93 (1H, m), 1. 75-1. 62 (1H, m) .** |

Example 20

cis-5-Acetyl-3-(7,8-dihydrofuro[3,2-e][1,3]benzothiazol-2-yl)octahydro-2H-imidazo[4,5-c]pyridin-2-one

[Formula 66]

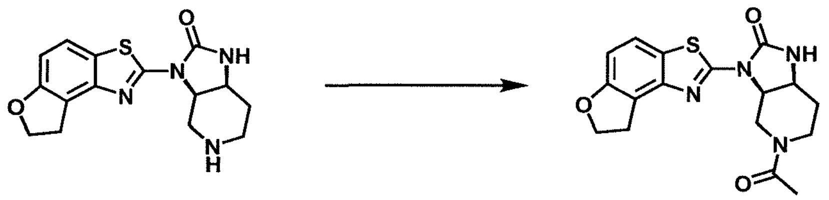

Triethylamine (0.028 mL) and acetic anhydride (0.011 mL) were added to a chloroform solution (2 mL) of the compound of Example 1 (32 mg), and the resulting mixture was stirred overnight. Saturated aqueous sodium bicarbonate was added to the reaction mixture, and the resulting mixture was extracted with chloroform/ethanol (3/1). The organic layer was dried over sodium sulfate, filtered, and then concentrated under reduced pressure. The residue was purified by reverse phase column chromatography (0.035% trifluoroacetic acid in acetonitrile/0.05% trifluoroacetic acid in water), amino silica gel column chromatography (chloroform/methanol), and silica gel column chromatography (chloroform/-methanol) to obtain the title compound (27 mg).
LC-MS $[M+H]^+$/Rt (min): 359.1/0.566 (Method B); $^1$H-NMR (DMSO-$d_6$, 100°C) δ: 7.59 (1H, br s), 7.54 (1H, d, J = 7.9 Hz), 6.72 (1H, d, J = 7.9 Hz), 4.83-4.78 (1H, m), 4.62 (2H, t, J = 8.8 Hz), 4.30-4.17 (2H, m), 3.85-3.78 (1H, m), 3.52-3.41 (2H, m), 3.39 (2H, t, J = 8.8 Hz), 2.13-2.02 (1H, m), 1.94-1.68 (4H, m).

Example 21

(3aR,7aR)-1-(2H-[1,3]dioxolo[4,5-e][1,3]benzothiazol-7-yl)hexahydropyrano[3,4-d]imidazol-2(3H)-one

[Formula 67]

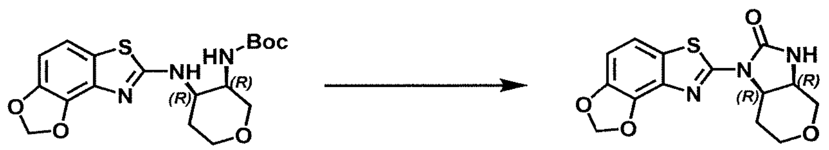

A 4 M hydrogen chloride/ethyl acetate solution (5 mL) was added to an ethyl acetate solution (5 mL) of the compound of Reference Example 47 (759 mg), and the resulting mixture was stirred at room temperature overnight. Hexane was added to the reaction mixture, the resulting solid was collected by filtration and washed with hexane, and then the solid was dried under reduced pressure. Triethylamine (1.41 mL) and di(N-succinimidyl) carbonate (519 mg) were added to a dimethylformamide solution (20 mL) of the resulting solid (668 mg), and the resulting mixture was stirred at room temperature for 1 hour. Water was added to the reaction mixture, and the resulting mixture was extracted with ethyl acetate. The organic layer was dried over sodium sulfate, filtered, and then concentrated under reduced pressure to obtain the title compound (364 mg).
LC-MS $[M+H]^+$/Rt (min): 320.0/1.990 (Method C); $^1$H-NMR (DMSO-$d_6$) δ: 7.96 (1H, s), 7.37 (1H, d, J = 8.2 Hz), 6.93 (1H, d, J = 8.2 Hz), 6.10 (2H, dd, J = 1.1, 5.0 Hz), 4.78 (1H, ddd, J = 6.1, 7.3, 8.6 Hz), 3.90-3.72 (3H, m), 3.67 (1H, dd, J = 2.7, 12.8 Hz), 3.44 (1H, ddd, J = 3.0, 10.2, 11.5), 2.46-2.28 (1H, m), 1.85-1.68 (1H, m).

Example 22

(4R, 5R)-1-(7,8-dihydrofuro[3,2-e][1,3]benzothiazol-2-yl)-5-[(dimethylamino)methyl]-4-methylimidazolidin-2-one

[Formula 68]

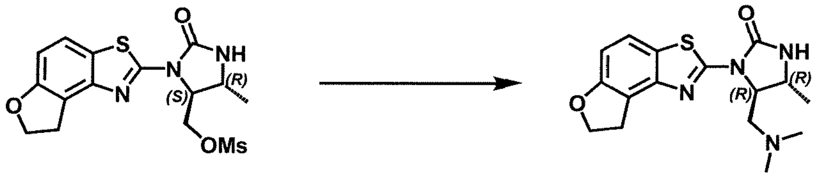

Dimethylamine (2.0 M tetrahydrofuran solution) (0.31 mL), potassium iodide (26.2 mg), and potassium carbonate (55.6 mg) were added to an acetonitrile solution (3 mL) of the compound of Reference Example 39 (59.4 mg), and the resulting mixture was stirred at 60°C for 15 hours. The reaction mixture was cooled to room temperature, then saturated aqueous sodium bicarbonate was added, and the resulting mixture was extracted with chloroform-methanol (10:1). The organic layer was dried over sodium sulfate, filtered, and then concentrated under reduced pressure. The residue was purified by silica gel column chromatography (chloroform/methanol) and amino silica gel column chromatography (chloroform/methanol) to obtain the title compound (22.6 mg).
LC-MS $[M+H]^+$/Rt (min): 333.2/0.569 (Method A); $^1$H-NMR (400 MHz, $CDCl_3$) δ: 7.45 (1H, d, J = 7.9 Hz), 6.76 (lH, d, J = 7.9 Hz), 4.93 (1H, s), 4.66 (2H, t, J = 8.9 Hz), 4.40-4.34 (1H, m), 3.90-3.84 (1H, m), 3.44 (2H, dt, J = 2.0, 8.9 Hz), 2.87 (1H, dd, J = 2.4, 11.9 Hz), 2.34 (1H, dd, J = 10.1, 11.9 Hz), 2.34 (6H, s), 1.35 (3H, d, J = 7.3 Hz).

Examples 23 to 26

The compounds of Examples 23 to 26 were obtained by using the corresponding raw material compounds according to the method described in Example 22.

[Table 56]

| Example | Chemical structure | Physical property data |
|---|---|---|
| 23 | 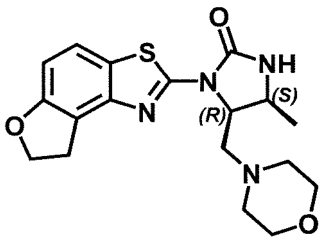 | **LC-MS $[M+H]^+$/Rt (min): 375.2/0.446 (Method B) ; $^1$H-NMR ($CDCl_3$) δ : 7.47 (1H, d, *J* = 8. 2 Hz), 6. 78 (1H, d, *J* = 8. 2 Hz), 4. 93 (1H, s), 4. 79-4. 73 (1H, m), 4. 68 (2H, t, *J* = 8. 9 Hz), 4. 29-4. 22 (1H, m), 3. 73-3. 64 (4H, m), 3.50-3.36 (2H, m), 2.84-2.72 (4H, m), 2.53-2.48 (2H, m), 1. 45 (3H, d, *J* = 6. 7 Hz).** |
| 24 | 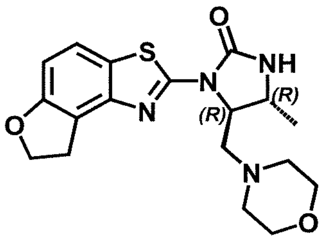 | **LC-MS $[M+H]^+$/Rt (min): 375.2/0.573 (Method A); $^1$H-NMR (400 MHz, $CDCl_3$) δ : 7. 45 (1H, d, *J* = 8. 7 Hz), 6. 76 (1H, d, *J* = 8. 7 Hz), 4. 93 (1H, s), 4. 67 (2H, t, *J* = 8. 7 Hz), 4. 42-4. 37 (1H, m), 3.91-3.85 (1H, m), 3.75-3.63 (4H, m), 3. 43 (2H, t, *J* = 8. 7 Hz), 3.05 (1H, dd, *J* = 3. 2, 12.3 Hz), 2. 73-2. 66 (2H, m), 2.55 (1H, dd, *J* = 9. 6, 12.3 Hz), 2.51-2.44 (2H, m), 1. 35 (3H, d, *J* = 6. 4 Hz).** |
| 25 | 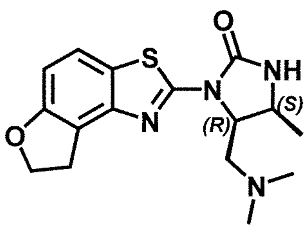 | **LC-MS $[M+H]^+$/Rt (min): 333.1/0.462 (Method B); $^1$H-NMR ($CDCl_3$) δ: 7. 47 (1H, d, J = 8. 5 Hz), 6.77 (1H, d, J = 8. 5 Hz), 4. 88 (1H, s), 4.75-4.65 (3H, m), 4.28-4.21 (1H, m), 3.51-3.37 (2H, m), 2.79 (1H, dd, *J* = 12.8, 9.8 Hz), 2.63 (1H, dd, *J* = 12. 8, 1. 8 Hz), 2.37 (6H, s), 1. 45 (3H, d, *J* = 6. 7 Hz).** |

(continued)

| Example | Chemical structure | Physical property data |
|---|---|---|
| **26** | 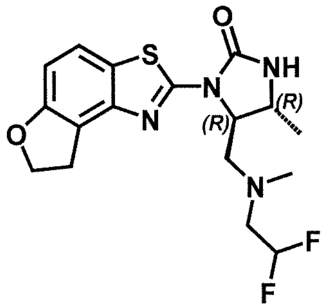 | **LC-MS [M+H]$^+$/Rt (min): 333.1/0.462 (Method B) ; 'H-NMR (CDCl$_3$) δ : 6. 95 (1H, d, *J* = 7. 9 Hz), 6. 43 (1H, d, *J* = 7. 9 Hz), 5. 79 (1H, tt, *J* = 4. 3, 56. 2 ($J_{H-F}$) Hz), 4. 69 (1H, d, *J* = 9. 2 Hz), 4.64-4.56 (3H, m), 4.15-4.08 (1H, m), 4.05 (t, *J* = 8. 5 Hz), 3. 79 (t, *J* = 8.5 Hz), 3. 64-3. 45 (2H, m), 3. 36-3. 21 (2H, m), 2. 85 (3H, s), 1. 36 (3H, d, *J* = 6. 7 Hz).** |

Example 27

(3aS,7aR)-3-(7,8-Dihydrofuro[3,2-e][1,3]benzothiazol-2-yl)-5-methyloctahydro-2H-imidazo[4,5-c]pyridin-2-one

Example 28

(3aR,7aS)-3-(7,8-Dihydrofuro[3,2-e][1,3]benzothiazol-2-yl)-5-methyloctahydro-2H-imidazo[4,5-c]pyridin-2-one

[Formula 69]

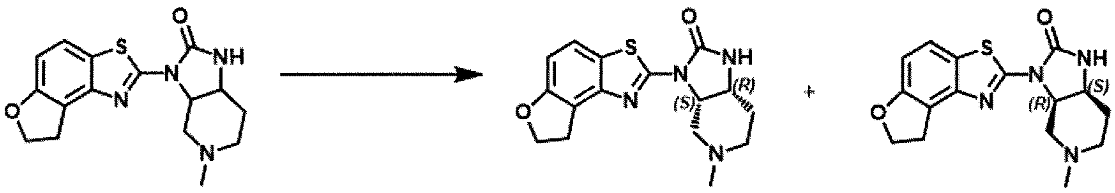

Example 7 Example 27 Example 28

The compound of Example 7 (30.0 mg) was optically fractionated under the following conditions to obtain the title compounds (Example 27: 12.2 mg-first peak: 15.3 min, Example 28: 8.1 mg-second peak: 16.4 min).
Column: CHIRALPAK (R) IG; Solvents: liquid A: chloroform, liquid B: 2-propanol; Mobile phase condition: A/B = 9/1; Flow rate: 5 mL/min; Detection UV: 254 nm; Column temperature: 40°C

[Table 57]

| Example | Physical property data |
|---|---|
| **27** | **LC-MS [M+H] $^+$/Rt (min): 331. 1/0. 434 (Method B); 'H-NMR (CDCl$_3$) δ : 7. 45 (1H, d, *J* = 8. 5 Hz), 6. 76 (1H, d, *J* = 8. 5 Hz), 4. 89-4. 82 (1H, m), 4. 74 (1H, s), 4.69-4.63 (2H, m), 4.08-4.03 (1H, m), 3.58-3.51 (1H, m), 3.44 (2H, t, *J* = 8. 9 Hz), 2. 66-2. 60 (1H, m), 2.29 (3H, s), 2.27-2.19 (1H, m), 2.12-2.02 (2H, m), 1. 94-1. 88 (1H, m).** |
| **28** | **LC-MS [M+H] $^+$/Rt (min): 331.1/0.430 (Method B); $^1$H-NMR (CDCl$_3$) δ : 7. 45 (1H, d, *J* = 8.5 Hz), 6. 76 (1H, d, *J* = 8.5 Hz), 4. 90-4. 82 (1H, m), 4. 76 (1H, s), 4.69-4.63 (2H, m), 4.09-4.03 (1H, m), 3.58-3.52 (1H, m), 3.44 (2H, t, *J* = 8.5 Hz), 2. 67-2. 60 (1H, m), 2.30 (3H, s), 2. 29-2. 20 (1H, m), 2.11-2.04 (2H, m), 1. 94-1. 88 (1H, m).** |

Examples 29 and 30

The compounds of Examples 29 and 30 were obtained by using the corresponding raw material compounds according to the method described in Example 1.

[Table 58]

| Example | Chemical structure | Physical property data |
|---|---|---|
| **29** | 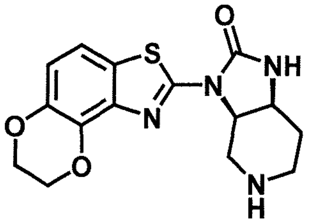 | **LC-MS [M+H]$^+$/Rt (min): 333.1/0.430 (Method B); $^1$H-NMR (DMSO-$d_6$) δ: 7.71 (1H, s), 7.26 (1H, d, J = 8. 6 Hz), 6. 78 (1H, d, *J* = 8. 6 Hz), 4.48-4.40 (1H, m), 4.38-4.24 (4H, m), 4.03-3.98 (1H, m), 3.49-3.42 (1H, m), 2.76-2.69 (1H, m), 2.62-2.48 (2H, m), 2.36-2.15 (1H, m), 1.83-1.66 (2H, m).** |
| **30** | 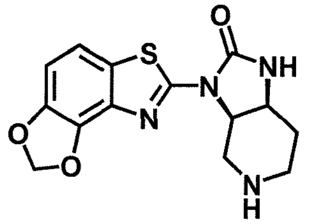 | **LC-MS [M+H]$^+$/Rt (min): 319.1/0.419 (Method B); ’H-NMR (DMSO-$d_6$) δ: 7.79 (1H, s), 7.34 (1H, d, *J* = 7. 9 Hz), 6. 90 (1H, d, *J* = 7.9 Hz), 6. 10-6. 07 (2H, m), 4. 50-4. 43 (1H, m), 4.04-4.01 (1H, m), 3.46-3.40 (1H, m), 2.75-2.72 (1H, m), 2.66-2.53 (2H, m), 2.43-2.21 (1H, m), 1.82-1.67 (2H, m).** |

Example 31

cis-5-Cyclopropyl-3-(7,8-dihydro[1,4]dioxino[2,3-e][1,3]benzothiazol-2-yl)octahydro-2H-imidazo[4,5-c]pyridin-2-one

[Formula 70]

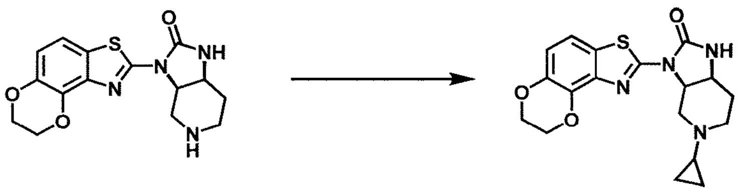

(1-Ethoxycyclopropoxy)trimethylsilane (0.121 mL), acetic acid (0.172 mL), and sodium cyanoborohydride (94 mg) were added to a methanol/tetrahydrofuran solution (1.5 mL/1.2 mL) of the compound of Example 29 (100 mg), and the resulting mixture was stirred at 60°C for 3 hours. The reaction mixture was cooled to room temperature, then saturated aqueous sodium bicarbonate was added, and the resulting mixture was extracted with chloroform/ethanol (3/1). The organic layer was dried over sodium sulfate, filtered, and then concentrated under reduced pressure. The residue was purified by silica gel column chromatography (chloroform/methanol) to obtain the title compound (77 mg).
LC-MS [M+H]$^+$/Rt (min): 373.3/0.585 (Method A); $^1$H-NMR (DMSO-$d_6$) δ: 7.72 (1H, s), 7.27 (1H, d, J = 8.5 Hz), 6.79 (1H, d, J = 8.5 Hz), 4.61-4.53 (1H, m), 4.41-4.25 (4H, m), 3.98-3.92 (lH, m), 3.49-3.41 (1H, m), 2.75-2.67 (1H, m), 2.45-2.36 (lH, m), 2.35-2.27 (1H, m), 1.86-1.75 (2H, m), 1.68-1.63 (1H, m), 0.43-0.37 (2H, m), 0.33-0.23 (2H, m).

Example 32

The compound of Example 32 was obtained by using the corresponding raw material compounds according to the method described in Example 31.

[Table 59]

| Example | Chemical structure | Physical property data |
|---|---|---|
| 32 | 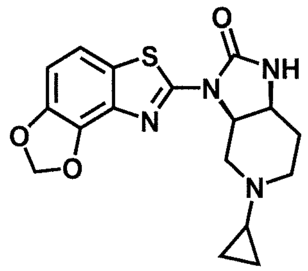 | **LC-MS [M+H]+/Rt (min): 359.2/0.613 (Method A); $^1$H-NMR (DMSO-$d_6$) δ : 7.80 (1H, s), 7.35 (1H, d, *J* = 8. 2 Hz), 6.91 (1H, d, *J* = 8.2 Hz), 6.12-6.08 (2H, m), 4. 62-4. 53 (1H, m), 4. 00-3. 94 (1H, m), 3.51-3.42 (1H, m), 2.76-2.67 (1H, m), 2. 46-2. 36 (1H, m), 2.35-2.27 (1H, m), 1. 87-1. 74 (2H, m), 1. 69-1. 64 (1H, m), 0.43-0. 38 (2H, m), 0.33-0.23 (2H, m).** |

Example 33

(3aS,7aR)-5-Cyclopropyl-3-(7,8-dihydro[1,4]dioxino[2,3-e][1,3]benzothiazol-2-yl)octahydro-2H-imidazo[4,5-c]pyridin-2-one

Example 34

(3aR,7aS)-5-Cyclopropyl-3-(7,8-dihydro[1,4]dioxino[2,3-e][1,3]benzothiazol-2-yl)octahydro-2H-imidazo[4,5-c]pyridin-2-one

[Formula 71]

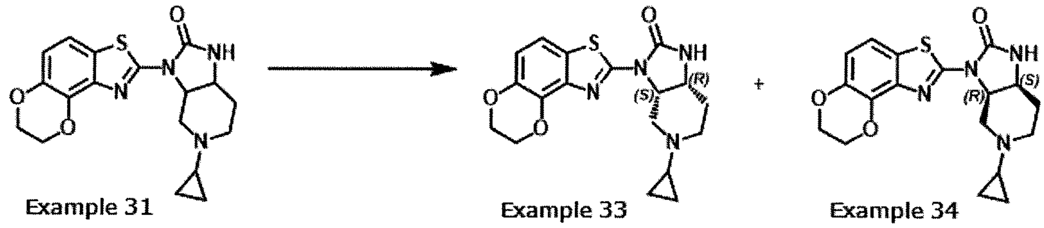

Example 31 Example 33 Example 34

The compound of Example 31 (104 mg) was optically fractionated under the following conditions to obtain the title compounds (Example 33: 37 mg-first peak: 13.0 min, Example 34: 36 mg-second peak: 14.9 min).
Column: CHIRALPAK (R) IG; Solvents: liquid A: chloroform, liquid B: methanol, liquid C: diethylamine; Mobile phase condition: A/B/C = 99/1/0.001; Flow rate: 0.5 mL/min; Detection UV: 280 nm; Column temperature: 25°C

[Table 60]

| Example | Physical property data |
|---|---|
| 33 | **LC-MS [M+H] +/Rt (min): 373. 3/0. 587 (Method A) ; $^1$H-NMR (DMSO-$d_6$) δ : 7. 72 (1H, s), 7. 27 (1H, d, *J* = 8. 5 Hz), 6. 79 (1H, d, *J* = 8. 5 Hz), 4.60-4.52 (1H, m), 4.41-4.28 (4H, m), 3.97-3.93 (1H, m), 3.47-3.41 (1H, m), 2.75-2.67 (1H, m), 2.45-2.37 (1H, m), 2.33-2.28 (1H, m), 1.86-1.75 (2H, m), 1.68-1.63 (1H, m), 0.43-0.33 (2H, m), 0.32-0.22 (2H, m).** |
| 34 | **LC-MS [M+H] +/Rt (min): 373. 2/0.585 (Method A) ; $^1$H-NMR (DMSO-$d_6$) δ : 7. 73 (1H, s), 7.28 (1H, d, *J* = 8.8 Hz), 6. 79 (1H, d, *J* = 8.8 Hz), 4.60-4.53 (1H, m), 4.41-4.25 (4H, m), 3.97-3.93 (1H, m), 3.48-3.41 (1H, m), 2.72-2.60 (1H, m), 2.45-2.36 (1H, m), 2.33-2.28 (1H, m), 1.86-1.75 (2H, m), 1.68-1.63 (1H, m), 0.45-0.36 (2H, m), 0.31-0.22 (2H, m).** |

Example 35

(3aS,7aR)-5-Cyclopropyl-3-(2H-[1,3]dioxolo[4,5-e][1,3]benzothiazol-7-yl)octahydro-2H-imidazo[4,5-c]pyridin-2-one

Example 36

(3aR,7aS)-5-Cyclopropyl-3-(2H-[1,3]dioxolo[4,5-e][1,3]benzothiazol-7-yl)octahydro-2H-imidazo[4,5-c]pyridin-2-one

[Formula 72]

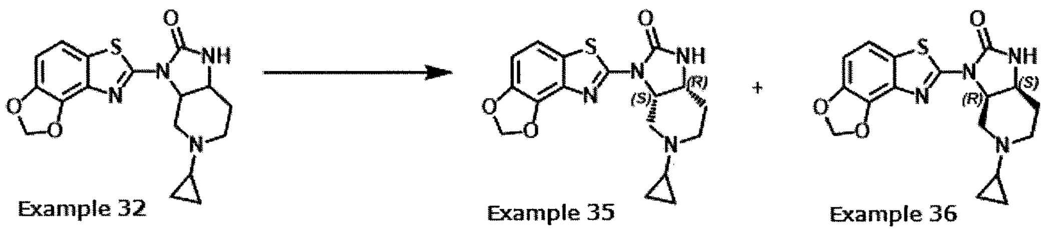

Example 32 Example 35 Example 36

The compound of Example 32 (85.0 mg) was optically fractionated under the following conditions to obtain the title compounds (Example 35: 37.1 mg-first peak: 10.7 min, Example 36: 36.4 mg-second peak: 12.3 min). Column: CHIRALPAK (R) IG; Solvents: liquid A: chloroform, liquid B: methanol, liquid C: diethylamine; Mobile phase condition: A/B/C = 99/1/0.002; Flow rate: 5 mL/min; Detection UV: 280 nm; Column temperature: 40°C

[Table 61]

| Example | Physical property data |
|---|---|
| 35 | **LC-MS [M+H] $^+$/Rt (min): 359. 2/0.657 (Method A); $^1$H-NMR (DMSO-$d_6$) δ : 7. 80 (1H, s), 7.35 (1H, d, *J* = 7. 9 Hz), 6.91 (1H, d, *J* = 7. 9 Hz), 6.12-6.09 (2H, m), 4.61-4.53 (1H, m), 3.98-3.95 (1H, m), 3.50-3.43 (1H, m), 2.75-2.68 (1H, m), 2.45-2.37 (1H, m), 2.35-2.27 (1H, m), 1.86-1.75 (2H, m), 1.69-1.64 (1H, m), 0.45-0.37 (2H, m), 0.33-0.23 (2H, m).** |
| 36 | **LC-MS [M+H] $^+$/Rt (min): 359. 3/0. 660 (Method A); $^1$H-NMR (DMSO-$d_6$) δ : 7.81 (1H, s), 7.35 (1H, d, *J* = 8. 3 Hz), 6. 91 (1H, d, *J* = 8. 3 Hz), 6. 10 (2H, d, *J* = 6.1 Hz), 4.61-4.53 (1H, m), 3.99-3.94 (1H, m), 3.50-3.42 (1H, m), 2.75-2.67 (1H, m), 2.44-2.36 (1H, m), 2.34-2.27 (1H, m), 1.86-1.75 (2H, m), 1.69-1.64 (1H, m), 0.44-0.37 (2H, m), 0.33-0.23 (2H, m).** |

Examples 37 to 43

The compounds of Examples 37 to 43 were obtained by using the corresponding raw material compounds according to the method described in Reference Example 14.

[Table 62-1]

| Example | Chemical structure | Physical property data |
|---|---|---|
| 37 | 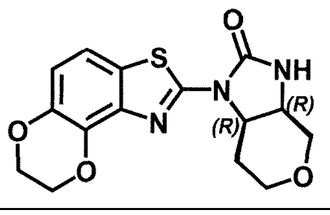 | **LC-MS [M+H]$^+$/Rt (min): 334.1/1.939 (Method C); $^1$H-NMR (DMSO-$d_6$) δ: 7.88 (1H, s), 7.29 (1H, d, *J* = 8. 6 Hz), 6. 80 (1H, d, *J* = 8. 6 Hz), 4. 79-4.73 (1H, m), 4.42-4.25 (4H, m), 3.87-3.71 (3H, m), 3.67 (1H, dd, *J* = 3.0, 13.0 Hz), 3. 44 (1H, ddd, *J* = 3. 0, 10. 2, 11.5 Hz), 2.41-2.29 (1H, m), 1. 87-1. 72 (1H, m).** |
| 38 | 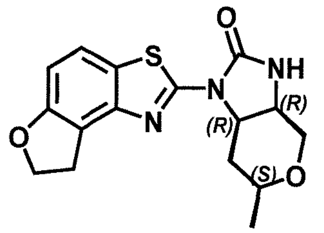 | **LC-MS [M+H]$^+$/Rt (min): 332.1/2.090 (Method C); $^1$H-NMR (DMSO-$d_6$) δ: 7.89 (1H, s), 7.59 (1H, d, *J* = 8.4 Hz), 6.74 (1H, d, *J* = 8.4 Hz), 4. 85-4.79 (1H, m), 4.67-4.49 (2H, m), 3.95 (1H, d, *J* = 13. 1 Hz), 3. 77 (1H, dd, *J* = 2. 4, 7. 1 Hz), 3.67 (1H, dd, *J* = 2.7, 13.2 Hz), 3.53-3.41 (1H, m), 3.41-3.31 (2H, m), 2.49-2.38 (1H, m), 1.19-1.13 (1H, m), 1.10 (3H, d, *J* = 6. 1 Hz).** |

(continued)

| Example | Chemical structure | Physical property data |
|---|---|---|
| 39 | 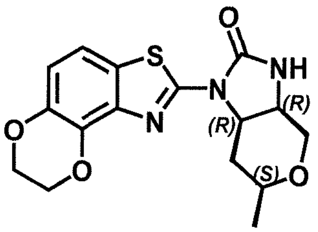 | LC-MS $[M+H]^+$/Rt (min): 348.1/2.040 (Method C); $^1$H-NMR (DMSO-$d_6$) δ: 7. 85 (1H, s), 7. 29 (1H, d, *J* = 8.6 Hz), 6. 80 (1H, d, *J* = 8.6 Hz), 4.81-4.75 (1H, m), 4.43-4.20 (4H, m), 3.95 (1H, d, *J* = 13. 1 Hz), 3. 77 (1H, dd, *J* = 2. 4, 7. 3 Hz), 3.67 (1H, dd, *J* = 2. 7, 13. 1 Hz), 3. 46 (1H, dqd, *J* = 1. 8, 6.1, 12. 3 Hz), 2. 44 (1H, ddd, *J* = 2.0, 6.7, 13.3 Hz), 1. 22-1. 11 (1H, m), 1.10 (3H, d, J = 6. 1 Hz). |
| 40 | 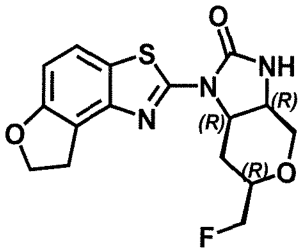 | LC-MS $[M+H]^+$/Rt (min): 350. 1/2. 073 (Method C) ; $^1$H-NMR (DMSO-$d_6$) δ : 7.93 (1H, s), 7.60 (1H, d, *J* = 8. 4 Hz), 6. 75 (1H, d, *J* = 8. 4 Hz), 4. 96-4.85 (1H, m), 4.68-4.56 (2H, m), 4.51-4.23 (2H, m), 4.01 (1H, d, *J* = 13.0 Hz), 3. 87-3. 80 (1H, m), 3.78-3.66 (2H, m), 3.41-3.35 (2H, m), 2.48-2.41 (1H, m), 1. 37-1. 24 (1H, m). |

[Table 62-2]

| | | |
|---|---|---|
| 41 | 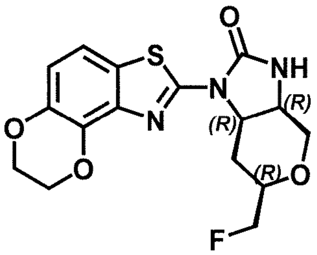 | LC-MS $[M+H]^+$/Rt (min): 366.1/2.023 (Method C); 'H-NMR (DMSO-$d_6$) δ: 7.91 (1H, brs), 7.30 (1H, d, *J* = 8. 6 Hz), 6.81 (1H, d, *J* = 8.6 Hz), 4. 90-4. 84 (1H, m), 4. 53-4. 22 (6H, m), 4.05-3.97 (1H, m), 3.87-3.80 (1H, m), 3.79-3.63 (2H, m), 2.46-2.38 (1H, m), 1.36-1. 21 (1H, m) . |
| 42 | 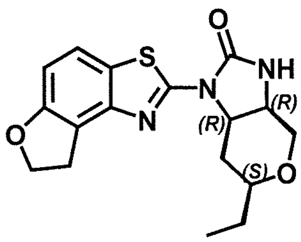 | LC-MS $[M+H]^+$/Rt (min): 346.1/2.257 (Method C); $^1$H-NMR (DMSO-$d_6$) δ: 7.87 (1H, s), 7.59 (1H, d, *J* = 8. 4 Hz), 6. 74 (1H, d, *J* = 8.4 Hz), 4.88-4.77 (1H, m), 4.67-4.55 (2H, m), 4.02-3.95 (1H, m), 3.82-3.76 (1H, m), 3.66 (1H, dd, J = 2. 6, 13. 1 Hz), 3. 43-3. 34 (2H, m), 3.30-3.21 (1H, m), 2.48-2.42 (1H, m), 1. 47-1. 36 (2H, m), 1. 28-1. 14 (1H, m), 0.86 (3H, t, *J* =*7. 4* Hz). |
| 43 | 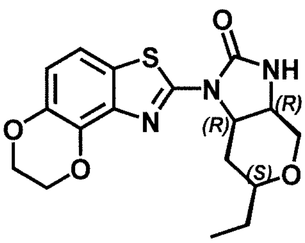 | LC-MS $[M+H]^+$/Rt (min): 362.2/2.140 (Method C); $^1$H-NMR (DMSO-$d_6$) δ: 7.85 (1H, s), 7.29 (1H, d, *J* = 8. 6 Hz), 6. 80 (1H, d, *J* = 8. 6 Hz), 4.86-4.73 (1H, m), 4.43-4.24 (4H, m), 4.03-3.93 (1H, m), 3.83-3.75 (1H, m), 3.66 (1H, dd, *J* = 2.7, 13.1 Hz), 3.30-3.20 (1H, m), 2.47-2.36 (1H, m), 1.47-1.37 (2H, m), 1.26-1.13 (1H, m), 0.86 (3H, t, *J* =7.4 Hz). |

Example 44

The compound of Example 44 was obtained by using the corresponding raw material compounds according to the method described in Reference Example 14.

[Table 63]

| **Example** | **Chemical structure** | **Physical property data** |
|---|---|---|
| **44** | 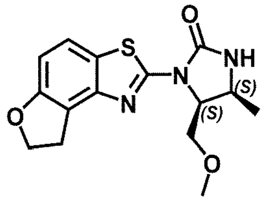 | **LC-MS [M+H]$^+$/Rt (min): 320.1/0.737 (Method B); $^1$H-NMR ($CDCl_3$) δ: 7. 46 (1H, d, *J* = 8. 7 Hz), 6. 76 (1H, d, *J* = 8. 7 Hz), 4. 89 (1H, br s), 4.78-4.74 (1H, m), 4.68-4.64 (2H, m), 4.25-4.18 (1H, m), 3.98 (1H, dd, *J* = 10.5, 6. 4 Hz), 3. 80 (1H, dd, *J* = 10. 5, 2. 1 Hz), 3.48-3.42 (2H, m), 3.32 (3H, s), 1. 43 (3H, d, *J* = 6. 4 Hz) .** |

Examples 45 to 66

The compounds of Examples 45 to 66 were obtained by using the corresponding raw material compounds according to the method described in Example 1.

[Table 64-1]

| Example | Chemical structure | Physical property data |
|---|---|---|
| 45 | 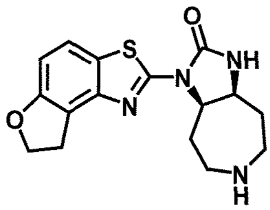 | LC-MS [M+H] $^+$/Rt (min): 331.2/0.610 (Method B) ; $^1$H-NMR ($CDCl_3$, 50°C) δ : 7.45 (1H, d, *J* = 7. 9 Hz), 6.75 (1H, d, *J* = 7.9 Hz), 4. 98-4.92 (1H, m), 4.86 (1H, br s), 4.68-4.61 (2H, m), 4.30-4.24 (1H, m), 3.47-3.41 (2H, m), 3.13-3.06 (1H, m), 3. 04-2. 98 (1H, m), 2.88-2.75 (2H, m), 2.51-2.44 (1H, m), 2.39-2.30 (1H, m), 2.11-2.04 (1H, m), 2.00-1.91 (1H, m) . |
| 46 | 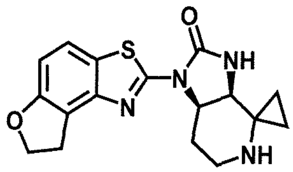 | LC-MS [M+H]$^+$/Rt (min): 343.1/0.476 (Method B); $^1$H-NMR ($CDCl_3$) δ : 7.46 (1H, d, *J* = 8.6 Hz), 6. 77 (1H, d, J = 8.6 Hz), 4.99 (1H, s), 4. 93-4. 85 (1H, m), 4. 72-4. 61 (2H, m), 3.45 (2H, t, *J* = 8.6 Hz), 3.16 (1H, d, *J* = 6.7 Hz), 3.05-2.99 (1H, m), 2.80-2.73 (1H, m), 2. 64-2. 56 (1H, m), 1. 67-1. 47 (2H, m), 0.86-0.73 (2H, m), 0.59-0.50 (2H, m). |
| 47 | 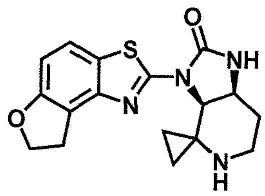 | LC-MS [M+H]$^+$/Rt (min): 343.1/0.484 (Method B); $^1$H-NMR ($CDCl_3$) δ: 7.45 (1H, d, *J* = 8.5 Hz), 6. 76 (1H, d, *J* = 8.5 Hz), 5.07 (1H, s), 4.66 (2H, t, J = 8.8 Hz), 4. 61 (1H, d, *J* = 8.3 Hz), 4.31-4.27 (1H, m), 3. 42 (2H, t, *J* = 8. 8 Hz), 3.16-3.08 (1H, m), 3.02-2.96 (1H, m), 2.19-2.06 (1H, m), 1.81-1.74 (1H, m), 1. 38-1. 28 (1H, m), 0.82-0. 77 (1H, m), 0.74-0.61 (2H, m). |
| 48 | 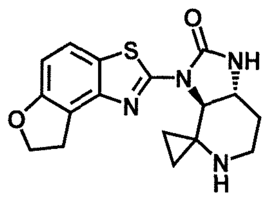 | LC-MS [M+H]$^+$/Rt (min): 343.2/0.481 (Method B); $^1$H-NMR ($CDCl_3$) δ: 7.45 (1H, d, J = 8.3 Hz), 6.80 (1H, d, *J* = 8.3 Hz), 5.22 (1H, s), 4. 72-4. 58 (2H, m), 4. 36 (1H, d, *J* = 11. 0 Hz), 3. 78-3. 70 (1H, m), 3. 56-3. 40 (2H, m), 3.17-3.11 (1H, m), 2.91-2.78 (1H, m), 2.13-2.06 (1H, m), 1. 88-1. 82 (1H, m), 1. 80-1. 68 (1H, m), 1. 40-1. 21 (1H, m), 0.76-0. 66 (1H, m), 0.58-0.46 (2H, m). |
| 49 | 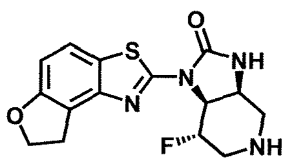 | LC-MS [M+H]$^+$/Rt (min): 335.1/0.476 (Method B). $^1$H-NMR (DMSO-$d_6$) δ: 7. 68 (1H, s), 7. 59 (1H, d, *J* = 7. 9 Hz), 6. 74 (1H, d, *J* = 7. 9 Hz), 5.25-5.08 (1H, m), 4.85-4.74 (1H, m), 4.65-4.55 (2H, m), 3.83-3.78 (1H, m), 3.46-3.33 (2H, m), 3.20-3.10 (1H, m), 3.04-2.98 (1H, m), 2. 88-2. 70 (2H, m), 2.05 (1H, br s). |

[Table 64-2]

| | | |
|---|---|---|
| 50 | 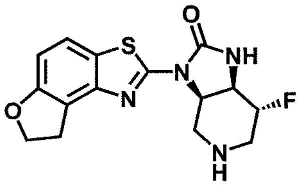 | LC-MS [M+H]$^+$/Rt (min): 335. 1/0. 444 (Method B). $^1$H-NMR (DMSO-$d_6$) δ: 8.02 (1H, s), 7.58 (1H, d, *J* = 7. 9 Hz), 6.73 (1H, d, *J* = 7. 9 Hz), 4. 81-4. 58 (4H, m), 4. 25-4. 19 (1H, m), 3. 50-3. 42 (1H, m), 3.36 (2H, t, *J = 9. 1* Hz), 3.03-2.95 (1H, m), 2.84-2.65 (2H, m). |
| 51 | 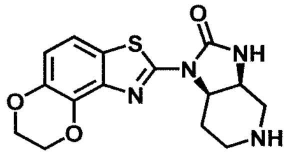 | LC-MS [M+H]$^+$/Rt (min): 333. 1/0. 443 (Method B); $^1$H-NMR (DMSO-$d_6$) δ : 7. 71 (1H, s), 7.27 (1H, d, *J* = 8. 6 Hz), 6. 78 (1H, d, *J* = 8. 6 Hz), 4.64-4.55 (1H, m), 4.37-4.26 (4H, m), 3.75-3.68 (1H, m), 2.97-2.90 (1H, m), 2.84-2.76 (2H, m), 2.46-2.40 (1H, m), 2.32-2.24 (1H, m), 1. 59-1. 50 (1H, m). |
| 52 | 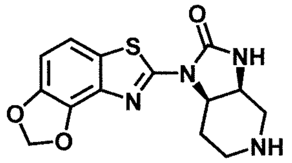 | LC-MS [M+H]$^+$/Rt (min): 319.1/0.464 (Method B); ’H-NMR (DMSO-$d_6$) δ: 7.79 (1H, s), 7.34 (1H, d, *J* = 8. 2 Hz), 6. 90 (1H, d, *J* = 8. 2 Hz), 6. 09-6. 07 (2H, m), 4. 66-4. 58 (1H, m), 3.77-3.72 (1H, m), 2.97-2.92 (1H, m), 2.85-2.76 (2H, m), 2.46-2.42 (1H, m), 2.31-2.06 (2H, m), 1. 59-1. 49 (1H, m). |
| 53 | 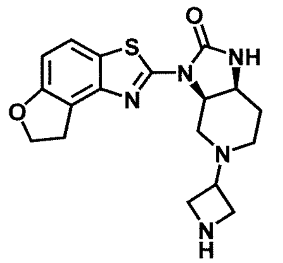 | LC-MS [M+H]$^+$/Rt (min): 372.3/0.547 (Method A); $^1$H-NMR (DMSO-$d_6$) δ: 7.76 (1H, s), 7.58 (1H, d, *J* = 8.5 Hz), 6.73 (1H, d, *J* = 8. 5 Hz), 4.65-4.56 (3H, m), 4.11-4.04 (1H, m), 4.00-3.94 (1H, m), 3.40-3.26 (2H, m), 3.21-3.08 (5H, m), 2.48-2.40 (1H, m), 2.17-2.04 (2H, m), 1.93-1.72 (2H, m). |
| 54 | 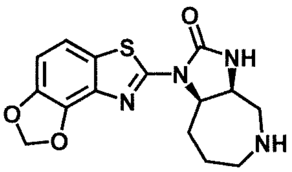 | LC-MS [M+H]$^+$/Rt (min): 333.2/0.576 (Method A); $^1$H-NMR (DMSO-$d_6$) δ: 7.83 (1H, s), 7.32 (1H, d, *J* = 7. 9 Hz), 6. 89 (1H, d, *J* = 7. 9 Hz), 6.08 (2H, s), 4.71-4.64 (1H, m), 4.05-3.97 (1H, m), 3.01-2.95 (1H, m), 2.80-2.57 (4H, m), 2. 32-2. 06 (2H, m), 1. 62-1. 36 (2H, m). |
| 55 | 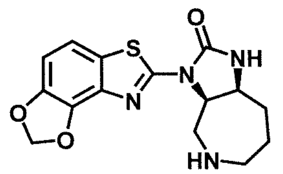 | LC-MS [M+H]$^+$/Rt (min): 333. 2/0. 507 (Method A); $^1$H-NMR (DMSO-$d_6$) δ: 7.88 (1H, s), 7.32 (1H, d, *J* = 8. 6 Hz), 6. 89 (1H, d, *J* = 8. 6 Hz), 6.08-6.06 (2H, m), 4.62-4.57 (1H, m), 4.19-4.13 (1H, m), 3.17-3.04 (1H, m), 2.79-2.54 (3H, m), 1. 89-1. 65 (3H, m), 1. 48-1. 37 (1H, m). |

[Table 64-3]

| | | |
|---|---|---|
| 56 | 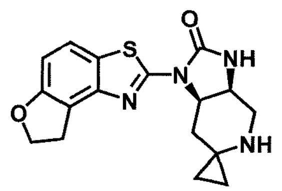 | LC-MS [M+H]$^+$/Rt (min): 343.2/0.495 (Method B); $^1$H-NMR (DMSO-$d_6$) δ : 7.80 (1H, brs), 7.56 (1H, d, *J* = 8. 2 Hz), 6. 71 (1H, d, *J* = 8. 2 Hz), 4. 73 (1H, q, *J* = 7.3 Hz), 4. 63-4. 53 (2H, m), 3.77-3.75 (1H, m), 3.40-3.31 (2H, m), 2. 97-2. 93 (2H, m), 1. 93-1. 84 (2H, m), 0.50-0.32 (4H, m) . |
| 57 | 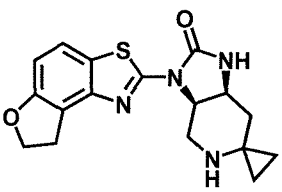 | LC-MS [M+H]$^+$/Rt (min): 343. 2/0. 452 (Method B) |

(continued)

| | | |
|---|---|---|
| 58 | 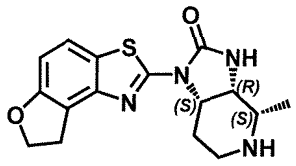 | LC-MS [M+H] $^{+}$/Rt (min): 331.3/0.497 (Method B) |
| 59 | 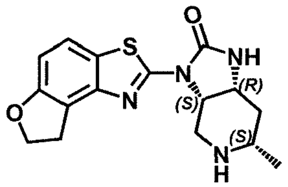 | LC-MS [M+H] $^{+}$/Rt (min) : 331. 2/0. 425 (Method B) |
| 60 | 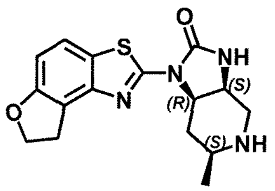 | LC-MS [M+H] $^{+}$/Rt (min): 331. 2/0. 460 (Method B) |
| 61 | 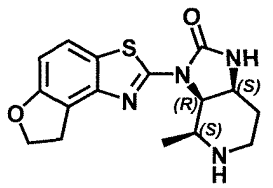 | LC-MS [M+H] $^{+}$/Rt (min) : 331. 2/0. 428 (Method B) |
| 62 | 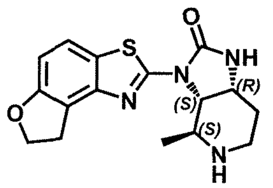 | LC-MS [M+H] $^{+}$/Rt (min) : 331. 2/0.446 (Method B) |
| 63 | 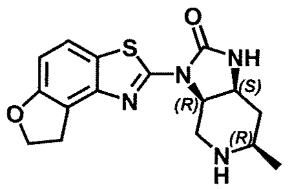 | LC-MS [M+H] $^{+}$/Rt (min) : 331. 2/0. 391 (Method B) |
| 64 | 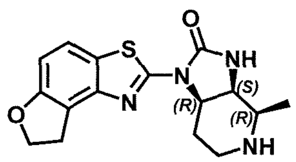 | LC-MS [M+H]$^{+}$/Rt (min) : 331. 2/0. 451 (Method B) |

[Table 64-4]

| | | |
|---|---|---|
| 65 | 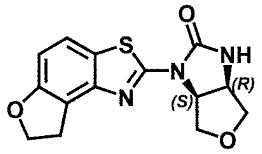 | LC-MS [M+H] $^{+}$/Rt (min) : 304. 3/1. 534 (Method D) ; $^{1}$H-NMR (DMSO-$d_6$) $\delta$: 8.14 (1H, s), 7.60 (1H, d, $J$ = 8. 5 Hz), 6. 75 (1H, d, $J$ = 8. 5 Hz), 5.08 (1H, dd, $J$ = 4. 4, 8.3 Hz), 4.61 (2H, t, $J$ = 9.0 Hz), 4. 40 (1H, dd, $J$ = 4. 1, 8.3 Hz), 4.19 (1H, d, $J$ = 10.4 Hz), 3.82 (1H, d, $J$ = 9. 8 Hz), 3. 71 (1H, dd, $J$ = 4.4, 10. 4 Hz), 3. 56 (1H, dd, $J$ = 4. 1, 9. 8 Hz), 3. 36 (2H, t, $J$ = 9. 0 Hz). |

(continued)

| | | |
|---|---|---|
| 66 | 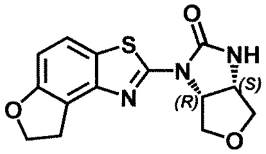 | LC-MS [M+H] +/Rt (min) : 304. 3/1. 534 (Method D) ; $^1$H-NMR (DMSO-$d_6$) $\delta$: 8.14 (1H, s), 7.60 (1H, d, *J* = 8. 5 Hz), 6. 75 (1H, d, *J* = 8. 5 Hz), 5.08 (1H, dd, *J* = 4. 5, 8.3 Hz), 4.61 (2H, t, *J* = 9.0 Hz), 4. 40 (1H, dd, *J* = 4.1, 8.3 Hz), 4.19 (1H, d, *J* = 10. 4 Hz), 3.82 (1H, d, *J* = 9. 8 Hz), 3. 71 (1H, dd, *J* = 4.5, 10.4 Hz), 3. 56 (1H, dd, *J* = 4.1, 9.8 Hz), 3. 36 (2H, t, *J* = 9.0 Hz). |

Examples 67 to 108

The compounds of Examples 67 to 108 were obtained by using the corresponding raw material compounds according to the method described in Example 7 or Example 8.

[Table 65-1]

| Example | Chemical structure | Physical property data |
|---|---|---|
| 67 | 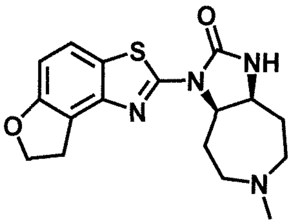 | LC-MS [M+H] +/Rt (min): 345.3/0.549 (Method A); $^1$H-NMR (DMSO-$d_6$) $\delta$: 7.90 (1H, s), 7.56 (1H, d, *J* = 8.5 Hz), 6.71 (1H, d, *J* = 8.5 Hz), 4.80-4.73 (1H, m), 4.64-4.54 (2H, m), 4.18-4.11 (1H, m), 3.34 (2H, t, *J* = 8. 9 Hz), 2.58-2.44 (2H, m), 2.42-2.32 (3H, m), 2.32-2.17 (1H, m), 2.20 (3H, s), 1.98-1.78 (2H, m). |
| 68 | 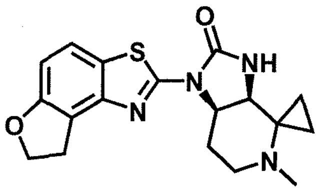 | LC-MS [M+H]+/Rt (min): 357.1/0.489 (Method B); $^1$H-NMR ($CDCl_3$) $\delta$: 7.46 (1H, d, *J* = 8.5 Hz), 6.77 (1H, d, *J* = 8.5 Hz), 4.97 (1H, s), 4. 93-4. 85 (1H, m), 4. 72-4. 62 (2H, m), 3. 45 (2H, t, *J* = 8. 7 Hz), 3. 16 (1H, d, *J* = 7. 3 Hz), 3. 04-2. 87 (2H, m), 2.54 (3H, s), 2.43-2.35 (1H, m), 2.07-1.97 (1H, m), 0.96-0.88 (1H, m), 0.76-0.71 (1H, m), 0.65-0.59 (1H, m), 0.52-0.47 (1H, m) . |
| 69 | 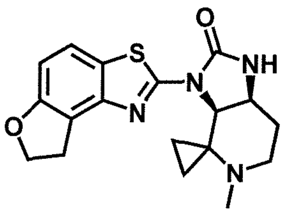 | LC-MS [M+H]+/Rt (min): 357.1/0.504 (Method B) ; $^1$H-NMR ($CDCl_3$) $\delta$: 7.45 (1H, d, *J* = 8. 5 Hz), 6.76 (1H, d, *J* = 8. 5 Hz), 4.85 (1H, s), 4.69-4.62 (3H, m), 4.28-4.24 (1H, m), 3.46 (2H, t, *J* = 8.9 Hz), 3.11-3.02 (1H, m), 2.98-2.91 (1H, m), 2.38 (3H, s), 2.37-2.30 (1H, m), 1.73-1.66 (1H, m), 1.37-1.22 (1H, m), 0.88-0.72 (2H, m), 0.65-0.59 (1H, m) . |
| 70 | 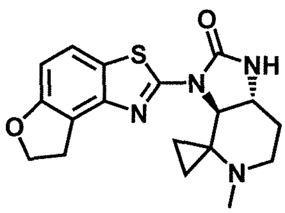 | LC-MS [M+H]+/Rt (min): 357.2/0.522 (Method B); $^1$H-NMR ($CDCl_3$) $\delta$: 7.45 (1H, d, *J* = 8.5 Hz), 6. 79 (1H, d, *J* = 8. 5 Hz), 5.19 (1H, s), 4. 67 (2H, t, *J* = 8. 7 Hz), 4. 56 (1H, d, *J* = 11.0 Hz), 3. 76-3. 65 (1H, m), 3. 56-3. 42 (2H, m), 3. 12-2. 90 (2H, m), 2. 69 (3H, s), 2.16-2.05 (1H, m), 1.89-1.83 (1H, m), 1.77-1.71 (1H, m), 0.78-0.69 (1H, m), 0.61-0.49 (2H, m) . |

(continued)

| Example | Chemical structure | Physical property data |
|---|---|---|
| 71 | 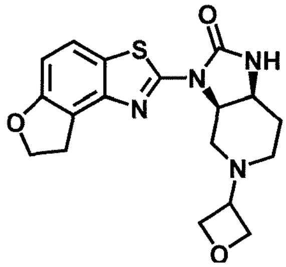 | LC-MS [M+H] +/Rt (min): 373.2/0.516 (Method B); $^1$H-NMR ($CDCl_3$) $\delta$: 7.45 (1H, d, *J* = 8. 2 Hz), 6. 76 (1H, d, *J* = 8.2 Hz), 4. 89-4. 83 (2H, m), 4.71-4.55 (6H, m), 4.13-4.07 (1H, m), 3. 54-3. 36 (4H, m), 2. 57-2. 54 (1H, m), 2.27-2.20 (1H, m), 2.16-2.04 (2H, m), 1.97-1. 92 (1H, m). |

[Table 65-2]

| | | |
|---|---|---|
| 72 | 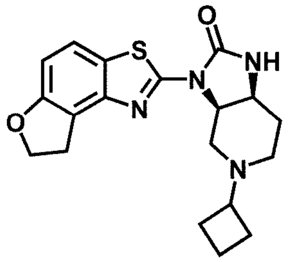 | LC-MS [M+H]+/Rt (min): 371.1/0.506 (Method B); $^1$H-NMR (DMSO-$d_6$) $\delta$: 7.74 (1H, s), 7.57 (1H, d, *J* = 8.2 Hz), 6.72 (1H, d, *J* = 8. 2 Hz), 4.66-4.56 (3H, m), 3.97-3.94 (1H, m), 3.48-3.23 (3H, m), 2.78-2.68 (1H, m), 2.56-2.45 (1H, m), 2.07-1.68 (8H, m), 1.65-1.50 (2H, m). |
| 73 | 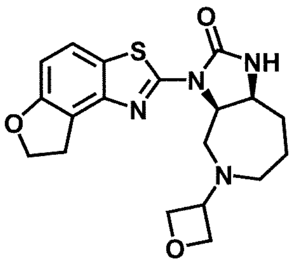 | LC-MS [M+H] +/Rt (min) : 387. 2/0. 496 (Method B); $^1$H-NMR (DMSO-$d_6$) $\delta$: 7.93 (1H, s), 7.56 (1H, d, *J* = 8.5 Hz), 6.71 (1H, d, *J* = 8.5 Hz), 4.74-4.67 (1H, m), 4.65-4.46 (5H, m), 4.27-4.21 (1H, m), 4.18-4.11 (1H, m), 3.71-3.64 (1H, m), 3.39-3.27 (2H, m), 3.26-3.17 (1H, m), 2.83-2.68 (1H, m), 2.53-2.40 (1H, m), 2.27-2.18 (1H, m), 2.00-1.89 (1H, m), 1.81-1.69 (2H, m), 1.53-1.45 (1H, m). |
| 74 | 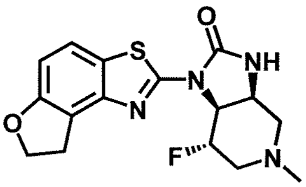 | LC-MS [M+H]+/Rt (min): 349.1/0.471 (Method B). $^1$H-NMR (DMSO-$d_6$) $\delta$: 7.83 (1H, s), 7.59 (1H, d, *J* = 8.2 Hz), 6.74 (1H, d, *J* = 8.2 Hz), 5.38-5.21 (1H, m), 4.76-4.65 (1H, m), 4.64-4.54 (2H, m), 4.04-3.99 (1H, m), 3.43-3.32 (2H, m), 3.04-2.94 (1H, m), 2.91-2.86 (1H, m), 2.43-2.27 (2H, m), 2.22 (3H, s). |
| 75 | 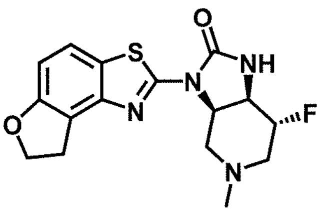 | LC-MS [M+H]+/Rt (min): 349. 1/0. 450 (Method B) . $^1$H-NMR (DMSO-$d_6$) $\delta$: 8.07 (1H, s), 7.59 (1H, d, *J* = 8.2 Hz), 6. 74 (1H, d, *J* = 8.2 Hz), 4.95-4.74 (2H, m), 4.66-4.55 (2H, m), 4.23-4.17 (1H, m), 3.45-3.33 (2H, m), 3.32-3.26 (1H, m), 2.93-2.85 (1H, m), 2.43-2.34 (1H, m), 2.29-2.22 (1H, m), 2.24 (3H, s). |
| 76 | 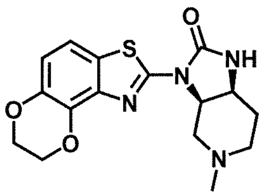 | LC-MS [M+H]+/Rt (min): 347.1/0.440 (Method B); $^1$H-NMR (DMSO-$d_6$) $\delta$: 7.71 (1H, s), 7.27 (1H, d, *J* = 8. 5 Hz), 6.79 (1H, d, *J* = 8.5 Hz), 4.64-4.57 (1H, m), 4.39-4.27 (4H, m), 3.99-3.93 (1H, m), 3.33-3.24 (1H, m), 2.56-2.50 (1H, m), 2.16 (3H, s), 2.13-2.04 (1H, m), 1.94-1.76 (3H, m). |

(continued)

| | | |
|---|---|---|
| 77 | 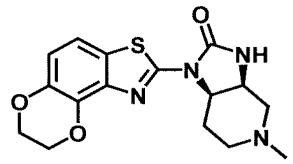 | LC-MS [M+H] +/Rt (min): 347.1/0.456 (Method B); 1H-NMR (DMSO-$d_6$) $\delta$: 7.82 (1H, s), 7.27 (1H, d, $J$ = 8. 5 Hz), 6. 78 (1H, d, $J$ = 8.5 Hz), 4.52-4.45 (1H, m), 4.38-4.23 (4H, m), 3.90-3.87 (1H, m), 2.76-2.71 (1H, m), 2.58-2.51 (1H, m), 2.38-2.25 (2H, m), 2.17 (3H, s), 2.04-1.95 (1H, m), 1.86-1.75 (1H, m). |

[Table 65-3]

| | | |
|---|---|---|
| 78 | 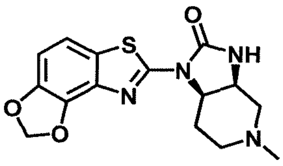 | LC-MS [M+H] +/Rt (min): 333. 1/0. 428 (Method B) ; 1H-NMR (DMSO-$d_6$) $\delta$: 7.90 (1H, s), 7.34 (1H, d, $J$ = 8. 2 Hz), 6.91 (1H, d, $J$ = 8.2 Hz), 6.10-6.07 (2H, m), 4.55-4.47 (1H, m), 3.93-3.88 (1H, m), 2.78-2.72 (1H, m), 2.59-2.52 (1H, m), 2. 37-2. 25 (2H, m), 2. 18 (3H, s), 2.03-1.96 (1H, m), 1. 83-1. 74 (1H, m). |
| 79 | 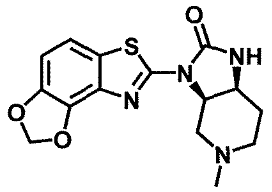 | LC-MS [M+H] +/Rt (min) : 333. 1/0. 440 (Method B) ; 1H-NMR (DMSO-$d_6$) $\delta$: 7.79 (1H, s), 7.35 (1H, d, $J$ = 8. 2 Hz), 6.91 (1H, d, $J$ = 8.2 Hz), 6. 11-6. 09 (2H, m), 4. 66-4. 59 (1H, m), 4.01-3.94 (1H, m), 3.30-3.24 (1H, m), 2.57-2.51 (1H, m), 2.17 (3H, s), 2.13-2.04 (1H, m), 1.97-1.77 (3H, m) . |
| 80 | 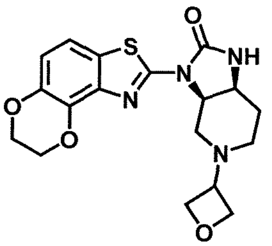 | LC-MS [M+H] +/Rt (min) : 389. 3/0. 450 (Method B) ; 1H-NMR (DMSO-$d_6$) $\delta$: 7. 76 (1H, s), 7. 27 (1H, d, $J$ = 8.6 Hz), 6. 78 (1H, d, $J$ = 8. 6 Hz), 4.66-4.60 (1H, m), 4.53-4.47 (2H, m), 4.40-4.25 (6H, m), 4.01-3.98 (1H, m), 3.52-3.46 (1H, m), 3.15-3.09 (1H, m), 2. 50-2. 48 (1H, m), 2. 24-2. 15 (2H, m), 1. 96-1. 76 (2H, m) . |
| 81 | 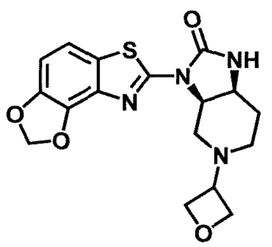 | LC-MS [M+H] +/Rt (min) : 375.2/0.458 (Method B) ;1H-NMR (DMSO-$d_6$) $\delta$: 7.84 (1H, s), 7.34 (1H, d, $J$ = 7.9 Hz), 6.91 (1H, d, $J$ = 7. 9 Hz), 6.11-6.08 (2H, m), 4.68-4.61 (1H, m), 4.54-4.48 (2H, m), 4.40-4.33 (2H, m), 4.03-4.00 (1H, m), 3.51-3.44 (1H, m), 3.16-3.11 (1H, m), 2.52-2.45 (1H, m), 2.21-2.12 (2H, m), 1.97-1.76 (2H, m). |
| 82 | 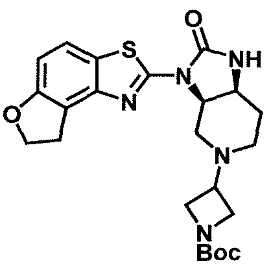 | LC-MS [M+H] +/Rt (min) : 472. 3/0. 723 (Method B); 1H-NMR ($CDCl_3$) $\delta$: 7.45 (1H, d, $J$ = 7. 9 Hz), 6. 77 (1H, d, $J$ = 7.9 Hz), 4. 99-4. 80 (2H, m), 4.67 (2H, t, $J$ = 9. 2 Hz), 4. 14-4.06 (1H, m), 3. 96-3. 74 (4H, m), 3. 53-3. 38 (1H, m), 3. 44 (2H, t, $J$ = 9. 2 Hz), 3. 16-3. 05 (1H, m), 2. 64-2. 54 (1H, m), 2. 29-1. 90 (4H, m), 1. 41 (9H, s). |

(continued)

| | | |
|---|---|---|
| 83 | 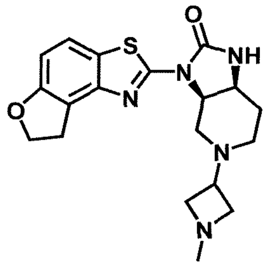 | LC-MS [M+H]$^+$/Rt (min) : 386. 3/0. 594 (Method A) ; $^1$H-NMR (CDCl$_3$) $\delta$: 7. 45 (1H, d, $J$ = 7. 9 Hz), 6. 77 (1H, d, $J$ = 7. 9 Hz), 5. 03 (1H, s), 4.83-4.76 (1H, m), 4.71-4.62 (2H, m), 4.11-4.04 (1H, m), 3.95-3.84 (2H, m), 3.55-3.22 (6H, m), 2. 60 (3H, s), 2. 55-2. 48 (1H, m), 2.36-2.25 (2H, m), 2.10-1.99 (1H, m), 1.97-1. 89 (1H, m) . |

[Table 65-4]

| | | |
|---|---|---|
| 84 | 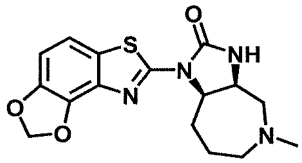 | LC-MS [M+H]$^+$/Rt (min) : 347. 2/0. 589 (Method A) ; $^1$H-NMR (DMSO-d$_6$) $\delta$: 7.86 (1H, s), 7.33 (1H, d, $J$ = 8. 2 Hz), 6. 90 (1H, d, $J$ = 8. 2 Hz), 6.08 (2H, s), 4.68-4.61 (1H, m), 4.12-4.05 (1H, m), 2.70-2.63 (1H, m), 2.60-2.51 (2H, m), 2. 39-2. 26 (2H, m), 2.30 (3H, s), 2.09-1.96 (1H, m), 1.69-1.44 (2H, m). |
| 85 | 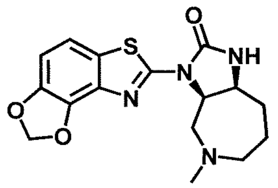 | LC-MS [M+H]$^+$/Rt (min) : 347. 2/0. 536 (Method A) ; $^1$H-NMR (DMSO-d$_6$) $\delta$: 7.90 (1H, s), 7.32 (1H, d, $J$ = 7. 9 Hz), 6. 89 (1H, d, $J$ = 7. 9 Hz), 6. 11-6. 07 (2H, m), 4. 68-4. 63 (1H, m), 4.18-4.11 (1H, m), 3.24-3.15 (1H, m), 2.92-2. 84 (1H, m), 2. 47-2. 36 (2H, m), 2.18 (3H, s), 1. 88-1. 69 (3H, m), 1. 51-1. 43 (1H, m) . |
| 86 | 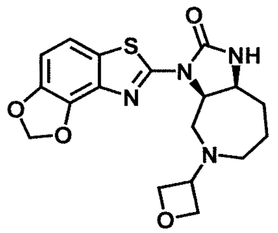 | LC-MS [M+H] $^+$/Rt (min): 389. 2/0. 562 (Method A); $^1$H-NMR (DMSO-d$_6$) $\delta$: 7.95 (1H, s), 7.33 (1H, d, $J$ = 7. 9 Hz), 6. 90 (1H, d, $J$ = 7. 9 Hz), 6.11-6.06 (2H, m), 4.71-4.66 (1H, m), 4.45-4.34 (3H, m), 4.21-4.16 (1H, m), 4.09-4.03 (1H, m), 3. 67-3. 60 (1H, m), 2. 99-2. 89 (2H, m), 2. 35-2. 26 (2H, m), 1. 92-1. 73 (3H, m), 1. 55-1. 45 (1H, m) . |
| 87 | 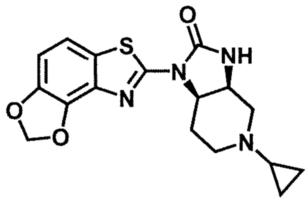 | LC-MS [M+H]$^+$/Rt (min): 359. 2/0. 582 (Method A); $^1$H-NMR (DMSO-d$_6$) $\delta$: 7.85 (1H, s), 7.34 (1H, d. $J$ = 8. 3 Hz), 6. 90 (1H, d, $J$ = 8. 3 Hz), 6. 10-6. 07 (2H, m), 4. 57-4. 50 (1H, m), 3.91-3.87 (1H, m), 2.98-2.89 (1H, m), 2.80-2.70 (1H, m), 2.61-2.53 (1H, m), 2.36-2.26 (2H, m), 1. 75-1. 65 (2H, m), 0.46-0. 39 (2H, m), 0.35-0. 24 (2H, m) . |
| 88 | 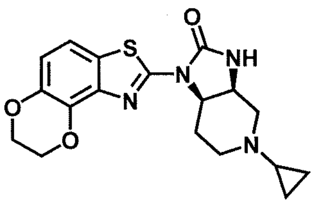 | LC-MS [M+H] $^+$/Rt (min) : 373. 2/0. 549 (Method A); $^1$H-NMR (DMSO-d$_6$) $\delta$: 7.77 (1H, s), 7.26 (1H, d, $J$ = 8. 6 Hz), 6. 78 (1H, d, $J$ = 8. 6 Hz), 4.54-4.47 (1H, m), 4.37-4.26 (4H, m), 3.90-3.83 (1H, m), 2.96-2.89 (1H, m), 2.76-2.70 (1H, m), 2.59-2.53 (1H, m), 2.35-2.25 (2H, m), 1. 75-1. 65 (2H, m), 0.45-0. 39 (2H, m), 0.34-0.23 (2H, m). |
| 89 | 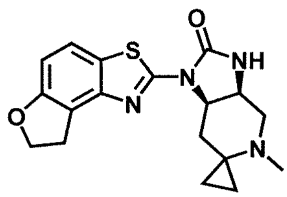 | LC-MS [M+H]$^+$/Rt (min): 357.1/0.507 (Method B) ; $^1$H-NMR (DMSO-d$_6$) $\delta$: 7.94 (1H, brs), 7.57 (1H, d, $J$ = 8. 2 Hz), 6. 72 (1H, d, $J$ = 8. 2 Hz), 4.74-4.67 (1H, m), 4.64-4.53 (2H, m), 3.83-3.80 (1H, m), 3.36-3.32 (2H, m), 3.01-2.89 (2H, m), 2.40 (3H, s), 2.16 (1H, m), 1.90-1.76 (1H, m), 0.58-0.30 (4H, m). |

[Table 65-5]

| | | |
|---|---|---|
| 90 | 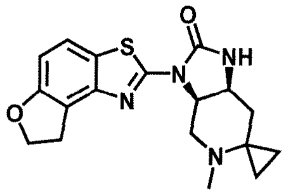 | LC-MS LC-MS [M+H] +/Rt (min): 357.2/0.462 (Method B) ; $^{1}$H-NMR (DMSO-$d_6$) $\delta$: 7.83 (1H, brs), 7.57 (1H, d, *J* = 8. 2 Hz), 6. 72 (1H, d, *J* = 8. 2 Hz), 4.81-4.72 (1H, m), 4.63-4.57 (2H, m), 4.08 (1H, m), 3. 40-3. 33 (2H, m), 2. 87-2. 81 (1H, m), 2.35-2.30 (1H, m), 2.34 (3H, s), 1.23-1.15 (1H, m), 0.58-0.28 (4H, m) . |
| 91 | 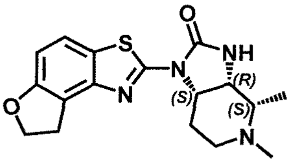 | LC-MS [M+H] +/Rt (min) : 345. 2/0. 450 (Method B) ; $^{1}$H-NMR (DMSO-$d_6$) $\delta$: 7.86 (1H, brs), 7. 57 (1H, d, J = 8. 2 Hz), 6. 73 (1H, d, J = 8. 2 Hz), 4.62-4.55 (3H, m), 3.77 (1H, m), 3.36 (2H, t, J = 8.9 Hz), 2.67 (1H, d, J = 11.0 Hz), 2.41-2.36 (1H, m), 2.18-2.14 (1H, m), 2.06-2.04 (1H, m), 1. 53-1. 50 (1H, m), 1. 13 (3H, d, J = 5.9 Hz). |
| 92 | 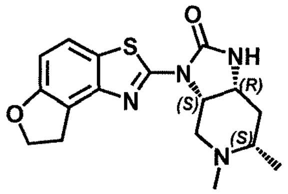 | LC-MS [M+H]+/Rt (min) : 345.3/0.442 (Method B) ; $^{1}$H-NMR ($CDCl_3$) $\delta$: 7.45 (1H, d, *J* = 8. 7 Hz), 6. 76 (1H, d, *J* = 8. 7 Hz), 4. 95 (1H, brs), 4. 70-4. 58 (3H, m), 3.95 (1H, dd, *J* = 13. 5, 7.1 Hz), 3.67 (1H, dd, *J* = 13.2, 5.0 Hz), 3.45 (2H, t, *J* = 8. 7 Hz), 2. 84 (1H, dd, *J* = 13. 2, 4.6 Hz), 2.60-2.38 (1H, m), 2.30 (3H, s), 2.13-2.01 (1H, m), 1.68-1.62 (1H, m), 1. 14 (3H, d, *J* = 6. 6 Hz). |
| 93 | 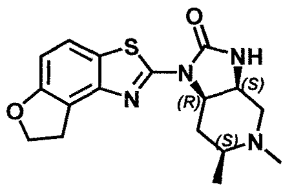 | LC-MS [M+H] + /Rt (min): 345.2/0.490 (Method B); $^{1}$H-NMR (DMSO-$d_6$) $\delta$: 7.82 (1H, brs), 7.58 (1H, d, *J* = 8.4 Hz), 6.72(1H, d, *J* = 8.4 Hz), 4. 69-4. 55 (3H, m), 3.90 (1H, m), 3. 45-3.34 (2H, m), 2.90 (1H, m), 2. 37-2. 30 (2H, m), 2. 17 (3H, s), 2. 15 (1H, s), 2.03-1.98 (1H, m), 1. 40-1. 32 (1H, m), 0.96 (3H, d, *J* = 6. 7 Hz). |
| 94 | 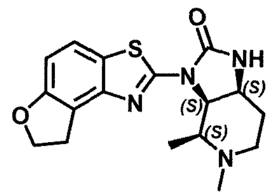 | LC-MS [M+H] +/Rt (min): 345. 2/0. 481 (Method B) ; $^{1}$H-NMR ($CDCl_3$) $\delta$: 7.46 (1H, d, *J* = 8.4 Hz), 6. 76 (1H, d, *J* = 8. 4 Hz), 4.91 (1H, t, *J* = 7. 1 Hz), 4. 72 (1H, brs), 4. 68-4. 61 (2H, m), 4.11 (1H, t, *J* = 6.2 Hz), 3. 87-3. 80 (1H, m), 3. 49-3. 42 (2H, m), 2. 79-2. 72 (1H, m), 2.42 (3H, s), 2.41-2.36 (1H, m), 2.15-2.05 (1H, m), 1. 87-1. 84 (1H, m), 0.89 (3H, d, *J* = 6. 8 Hz). |

[Table 65-6]

| | | |
|---|---|---|
| 95 | 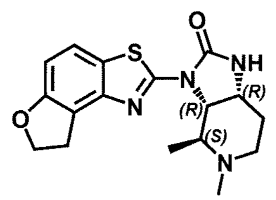 | LC-MS [M+H]+/Rt (min): 345.2/0.474 (Method B); $^{1}$H-NMR ($CDCl_3$) $\delta$: 7.47 (1H, d, *J* = 8.8 Hz), 6. 77 (1H, d, *J* = 8.8 Hz), 5. 30 (1H, brs), 4. 84-4. 77 (1H, m), 4.66 (2H, t, *J* = 8.7 Hz), 3. 55-3. 51 (1H, m), 3.47 (2H, t, *J* = 8.7 Hz), 3. 38-3. 33 (1H, m), 2.66-2.59 (1H, m), 2.47 (3H, s), 2.30-2.25 (1H, m), 2.18-2. 10 (1H, m), 1. 76-1. 66 (1H, m), 1.59 (3H, d, *J* = 6.0 Hz). |
| 96 | 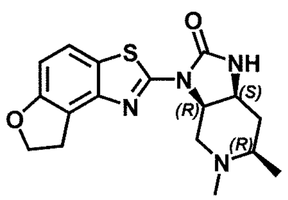 | LC-MS [M+H]+/Rt (min) : 345.1/0.449 (Method B); $^{1}$H-NMR ($CDCl_3$) $\delta$: 7.47 (1H, d, *J* = 8. 8 Hz), 6. 78 (1H, d, *J* = 8.8 Hz), 5.13 (1H, brs), 4.84-4.47 (3H, m), 3.95 (1H, dd, *J* = 12.8, 7.3 Hz), 3.70-3.61 (1H, m), 3.47-3.42 (2H, m), 2.84 (1H, dd, *J* = 13. 5, 4.3 Hz), 2. 60-2. 38 (1H, m), 2.31 (3H, s), 2.12-2.02 (1H, m), 1. 68-1. 61 (1H, m), 1. 14 (3H, d, *J* = 6. 4 Hz). |
| 97 | 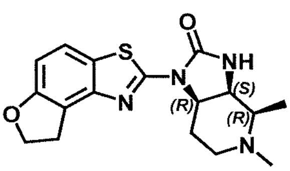 | LC-MS [M+H] +/Rt (min) : 345.1/0.453 (Method B); $^{1}$H-NMR ($CDCl_3$) $\delta$: 7.45 (1H, d, *J* = 8.8 Hz), 6. 76 (1H, d, *J* = 8.8 Hz), 5.01 (1H, brs), 4.74-4.61 (3H, m), 3.81-3.79 (1H, m), 3.44 (2H, t, *J* = 8.8 Hz), 2.87-2.82 (1H, m), 2.65-2.59 (1H, m), 2.31 (3H, s), 2.30-2.25 (1H, m), 2.19-2.12 (1H, m), 1.82-1.71 (1H, m), 1.26 (3H, d, *J* = 6.9 Hz). |

(continued)

| | | |
|---|---|---|
| 98 | 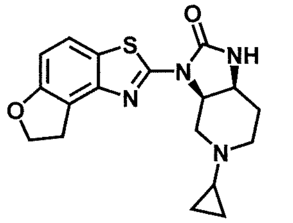 | LC-MS [M+H] +/Rt (min) : 357. 1/0. 489 (Method B); 1H-NMR ($CDCl_3$) $\delta$: 7.45 (1H, d, $J$ = 8.8 Hz), 6. 76 (1H, d, $J$ = 8.8 Hz), 4.81-4.74 (1H, m), 4. 69-4. 64 (3H, m), 4. 05-4.00 (1H, m), 3.73-3.69 (1H, m), 3.45 (2H, t, $J$ = 8.7 Hz), 2. 85-2. 81 (1H, m), 2. 53-2. 46 (1H, m), 2.39-2.29 (1H, m), 2.01-1.83 (2H, m), 1.69-1.61 (1H, m), 0.48-0.38 (4H, m) . |
| 99 | 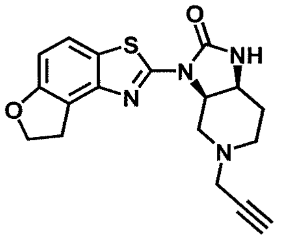 | LC-MS [M+H]+/Rt (min) : 355.1/0.530 (Method B); 1H-NMR ($CDCl_3$) $\delta$: 7.47 (1H, d, $J$ = 8. 4 Hz), 6. 76 (1H, d, $J$ = 8.4 Hz), 4.91-4.85 (1H, m), 4.74 (1H, brs), 4.68-4.62 (2H, m), 4.09-4.05 (1H, m), 3.59-3.51 (1H, m), 3.49-3.41 (3H, m), 3.37-3.35 (2H, m), 2.72-2.68 (1H, m), 2.57-2.51 (1H, m), 2. 24-2. 22 (1H, m), 2. 13-2. 03 (1H, m), 1. 96-1. 92 (1H, m). |

[Table 65-7]

| | | |
|---|---|---|
| 100 | 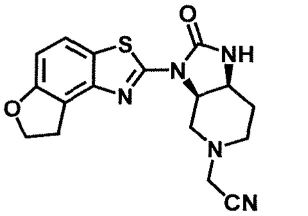 | LC-MS [M+H] +/Rt (min) : 356. 1/0. 681 (Method B) ; 1H-NMR ($CDCl_3$) $\delta$ : 7.46 (1H, d, $J$ = 8. 2 Hz), 6. 78 (1H, d, $J$ = 8. 2 Hz), 4. 90-4. 82 (2H, m), 4. 67 (2H, t, $J$ = 8. 2 Hz), 4. 11-4. 08 (1H, m), 3. 59-3. 43 (3H, m), 3. 47-3. 43 (2H, m), 2.71-2.66 (2H, m), 2.63-2.58 (1H, m), 2.15-2.03 (1H, m), 2.00-1.94 (1H, m). |
| 101 | 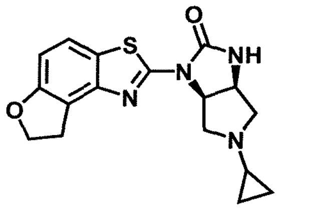 | LC-MS [M+H]+/Rt (min): 343.1/0.502 (Method B); 1H-NMR (DMSO-$d_6$) $\delta$: 8.00 (1H, brs), 7.56 (1H, d, $J$ = 8. 2 Hz), 6. 72 (1H, d, $J$ = 8. 2 Hz), 4. 95-4. 89 (1H, m), 4. 62-4. 57 (2H, m), 4. 26-4. 22 (1H, m), 3. 39-3. 22 (4H, m), 2.86 (1H, d, $J$ = 10.1 Hz), 2. 68-2. 64 (1H, m), 2. 53-2. 49 (1H, m), 1.65-1.60 (1H, m), 0.44-0.23 (4H, m). |
| 102 | 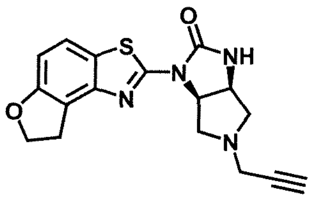 | LC-MS [M+H]+/Rt (min): 341. 1/0. 544 (Method B) ; 1H-NMR (DMSO-$d_6$) $\delta$: 8. 06 (1H, s), 7. 57 (1H, d, $J$ = 8. 2 Hz), 6. 72 (1H, d, $J$ = 8. 2 Hz), 4. 97-4. 92 (1H, m), 4.62-4.57 (2H, m), 4. 29-4. 25 (1H, m), 3. 43-3. 42 (2H, m), 3. 36 (2H, t, $J$ = 8. 9 Hz), 3. 26-3. 21 (1H, m), 3. 16 (1H, t, $J$ = 2. 3 Hz), 2. 83-2. 81 (1H, m), 2. 58-2.54 (1H, m), 2.46-2.41 (1H, m). |
| 103 | 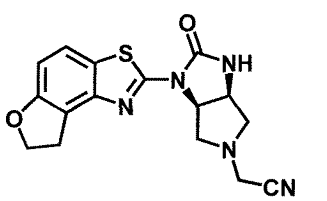 | LC-MS [M+H] +/Rt (min) : 342. 1/0. 656 (Method B) ; 1H-NMR (DMSO-$d_6$) $\delta$: 8. 11 (1H, s), 7. 58 (1H, d, $J$ = 8. 2 Hz), 6. 73 (1H, d, $J$ = 8. 2 Hz), 5.01-4.97 (1H, m), 4.61-4.57 (2H, m), 4.34-4.31 (1H, m), 3.96-3.85 (2H, m), 3.39-3. 31 (3H, m), 2.88 (1H, d, $J$ = 10.1 Hz), 2.60-2.55 (1H, m), 2.51-2.44 (1H, m) . |

(continued)

| | | |
|---|---|---|
| 104 | 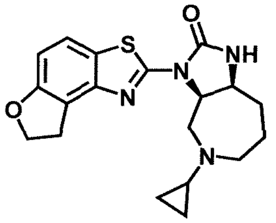 | LC-MS [M+H]+/Rt (min) : 371. 2/0. 525 (Method B); $^1$H-NMR ($CDCl_3$) $\delta$: 7.46 (1H, d, *J* = 8.5 Hz), 6. 76 (1H, d, *J* = 8. 5 Hz), 4. 82-4. 77 (2H, m), 4.67 (2H, t, *J* = 8.8 Hz), 4. 21-4. 10 (1H, m), 3.69-3.64 (1H, m), 3.47 (2H, t, *J* = 8.5 Hz), 3.01-2.96 (2H, m), 2.60-2.56 (1H, m), 2. 05-1. 46 (5H, m), 0.65-0. 32 (4H, m) . |
| 105 | 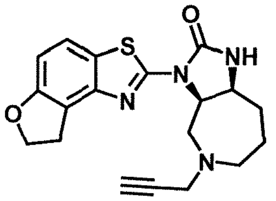 | LC-MS [M+H] +/Rt (min) : 369. 3/0. 528 (Method B) ; $^1$H-NMR ($CDCl_3$) $\delta$: 7.45 (1H, d, *J* = 8.5 Hz), 6. 79 (1H, d, *J* = 8.5 Hz), 5. 89-5. 78 (1H, m), 5.09-5.00 (1H, m), 4.67 (2H, t, *J* = 10.4 Hz), 4.54-4.40 (2H, m), 4.03-3.92 (2H, m), 3. 64-3. 52 (1H, m), 3. 50-3. 37 (3H, m), 3.28-3.18 (1H, m), 2.47-1.82 (5H, m). |

[Table 65-8]

| | | |
|---|---|---|
| 106 | 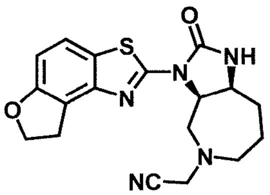 | LC-MS [M+H] + /Rt (min): 370. 2/0.731 (Method B); $^1$H-NMR ($CDCl_3$) $\delta$ : 7.46 (1H, d, *J* = 8.5 Hz), 6. 77 (1H, d, *J* = 8.5 Hz), 4. 86-4. 78 (2H, m), 4. 67 (2H, t, *J* = 8. 8 Hz), 4. 28-4. 24 (1H, m), 3. 65-3. 58 (1H, m), 3. 53-3. 36 (4H, m), 3. 19-3. 16 (1H, m), 2. 72-2. 71 (2H, m), 1.90-1.74 (4H, m). |
| 107 | 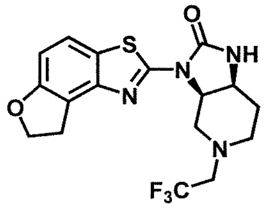 | LC-MS [M+H] + /Rt (min): 399. 2/0.881 (Method B); $^1$H-NMR ($CDCl_3$) $\delta$ : 7.45 (1H, d, *J* = 7. 3 Hz), 6.77 (1H, d, *J* = 8.5 Hz), 4. 89-4. 83 (1H, m), 4. 70-4. 63 (3H, m), 4.10-4.06 (1H, m), 3.70-3.67 (1H, m), 3.45 (2H, t, *J* = 8.8 Hz), 3.08-2.99 (2H, m), 2.86-2.59 (3H, m), 2.14-1.88 (2H, m). |
| 108 | 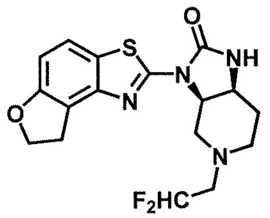 | LC-MS [M+H] + /Rt (min): 381. 1/0. 730 (Method B); $^1$H-NMR ($CDCl_3$) $\delta$ : 7.45 (1H, d, *J* = 8.4 Hz), 6. 77 (1H, d, *J* = 8.4 Hz), 6. 05-5. 77 (1H, m), 4. 92-4. 78 (2H, m), 4. 67 (2H, t, *J* = 9.2 Hz), 4. 09-4.05 (1H, m), 3. 68-3. 65 (1H, m), 3. 45 (2H, t, *J* = *9. 2* Hz), 2. 84-2. 77 (3H, m), 2. 63-2. 50 (2H, m), 2.21-2.04 (1H, m), 1. 94-1. 90 (1H, m). |

Example 109

(3aS,7aR)-5-Cyclopropyl-3-(7,8-dihydrofuro[3,2-e][1,3]benzothiazol-2-yl)octahydro-2H-imidazo[4,5-c]pyridin-2-one

Example 110

(3aR,7aS)-5-Cyclopropyl-3-(7,8-dihydrofuro[3,2-e][1,3]benzothiazol-2-yl)octahydro-2H-imidazo[4,5-c]pyridin-2-one

[Formula 73]

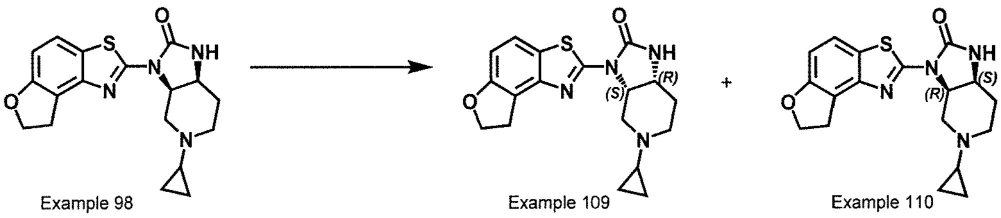

Example 98 Example 109 Example 110

The compound of Example 98 (12.0 mg) was optically fractionated under the following conditions to obtain the title compounds (Example 109: 1.5 mg-first peak: 9.40 min, Example 110: 1.1 mg-second peak: 10.8 min). Column: CHIRALPAK (R) IG; Solvents: liquid A: chloroform, liquid B: methanol, liquid C: diethylamine; Mobile phase condition: A/B/C = 99/1/0.002; Flow rate: 7 mL/min; Detection UV: 280 nm; Column temperature: 40°C

[Table 66]

| Example | Physical property data |
|---|---|
| 109 | LC-MS $[M+H]^+$/Rt (min): 357.2/0.515 (Method B); $^1$H-NMR ($CDCl_3$) δ: 7.45 (1H, d, *J* = 8.2 Hz), 6.76 (1H, d, *J* = 8.2 Hz), 4.82-4.62 (4H, m), 4.07-4. 01 (1H, m), 3. 76-3. 65 (1H, m), 3. 56-3. 42 (2H, m), 2. 87-2. 78 (1H, m), 2.55-2.33 (2H, m), 2.02-1.84 (2H, m), 1.67-1.60 (1H, m), 0.48-0.35 (4H, m). |
| 110 | LC-MS $[M+H]^+$ /Rt (min): 357. 1/0. 513 (Method B) ; 'H-NMR ($CDCl_3$) δ : 7. 45 (1H, d, *J* = 8.5 Hz), 6.76 (1H, d, *J* = 8.5 Hz), 4. 83-4. 62 (4H, m), 4.07-4.00 (1H, m), 3.75-3.68 (1H, m), 3.57-3.42 (2H, m), 2.88-2.79 (1H, m), 2.56-2.32 (2H, m), 2.02-1.84 (2H, m), 1.68-1.61 (1H, m), 0.49-0.36 (4H, m). |

Example 111

[(3aS,7aR)-3-(7,8-Dihydrofuro[3,2-e] [1,3]benzothiazol-2-yl)-2-oxooctahydro-5H-imidazo[4,5-c]pyridin-5-yl]acetonitrile

Example 112

[(3aR,7aS)-3-(7,8-Dihydrofuro[3,2-e][1,3]benzothiazol-2-yl)-2-oxooctahydro-5H-imidazo[4,5-c]pyridin-5-yl]acetonitrile

[Formula 74]

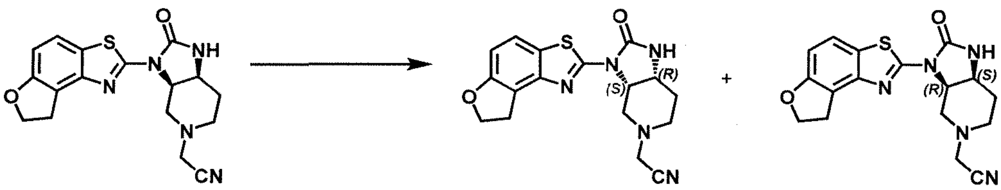

Example 100 Example 111 Example 112

The compound of Example 100 (30.0 mg) was optically fractionated under the following conditions to obtain the title compounds (Example 111: 13.8 mg-First Peak: 13.0 min, Example 112: 12.2 mg-Second Peak: 14.5 min). Column: CHIRALPAK (R) IC; Solvents: liquid A: chloroform, liquid B: methanol, liquid C: diethylamine; Mobile phase condition: A/B/C = 98/1/0.001; Flow rate: 10 mL/min; Detection UV: 254 nm; Column temperature: 40°C

[Table 67]

| Example | Physical property data |
|---|---|
| 111 | LC-MS $[M+H]^+$/Rt (min): 356.2/0.755 (Method A); $^1$H-NMR (DMSO-$d_6$) δ: 7.81 (1H, s), 7. 59 (1H, d, *J* = 8.5 Hz), 6. 74 (1H, d, *J* = 8.5 Hz), 4. 75-4. 68 (1H, m), 4.66-4.56 (2H, m), 4.03-3.98 (1H, m), 3.78 (2H, s), 3.43-3.34 (3H, m), 2. 66-2. 59 (1H, m), 2. 42-2. 35 (1H, m), 2.22 (1H, dd, *J* = 8. 9, 11.3 Hz), 1. 98-1. 81 (2H, m). |

(continued)

| Example | Physical property data |
|---|---|
| 112 | LC-MS [M+H]+/Rt (min): 356.2/0.755 (Method A) ; $^1$H-NMR (DMSO-$d_6$) δ: 7.81 (1H, s), 7. 59 (1H, d, *J* = 8.5 Hz), 6. 74 (1H, d, *J* = 8.5 Hz), 4. 75-4. 68 (1H, m), 4. 66-4. 56 (2H, m), 4. 03-3. 98 (1H, m), 3. 78 (2H, s), 3. 43-3. 34 (3H, m), 2.66-2.59 (1H, m), 2.42-2.35 (1H, m), 2.22 (1H, dd, *J* = 9.2, 11. 0 Hz), 1. 98-1. 81 (2H, m). |

Example 113

(3aS,7aR)-3-(7,8-Dihydrofuro[3,2-e][1,3]benzothiazol-2-yl)-5-(2-propyn-1-yl)octahydro-2H-imidazo[4,5-c]pyridin-2-one

Example 114

(3aR,7aS)-3-(7,8-Dihydrofuro[3,2-e][1,3]benzothiazol-2-yl)-5-(2-propyn-1-yl)octahydro-2H-imidazo[4,5-c]pyridin-2-one

[Formula 75]

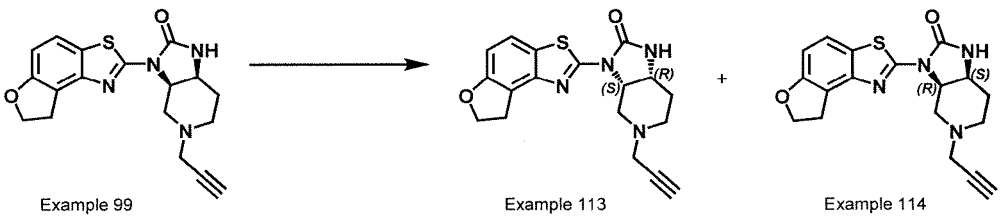

Example 99 Example 113 Example 114

The compound of Example 99 (30.0 mg) was optically fractionated under the following conditions to obtain the title compounds (Example 113: 15.4 mg-First Peak: 10.2 min, Example 114: 13.7 mg-Second Peak: 11.3 min). Column: CHIRALPAK (R) IC; Solvents: liquid A: chloroform, liquid B: methanol, liquid C: diethylamine; Mobile phase condition: A/B/C = 99/1/0.001; Flow rate: 10 mL/min; Detection UV: 254 nm; Column temperature: 40°C

[Table 68]

| Example | Physical property data |
|---|---|
| 113 | LC-MS [M+H] + /Rt (min): 355. 2/0. 608 (Method A) ; $^1$H-NMR (DMSO-$d_6$) δ: 7. 76 (1H, s), 7. 58 (1H, d, *J* = 8.5 Hz), 6. 73 (1H, d, *J* = 8.5 Hz), 4. 70-4. 56 (3H, m), 4. 01-3. 95 (1H, m), 3. 41-3. 35 (3H, m), 3. 32-3. 30 (2H, m), 3.14 (1H, t, *J* = 2. 1 Hz), 2. 62-2. 55 (1H, m), 2. 38-2. 30 (1H, m), 2.17 (1H, dd, *J* = 9.2, 11.6 Hz), 1.94-1.79 (2H, m). |
| 114 | LC-MS [M+H]+/Rt (min): 355.2/0.610 (Method A); $^1$H-NMR (DMSO-$d_6$) δ: 7.76 (1H, s), 7.58 (1H, d, *J* = 8.5 Hz), 6.73 (1H, d, *J* = 8.5 Hz), 4.70-4.56 (3H, m), 4.01-3.95 (1H, m), 3.41-3.35 (3H, m), 3.32 (2H, d *J* = 2.1 Hz), 3.14 (1H, t, *J* = 2.1 Hz), 2.62-2.55 (1H, m), 2.38-2.30 (1H, m), 2.17 (1H, dd, *J* = 9.2, 11.6 Hz), 1. 94-1. 79 (2H, m). |

Examples 115 to 118

The compounds of Examples 115 to 118 were obtained by using the corresponding raw material compounds according to the method described in Example 19.

[Table 69]

| Example | Chemical structure | Physical property data |
|---|---|---|
| 115 | 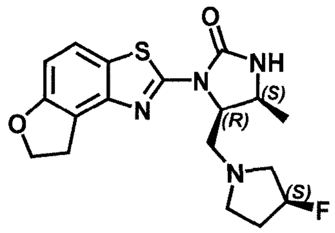 | LC-MS [M+H] + /Rt (min): 377.2/0.491 (Method B); $^{1}$H-NMR ($CDCl_3$) δ : 7. 46 (1H, d, *J* = 8.4 Hz), 6. 76 (1H, d, *J* = 8.4 Hz), 5.21-5.07 (1H, m), 4.81-4.73 (2H, m), 4.67 (2H, t, *J* = 8.8 Hz), 4.28-4.19 (1H, m), 3.51-3.35 (2H, m), 3.15-3.04 (2H, m), 2. 96-2. 75 (3H, m), 2.61-2.54 (1H, m), 2.18-1.93 (2H, m), 1.46 (3H, d, *J* = 6. 7 Hz) . |
| 116 | 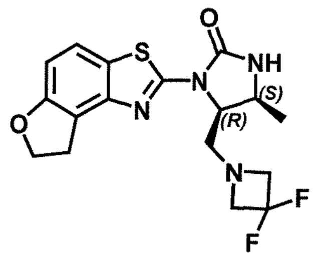 | LC-MS [M+H]$^+$/Rt (min): 381.2/0.652 (Method B); $^{1}$H-NMR ($CDCl_3$) δ : 7.48 (1H, d, *J* = 8.4 Hz), 6. 78 (1H, d, *J* = 8.4 Hz), 4. 85 (1H, brs), 4.69 (2H, t, *J* = 7.9 Hz), 4.59-4.50 (2H, m), 4. 29-4. 21 (1H, m), 4. 03-3. 63 (3H, m), 3. 47-3. 42 (2H, m), 3. 33-3. 05 (2H, m), 1.47 (3H, d, *J* = 6. 7 Hz). |
| 117 | 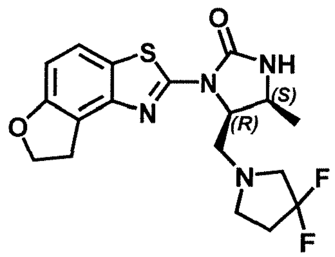 | LC-MS [M+H]$^+$/Rt (min): 395. 2/0. 685 (Method B); $^{1}$H-NMR ($CDCl_3$) δ: 7.46 (1H, d, *J* = 8.8 Hz), 6. 78 (1H, d, *J* = 8.8 Hz), 4. 87 (1H, br s), 4. 79-4. 65 (4H, m), 4. 34-4. 21 (1H, m), 3. 46-3. 40 (2H, m), 3. 32-2. 81 (5H, m), 2.38-2.20 (2H, m), 1. 47 (3H, d, *J* = 7. 0 Hz). |
| 118 | 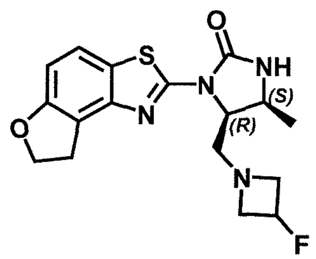 | LC-MS [M+H]$^+$/Rt (min): 363.1/0.490 (Method B); $^{1}$H-NMR ($CDCl_3$) δ: 7.46 (1H, d, *J* = 8.5 Hz), 6.77 (1H, d, *J* = 8.5 Hz), 5.13-4.98 (1H, m), 4.86 (1H, brs), 4.68 (2H, t, J = 8.8 Hz), 4.59-4.54 (1H, m), 4.22-4.16 (1H, m), 3. 78-3. 62 (2H, m), 3. 55-3. 34 (3H, m), 3.21-3.02 (2H, m), 2.92-2.88 (1H, m), 1.42 (3H, d, *J* = 6. 7 Hz) . |

Examples 119 to 124

The compounds of Examples 119 to 124 were obtained by using the corresponding raw material compounds according to the method described in Example 21.

[Table 70]

| Example | Chemical structure | Physical property data |
|---|---|---|
| 119 | 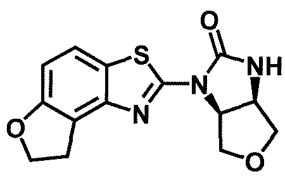 | LC-MS [M+H]$^+$/Rt (min): 304.0/2.907 (Method C); $^{1}$H-NMR (DMSO-$d_6$) δ: 8.15 (1H, s), 7.60 (1H, td, *J* = 0. 8, 8.4 Hz), 6.75 (1H, d, *J* = 8.4 Hz), 5.08 (1H, dd, *J* = 4. 4, 8. 3 Hz), 4. 65-4. 54 (2H, m), 4. 40 (1H, dd, *J* = 4. 1, 8. 3 Hz), 4. 19 (1H, d, *J* = 10. 4 Hz), 3. 82 (1H, d, *J* = 10.0 Hz), 3. 70 (1H, dd, *J* = 4. 7, 10. 4 Hz), 3. 56 (1H, dd, *J* = 4.1, 10.0 Hz), 3. 41-3. 26 (2H, m). |

(continued)

| Example | Chemical structure | Physical property data |
|---|---|---|
| 120 | 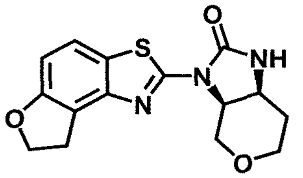 | LC-MS $[M+H]^+$ /Rt (min): 318. 2/1. 43 (Method E); $^1$H-NMR (DMSO-$d_6$) δ: 7.90 (1H, s), 7. 61-7.57 (1H, m), 6.74 (1H, d, *J* = 8.4 Hz), 4.70-4.55 (3H, m), 4.21-4.10 (2H, m), 3.77-3.67 (2H, m), 3.65-3.56 (1H, m), 3.41-3.34 (2H, m), 2. 07-1. 95 (1H, m), 1. 76-1. 68 (1H, m). |
| 121 | 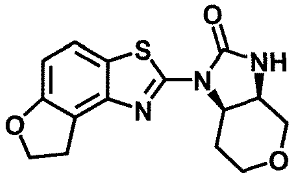 | LC-MS $[M+H]^+$ /Rt (min): 318. 2/1. 38 (Method E); $^1$H-NMR (DMSO-$d_6$) δ: 7. 90 (1H, s), 7.59 (1H, dd, *J* = 0.9, 8.4 Hz), 6.74 (1H, d, *J* = 8.4 Hz), 4.84-4.74 (1H, m), 4.68-4.54 (2H, m), 3.87-3.72 (3H, m), 3. 71-3. 62 (1H, m), 3. 50-3. 27 (3H, m), 2. 43-2. 31 (1H, m), 1. 85-1. 70 (1H, m) . |
| 122 | 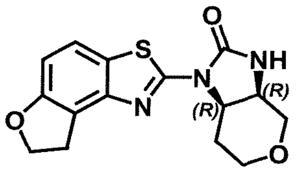 | LC-MS $[M+H]^+$/Rt (min): 318.2/1.57 (Method E); $^1$H-NMR (DMSO-$d_6$) δ: 7.91 (1H, s), 7.60 (1H, d, *J* = 8.4 Hz), 6.74 (1H, d, *J* = 8.4 Hz), 4.84-4.74 (1H, m) , 4.61 (1H, td, *J* = 2. 8, 8.8 Hz), 3. 87-3. 69 (3H, m), 3.66 (1H, dd, *J* = 3. 1, 13.2 Hz), 3. 53-3. 29 (3H, m), 2.43-2.31 (1H, m), 1.83-1.72 (1H, m). |
| 123 | 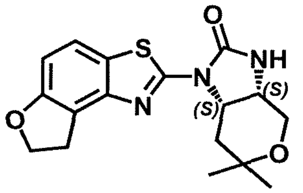 | LC-MS $[M+H]^+$ /Rt (min): 346. 2/2. 157 (Method C); $^1$H-NMR (DMSO-$d_6$) δ: 7.91 (1H, s), 7. 58 (1H, d, *J* = 8. 4 Hz), 6. 73 (1H, d, *J* = 8. 4 Hz), 4.93-4.77 (1H, m), 4.68-4.52 (2H, m), 3.92-3.89 (1H, m), 3.75 (1H, dd, *J* = 3.1, 12.9 Hz), 3.55 (1H, dd, *J* = 2. 6, 12.9 Hz), 3.38 (1H, t, *J* = 9.2 Hz), 2. 16-2. 04 (2H, m), 1. 19 (3H, s), 1. 04 (3H, s). |
| 124 | 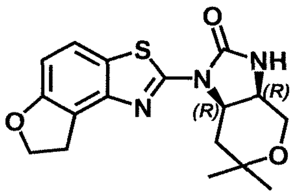 | LC-MS $[M+H]^+$/Rt (min): 346. 1/2. 157 (Method C); $^1$H-NMR (DMSO-$d_6$) δ: 7.92 (1H, s), 7.62-7.55 (1H, m), 6.74 (1H, d, *J* = 8.4 Hz), 4.88-4.83 (1H, m), 4.69-4.50 (2H, m), 3.92-3. 89 (1H, m), 3.75 (1H, dd, *J* = 3.2, 12.9 Hz), 3.55 (1H, dd, *J* = 2.6, 12.9 Hz), 3.38 (1H, t, *J* = 9. 2 Hz), 2.16-2.04 (2H, m), 1.19 (3H, s), 1. 04 (3H, s). |

Examples 125 to 140

The compounds of Examples 125 to 140 were obtained by using the corresponding raw material compounds according to the method described in Reference Example 14.

[Table 71-1]

| Example | Chemical structure | Physical property data |
|---|---|---|
| 125 | 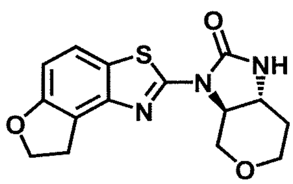 | LC-MS $[M+H]^+$ /Rt (min): 318.2/1.69 (Method E); $^1$H-NMR (DMSO-$d_6$) δ: 8.07 (1H, s), 7.65-7.59 (1H, m), 6.77 (1H, d, *J* = 8.4 Hz), 5.10-5.03 (1H, m), 4.66-4.57 (2H, m), 4.04-3.97 (1H, m), 3. 79-3.65 (2H, m), 3. 64-3. 46 (2H, m), 3.38 (2H, t, *J* = 8.8 Hz), 2.00-1.92 (1H, m), 1.85-1.72 (1H, m). |
| 126 | 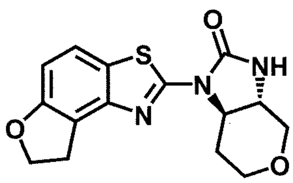 | LC-MS $[M+H]^+$ /Rt (min): 318.2/1.64 (Method E); $^1$H-NMR (DMSO-$d_6$) δ: 7. 87 (1H, s), 7.65-7.59 (1H, m), 6.77 (1H, d, *J* = 8.4 Hz), 4. 64-4. 57 (2H, m), 4. 11-4.00 (2H, m), 3.98-3.89 (1H, m), 3.53-3. 41 (2H, m), 3. 36 (2H, t, *J* = 8. 8 Hz), 3.07-2.96 (1H, m), 2.09-1.96 (1H, m). |

(continued)

| Example | Chemical structure | Physical property data |
|---|---|---|
| 127 | 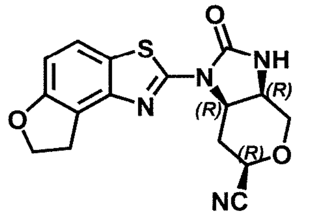 | LC-MS [M+H] + /Rt (min): 343. 1/2. 023 (Method C); $^1$H-NMR (DMSO-$d_6$) δ: 8.02 (1H, s), 7.61 (1H, d, *J* = 8. 4 Hz), 6. 76 (1H, d, *J* = 8. 4 Hz), 4. 97-4. 83 (2H, m), 4. 62 (1H, ddd, *J* = 3. 5, 8. 0, 9. 3 Hz), 3. 96 (1H, dd, *J* = 2. 5, 8. 5 Hz) , 3.82 (2H, d, *J* = 2. 4 Hz), 3. 46-3. 33 (2H, m), 2.77-2.63 (1H, m), 2.32 (1H, td, *J= 7. 4,* 14. 5 Hz). |
| 128 | 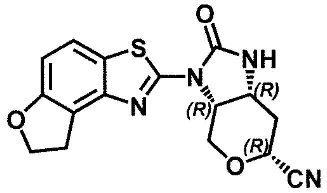 | LC-MS [M+H] + /Rt (min): 343. 1/2. 057 (Method C); $^1$H-NMR (DMSO-$d_6$) δ: 8. 15 (1H, s), 7.61 (1H, d, *J* = 8.4 Hz), 6.75 (1H, d, *J* = 8.4 Hz), 5.08 (1H, dd, *J* = 3.8, 6.8 Hz), 4.74 (1H, td, *J* = 4. 8, 8.6 Hz), 4. 67-4. 55 (2H, m), 4. 27-4. 08 (3H, m), 3.44-3.31 (2H, m), 2.45-2.24 (1H, m), 2.21-1.99 (1H, m). |
| 129 | 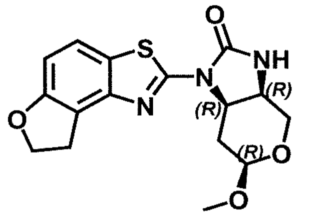 | LC-MS [M+H] + /Rt (min): 348. 1/1. 49 (Method E); $^1$H-NMR (DMSO-$d_6$) δ : 7.94 (1H, s), 7.59 (1H, d, *J* = 8. 4 Hz), 6. 74 (1H, d, *J* = 8. 4 Hz), 4. 78 (1H, tdd, *J* = 2. 7, 5. 3, 8.8 Hz), 3. 86-3. 71 (2H, m), 3.38 (1H, dt, *J* = 4. 9, 8.6 Hz), 3.30 (3H, s), 2. 65 (1H, ddd, *J* = 3. 7, 5.9, 13.7 Hz), 1. 71 (1H, ddd, *J* = 8. 0, 9. 5, 13. 7 Hz). |

[Table 71-2]

| | | |
|---|---|---|
| 130 | 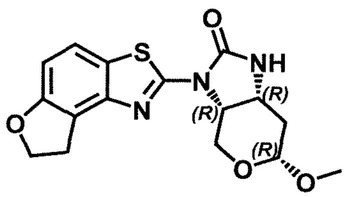 | LC-MS [M+H] + /Rt (min): 348.1/1.80 (Method E); $^1$H-NMR (DMSO-$d_6$) δ: 7.94 (1H, s), 7.59 (1H, d, *J* = 8.4 Hz), 6.74 (1H, d, *J* = 8.4 Hz), 4.78 (1H, tdd, *J* = 2.7, 5.3, 8.8 Hz), 3. 86-3. 71 (2H, m), 3.38 (1H, dt, *J* = 4.9, 8.6 Hz), 3.30 (3H, s), 2.65 (1H, ddd, *J* = 3.7, 5.9, 13.7 Hz), 1.71 (1H, ddd, *J* = 8. 0, 9.5, 13.7 Hz). |
| 131 | 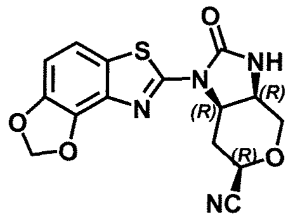 | LC-MS [M+H] + /Rt (min): 345. 1/2. 023 (Method C) ; $^1$H-NMR (DMSO-$d_6$) δ: 8.08 (1H, s), 7. 38 (1H, d, *J* = 8. 3 Hz), 6. 94 (1H, d, *J* = 8. 3 Hz), 6.17-6.02 (2H, m), 4.99-4. 78 (2H, m), 3.99 (1H, dd, *J* = 2. 7, 8.7 Hz), 3.84 (1H, dd, *J* = 2. 7, 13.0 Hz), 3. 78 (1H, dd, *J* = 2. 3, 13.0 Hz), 2. 72-2.61 (1H, m), 2.44-2.35 (1H, m). |
| 132 | 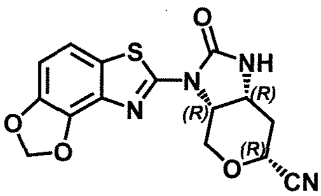 | LC-MS [M+H] + /Rt (min): 345. 1/2. 023 (Method C) ; $^1$H-NMR (DMSO-$d_6$) δ : 8.20 (1H, s), 7. 38 (1H, d, *J* = 8. 3 Hz), 6. 94 (1H, d, *J* = 8. 3 Hz), 6.18-6.01 (2H, m), 5. 08 (1H, dd, *J* = 3. 6, 7.0 Hz), 4. 73 (1H, td, *J* = 4. 5, 8.9 Hz), 4. 22 (1H, dd, *J* = 4. 3, 9.0 Hz), 4. 12 (1H, d, *J* = 4.5), 2. 46-2. 34 (1H, m), 2.05 (1H, td, *J* = 3. 9, 15.2 Hz). |
| 133 | 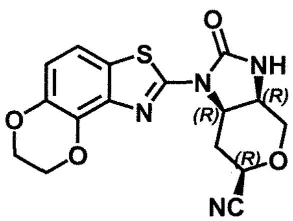 | LC-MS [M+H] + /Rt (min): 359. 1/1. 973 (Method C) ; $^1$H-NMR (DMSO-$d_6$) δ: 8.00 (1H, s), 7.30 (1H, d, *J* = 8.6 Hz), 6.81 (1H, d, *J* = 8. 6 Hz), 4. 96-4. 80 (2H, m), 4. 43-4.24 (4H, m), 4.02-3.92 (1H, m), 3.83 (1H, dd, *J* = 2.8, 13.0 Hz), 3.78 (1H, dd, *J* = 2.4, 13.0 Hz), 2.67 (1H, td, *J* = 5.1, 14. 2 Hz), 2.40 (1H, td, *J* = 6. 8, 14.2 Hz). |

(continued)

| | | |
|---|---|---|
| 134 | 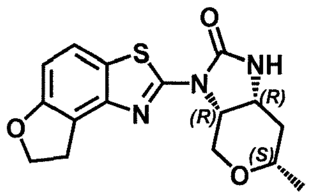 | LC-MS [M+H] + /Rt (min) : 332. 1/2. 124 (Method C); 1H-NMR (DMSO-$d_6$) δ : 8. 09 (1H, s), 7. 60 (1H, d, *J* = 8.4 Hz), 6.75 (1H, d, *J* = 8. 4 Hz), 4. 99 (1H, dd, *J* = 2. 3, 13. 1 Hz), 4.61 (2H, t, *J* = 8.8 Hz), 4. 38 (1H, td, *J* = 2. 7, 8.0 Hz), 3.98 (1H, dt, *J* = 6.0, 8. 4 Hz), 3. 81 (1H, dd, *J* = 3. 2, 13.1 Hz), 3.59 (1H, ddd, *J* = 3.6, 6.4, 9. 7 Hz), 3. 37 (2H, dt, *J* = 3.0, 8. 7 Hz), 2.04 (1H, ddd, *J* = 3. 6, 5.9, 13. 7 Hz), 1. 35-1. 21 (1H, m), 1. 13 (3H, d, *J* = 6.2 Hz). |

[Table 71-3]

| | | |
|---|---|---|
| 135 | 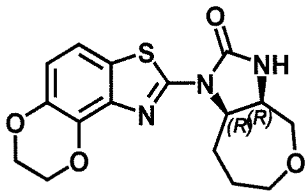 | LC-MS [M+H] + /Rt (min): 348. 1/2. 023 (Method C); 1H-NMR (DMSO-$d_6$) δ: 7.89 (1H, s), 7.28 (1H, d, *J* = 8.5 Hz), 6. 79 (1H, d, *J* = 8.5 Hz), 4.78 (1H, ddd, *J* = 2.7, 7.4, 9.9 Hz), 4.43-4.25 (4H, m), 4.12-4.02 (1H, m), 3. 73-3. 69 (2H, m), 3. 69-3. 64 (1H, m), 3.52 (1H, dt, J= 5. 5, 11.6 Hz), 2.61-2.56 (1H, m), 2.27-2.19 (1H, m), 1.61-1.50 (2H, m). |
| 136 | 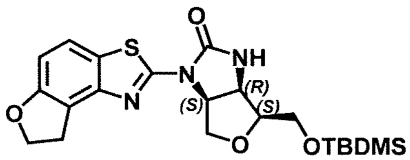 | LC-MS [M+H] + /Rt (min): 448.2/2.773 (Method C) |
| 137 | 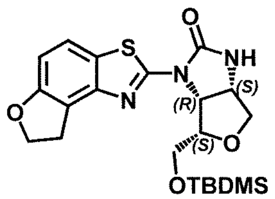 | LC-MS [M+H] + /Rt (min): 448. 2/2. 690 (Method C) |
| 138 | 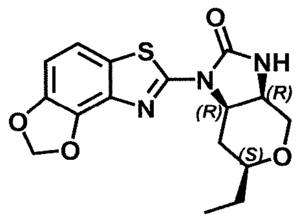 | LC-MS [M+H] + /Rt (min): 348. 1/2. 223 (Method C); 1H-NMR (DMSO-$d_6$) δ : 7.92 (1H, s), 7. 36 (1H, d, *J* = 8. 3 Hz), 6.92 (1H, d, *J* = 8. 3 Hz), 6. 15-6. 06 (2H, m), 4. 86-4. 76 (1H, m), 4. 03-3.93 (1H, m), 3. 84-3. 76 (1H, m), 3. 70-3.62 (1H, m), 3. 29-3. 21 (1H, m), 2.47-2.38 (1H, m), 1. 48-1. 37 (2H, m), 1. 28-1. 13 (1H, m), 0.86 (3H, t, *J* = 7. 4 Hz) . |
| 139 | 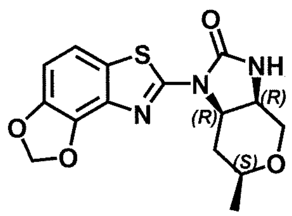 | LC-MS [M+H] + /Rt (min): 334. 1/2. 107 (Method C); 1H-NMR (DMSO-$d_6$) δ : 7.92 (1H, s), 7.36 (1H, d, *J* = 8. 3 Hz), 6.93 (1H, d, *J* = 8. 3 Hz), 6.11 (1H, d, *J* = 1. 1 Hz), 6. 09 (1H, d, *J* = 1. 1 Hz), 4. 83-4. 77 (1H, m), 3. 99-3. 89 (1H, m), 3. 79 (1H, d, *J* = 7. 2 Hz), 3. 67 (1H, dd, *J* = 2. 7, 13. 2 Hz), 3. 49-3. 42 (1H, m), 2.47-2.42 (1H, m), 1.23-1.14 (1H, m), 1.10 (3H, t, *J* = 6. 1 Hz). |
| 140 | 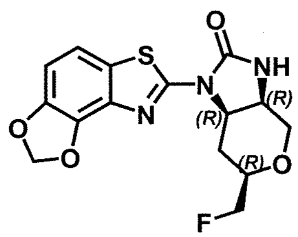 | LC-MS [M+H] + /Rt (min): 352. 1/2. 073 (Method C) ; 1H-NMR (DMSO-$d_6$) δ: 7.97 (s, 1H), 7.37 (d, *J* = 8.3 Hz, 1H), 6. 93 (d, *J* = 8. 3 Hz, 1H), 6. 11 (d, *J* = 1. 1 Hz, 1H), 6. 09 (d, *J* = 1. 1 Hz, 1H), 4. 88 (dt, *J* = 9. 8, 6.9 Hz, 1H), 4. 54 - 4. 16 (m, 2H), 4. 06 - 3. 92 (m, 1H), 3. 85 (d, *J* = 7.4 Hz, 1H), 3. 79 - 3.60 (m, 2H), 2.43 (ddd, *J* = 13. 3, 6. 7, 2.3 Hz, 1H), 1. 40 - 1. 19 (m, 1H). |

Examples 141 to 143

The compounds of Examples 141 to 143 were obtained by using the corresponding raw material compounds according to the method described in Reference Example 70.

[Table 72]

| Example | Chemical structure | Physical property data |
|---|---|---|
| 141 | 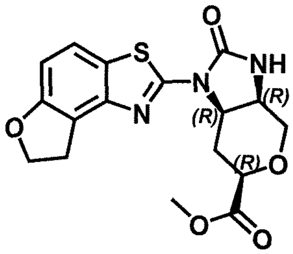 | **LC-MS [M+H]+/Rt (min): 376.1/2.006 (Method C); 1H-NMR (DMSO-$d_6$) δ: 7.96 (1H, s), 7.60 (1H, d, *J* = 8.4 Hz), 6. 75 (1H, d, *J* = 8.4 Hz), 4.92 (1H, q, *J* = 7.7 Hz), 4.66-4. 53 (2H, m), 4.28 (1H, dd, *J* = 3.5, 10.2 Hz), 3.95 (1H, dd, *J* = 1.6, 13.0 Hz), 3.91-3.84 (1H, m), 3.78 (1H, dd, *J* = 2. 7, 13.0 Hz), 3.55 (3H, s), 3.43-3.34 (2H, m), 2.69 (1H, ddd, *J* = 3.6, 6.0, 13.6 Hz), 1.87-1.67 (1H, m).** |
| 142 | 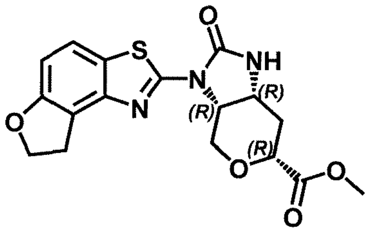 | **LC-MS [M+H]+/Rt (min): 376.1/2.007 (Method C)** |
| 143 | 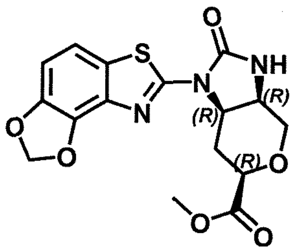 | **LC-MS [M+H]+/Rt (min): 378.1/2.006 (Method C)** |

Examples 144 to 147

The compounds of Examples 144 to 147 were obtained by using the corresponding raw material compounds according to the method described in Reference Example 72.

[Table 73]

| Example | Chemical structure | Physical property data |
|---|---|---|
| 144 | 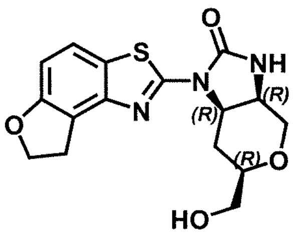 | **LC-MS [M+H]+/Rt (min): 348.2/1.856 (Method C) ; 1H-NMR (DMSO-$d_6$) δ: 7.90 (1H, s), 7.60 (1H, d, *J* = 8.4 Hz), 6.74 (1H, d, *J* = 8.4 Hz), 4.85 (1H, td, *J* = 6.8, 9.7 Hz), 4.70 (1H, t, *J* = 5.6 Hz), 4.66-4.53 (2H, m), 3.98 (1H, d, *J* = 13.1 Hz), 3.80 (1H, d, *J* = 7.2 Hz), 3.68 (1H, dd, *J* = 2.7, 13.0 Hz), 3.45-3.33 (5H, m), 263-2.40 (1H, m), 1.33-1.15 (1H, m).** |
| 145 | 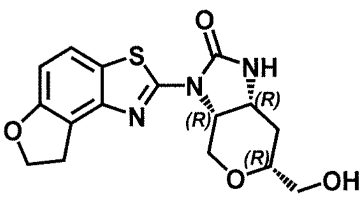 | **LC-MS [M+H]+/Rt (min): 348.1/1.873 (Method C) ; 1H-NMR (DMSO-$d_6$) δ: 8.08 (1H, s), 7.60 (1H, d, *J* = 8.4 Hz), 6.75 (1H, d, *J* = 8.4 Hz), 4.70 (1H, t, *J* = 5.8 Hz), 4.61 (2H, t, *J* = 8.8 Hz), 4.46 (1H, td, *J* = 3.2, 8.4 Hz), 4.04 (2H, dt, *J* = 5.7, 8.2 Hz), 3.88 (1H, dd, *J* = 3.5, 12.9 Hz), 3.54 (1H, td, *J* = 4.8, 9.6 Hz), 3.49-3.26 (4H, m), 1.99 (1H, ddd, *J* = 4.3, 5.8, 13.9 Hz), 1.43 (1H, td, *J* = 8.8, 13.9 Hz).** |

(continued)

| Example | Chemical structure | Physical property data |
|---|---|---|
| 146 | 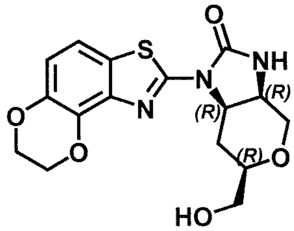 | LC-MS [M+H]+/Rt (min): 364. 1/1. 840 (Method C) ; $^1$H-NMR (DMSO-$d_6$) δ: 7.87 (1H, s), 7.29 (1H, d, *J* = 8.6 Hz), 6.80 (1H, d, *J* = 8.6 Hz), 4.81 (1H, td, *J* = 6.8, 9.9 Hz), 4.76-4.66 (1H, m), 4.42-4. 23 (4H, m), 4.02-3.90 (1H, m), 3.80 (1H, d, *J* = 7.4 Hz), 3.68 (1H, dd, *J* = 2.8, 13.0 Hz), 3.46-3.24 (3H, m), 2.50-2.40 (1H, m), 1.30-1.15 (1H, m). |
| 147 | 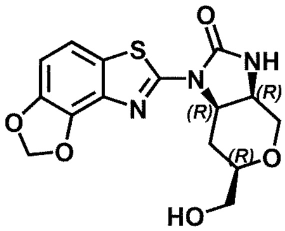 | LC-MS [M+H]+/Rt (min): 350.1/1.890 (Method C) ; $^1$H-NMR (DMSO-$d_6$) δ: 7.95 (1H, s), 7.37 (1H, dd, *J* = 1.9, 8.3 Hz), 6.93 (1H, dd, *J* = 1. 9, 8. 3 Hz), 6.11-6.09 (2H, m), 4.87-4.79 (1H, m), 4.72-4.67 (1H, m), 3.98 (1H, d, *J* = 12.7 Hz), 3.87-3.76 (1H, m), 3.68 (1H, d, *J* = 13.0 Hz), 3.47-3.31 (3H, m), 2.61-2.42 (1H, m), 1.35-1.14 (1H, m). |

Example 148

The compound of Example 148 was obtained by using the corresponding raw material compounds according to the method described in Reference Example 75.

[Table 74]

| Example | Chemical structure | Physical property data |
|---|---|---|
| 148 | 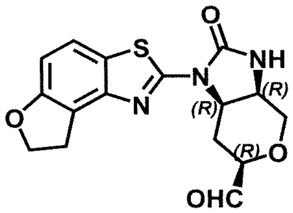 | LC-MS [M+H]+/Rt (min): 346.1/1.907 (Method C) |

Example 149

The compound of Example 149 was obtained by using the corresponding raw material compounds according to the method described in Reference Example 76.

[Table 75]

| Example | Chemical structure | Physical property data |
|---|---|---|
| 149 | 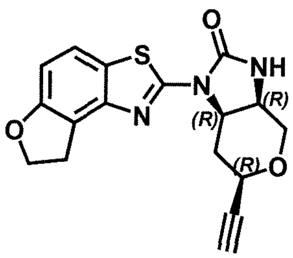 | LC-MS [M+H]+/Rt (min) : 342.1/2.123 (Method C); $^1$H-NMR (DMSO-$d_6$) $\delta$ : 7.93 (1H, s), 7.60 (1H, d, *J* = 8.4 Hz), 6.75 (1H, d, *J* = 8.4 Hz), 4.93-4.80 (1H, m), 4.66-4.50 (2H, m), 4.34 (1H, td, *J* = 2.6, 10.5 Hz), 3.95-3.86 (1H, m), 3.83 (1H, d, *J* = 7.6 Hz), 3.72 (1H, dd, *J* = 2.8, 13.1 Hz), 3.45 (1H, d, *J* = 2.1 Hz), 3.41-3.33 (2H, m), 2. 72-2. 57 (1H, m), 1.76-1.60 (1H, m). |

Example 150

(3aR,7aR)-1-(7,8-Dihydrofuro[3,2-e][1,3]benzothiazol-2-yl)-6-hydroxyhexahydropyrano[3,4-d]imidazol-2(3H)-one

[Formula 76]

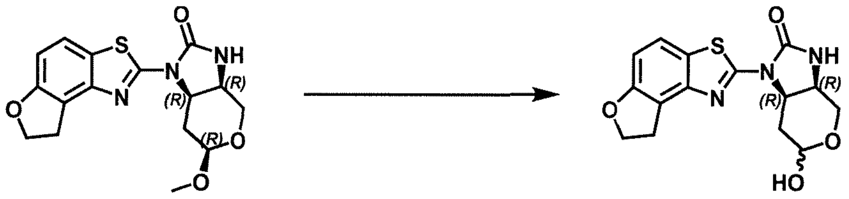

1 N Hydrochloric acid (4 mL) was added to a 1,4-dioxane solution (3 mL) of the compound of Example 129 (53.0 mg) at room temperature, and the resulting mixture was stirred at room temperature for 1 hour and at 60°C for 2 hours. The reaction mixture was cooled to room temperature, then saturated aqueous sodium bicarbonate was added, and the resulting mixture was extracted with ethyl acetate. The organic layer was dried over sodium sulfate, filtered, and then concentrated under reduced pressure. The residue was washed with ethyl acetate to obtain the title compound (8.0 mg). LC-MS $[M+H]^+$/Rt (min): 334.1/2.761 (Method C); $^1$H-NMR (DMSO-$d_6$) δ: 7.95 (1H, s), 7.59 (1H, d, J = 8.4 Hz), 6.74 (1H, d, J = 8.4 Hz), 6.40 (1H, d, J = 4.9 Hz), 5.00-4.85 (1H, m), 4.81 (1H, td, J = 4.4, 9.1 Hz), 4.67-4.52 (2H, m), 3.98-3.84 (2H, m), 3.49-3.43 (1H, m), 3.42-3.34 (1H, m), 2.77-2.68 (1H, m), 2.00-1.90 (2H, m).

Example 151

(3aR,7aR)-1-(7,8-Dihydrofuro[3,2-e][1,3]benzothiazol-2-yl)-6-fluorohexahydropyrano[3,4-d]imidazol-2(3H)-one

[Formula 77]

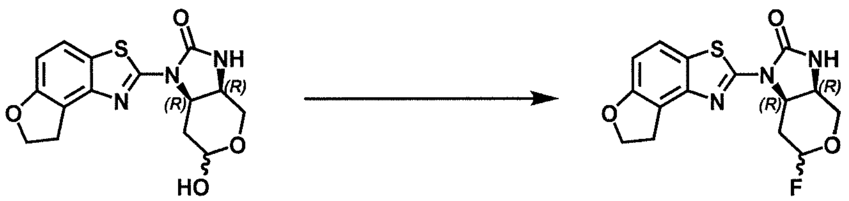

Diethylaminosulfur trifluoride (0.041 mL) was added to a dichloromethane/1,4-dioxane solution (6 mL/9 mL) of Example 150 (69.0 mg) under ice cooling, and the resulting mixture was stirred under ice cooling for 15 minutes. Saturated aqueous sodium bicarbonate was added to the reaction mixture under ice cooling, and the resulting mixture was extracted with chloroform. The organic layer was dried over sodium sulfate, filtered, and then concentrated under reduced pressure. The residue was purified by silica gel column chromatography (chloroform/methanol) to obtain the title compound (20.0 mg). LC-MS $[M+H]^+$/Rt (min): 336.1/2.123 (Method C); $^1$H-NMR (DMSO-$d_6$) δ: 8.08 (1H, s), 7.60 (1H, d, J = 8.4 Hz), 6.75 (1H, d, J = 8.4 Hz), 5.67 (1H, ddd, J = 5.0, 6.9, 60.9 Hz), 4.94-4.83 (1H, m), 4.67-4.54 (2H, m), 4.13-3.96 (2H, m), 3.68 (1H, d, J = 12.6 Hz), 3.45-3.33 (2H, m), 3.22-3.02 (1H, m), 2.32-2.14 (1H, m).

Example 152

The compound of Example 152 was obtained by using the corresponding raw material compounds according to the method described in Reference Example 75.

[Table 76]

| Example | Chemical structure | Physical property data |
|---|---|---|
| 152 | 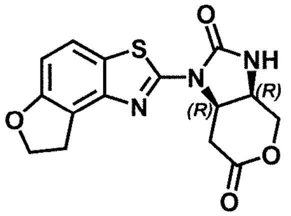 | LC-MS [M+H]+/Rt (min) : 332.1/1.956 (Method C); $^1$H-NMR (DMSO-$d_6$) δ : 8.24 (1H, s), 7.61 (1H, d, *J* = 8.4 Hz), 6.77 (1H, d, *J* = 8.4 Hz), 5.17 (1H, ddd, *J* = 2.2, 5.2, 10.1 Hz) , 4.68-4.55 (2H, m), 4. 48 (1H, dd, *J* = 2.1, 12.5 Hz), 4.36 (1H, d, *J* = 10.2 Hz), 4.22 (1H, dd, *J* = 1.7, 12.5 Hz), 3.53-3.32 (3H, m), 3.18 (1H, dd, *J* = 5.2, 15.8 Hz). |

Examples 153 and 154

The compounds of Examples 153 and 154 were obtained by using the corresponding raw material compounds according to the method described in Reference Example 147.

[Table 77]

| Example | Chemical structure | Physical property data |
|---|---|---|
| 153 | 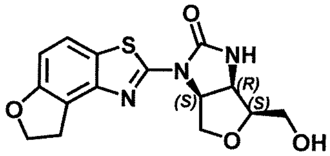 | LC-MS [M+H]+/Rt (min): 334.1/1.890 (Method C) ; $^1$H-NMR (DMSO-$d_6$) δ: 8.01 (1H, s), 7.60 (1H, d, *J* = 8. 4 Hz), 6. 75 (1H, d, *J* = 8. 4 Hz), 5.14-5.06 (1H, m), 4.79 (1H, t, *J* = 5.5 Hz), 4.67-4.53 (2H, m), 4.40-4.31 (1H, m), 4.23-4.16 (1H, m), 3.82-3.74 (1H, m), 3.70-3.51 (3H, m), 3.39-3.33 (3H, m). |
| 154 | 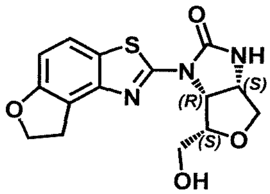 | LC-MS [M+H]+/Rt (min): 334.1/1.890 (Method C); $^1$H-NMR (DMSO-$d_6$) δ : 8.21 (1H, s), 7.61 (1H, d, *J* = 8. 4 Hz), 6. 77 (1H, d, *J* = 8. 4 Hz), 5.17-5.10 (1H, m), 4.97-4.90 (1H, m), 4.63 (1H, t, *J* = 8.8 Hz), 4.49-4.42 (1H, m), 3.87-3.80 (2H, m), 3.75-3.66 (1H, m), 3.62-3.55 (1H, m), 3.43-3.28 (3H, m). |

Examples 155 and 156

The compounds of Examples 155 and 156 were obtained by using the corresponding raw material compounds according to the method described in Reference Example 14.

[Table 78]

| Example | Chemical structure | Physical property data |
|---|---|---|
| 155 | 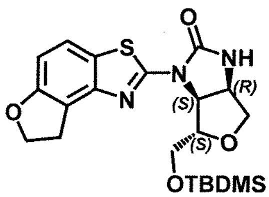 | LC-MS [M+H] + /Rt (min): 448.0/2.840 (Method C) |
| 156 | 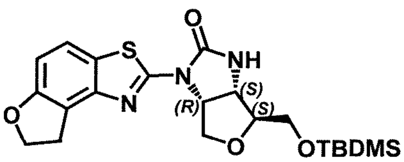 | LC-MS [M+H] + /Rt (min): 448.2/2.823 (Method C) |

Examples 157 and 158

The compounds of Examples 157 and 158 were obtained by using the corresponding raw material compounds

according to the method described in Reference Example 147.

[Table 79]

| Example | Chemical structure | Physical property data |
|---|---|---|
| 157 | 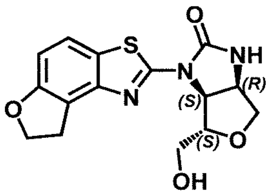 | LC-MS [M+H]$^+$/Rt (min): 334.1/1.907 (Method C) ; $^1$H-NMR (DMSO-$d_6$) $\delta$ : 8.19 (1H, s), 7.60 (1H, d, $J$ = 8.4 Hz), 6.75 (1H, d, $J$ = 8.4 Hz), 4.96 (1H, dd, $J$ = 5.4, 5.4 Hz), 4.92 (1H, dd, $J$ = 2.3, 8.5 Hz), 4. 68-4.56 (2H, m), 4.44-4.37 (1H, m), 4.18-4.12 (1H, m), 4.10-4.05 (1H, m), 3.84-3.69 (3H, m), 3.39-3.33 (2H, m). |
| 158 | 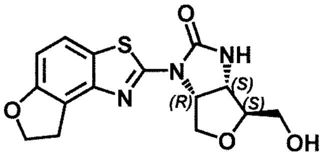 | LC-MS [M+H]$^+$/Rt (min): 334.1/1.890 (Method C) ; $^1$H-NMR (DMSO-$d_6$) $\delta$ : 8.26 (1H, s), 7.60 (1H, d, $J$ = 8.4 Hz), 6.75 (1H, d, $J$ = 8.4 Hz), 5.11-5.03 (1H, m), 4.95 (1H, dd, $J$ = 5.4, 5.4 Hz), 4.66-4.55 (2H, m), 4.28-4.17 (2H, m), 4.01-3.95 (1H, m), 3.88-3.81 (1H, m), 3.55-3.47 (2H, m), 3.39-3.33 (2H, m). |

Example 159

The compound of Example 159 was obtained by using the corresponding raw material compounds according to the method described in Reference Example 74.

[Table 80]

| Example | Chemical structure | Physical property data |
|---|---|---|
| 159 | 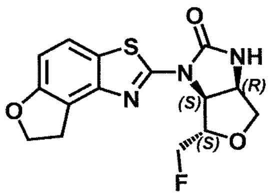 | LC-MS [M+H] $^+$/Rt (min): 336.1/2.157 (Method C) ; $^1$H-NMR (DMSO-$d_6$) $\delta$ : 8.26 (1H, s), 7.61 (1H, d, $J$ = 8.4 Hz), 6.76 (1H, d, $J$ = 8.4 Hz), 4.94 (1H, dd, $J$ = 3.0, 8.7 Hz), 4.92-4.72 (2H, m), 4.67-4.56 (2H, m), 4.46-4.40 (1H, m), 4.40-4.30 (1H, m), 4.11-4.03 (1H, m), 3.79-3.73 (1H, m), 3.41-3.34 (2H, m). |

Example 160

The compound of Example 160 was obtained by using the corresponding raw material compounds according to the method described in Reference Example 14.

[Table 81]

| Example | Chemical structure | Physical property data |
|---|---|---|
| 160 | 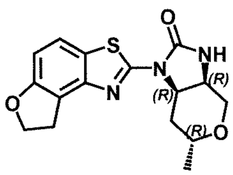 | LC-MS [M+H]$^+$/Rt (min): 332.1/2.123 (Method C) ; $^1$H-NMR (DMSO-$d_6$) $\delta$ : 7.87 (1H, s), 7.59 (1H, d, $J$ = 8. 4 Hz), 6. 74 (1H, d, $J$ = 8. 4 Hz), 4.70-4.53 (3H, m), 4.30 (1H, dd, $J$ = 5. 6, 11.8 Hz), 4.19-4.08 (1H, m), 3.78-3.63 (1H, m), 3. 45 (1H, dd, $J$ = 8. 2, 11. 8 Hz), 3.40-3.33 (2H, m), 1.87-1.82 (1H, m), 1. 64 (1H, ddd, $J$ = 4. 2, 11. 3, 15. 1 Hz), 1. 13 (3H, d, $J$ = 6.2 Hz) . |

Example 161

(3aR,6R,6aS)-1-(7,8-Dihydrofuro[3,2-e][1,3]benzothiazol-2-yl)-6-methyltetrahydro-1H-furo[3,4-d]imidazol-2(3H)-one

[Formula 78]

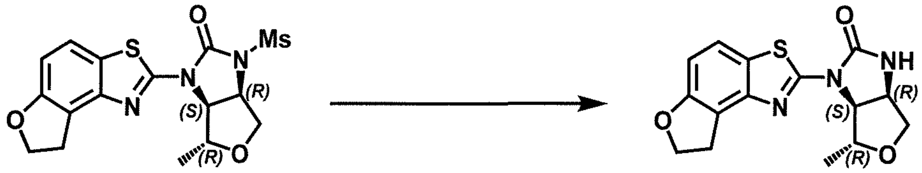

1.0 M Tetrahydrofuran solution (0.024 mL) of tetrabutylammonium fluoride was added to a tetrahydrofuran solution (3 mL) of Reference Example 290 (1.0 mg) at room temperature, and the resulting mixture was stirred at room temperature for 2 days. The reaction mixture was concentrated under reduced pressure, and then the residue was purified by silica gel column chromatography (hexane/ethyl acetate) to obtain the title compound (0.6 mg).
LC-MS $[M+H]^+$/Rt (min): 318.1/2.123 (Method C); $^1$H-NMR (DMSO-$d_6$) δ: 8.17 (1H, s), 7.60 (1H, d, J = 8.4 Hz), 6.75 (1H, d, J = 8.4 Hz), 4.68 (1H, dd, J = 2.4, 8.7 Hz), 4.65-4.58 (2H, m), 4.46-4.38 (1H, m), 4.29-4.21 (1H, m), 4.02-3.94 (1H, m), 3.65 (1H, dd, J = 2.6, 9.9 Hz), 3.50-3.38 (2H, m), 1.38 (3H, d, J = 6.6 Hz).

Example 162

[(3aS,4R,6aR)-3-(7,8-Dihydrofuro[3,2-e][1,3]benzothiazol-2-yl)-2-oxohexahydro-1H-furo[3,4-d]imidazol-4-yl]acetonitrile

[Formula 79]

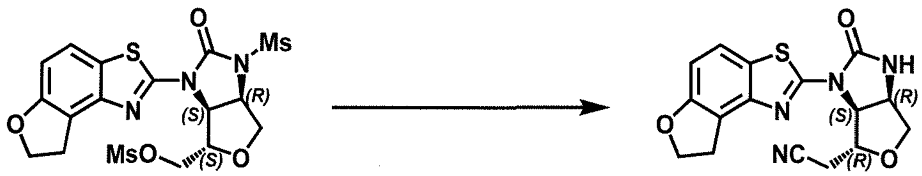

Potassium cyanide (4.0 mg) was added to a dimethyl sulfoxide solution (2 mL) of Reference Example 285 (20.0 mg) at room temperature, and the resulting mixture was stirred at 80°C for 1 hour. Potassium cyanide (16.5 mg) was further added, and the resulting mixture was stirred at 80°C for 35 minutes, then 1.0 M tetrahydrofuran solution (0.204 mL) of tetrabutylammonium fluoride was added at room temperature, and the resulting mixture was stirred at 70°C for 5 hours. The reaction mixture was concentrated under reduced pressure, and then the residue was purified by silica gel column chromatography (hexane/ethyl acetate) to obtain the title compound (2.4 mg).
LC-MS $[M+H]^+$/Rt (min): 343.1/2.090 (Method C); $^1$H-NMR (DMSO-$d_6$) δ: 8.27 (1H, s), 7.61 (1H, d, J = 8.4 Hz), 6.76 (1H, d, J = 8.4 Hz), 4.82 (1H, dd, J = 3.1, 8.9 Hz), 4.69-4.56 (2H, m), 4.49-4.56 (2H, m), 4.49-4.41 (1H, m), 4.37 (1H, dt, J = 3.1, 6.2 Hz), 4.13 (1H, dd, J = 5.2, 9.9 Hz), 3.72 (1H, dd, J = 3.3, 10.0 Hz 3.45-3.36 (2H, m), 3.19-3.17 (2H, m).

Test Example 1

[Inhibition Test on Activity of DYRK Family (DYRK1A, DYRK1B, DYRK2, and DYRK3)]

(Method for Measuring Kinase Activity)

The kinase activity was measured by mobility shift assay (MSA) method using QuickScout Screening Assist(TM) MSA (commercially available kit manufactured by Carna Biosciences, Inc.). The substrate of the kinase reaction used was an FITC-labeled DYRKtide peptide included in the kit. An assay buffer [20 mM HEPES, 0.01% Triton X-100 (TM), 2 mM dithiothreitol, pH 7.5] was used to create a substrate mixture solution with a substrate (4 μM), $MgCl_2$(20 mM), and ATP (DYRKLA: 100 μM; DYRK1B: 200 μM; DYRK2: 40 μM; and DYRK3: 20 μM). In addition, kinases (DYRK1A: manufactured by Carna Biosciences, Inc., Cat. No. 04-130; DYRK1B: manufactured by Carna Biosciences, Inc., Cat. No. 04-131; DYRK2; manufactured by Carna Biosciences, Inc., Cat. No. 04-132; and DYRK3; manufactured by Carna Biosciences, Inc., Cat. No. 04-133) were diluted with the assay buffer to prepare enzyme solutions (DYRK1A: 0.2 ng/μL; DYRK1B: 0.08 ng/μL; DYRK2: 0.04 ng/μL; and DYRK3: 0.25 ng/μL). The 10 mM solution of the test compound in DMSO was further diluted with DMSO to 10 levels of the concentration (0.00003 mM, 0.0001 mM, 0.0003 mM, 0.001 mM, 0.003 mM, 0.01 mM,

0.03 mM, 0.1 mM, 0.3 mM, and 1 mM), each of which was subjected to 25-fold dilution with the assay buffer to obtain a drug solution (4% DMSO solution). 5 μL of the drug solution or a control solution (4% DMSO-assay buffer), 5 μL of the substrate mixture solution, and 10 μL of the enzyme solution were mixed in the wells of a polypropylene 384-well plate and allowed to react at room temperature for 1 hour, and then the reaction was quenched by adding 60 μL of the termination buffer included in the kit. Subsequently, the quantities of the substrates (S) and the phosphorylated substrate (P) in the reaction solution were measured using LabChip EZ Reader II system (manufactured by Caliper Life Sciences) according to the protocol of the assay kit.

(Method for Evaluating Inhibitory Activity)

The heights of the peaks of the "substrate" and the "phosphorylated substrate" were expressed as S and P, respectively, and a blank containing the assay buffer instead of the enzyme solution was also measured.
The inhibition rate (%) of the test compound was calculated according to the following equation:

$$\text{Inhibition rate (\%)} = (1 - (C - A)/(B - A)) \times 100$$

wherein, A, B, and C represent P/(P + S) of the blank well, P/(P + S) of the control solution well, and P/(P + S) of the compound-containing well, respectively.
The $IC_{50}$ value was calculated via a regression analysis of the inhibition rate and the test compound concentration (logarithmic value).

(Evaluation Result)

The inhibiting activities of representative compounds of the present invention are shown against DYRK1A, DYRK1B, DYRK2, and DYRK3 in Tables. The kinase activity inhibitory effect was indicated with the mark *** at an $IC_{50}$ value of less than 0.01 μM; the mark ** at 0.01 μM or more and less than 0.1 μM; the mark * at 0.1 μM or more and less than 1 μM; and the mark - at 1 μM or more.

[Table 82]

| Test Compound | Inhibitory activity | | | |
|---|---|---|---|---|
| Example No. | DYRK 1A | DYRK 1B | DYRK 2 | DYRK 3 |
| 1 | ** | NT | - | * |
| 2 | ** | NT | * | ** |
| 3 | * | * | - | * |
| 4 | * | NT | - | * |
| 5 | ** | NT | * | * |
| 7 | ** | ** | - | - |
| 8 | ** | ** | - | - |
| 9 | ** | ** | * | * |
| 10 | * | NT | - | - |
| 11 | ** | ** | - | - |
| 12 | ** | NT | * | * |
| 13 | ** | NT | - | - |
| 14 | ** | ** | * | * |
| 15 | ** | NT | - | - |
| 16 | * | NT | - | - |
| 17 | ** | NT | - | - |
| 18 | ** | NT | * | * |
| 19 | ** | NT | * | * |

(continued)

| Test Compound | Inhibitory activity | | | |
|---|---|---|---|---|
| Example No. | DYRK 1A | DYRK 1B | DYRK 2 | DYRK 3 |
| 20 | * | NT | - | - |
| 21 | *** | NT | * | * |
| 22 | * | NT | - | - |
| 23 | *** | *** | * | * |
| 24 | ** | ** | - | - |
| 25 | ** | NT | - | - |
| 26 | - | NT | - | - |
| 27 | - | - | - | - |
| 28 | ** | ** | - | - |

[Table 83]

| Test Compound | Inhibitory activity | | | |
|---|---|---|---|---|
| Example No. | DYRK 1A | DYRK 1B | DYRK 2 | DYRK 3 |
| 29 | ** | NT | - | - |
| 30 | ** | NT | - | - |
| 31 | *** | NT | - | - |
| 32 | *** | NT | - | - |
| 33 | * | NT | - | - |
| 34 | *** | ** | - | - |
| 35 | - | NT | - | - |
| 36 | *** | *** | - | - |
| 37 | *** | NT | * | * |
| 38 | *** | *** | * | * |
| 39 | *** | *** | * | * |
| 40 | *** | *** | * | ** |
| 41 | *** | *** | * | ** |
| 42 | *** | *** | * | * |
| 43 | *** | *** | * | * |

[Table 84]

| Test Compound | Inhibitory activity | | | |
|---|---|---|---|---|
| Example No. | DYRK 1A | DYRK 1B | DYRK 2 | DYRK 3 |
| 44 | *** | NT | * | * |
| 45 | ** | NT | * | ** |
| 46 | ** | NT | * | * |
| 47 | * | NT | - | * |
| 48 | ** | NT | * | ** |
| 49 | ** | NT | * | * |
| 50 | * | NT | - | * |

(continued)

| Test Compound | Inhibitory activity | | | |
|---|---|---|---|---|
| Example No. | DYRK 1A | DYRK 1B | DYRK 2 | DYRK 3 |
| 51 | ** | NT | * | * |
| 52 | ** | NT | * | * |
| 53 | * | NT | - | * |
| 54 | ** | NT | * | * |
| 55 | * | NT | - | * |
| 56 | * | NT | - | * |
| 57 | * | NT | - | * |
| 65 | *** | NT | ** | ** |
| 66 | * | NT | - | - |
| 67 | * | NT | - | * |
| 68 | * | NT | - | * |
| 69 | ** | NT | - | - |
| 70 | ** | NT | - | * |
| 71 | ** | NT | - | - |
| 72 | ** | NT | - | - |
| 73 | *** | NT | * | - |
| 74 | * | NT | - | * |
| 75 | ** | NT | - | - |
| 76 | ** | NT | - | - |
| 77 | ** | NT | - | * |
| 78 | ** | NT | - | * |
| 79 | ** | NT | - | - |
| 80 | ** | NT | - | - |
| 81 | ** | NT | - | - |
| 82 | ** | NT | - | - |
| 83 | * | NT | - | * |
| 84 | ** | NT | * | * |
| 85 | ** | NT | - | - |
| 86 | ** | NT | - | - |
| 87 | * | NT | * | - |
| 88 | * | NT | - | - |
| 89 | * | NT | - | - |
| 90 | ** | NT | * | * |
| 91 | ** | NT | * | ** |

[Table 85]

| Test Compound | Inhibitory activity |
|---|---|

(continued)

| Example No. | DYRK 1A | DYRK 1B | DYRK 2 | DYRK 3 |
|---|---|---|---|---|
| 92 | * | NT | - | - |
| 93 | ** | NT | - | * |
| 94 | * | NT | - | - |
| 95 | * | NT | * | * |
| 96 | * | NT | - | - |
| 97 | * | NT | - | - |
| 98 | *** | NT | - | - |
| 99 | *** | NT | * | * |
| 100 | *** | ** | - | - |
| 101 | ** | NT | - | - |
| 102 | ** | NT | - | * |
| 103 | ** | NT | - | * |
| 104 | *** | NT | * | * |
| 105 | ** | NT | - | - |
| 106 | ** | NT | - | * |
| 107 | ** | NT | - | - |
| 108 | ** | NT | - | - |
| 109 | - | NT | - | - |
| 110 | *** | NT | - | - |
| 111 | * | NT | - | - |
| 112 | *** | NT | - | * |
| 113 | * | NT | - | - |
| 114 | *** | NT | * | * |
| 115 | ** | NT | * | - |
| 116 | ** | NT | - | - |
| 117 | ** | NT | - | - |
| 118 | ** | NT | - | - |
| 119 | ** | ** | ** | ** |
| 120 | ** | NT | * | * |
| 121 | ** | NT | * | * |
| 122 | *** | *** | * | * |
| 123 | - | NT | - | - |
| 124 | *** | NT | * | * |
| 125 | ** | NT | * | * |
| 126 | * | NT | * | * |
| 127 | *** | NT | * | * |
| 128 | - | NT | - | - |
| 129 | ** | NT | * | * |
| 130 | * | NT | - | - |

(continued)

| Example No. | DYRK 1A | DYRK 1B | DYRK 2 | DYRK 3 |
|---|---|---|---|---|
| 131 | *** | NT | * | * |
| 132 | - | NT | - | - |

[Table 86]

| Test Compound | Inhibitory activity | | | |
|---|---|---|---|---|
| Example No. | DYRK 1A | DYRK 1B | DYRK 2 | DYRK 3 |
| 133 | *** | NT | * | * |
| 134 | * | NT | - | - |
| 135 | ** | NT | * | * |
| 138 | *** | *** | * | * |
| 139 | *** | *** | * | ** |
| 140 | *** | *** | ** | ** |
| 141 | ** | NT | - | - |
| 144 | *** | NT | - | * |
| 145 | * | NT | - | - |
| 146 | *** | NT | - | * |
| 147 | *** | NT | - | * |
| 149 | *** | NT | - | * |
| 150 | ** | NT | * | * |
| 151 | *** | NT | * | ** |
| 152 | *** | NT | ** | ** |
| 153 | ** | NT | * | * |
| 154 | - | NT | - | - |
| 157 | ** | ** | * | * |
| 158 | * | * | - | - |
| 159 | *** | ** | ** | ** |
| 160 | * | * | - | - |
| 161 | ** | ** | ** | * |
| 162 | ** | ** | * | * |

These results have shown that the test compounds (Compounds (1) of the present invention) have potent DYRK-inhibitory activities.

Test Example 2

[Hematological test by oral administration using mice]

In the present test, mice aged 8 to 12 weeks (B6C3F1, female, Charles River Laboratories Japan, Inc.) were used. A 0.5% methylcellulose solution or an Example compound suspended in a 0.5% methylcellulose solution was orally administered as a single dose or as repeated doses of 10 mL/kg to the mice, and on or after the day after the final administration, the blood collected from the posterior vena cava under isoflurane anesthesia was subjected to anticoagulation treatment with a blood collection tube containing EDTA-2K, and the reticulocyte count was quantified by using a multi-item automated hematology analyzer (manufactured by Sysmex Corporation).

INDUSTRIAL APPLICABILITY

The compound provided by the present invention is useful as a prophylactic or therapeutic agent for disease which is known to be involved in abnormal cell response through DYRK1A, for example, Alzheimer's disease, Parkinson's disease, Down's syndrome, mental retardation, memory impairment, memory loss, neuropsychiatric disorder such as depression, and cancers such as brain tumors. The compound is a DYRK1B inhibitor also useful as a prophylactic or therapeutic pharmaceutical (pharmaceutical composition) for cancers such as pancreatic cancer. Since DYRK2 controls p53 to induce apoptosis in response to DNA damages, the compound provided by the present invention is further useful as a prophylactic or therapeutic pharmaceutical (pharmaceutical composition) for bone resorption disease and osteoporosis. The compound provided by the present invention is a DYRK3 inhibitor also useful as a prophylactic or therapeutic pharmaceutical (pharmaceutical composition) for sickle-cell anemia, bone resorption disease in chronic kidney disease, and osteoporosis. The compound is also useful, as a compound inhibiting DYRK, for reagents to be used in pathological imaging and for reagents for basic experiments and research regarding the above diseases.

## Claims

1. A compound represented by the following formula (1):

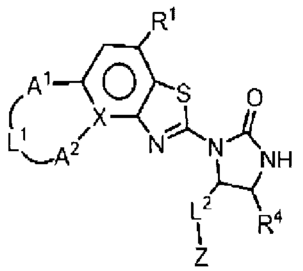

(1)

wherein

$A^1$ represents an oxygen atom or a nitrogen atom (=N-),
$A^2$ represents $CR^B$, $CR^CR^D$, an oxygen atom, or $NR^{A1}$,
$L^1$ represents optionally substituted methylene, optionally substituted ethylene, optionally substituted methine, optionally substituted ethanediylidene, =N-, or $NR^{N2}$, $R^{A1}$, $R^{A2}$, $R^B$, $R^C$, and $R^D$ each independently represent a hydrogen atom or optionally substituted $C_{1-6}$ alkyl,
$R^1$ represents a hydrogen atom, a halogen atom, or optionally substituted $C_{1-6}$ alkyl,
X represents a carbon atom or a nitrogen atom,
$L^2$ represents optionally substituted $C_{1-4}$ alkylene,
$R^E$ represents optionally substituted $C_{1-6}$ alkyl,
Z represents $-NR^2R^3$ or $-OR^7$,
$R^7$ represents optionally substituted $C_{1-6}$ alkyl, or optionally substituted $C_{1-7}$ alkylene formed together with $R^4$, wherein $R^4$ and $R^7$, together with the carbon atom and the oxygen atom to which they, respectively, are attached, form an optionally substituted 5- to 11-membered saturated heterocycle,
$R^2$ represents a hydrogen atom, optionally substituted $C_{1-6}$ alkyl, $C(O)-R^E$, $C_{3-10}$ cycloalkyl, $C_{2-6}$ alkynyl, or a cyclic group of a 4- to 11-membered saturated heterocycle,
$R^3$ represents a hydrogen atom, optionally substituted $C_{1-6}$ alkyl, or $C(O)-R^E$, and
$R^4$ represents optionally substituted $C_{1-6}$ alkyl,
wherein $R^2$ and $R^3$, together with the nitrogen atom to which they are attached, may form an optionally substituted 4- to 11-membered saturated heterocycle, or $R^3$ and $R^4$, together with the nitrogen atom and the carbon atom to which they, respectively, are attached, may form an optionally substituted 4- to 11-membered saturated heterocycle, or any carbon atom on the saturated heterocycle constituted by $R^2$, $R^3$, and the nitrogen atom, and $R^4$ together may form a 4- to 11-membered saturated heterocycle,
or a pharmaceutically acceptable salt thereof.

2. The compound according to claim 1 or a pharmaceutically acceptable salt thereof, wherein Z represents $-NR^2R^3$, and $R^2$ and $R^3$ each independently represent a hydrogen atom, optionally substituted $C_{1-6}$ alkyl, or $C(O)-R^E$, wherein $R^2$ and $R^3$, together with the nitrogen atom to which they are attached, may form an optionally substituted 4- to 8-membered saturated heterocycle.

3. The compound according to claim 1 or a pharmaceutically acceptable salt thereof, wherein Z represents $-NR^2R^3$, and $R^3$ and $R^4$, together with the nitrogen atom and the carbon atom to which they, respectively, are attached, form an optionally substituted 4- to 8-membered saturated heterocycle.

4. The compound according to any one of claims 1 to 3 or a pharmaceutically acceptable salt thereof, wherein $R^1$ is a hydrogen atom.

5. The compound according to any one of claims 1 to 4 or a pharmaceutically acceptable salt thereof, wherein X is a carbon atom.

6. The compound according to claim 5 or a pharmaceutically acceptable salt thereof, wherein $A^1$ is an oxygen atom, $A^2$ is methylene, and $L^1$ is methylene.

7. The compound according to claim 1, 2, 4, 5, or 6 or a pharmaceutically acceptable salt thereof, wherein formula (1) is represented by the following formula (1a):

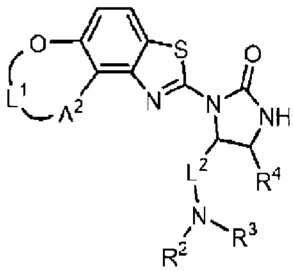
(1a)

wherein

$A^2$ represents optionally substituted methylene or an oxygen atom,
$L^1$ represents optionally substituted methylene or optionally substituted ethylene,
$L^2$ represents optionally substituted $C_{1-4}$ alkylene,
$R^2$ and $R^3$ each independently represent a hydrogen atom,
optionally substituted $C_{1-6}$ alkyl, or C(O) $-R^E$, wherein $R^2$ and
$R^3$, together with the nitrogen atom to which they are attached,
may form an optionally substituted 4- to 8-membered saturated heterocycle,
$R^E$ represents optionally substituted $C_{1-6}$ alkyl, and
$R^4$ is optionally substituted $C_{1-6}$ alkyl.

8. The compound according to claim 7, wherein $R^2$ and $R^3$ each independently are optionally substituted $C_{1-6}$ alkyl, or a pharmaceutically acceptable salt thereof.

9. The compound according to claim 7 or a pharmaceutically acceptable salt thereof, wherein $R^2$ and $R^3$, together with the nitrogen atom to which they are attached, form an optionally substituted 4- to 8-membered saturated heterocycle.

10. The compound according to any one of claims 7 to 9 or a pharmaceutically acceptable salt thereof, wherein $L^2$ is $C_{1-3}$ alkylene.

11. The compound according to claim 1, 3, 4, 5, or 6 or a pharmaceutically acceptable salt thereof, wherein formula (1) is represented by the following formula (1b):

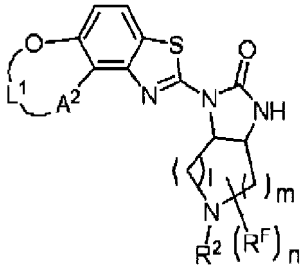

(1b)

wherein

$A^2$ represents optionally substituted methylene or an oxygen atom,
$L^1$ represents optionally substituted methylene or optionally substituted ethylene,
1 and m each independently represent 1, 2, or 3, wherein a sum of 1 and m is 5 or less,
n represents 1, 2, 3, or 4,
$R^2$ represents a hydrogen atom, optionally substituted $C_{1-6}$ alkyl, $C_{3-10}$ cycloalkyl, or C(O)-$R^E$,
$R^E$ represents optionally substituted $C_{1-6}$ alkyl,
$R^F$ represents a hydrogen atom, a halogen atom, or optionally substituted $C_{1-6}$ alkyl,
when n is 2, 3, or 4, each $R^F$ may be the same or different, and two $R^F$ on the same carbon atom, together with the carbon atom to which they are each attached, may form a spiro ring consisting of a 4- to 8-membered saturated heterocycle or a 3-to 8-membered saturated carbocycle, or two $R^F$ on different carbon atoms may bond together to form a crosslink.

**12.** The compound according to claim 11 or a pharmaceutically acceptable salt thereof, wherein $R^2$ is a hydrogen atom, optionally substituted $C_{1-6}$ alkyl, or $C_{3-10}$ cycloalkyl.

**13.** The compound according to claim 11 or a pharmaceutically acceptable salt thereof, wherein $R^2$ is optionally substituted $C_{1-6}$ alkyl.

**14.** The compound according to any one of claims 11 to 13 or a pharmaceutically acceptable salt thereof, wherein 1 and m are each independently 1 or 2.

**15.** The compound according to claim 1, wherein Z is - $OR^7$, and $R^4$ and $R^7$, together with the carbon atom and the oxygen atom to which they, respectively, are attached, form an optionally substituted 5- to 8- membered saturated heterocycle, or a pharmaceutically acceptable salt thereof.

**16.** The compound according to claim 1 or 15 or a pharmaceutically acceptable salt thereof, wherein formula (1) is represented by the following formula (1c):

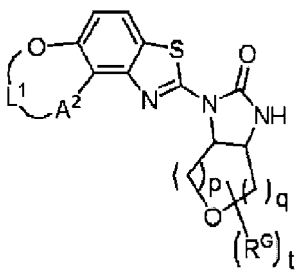

(1c)

wherein

$A^2$ represents optionally substituted methylene or an oxygen atom,
$L^1$ represents optionally substituted methylene or optionally substituted ethylene,
p and q each independently represent 1, 2, or 3, wherein a sum of p and q is 5 or less,
t represents 1, 2, 3, or 4,
$R^G$ represents a hydrogen atom, a halogen atom, optionally substituted $C_{1-6}$ alkyl, optionally substituted $C_{1-6}$ alkoxy, or a CN group,

when t is 2, 3, or 4, each $R^G$ may be the same or different, and two $R^G$ on the same carbon atom, together with the carbon atom to which they are each attached, may form a spiro ring consisting of a 4- to 8-membered saturated heterocycle or a 3-to 8-membered saturated carbocycle, or two $R^G$ on different carbon atoms may bond together to form a cross-link.

**17.** The compound according to any one of claims 1 to 16 or a pharmaceutically acceptable salt thereof, wherein the compound is selected from the group of the following compounds:

cis-3-(7,8-dihydrofuro[3,2-e] [1,3]benzothiazol-2-yl)-5-methyloctahydro-2H-imidazo[4,5-c]pyridin-2-one (Example 7);
cis-3-(7,8-dihydrofuro[3,2-e][1,3]benzothiazol-2-yl)-5-(2-methoxyethyl)octahydro-2H-imidazo[4,5-c]pyridin-2-one (Example 8);
cis-1-(7,8-dihydrofuro[3,2-e][1,3]benzothiazol-2-yl)-5-methyloctahydro-2H-imidazo[4,5-c]pyridin-2-one (Example 9);
cis-3-(7,8-dihydrofuro[3,2-e][1,3]benzothiazol-2-yl)-5-ethyloctahydro-2H-imidazo[4,5-c]pyridin-2-one (Example 11);
cis-1-(7,8-dihydrofuro[3,2-e][1,3]benzothiazol-2-yl)-5-methylhexahydropyrrolo[3,4-d]imidazol-2(1H)-one (Example 14);
cis-3-(7,8-dihydrofuro[3,2-e][1,3]benzothiazol-2-yl)-5-(propan-2-yl)octahydro-2H-imidazo[4,5-c]pyridin-2-one (Example 15);
cis-3-(7,8-dihydrofuro[3,2-e][1,3]benzothiazol-2-yl)-5-methyloctahydroimidazo[4,5-c]azepin-2(1H)-one (Example 17);
(3aS,7aS)-3-(7,8-dihydrofuro[3,2-e][1,3]benzothiazol-2-yl)-5-(oxetan-3-yl)octahydro-2H-imidazo[4,5-c]pyridin-2-one (Example 19);
(3aR,7aR)-1-(2H-[1,3]dioxolo[4,5-e][1,3]benzothiazol-7-yl)hexahydropyrano[3,4-d]imidazol-2(3H)-one (Example 21);
(4S,5R)-1-(7,8-dihydrofuro[3,2-e][1,3]benzothiazol-2-yl)-4-methyl-5-[(morpholin-4-yl)methyl]imidazolidin-2-one (Example 23);
(4R,5R)-1-(7,8-dihydrofuro[3,2-e][1,3]benzothiazol-2-yl)-4-methyl-5-[(morpholin-4-yl)methyl]imidazolidin-2-one (Example 24);
(3aR,7aS)-3-(7,8-dihydrofuro[3,2-e][1,3]benzothiazol-2-yl)-5-methyloctahydro-2H-imidazo[4,5-c]pyridin-2-one (Example 28);
(3aR,7aS)-5-cyclopropyl-3-(7,8-dihydro[1,4]dioxino[2,3-e][1,3]benzothiazol-2-yl)octahydro-2H-imidazo[4,5-c]pyridin-2-one (Example 34);
(3aR,7aS)-5-cyclopropyl-3-(2H-[1,3]dioxolo[4,5-e][1,3]benzothiazol-7-yl)octahydro-2H-imidazo[4,5-c]pyridin-2-one (Example 36);
(3aR,7aR)-1-(7,8-dihydro[1,4]dioxino[2,3-e][1,3]benzothiazol-2-yl)hexahydropyrano[3,4-d]imidazol-2(3H)-one (Example 37);
(3aR,6S,7aR)-1-(7,8-dihydrofuro[3,2-e][1,3]benzothiazol-2-yl)-6-methylhexahydropyrano[3,4-d]imidazol-2(3H)-one (Example 38);
(3aR,6S,7aR)-1-(7,8-dihydro[1,4]dioxino[2,3-e][1,3]benzothiazol-2-yl)-6-methylhexahydropyrano[3,4-d]imidazol-2(3H)-one (Example 39);
(3aR,6R,7aR)-1-(7,8-dihydrofuro[3,2-e][1,3]benzothiazol-2-yl)-6-(fluoromethyl)hexahydropyrano[3,4-d]imidazol-2(3H)-one (Example 40);
(3aR,6R,7aR)-1-(7,8-dihydro[1,4]dioxino[2,3-e][1,3]benzothiazol-2-yl)-6-(fluoromethyl)hexahydropyrano[3,4-d]imidazol-2(3H)-one (Example 41);
(3aR,6S,7aR)-1-(7,8-dihydrofuro[3,2-e][1,3]benzothiazol-2-yl)-6-ethylhexahydropyrano[3,4-d]imidazol-2(3H)-one (Example 42);
(3aR,6S,7aR)-1-(7,8-dihydro[1,4]dioxino[2,3-e][1,3]benzothiazol-2-yl)-6-ethylhexahydropyrano[3,4-d]imidazol-2(3H)-one (Example 43);
(4S,5S)-1-(7,8-dihydrofuro[3,2-e][1,3]benzothiazol-2-yl)-5-(methoxymethyl)-4-methylimidazolidin-2-one (Example 44);
(3aR,6aS)-1-(7,8-dihydrofuro[3,2-e][1,3]benzothiazol-2-yl)tetrahydro-1H-furo[3,4-d]imidazol-2(3H)-one (Example 65);
rac-(3aR,7aS)-3-(7,8-dihydrofuro[3,2-e][1,3]benzothiazol-2-yl)-5-(oxetan-3-yl)octahydro-2H-imidazo[4,5-c]pyridin-2-one (Example 71);
rac-(3aR,7aS)-5-cyclobutyl-3-(7,8-dihydrofuro[3,2-e][1,3]benzothiazol-2-yl)octahydro-2H-imidazo[4,5-c]pyridin-2-one (Example 72);

rac-(3aR,8aS)-3-(7,8-dihydrofuro[3,2-e][1,3]benzothiazol-2-yl)-5-(oxetan-3-yl)octahydroimidazo[4,5-c]azepin-2(1H)-one (Example 73);
rac-(3aR,7aS)-3-(7,8-dihydro[1,4]dioxino[2,3-e][1,3]benzothiazol-2-yl)-5-methyloctahydro-2H-imidazo[4,5-c]pyridin-2-one (Example 76);
rac-(3aR,7aS)-1-(7,8-dihydro[1,4]dioxino[2,3-e][1,3]benzothiazol-2-yl)-5-methyloctahydro-2H-imidazo[4,5-c]pyridin-2-one (Example 77);
rac-(3aR,7aS)-1-(2H-[1,3]dioxolo[4,5-e][1,3]benzothiazol-7-yl)-5-methyloctahydro-2H-imidazo[4,5-c]pyridin-2-one (Example 78);
rac-(3aR,7aS)-3-(2H-[1,3]dioxolo[4,5-e] [1,3] benzothiazol-7-yl)-5-methyloctahydro-2H-imidazo[4,5-c]pyridin-2-one (Example 79);
rac-(3aR,7aS)-3-(7,8-dihydro[1,4]dioxino[2,3-e][1,3]benzothiazol-2-yl)-5-(oxetan-3-yl)octahydro-2H-imidazo[4,5-c]pyridin-2-one (Example 80);
rac-(3aR,7aS)-3-(2H-[1,3]dioxolo[4,5-e][1,3]benzothiazol-7-yl)-5-(oxetan-3-yl)octahydro-2H-imidazo[4,5-c]pyridin-2-one (Example 81);
rac-(3aR,8aS)-3-(2H-[1,3]dioxolo[4,5-e][1,3]benzothiazol-7-yl)-5-methyloctahydroimidazo[4,5-c]azepin-2(1H)-one (Example 85);
rac-(3aR,8aS)-3-(2H-[1,3]dioxolo[4,5-e][1,3]benzothiazol-7-yl)-5-(oxetan-3-yl)octahydroimidazo[4,5-c]azepin-2(1H)-one (Example 86);
(3aS,6S,7aR)-1-(7,8-dihydrofuro[3,2-e] [1,3]benzothiazol-2-yl)-5,6-dimethyloctahydro-2H-imidazo[4,5-c]pyridin-2-one (Example 93);
rac-(3aR,7aS)-5-cyclopropyl-3-(7,8-dihydrofuro[3,2-e][1,3]benzothiazol-2-yl)octahydro-2H-imidazo[4,5-c]pyridin-2-one (Example 98);
rac-(3aR,7aS)-3-(7,8-dihydrofuro[3,2-e] [1,3]benzothiazol-2-yl)-5-(2-propyn-1-yl)octahydro-2H-imidazo[4,5-c]pyridin-2-one (Example 99);
rac-[(3aR,7aS)-3-(7,8-dihydrofuro[3,2-e][1,3]benzothiazol-2-yl)-2-oxooctahydro-5H-imidazo[4,5-c]pyridin-5-yl]acetonitrile (Example 100);
rac-(3aR,6aS)-1-(7,8-dihydrofuro[3,2-e][1,3]benzothiazol-2-yl)-5-(2-propyn-1-yl)hexahydropyrrolo[3,4-d]imidazol-2(1H)-one (Example 102);
rac-[(3aR,6aS)-1-(7,8-dihydrofuro[3,2-e][1,3]benzothiazol-2-yl)-2-oxohexahydropyrrolo[3,4-d]imidazol-5(1H)-yl]acetonitrile (Example 103);
rac-(3aR,8aS)-5-cyclopropyl-3-(7,8-dihydrofuro[3,2-e][1,3]benzothiazol-2-yl)octahydroimidazo[4,5-c]azepin-2(1H)-one (Example 104);
rac-(3aR,8aS)-3-(7,8-dihydrofuro[3,2-e][1,3]benzothiazol-2-yl)-5-(2-propyn-1-yl)octahydroimidazo[4,5-c]azepin-2(1H)-one (Example 105);
rac-[(3aR,8aS)-3-(7,8-dihydrofuro[3,2-e][1,3]benzothiazol-2-yl)-2-oxooctahydroimidazo[4,5-c]azepin-5(1H)-yl]acetonitrile (Example 106);
rac-(3aR,7aS)-3-(7,8-dihydrofuro[3,2-e] [1,3]benzothiazol-2-yl)-5-(2,2,2-trifluoroethyl)octahydro-2H-imidazo[4,5-c]pyridin-2-one (Example 107);
rac-(3aR,7aS)-5-(2,2-difluoroethyl)-3-(7,8-dihydrofuro[3,2-e][1,3]benzothiazol-2-yl)octahydro-2H-imidazo[4,5-c]pyridin-2-one (Example 108);
(3aR,7aS)-5-cyclopropyl-3-(7,8-dihydrofuro[3,2-e][1,3]benzothiazol-2-yl)octahydro-2H-imidazo[4,5-c]pyridin-2-one (Example 110);
[(3aR,7aS)-3-(7,8-dihydrofuro[3,2-e][1,3]benzothiazol-2-yl)-2-oxooctahydro-5H-imidazo[4,5-c]pyridin-5-yl]acetonitrile (Example 112);
(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Example 114);
(4S,5R)-1-(7,8-dihydrofuro[3,2-e][1,3]benzothiazol-2-yl)-5-{[(3S)-3-fluoropyrrolidin-1-yl]methyl}-4-methylimidazolidin-2-one (Example 115);
(4S,5R)-5-[(3,3-difluoroazetidin-1-yl)methyl]-1-(7,8-dihydrofuro[3,2-e][1,3]benzothiazol-2-yl)-4-methylimidazolidin-2-one (Example 116);
(4S,5R)-5-[(3,3-difluoropyrrolidin-1-yl)methyl]-1-(7,8-dihydrofuro[3,2-e][1,3]benzothiazol-2-yl)-4-methylimidazolidin-2-one (Example 117);
(4S,5R)-1-(7,8-dihydrofuro[3,2-e][1,3]benzothiazol-2-yl)-5-[(3-fluoroazetidin-1-yl)methyl]-4-methylimidazolidin-2-one (Example 118);
rac-(3aR,7aR)-3-(7,8-dihydrofuro[3,2-e][1,3]benzothiazol-2-yl)hexahydropyrano[3,4-d]imidazol-2(3H)-one (Example 120);
rac-(3aR,7aR)-1-(7,8-dihydrofuro[3,2-e][1,3]benzothiazol-2-yl)hexahydropyrano[3,4-d]imidazol-2(3H)-one (Example 121);

(3aR,7aR)-1-(7,8-dihydrofuro[3,2-e][1,3]benzothiazol-2-yl)hexahydropyrano[3,4-d]imidazol-2(3H)-one (Example 122);
(3aR,7aR)-1-(7,8-dihydrofuro[3,2-e][1,3]benzothiazol-2-yl)-6,6-dimethylhexahydropyrano[3,4-d]imidazol-2(3H)-one (Example 124);
(3aR,6R,7aR)-1-(7,8-dihydrofuro[3,2-e][1,3]benzothiazol-2-yl)-2-oxooctahydropyrano[3,4-d]imidazole-6-carbonitrile (Example 127);
(3aR,6R,7aR)-1-(7,8-dihydrofuro[3,2-e][1,3]benzothiazol-2-yl)-6-methoxyhexahydropyrano[3,4-d]imidazol-2(3H)-one (Example 129);
(3aR,6R,7aR)-1-(2H-[1,3]dioxolo[4,5-e][1,3]benzothiazol-7-yl)-2-oxooctahydropyrano[3,4-d]imidazole-6-carbonitrile (Example 131);
(3aR,6R,7aR)-1-(7,8-dihydro[1,4]dioxino[2,3-e][1,3]benzothiazol-2-yl)-2-oxooctahydropyrano[3,4-d]imidazole-6-carbonitrile (Example 133);
(3aR,8aR)-1-(7,8-dihydro[1,4]dioxino[2,3-e][1,3]benzothiazol-2-yl)hexahydro-1H-oxepino[3,4-d]imidazol-2(3H)-one (Example 135);
(3aR,6S,7aR)-1-(2H-[1,3]dioxolo[4,5-e][1,3]benzothiazol-7-yl)-6-ethylhexahydropyrano[3,4-d]imidazol-2(3H)-one (Example 138);
(3aR,6S,7aR)-1-(2H-[1,3]dioxolo[4,5-e][1,3]benzothiazol-7-yl]-6-methylhexahydropyrano[3,4-d]imidazol-2(3H)-one (Example 139);
(3aR,6R,7aR)-1-(2H-[1,3]dioxolo[4,5-e][1,3]benzothiazol-7-yl]-6-(fluoromethyl)hexahydropyrano[3,4-d]imidazol-2(3H)-one (Example 140);
(3aR,6R,7aR)-1-(7,8-dihydrofuro[3,2-e][1,3]benzothiazol-2-yl)-6-(hydroxymethyl)hexahydropyrano[3,4-d]imidazol-2(3H)-one (Example 144);
(3aR,6R,7aR)-1-(7,8-dihydro[1,4]dioxino[2,3-e][1,3]benzothiazol-2-yl)-6-(hydroxymethyl)hexahydropyrano[3,4-d]imidazol-2(3H)-one (Example 146);
(3aR,6R,7aR)-1-(2H-[1,3]dioxolo[4,5-e][1,3]benzothiazol-7-yl)-6-(hydroxymethyl)hexahydropyrano[3,4-d]imidazol-2(3H)-one (Example 147);
(3aR,6R,7aR)-1-(7,8-dihydrofuro[3,2-e][1,3]benzothiazol-2-yl)-6-ethynylhexahydropyrano[3,4-d]imidazol-2(3H)-one (Example 149);
(3aR,7aR)-1-(7,8-dihydrofuro[3,2-e][1,3]benzothiazol-2-yl)-6-fluorohexahydropyrano[3,4-d]imidazol-2(3H)-one (Example 151);
(3aR,6S,6aS)-1-(7,8-dihydrofuro[3,2-e] [1,3]benzothiazol-2-yl)-6-(hydroxymethyl)tetrahydro-1H-furo[3,4-d]imidazol-2(3H)-one (Example 157);
(3aR,6S,6aS)-1-(7,8-dihydrofuro[3,2-e] [1,3]benzothiazol-2-yl)-6-(fluoromethyl)tetrahydro-1H-furo[3,4-d]imidazol-2(3H)-one (Example 159);
(3aR,6R,6aS)-1-(7,8-dihydrofuro[3,2-e][1,3]benzothiazol-2-yl)-6-methyltetrahydro-1H-furo[3,4-d]imidazol-2(3H)-one (Example 161); and
[(3aS,4R,6aR)-3-(7,8-dihydrofuro[3,2-e][1,3]benzothiazol-2-yl)-2-oxohexahydro-1H-furo[3,4-d]imidazol-4-yl] acetonitrile (Example 162).

**18.** A medicament comprising the compound according to any one of claims 1 to 17 or a pharmaceutically acceptable salt thereof as an active ingredient.

**19.** A pharmaceutical composition comprising the compound according to any one of claims 1 to 17 or a pharmaceutically acceptable salt thereof as an active ingredient.

**20.** A therapeutic agent and/or a prophylactic agent for use in preventing or treating a disease involving DYRK, comprising the compound according to any one of claims 1 to 17 or a pharmaceutically acceptable salt thereof as an active ingredient.

**21.** The therapeutic agent and/or the prophylactic agent according to claim 20, wherein the disease involving DYRK is frontotemporal dementia, progressive supranuclear palsy, corticobasal degeneration, Lewy body dementia, vascular dementia, traumatic brain injury, chronic traumatic encephalopathy, stroke, Alzheimer's disease, Parkinson's disease, Down's disease, or depression, and mental retardation, memory impairment, memory loss, learning disability, intellectual disability, cognitive dysfunction, mild cognitive impairment, or dementia symptom associated therewith, or brain tumor, pancreatic cancer, ovarian cancer, osteosarcoma, large intestine cancer, lung cancer, bone resorption disease, osteoporosis, sickle cell anemia, chronic renal disease, or bone resorption disease.

## Patentansprüche

1. Eine Verbindung, dargestellt durch die folgende Formel (1):

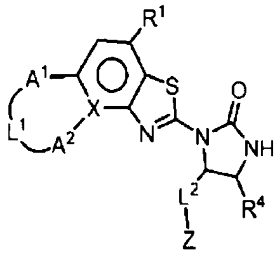

(1)

wobei

$A^1$ ein Sauerstoffatom oder ein Stickstoffatom (=N-) darstellt,
$A^2$ $CR^B$, $CR^CR^D$, ein Sauerstoffatom oder $NR^{A1}$ darstellt,
$L^1$ gegebenenfalls substituiertes Methylen, gegebenenfalls substituiertes Ethylen, gegebenenfalls substituiertes Methin, gegebenenfalls substituiertes Ethandiyliden, =N- oder $NR^{A2}$ darstellt,
$R^{A1}$, $R^{A2}$, $R^B$, $R^C$ und $R^D$ jeweils unabhängig ein Wasserstoffatom oder gegebenenfalls substituiertes $C_{1-6}$-Alkyl darstellen,
$R^1$ ein Wasserstoffatom, ein Halogenatom oder gegebenenfalls substituiertes $C_{1-6}$-Alkyl darstellt,
X ein Kohlenstoffatom oder ein Stickstoffatom darstellt,
$L^2$ gegebenenfalls substituiertes $C_{1-4}$-Alkylen darstellt,
$R^E$ gegebenenfalls substituiertes $C_{1-6}$-Alkyl darstellt,
Z $-NR^2R^3$ oder $-OR^7$ darstellt,
$R^7$ gegebenenfalls substituiertes $C_{1-6}$-Alkyl oder gegebenenfalls substituiertes $C_{1-7}$-Alkylen, das zusammen mit $R^4$ gebildet ist, darstellt, wobei $R^4$ und $R^7$ zusammen mit dem Kohlenstoffatom und dem Sauerstoffatom, an das sie jeweils gebunden sind, einen gegebenenfalls substituierten 5- bis 11-gliedrigen gesättigten Heterocyclus bilden,
$R^2$ ein Wasserstoffatom, gegebenenfalls substituiertes $C_{1-6}$-Alkyl, $C(O)-R^E$, $C_{3-10}$-Cycloalkyl, $C_{2-6}$-Alkinyl oder eine cyclische Gruppe eines 4- bis 11-gliedrigen gesättigten Heterocyclus darstellt,
$R^3$ ein Wasserstoffatom, gegebenenfalls substituiertes $C_{1-6}$-Alkyl oder $C(O)-R^E$ darstellt und
$R^4$ gegebenenfalls substituiertes $C_{1-6}$-Alkyl darstellt,
wobei $R^2$ und $R^3$ zusammen mit dem Stickstoffatom, an das sie gebunden sind, einen gegebenenfalls substituierten 4- bis 11-gliedrigen gesättigten Heterocyclus bilden können oder $R^3$ und $R^4$ zusammen mit dem Stickstoffatom und dem Kohlenstoffatom, an das sie jeweils gebunden sind, einen gegebenenfalls substituierten 4- bis 11-gliedrigen gesättigten Heterocyclus bilden können oder ein Kohlenstoffatom an dem durch $R^2$, $R^3$ und das Stickstoffatom gebildeten gesättigten Heterocyclus und $R^4$ zusammen einen 4- bis 11-gliedrigen gesättigten Heterocyclus bilden können oder ein pharmazeutisch verträgliches Salz davon.

2. Die Verbindung nach Anspruch 1 oder ein pharmazeutisch verträgliches Salz davon, wobei Z $-NR^2R^3$ darstellt und $R^2$ und $R^3$ jeweils unabhängig ein Wasserstoffatom, gegebenenfalls substituiertes $C_{1-6}$-Alkyl oder $C(O)-R^E$ darstellen, wobei $R^2$ und $R^3$ zusammen mit dem Stickstoffatom, an das sie gebunden sind, einen gegebenenfalls substituierten 4- bis 8-gliedrigen gesättigten Heterocyclus bilden können.

3. Die Verbindung nach Anspruch 1 oder ein pharmazeutisch verträgliches Salz davon, wobei Z $-NR^2R^3$ darstellt und $R^3$ und $R^4$ zusammen mit dem Stickstoffatom und dem Kohlenstoffatom, an das sie jeweils gebunden sind, einen gegebenenfalls substituierten 4- bis 8-gliedrigen gesättigten Heterocyclus bilden.

4. Die Verbindung nach einem der Ansprüche 1 bis 3 oder ein pharmazeutisch verträgliches Salz davon, wobei $R^1$ ein Wasserstoffatom ist.

5. Die Verbindung nach einem der Ansprüche 1 bis 4 oder ein pharmazeutisch verträgliches Salz davon, wobei X ein Kohlenstoffatom ist.

6. Die Verbindung nach Anspruch 5 oder ein pharmazeutisch verträgliches Salz davon, wobei $A^1$ ein Sauerstoffatom ist,

$A^2$ Methylen ist und $L^1$ Methylen ist.

7. Die Verbindung nach Anspruch 1, 2, 4, 5 oder 6 oder ein pharmazeutisch verträgliches Salz davon, wobei Formel (1) durch die folgende Formel (1a) dargestellt ist:

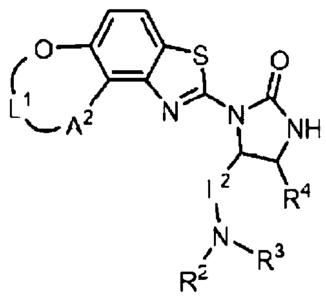
(1a)

wobei

$A^2$ gegebenenfalls substituiertes Methylen oder ein Sauerstoffatom darstellt,
$L^1$ gegebenenfalls substituiertes Methylen oder gegebenenfalls substituiertes Ethylen darstellt,
$L^2$ gegebenenfalls substituiertes $C_{1-4}$-Alkylen darstellt,
$R^2$ und $R^3$ jeweils unabhängig ein Wasserstoffatom, gegebenenfalls substituiertes $C_{1-6}$-Alkyl oder C(O)-$R^E$ darstellen, wobei $R^2$ und $R^3$ zusammen mit dem Stickstoffatom, an das sie gebunden sind, einen gegebenenfalls substituierten 4- bis 8-gliedrigen gesättigten Heterocyclus bilden können,
$R^E$ gegebenenfalls substituiertes $C_{1-6}$-Alkyl darstellt und
$R^4$ gegebenenfalls substituiertes $C_{1-6}$-Alkyl darstellt.

8. Die Verbindung nach Anspruch 7, wobei $R^2$ und $R^3$ jeweils unabhängig gegebenenfalls substituiertes $C_{1-6}$-Alkyl darstellen, oder ein pharmazeutisch verträgliches Salz davon.

9. Die Verbindung nach Anspruch 7 oder ein pharmazeutisch verträgliches Salz davon, wobei $R^2$ und $R^3$ zusammen mit dem Stickstoffatom, an das sie gebunden sind, einen gegebenenfalls substituierten 4- bis 8-gliedrigen gesättigten Heterocyclus bilden.

10. Die Verbindung nach einem der Ansprüche 7 bis 9 oder ein pharmazeutisch verträgliches Salz davon, wobei $L^2$ $C_{1-3}$-Alkylen ist.

11. Die Verbindung nach Anspruch 1, 3, 4, 5 oder 6 oder ein pharmazeutisch verträgliches Salz davon, wobei Formel (1) durch die folgende Formel (1b) dargestellt ist:

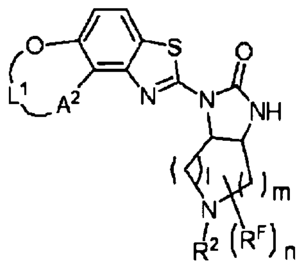
(1b)

wobei

$A^2$ gegebenenfalls substituiertes Methylen oder ein Sauerstoffatom darstellt,
$L^1$ gegebenenfalls substituiertes Methylen oder gegebenenfalls substituiertes Ethylen darstellt,
l und m jeweils unabhängig 1, 2 oder 3 darstellen, wobei eine Summe von l und m 5 oder weniger beträgt,
n 1, 2, 3 oder 4 darstellt,
$R^2$ ein Wasserstoffatom, gegebenenfalls substituiertes $C_{1-6}$-Alkyl, $C_{3-10}$-Cycloalkyl oder C(O)-$R^E$ darstellt,
$R^E$ gegebenenfalls substituiertes $C_{1-6}$-Alkyl darstellt,
$R^F$ ein Wasserstoffatom, ein Halogenatom oder gegebenenfalls substituiertes $C_{1-6}$-Alkyl darstellt,

wenn n 2, 3 oder 4 ist, jedes $R^F$ gleich oder verschieden sein kann, und zwei $R^F$ an demselben Kohlenstoffatom zusammen mit dem Kohlenstoffatom, an das sie jeweils gebunden sind, einen Spiro-Ring bilden können, der aus einem 4- bis 8-gliedrigen gesättigten Heterocyclus oder einem 3- bis 8-gliedrigen gesättigten Carbocyclus besteht, oder zwei $R^F$ an verschiedenen Kohlenstoffatomen aneinander gebunden sein können, um eine Querverbindung zu bilden.

**12.** Die Verbindung nach Anspruch 11 oder ein pharmazeutisch verträgliches Salz davon, wobei $R^2$ ein Wasserstoffatom, gegebenenfalls substituiertes $C_{1-6}$-Alkyl oder $C_{3-10}$-Cycloalkyl ist.

**13.** Die Verbindung nach Anspruch 11 oder ein pharmazeutisch verträgliches Salz davon, wobei $R^2$ gegebenenfalls substituiertes $C_{1-6}$-Alkyl ist.

**14.** Die Verbindung nach einem der Ansprüche 11 bis 13 oder ein pharmazeutisch verträgliches Salz davon, wobei l und m jeweils unabhängig 1 oder 2 sind.

**15.** Die Verbindung nach Anspruch 1, wobei Z -$OR^7$ ist und $R^4$ und $R^7$ zusammen mit dem Kohlenstoffatom und dem Sauerstoffatom, an das sie jeweils gebunden sind, einen gegebenenfalls substituierten 5- bis 8-gliedrigen gesättigten Heterocyclus bilden, oder ein pharmazeutisch verträgliches Salz davon.

**16.** Die Verbindung nach Anspruch 1 oder 15 oder ein pharmazeutisch verträgliches Salz davon, wobei Formel (1) durch die folgende Formel (1c) dargestellt ist:

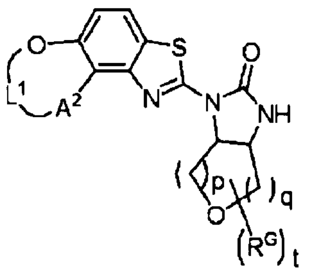

(1c)

wobei

$A^2$ gegebenenfalls substituiertes Methylen oder ein Sauerstoffatom darstellt,
$L^1$ gegebenenfalls substituiertes Methylen oder gegebenenfalls substituiertes Ethylen darstellt,
p und q jeweils unabhängig 1, 2 oder 3 darstellen, wobei eine Summe von p und q 5 oder weniger beträgt,
t 1, 2, 3 oder 4 darstellt,
$R^G$ ein Wasserstoffatom, ein Halogenatom, gegebenenfalls substituiertes $C_{1-6}$-Alkyl, gegebenenfalls substituiertes $C_{1-6}$-Alkoxy oder eine CN-Gruppe darstellt,
wenn t 2, 3 oder 4 ist, jedes $R^G$ gleich oder verschieden sein kann und zwei $R^G$ an demselben Kohlenstoffatom zusammen mit dem Kohlenstoffatom, an das sie jeweils gebunden sind, einen Spiro-Ring bilden können, der aus einem 4- bis 8-gliedrigen gesättigten Heterocyclus oder einem 3- bis 8-gliedrigen gesättigten Carbocyclus besteht, oder zwei $R^G$ an verschiedenen Kohlenstoffatomen aneinander gebunden sein können, um eine Querverbindung zu bilden.

**17.** Die Verbindung nach einem der Ansprüche 1 bis 16 oder ein pharmazeutisch verträgliches Salz davon, wobei die Verbindung aus der Gruppe der folgenden Verbindungen ausgewählt ist:

cis-3-(7,8-Dihydrofuro[3,2-e][1,3]benzothiazol-2-yl)-5-methyloctahydro-2H-imidazo[4,5-c]pyridin-2-on (Beispiel 7);
cis-3-(7,8-Dihydrofuro[3,2-e][1,3]benzothiazol-2-yl)-5-(2-methoxyethyl)octahydro-2H-imidazo[4,5-c]pyridin-2-on (Beispiel 8);
cis-1-(7,8-Dihydrofuro[3,2-e][1,3]benzothiazol-2-yl)-5-methyloctahydro-2H-imidazo[4,5-c]pyridin-2-on (Beispiel 9);
cis-3-(7,8-Dihydrofuro[3,2-e][1,3]benzothiazol-2-yl)-5-ethyloctahydro-2H-imidazo[4,5-c]pyridin-2-on (Beispiel 11);
cis-1-(7,8-Dihydrofuro[3,2-e][1,3]benzothiazol-2-yl)-5-methylhexahydropyrrolo[3,4-d]imidazol-2(1H)-on (Beispiel 14);
cis-3-(7,8-Dihydrofuro[3,2-e][1,3]benzothiazol-2-yl)-5-(propan-2-yl)octahydro-2H-imidazo[4,5-c]pyridin-2-on (Beispiel 15);
cis-3-(7,8-Dihydrofuro[3,2-e][1,3]benzothiazol-2-yl)-5-methyloctahydroimidazo[4,5-c]azepin-2(1H)-on (Beispiel 17);
(3aS,7aS)-3-(7,8-Dihydrofuro[3,2-e][1,3]benzothiazol-2-yl)-5-(oxetan-3-yl)octahydro-2H-imidazo[4,5-c]pyridin-2-on (Beispiel 19);
(3aR,7aR)-1-(2H-[1,3]Dioxolo[4,5-e][1,3]benzothiazol-7-yl)hexahydropyrano[3,4-d]imidazol-2(3H)-on (Beispiel 21);
(4S,5R)-1-(7,8-Dihydrofuro[3,2-e][1,3]benzothiazol-2-yl)-4-methyl-5-[(morpholin-4-yl)methyl]imidazolidin-2-on (Beispiel 23);
(4R,5R)-1-(7,8-Dihydrofuro[3,2-e][1,3]benzothiazol-2-yl)-4-methyl-5-[(morpholin-4-yl)methyl]imidazolidin-2-on (Beispiel 24);
(3aR,7aS)-3-(7,8-Dihydrofuro[3,2-e][1,3]benzothiazol-2-yl)-5-methyloctahydro-2H-imidazo[4,5-c]pyridin-2-on (Beispiel 28);
(3aR,7aS)-5-Cyclopropyl-3-(7,8-dihydro[1,4]dioxino[2,3-e][1,3]benzothiazol-2-yl)octahydro-2H-imidazo[4,5-c]pyridin-2-on (Beispiel 34);
(3aR,7aS)-5-Cyclopropyl-3-(2H-[1,3]dioxolo[4,5-e][1,3]benzothiazol-7-yl)octahydro-2H-imidazo[4,5-c]pyridin-2-on (Beispiel 36);
(3aR,7aR)-1-(7,8-Dihydro[1,4]dioxino[2,3-e][1,3]benzothiazol-2-yl)hexahydropyrano[3,4-d]imidazol-2(3H)-on (Beispiel 37);
(3aR,6S,7aR)-1-(7,8-Dihydrofuro[3,2-e][1,3]benzothiazol-2-yl)-6-methylhexahydropyrano[3,4-d]imidazol-2(3H)-on (Beispiel 38);
(3aR,6S,7aR)-1-(7,8-Dihydro[1,4]dioxino[2,3-e][1,3]benzothiazol-2-yl)-6-methylhexahydropyrano[3,4-d]imidazol-2(3H)-on (Beispiel 39);
(3aR,6R,7aR)-1-(7,8-Dihydrofuro[3,2-e][1,3]benzothiazol-2-yl)-6-(fluormethyl)hexahydropyrano[3,4-d]imidazol-2(3H)-on (Beispiel 40);
(3aR,6R,7aR)-1-(7,8-Dihydro[1,4]dioxino[2,3-e][1,3]benzothiazol-2-yl)-6-(fluormethyl)hexahydropyrano[3,4-d]imidazol-2(3H)-on (Beispiel 41);
(3aR,6S,7aR)-1-(7,8-Dihydrofuro[3,2-e][1,3]benzothiazol-2-yl)-6-ethylhexahydropyrano[3,4-d]imidazol-2(3H)-on (Beispiel 42);
(3aR,6S,7aR)-1-(7,8-Dihydro[1,4]dioxino[2,3-e][1,3]benzothiazol-2-yl)-6-ethylhexahydropyrano[3,4-d]imidazol-2(3H)-on (Beispiel 43);
(4S,5S)-1-(7,8-Dihydrofuro[3,2-e][1,3]benzothiazol-2-yl)-5-(methoxymethyl)-4-methylimidazolidin-2-on (Beispiel 44);
(3aR,6aS)-1-(7,8-Dihydrofuro[3,2-e][1,3]benzothiazol-2-yl)tetrahydro-1H-furo[3,4-d]imidazol-2(3H)-on (Beispiel 65);
rac-(3aR,7aS)-3-(7,8-Dihydrofuro[3,2-e][1,3]benzothiazol-2-yl)-5-(oxetan-3-yl)octahydro-2H-imidazo[4,5-c]pyridin-2-on (Beispiel 71);
rac-(3aR,7aS)-5-Cyclobutyl-3-(7,8-dihydrofuro[3,2-e][1,3]benzothiazol-2-yl)octahydro-2H-imidazo[4,5-c]pyridin-2-on (Beispiel 72);
rac-(3aR,8aS)-3-(7,8-Dihydrofuro[3,2-e][1,3]benzothiazol-2-yl)-5-(oxetan-3-yl)octahydroimidazo[4,5-c]azepin-2(1H)-on (Beispiel 73);
rac-(3aR,7aS)-3-(7,8-Dihydro[1,4]dioxino[2,3-e][1,3]benzothiazol-2-yl)-5-methyloctahydro-2H-imidazo [4,5-c]pyridin-2-on (Beispiel 76);
rac-(3aR,7aS)-1-(7,8-Dihydro[1,4]dioxino[2,3-e][1,3]benzothiazol-2-yl)-5-methyloctahydro-2H-imidazo[4,5-c]pyridin-2-on (Beispiel 77);
rac-(3aR,7aS)-1-(2H-[1,3]Dioxolo[4,5-e][1,3]benzothiazol-7-yl)-5-methyloctahydro-2H-imidazo[4,5-c]pyridin-2-on (Beispiel 78);
rac-(3aR,7aS)-3-(2H-[1,3]Dioxolo[4,5-e][1,3]benzothiazol-7-yl)-5-methyloctahydro-2H-imidazo[4,5-c]pyridin-2-on (Beispiel 79);
rac-(3aR,7aS)-3-(7,8-Dihydro[1,4]dioxino[2,3-e][1,3]benzothiazol-2-yl)-5-(oxetan-3-yl)octahydro-2H-imidazo [4,5-c]pyridin-2-on (Beispiel 80);
rac-(3aR,7aS)-3-(2H-[1,3]Dioxolo[4,5-e][1,3]benzothiazol-7-yl)-5-(oxetan-3-yl)octahydro-2H-imidazo[4,5-c]pyridin-2-on (Beispiel 81);
rac-(3aR,8aS)-3-(2H-[1,3]Dioxolo[4,5-e][1,3]benzothiazol-7-yl)-5-methyloctahydroimidazo[4,5-c]azepin-2(1H)-on (Beispiel 85);
rac-(3aR,8aS)-3-(2H-[1,3]Dioxolo[4,5-e][1,3]benzothiazol-7-yl)-5-(oxetan-3-yl)octahydroimidazo[4,5-c]azepin-2(1H)-on (Beispiel 86);
(3aS,6S,7aR)-1-(7,8-Dihydrofuro[3,2-e][1,3]benzothiazol-2-yl)-5,6-dimethyloctahydro-2H-imidazo[4,5-c]pyridin-2-on (Beispiel 93);
rac-(3aR,7aS)-5-Cyclopropyl-3-(7,8-dihydrofuro[3,2-e][1,3]benzothiazol-2-yl)octahydro-2H-imidazo[4,5-c]pyridin-2-on (Beispiel 98);
rac-(3aR,7aS)-3-(7,8-Dihydrofuro[3,2-e][1,3]benzothiazol-2-yl)-5-(2-propin-1-yl)octahydro-2H-imidazo[4,5-c]pyridin-2-on (Beispiel 99);
rac-[(3aR,7aS)-3-(7,8-Dihydrofuro[3,2-e][1,3]benzothiazol-2-yl)-2-oxooctahydro-5H-imidazo[4,5-c]pyridin-5-yl]acetonitril (Beispiel 100);
rac-(3aR,6aS)-1-(7,8-Dihydrofuro[3,2-e][1,3]benzothiazol-2-yl)-5-(2-propin-1-yl)hexahydropyrrolo[3,4-d]imidazol-2(1H)-on (Beispiel 102) ;
rac-[(3aR,6aS)-1-(7,8-Dihydrofuro[3,2-e][1,3]benzothiazol-2-yl)-2-oxohexahydropyrrolo[3,4-d]imidazol-5(1H)-yl]acetonitril (Beispiel 103);
rac-(3aR,8aS)-5-Cyclopropyl-3-(7,8-dihydrofuro[3,2-e][1,3]benzothiazol-2-yl)octahydroimidazo[4,5-c]azepin-2(1H)-on (Beispiel 104);
rac-(3aR,8aS)-3-(7,8-Dihydrofuro[3,2-e][1,3]benzothiazol-2-yl)-5-(2-propin-1-yl)octahydroimidazo[4,5-c]azepin-2(1H)-on (Beispiel 105);
rac-[(3aR,8aS)-3-(7,8-Dihydrofuro[3,2-e][1,3]benzothiazol-2-yl)-2-oxooctahydroimidazo[4,5-c]azepin-5(1H)-yl]acetonitril (Beispiel 106);
rac-(3aR,7aS)-3-(7,8-Dihydrofuro[3,2-e][1,3]benzothiazol-2-yl)-5-(2,2,2-trifluorethyl)octahydro-2H-imidazo[4,5-c]pyridin-2-on (Beispiel 107);
rac-(3aR,7aS)-5-(2,2-Difluorethyl)-3-(7,8-dihydrofuro[3,2-e][1,3]benzothiazol-2-yl)octahydro-2H-imidazo[4,5-c]pyridin-2-on (Beispiel 108);
(3aR,7aS)-5-Cyclopropyl-3-(7,8-dihydrofuro[3,2-e][1,3]benzothiazol-2-yl)octahydro-2H-imidazo[4,5-c]pyridin-2-on (Beispiel 110);
[(3aR,7aS)-3-(7,8-Dihydrofuro[3,2-e][1,3]benzothiazol-2-yl)-2-oxooctahydro-5H-imidazo[4,5-c]pyridin-5-yl]acetonitril (Beispiel 112);
(3aR,7aS)-3-(7,8-Dihydrofuro[3,2-e][1,3]benzothiazol-2-yl)-5-(2-propin-1-yl)octahydro-2H-imidazo[4,5-c]pyridin-2-on (Beispiel 114);
(4S,5R)-1-(7,8-Dihydrofuro[3,2-e][1,3]benzothiazol-2-yl)-5-{[(3S)-3-fluorpyrrolidin-1-yl]methyl}-4-methylimidazolidin-2-on (Beispiel 115) ;
(4S,5R)-5-[(3,3-Difluorazetidin-1-yl)methyl]-1-(7,8-dihydrofuro[3,2-e][1,3]benzothiazol-2-yl)-4-methylimidazolidin-2-on (Beispiel 116);
(4S,5R)-5-[(3,3-Difluorpyrrolidin-1-yl)methyl]-1-(7,8-dihydrofuro[3,2-e][1,3]benzothiazol-2-yl)-4-methylimidazolidin-2-on (Beispiel 117);
(4S,5R)-1-(7,8-Dihydrofuro[3,2-e][1,3]benzothiazol-2-yl)-5-[(3-fluorazetidin-1-yl)methyl]-4-methylimidazolidin-2-on (Beispiel 118);
rac-(3aR,7aR)-3-(7,8-Dihydrofuro[3,2-e][1,3]benzothiazol-2-yl)hexahydropyrano[3,4-d]imidazol-2(3H)-on (Beispiel 120);
rac-(3aR,7aR)-1-(7,8-Dihydrofuro[3,2-e][1,3]benzothiazol-2-yl)hexahydropyrano[3,4-d]imidazol-2(3H)-on (Beispiel 121);
(3aR,7aR)-1-(7,8-Dihydrofuro[3,2-e][1,3]benzothiazol-2-yl)hexahydropyrano[3,4-d]imidazol-2(3H)-on (Beispiel 122);
(3aR,7aR)-1-(7,8-Dihydrofuro[3,2-e][1,3]benzothiazol-2-yl)-6,6-dimethylhexahydropyrano[3,4-d]imidazol-2(3H)-on (Beispiel 124);
(3aR,6R,7aR)-1-(7,8-Dihydrofuro[3,2-e][1,3]benzothiazol-2-yl)-2-oxooctahydropyrano[3,4-d]imidazol-6-carbonitril (Beispiel 127);
(3aR,6R,7aR)-1-(7,8-Dihydrofuro[3,2-e][1,3]benzothiazol-2-yl)-6-methoxyhexahydropyrano[3,4-d]imidazol-2(3H)-on (Beispiel 129);
(3aR,6R,7aR)-1-(2H-[1,3]dioxolo[4,5-e][1,3]benzothiazol-7-yl)-2-oxooctahydropyrano[3,4-d]imidazol-6-carbonitril (Beispiel 131);
(3aR,6R,7aR)-1-(7,8-Dihydro[1,4]dioxino[2,3-e][1,3]benzothiazol-2-yl)-2-oxooctahydropyrano[3,4-d]imidazol-6-carbonitril (Beispiel 133);
(3aR,8aR)-1-(7,8-Dihydro[1,4]dioxino[2,3-e][1,3]benzothiazol-2-yl)hexahydro-1H-oxepino[3,4-d]imidazol-2(3H)-on (Beispiel 135);
(3aR,6S,7aR)-1-(2H-[1,3]Dioxolo[4,5-e][1,3]benzothiazol-7-yl)-6-ethylhexahydropyrano[3,4-d]imidazol-2(3H)-on (Beispiel 138);
(3aR,6S,7aR)-1-(2H-[1,3]Dioxolo[4,5-e][1,3]benzothiazol-7-yl]-6-methylhexahydropyrano[3,4-d]imidazol-2(3H)-on (Beispiel 139);
(3aR,6R, 7aR)-1-(2H-[1,3]Dioxolo[4,5-e][1,3]benzothiazol-7-yl]-6-(fluormethyl)hexahydropyrano[3,4-d]imidazol-2 (3H)-on (Beispiel 140);
(3aR,6R,7aR)-1-(7,8-Dihydrofuro[3,2-e][1,3]benzothiazol-2-yl)-6-(hydroxymethyl)hexahydropyrano[3,4-d]imidazol-2(3H)-on (Beispiel 144);
(3aR,6R,7aR)-1-(7,8-Dihydro[1,4]dioxino[2,3-e][1,3]benzothiazol-2-yl)-6-(hydroxymethyl)hexahydropyrano [3,4-d]imidazol-2(3H)-on (Beispiel 146);
(3aR,6R, 7aR)-1-(2H-[1,3]Dioxolo[4,5-e][1,3]benzothiazol-7-yl)-6-(hydroxymethyl)hexahydropyrano[3,4-d]imidazol-2(3H)-on (Beispiel 147);
(3aR,6R,7aR)-1-(7,8-Dihydrofuro[3,2-e][1,3]benzothiazol-2-yl)-6-ethinylhexahydropyrano[3,4-d]imidazol-2(3H)-on (Beispiel 149);
(3aR,7aR)-1-(7,8-Dihydrofuro[3,2-e][1,3]benzothiazol-2-yl)-6-fluorhexahydropyrano[3,4-d]imidazol-2(3H)-on (Beispiel 151);
(3aR,6S,6aS)-1-(7,8-Dihydrofuro[3,2-e][1,3]benzothiazol-2-yl)-6-(hydroxymethyl)tetrahydro-1H-furo[3,4-d]imidazol-2(3H)-on (Beispiel 157);
(3aR,6S,6aS)-1-(7,8-Dihydrofuro[3,2-e][1,3]benzothiazol-2-yl)-6-(fluormethyl)tetrahydro-1H-furo[3,4-d]imidazol-2(3H)-on (Beispiel 159);
(3aR,6R,6aS)-1-(7,8-Dihydrofuro[3,2-e][1,3]benzothiazol-2-yl)-6-methyltetrahydro-1H-furo[3,4-d]imidazol-2(3H)-on (Beispiel 161); und
[(3aS,4R,6aR)-3-(7,8-Dihydrofuro[3,2-e][1,3]benzothiazol-2-yl)-2-oxohexahydro-1H-furo[3,4-d]imidazol-4-yl] acetonitril (Beispiel 162).

**18.** Ein Medikament, umfassend die Verbindung nach einem der Ansprüche 1 bis 17 oder ein pharmazeutisch verträgliches Salz davon als einen Wirkstoff.

**19.** Ein Arzneimittel, umfassend die Verbindung nach einem der Ansprüche 1 bis 17 oder ein pharmazeutisch verträgliches Salz davon als einen Wirkstoff.

**20.** Ein Therapeutikum und/oder ein Prophylaktikum zur Verwendung bei der Vorbeugung oder Behandlung einer DYRK-assoziierten Erkrankung, umfassend die Verbindung nach einem der Ansprüche 1 bis 17 oder ein pharmazeutisch verträgliches Salz davon als einen Wirkstoff.

**21.** Das Therapeutikum und/oder das Prophylaktikum nach Anspruch 20, wobei die DYRKassoziierte Erkrankung frontotemporale Demenz, progressive supranukleäre Lähmung, kortikobasale Degeneration, Lewy-Körper-Demenz, vaskuläre Demenz, traumatische Hirnverletzung, chronisch-traumatische Enzephalopathie, Schlaganfall, Alzheimer-Krankheit, Parkinson-Krankheit, Down-Syndrom oder Depression und mentale Retardierung, Gedächtnisbeeinträchtigung, Gedächtnisverlust, Lernbehinderung, intellektuelle Behinderung, kognitive Dysfunktion, leichte kognitive Beeinträchtigung oder damit zusammenhängende Demenzsymptome, oder Hirntumor, Bauchspeicheldrüsenkrebs, Eierstockkrebs, Osteosarkom, Dickdarmkrebs, Lungenkrebs, Knochenresorptionserkrankung, Osteoporose, Sichelzellenanämie, chronische Nierenerkrankung oder Knochenresorptionserkrankung ist.

## Revendications

**1.** Composé représenté par la formule suivante (1) :

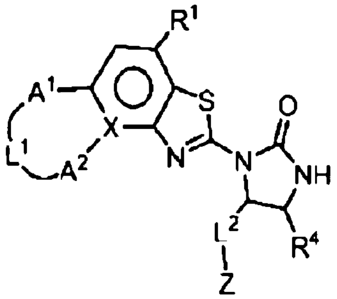

(1)

dans laquelle

$A^1$ représente un atome d'oxygène ou un atome d'azote (=N-),
$A^2$ représente $CR^B$, $CR^CR^D$, un atome d'oxygène, ou $NR^{A1}$,
$L^1$ représente un méthylène éventuellement substitué, un éthylène éventuellement substitué, une méthine éventuellement substituée, un éthanediylidène éventuellement substitué, =N-, ou $NR^{A2}$,
$R^{A1}$, $R^{A2}$, $R^B$, $R^C$, et $R^D$ représentent chacun indépendamment un atome d'hydrogène ou un alkyle en $C_{1-6}$ éventuellement substitué,
$R^1$ représente un atome d'hydrogène, un atome d'halogène ou un alkyle en $C_{1-6}$ éventuellement substitué,
X représente un atome de carbone ou un atome d'azote,
$L^2$ représente un alkylène en $C_{1-4}$ éventuellement substitué,
$R^E$ représente un alkyle en $C_{1-6}$ éventuellement substitué,
Z représente $-NR^2R^3$ ou $-OR^7$,
$R^7$ représente un alkyle en $C_{1-6}$ éventuellement substitué, ou un alkylène en $C_{1-7}$ éventuellement substitué formé conjointement avec $R^4$, dans lequel $R^4$ et $R^7$, conjointement avec l'atome de carbone et l'atome d'oxygène auxquels ils sont respectivement liés, forment un hétérocycle saturé de 5 à 11 chaînons éventuellement substitué,
$R^2$ représente un atome d'hydrogène, un alkyle en $C_{1-6}$ éventuellement substitué, $C(O)-R^E$, un cycloalkyle en $C_{3-10}$, un alcynyle en $C_{2-6}$, ou un groupe cyclique d'un hétérocycle saturé de 4 à 11 chaînons,
$R^3$ représente un atome d'hydrogène, un alkyle en $C_{1-6}$ éventuellement substitué, ou $C(O)-R^E$, et
$R^4$ représente un alkyle en $C_{1-6}$ éventuellement substitué,
dans lequel $R^2$ et $R^3$, conjointement avec l'atome d'azote auquel ils sont liés, peuvent former un hétérocycle saturé de 4 à 11 chaînons éventuellement substitué, ou $R^3$ et $R^4$, conjointement avec l'atome d'azote et l'atome de carbone auxquels ils sont respectivement liés, peuvent former un hétérocycle saturé de 4 à 11 chaînons éventuellement substitué, ou tout atome de carbone sur l'hétérocycle saturé constitué par $R^2$, $R^3$, et l'atome d'azote, et $R^4$ conjointement peuvent former un hétérocycle saturé de 4 à 11 chaînons,
ou un sel pharmaceutiquement acceptable de celui-ci.

2. Composé selon la revendication 1 ou un sel pharmaceutiquement acceptable de celui-ci, dans lequel Z représente $-NR^2R^3$, et $R^2$ et $R^3$ représentent chacun indépendamment un atome d'hydrogène, un alkyle en $C_{1-6}$ éventuellement substitué, ou $C(O)-R^E$, dans lequel $R^2$ et $R^3$, conjointement avec l'atome d'azote auquel ils sont liés, peuvent former un hétérocycle saturé de 4 à 8 chaînons éventuellement substitué.

3. Composé selon la revendication 1 ou un sel pharmaceutiquement acceptable de celui-ci, dans lequel Z représente $-NR^2R^3$, et $R^3$ et $R^4$, conjointement avec l'atome d'azote et l'atome de carbone auxquels ils sont respectivement liés, forment un hétérocycle saturé de 4 à 8 chaînons éventuellement substitué.

4. Composé selon l'une quelconque des revendications 1 à 3 ou un sel pharmaceutiquement acceptable de celui-ci, dans lequel $R^1$ est un atome d'hydrogène.

5. Composé selon l'une quelconque des revendications 1 à 4 ou un sel pharmaceutiquement acceptable de celui-ci, dans lequel X est un atome de carbone.

6. Composé selon la revendication 5 ou un sel pharmaceutiquement acceptable de celui-ci, dans lequel $A^1$ est un atome d'oxygène, $A^2$ est un méthylène, et $L^1$ est un méthylène.

7. Composé selon la revendication 1, 2, 4, 5 ou 6 ou un sel pharmaceutiquement acceptable de celui-ci, dans lequel la formule (1) est représentée par la formule suivante (1a) :

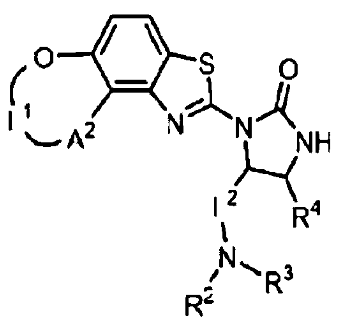

(1a)

dans laquelle

$A^2$ représente un méthylène éventuellement substitué ou un atome d'oxygène,
$L^1$ représente un méthylène éventuellement substitué ou un éthylène éventuellement substitué,
$L^2$ représente un alkylène en $C_{1-4}$ éventuellement substitué,
$R^2$ et $R^3$ représentent chacun indépendamment un atome d'hydrogène, un alkyle en $C_{1-6}$ éventuellement substitué, ou C(O)-$R^E$, dans lequel $R^2$ et $R^3$, conjointement avec l'atome d'azote auquel ils sont liés, peuvent former un hétérocycle saturé de 4 à 8 chaînons éventuellement substitué,
$R^E$ représente un alkyle en $C_{1-6}$ éventuellement substitué, et
$R^4$ est un alkyle en $C_{1-6}$ éventuellement substitué.

**8.** Composé selon la revendication 7, dans lequel $R^2$ et $R^3$ sont chacun indépendamment un alkyle en $C_{1-6}$ éventuellement substitué, ou un sel pharmaceutiquement acceptable de celui-ci.

**9.** Composé selon la revendication 7 ou un sel pharmaceutiquement acceptable de celui-ci, dans lequel $R^2$ et $R^3$, conjointement avec l'atome d'azote auquel ils sont liés, forment un hétérocycle saturé de 4 à 8 chaînons éventuellement substitué,

**10.** Composé selon l'une quelconque des revendications 7 à 9 ou un sel pharmaceutiquement acceptable de celui-ci, dans lequel $L^2$ est un alkylène en $C_{1-3}$.

**11.** Composé selon la revendication 1, 3, 4, 5, ou 6 ou un sel pharmaceutiquement acceptable de celui-ci, dans lequel la formule (1) est représentée par la formule suivante (1b) :

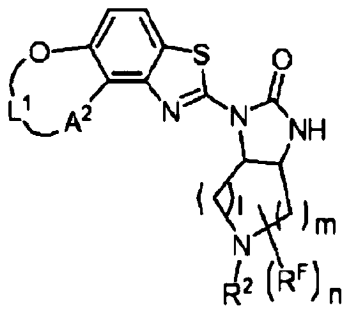

(1b)

dans laquelle

$A^2$ représente un méthylène éventuellement substitué ou un atome d'oxygène,
$L^1$ représente un méthylène éventuellement substitué ou un éthylène éventuellement substitué,
1 et m représentent chacun indépendamment 1, 2, ou 3, dans lequel la somme de 1 et m est égale à 5 ou moins,
n représente 1, 2, 3, ou 4,
$R^2$ représente un atome d'hydrogène, un alkyle en $C_{1-6}$ éventuellement substitué, un cycloalkyle en $C_{3-10}$, ou C(O)-$R^E$,
$R^E$ représente un alkyle en $C_{1-6}$ éventuellement substitué,
$R^F$ représente un atome d'hydrogène, un atome d'halogène, ou un alkyle en $C_{1-6}$ éventuellement substitué, lorsque n est égal à 2, 3, ou 4, chaque $R^F$ peut être identique ou différent, et deux $R^F$ sur le même atome de carbone, conjointement avec l'atome de carbone auquel ils sont chacun liés, peuvent former un cycle spirannique consistant en un hétérocycle saturé de 4 à 8 chaînons ou un cycle carboné saturé de 3 à 8 chaînons, ou deux $R^F$ sur différents atomes de carbone peuvent se lier ensemble pour former une liaison de réticulation.

**12.** Composé selon la revendication 11 ou un sel pharmaceutiquement acceptable de celui-ci, dans lequel $R^2$ est un atome d'hydrogène, un alkyle en $C_{1-6}$ éventuellement substitué, ou un cycloalkyle en $C_{3-10}$.

**13.** Composé selon la revendication 11 ou un sel pharmaceutiquement acceptable de celui-ci, dans lequel $R^2$ est un alkyle en $C_{1-6}$ éventuellement substitué.

**14.** Composé selon l'une quelconque des revendications 11 à 13 ou un sel pharmaceutiquement acceptable de celui-ci, dans lequel 1 et m sont chacun indépendamment égaux à 1 ou 2.

**15.** Composé selon la revendication 1, dans lequel Z est $-OR^7$, et $R^4$ et $R^7$, conjointement avec l'atome de carbone et l'atome d'oxygène auxquels ils sont respectivement liés, forment un hétérocycle saturé de 5 à 8 chaînons éventuellement substitué, ou un sel pharmaceutiquement acceptable de celui-ci.

**16.** Composé selon la revendication 1 ou 15 ou un sel pharmaceutiquement acceptable de celui-ci, dans lequel la formule (1) est représentée par la formule suivante (1c) :

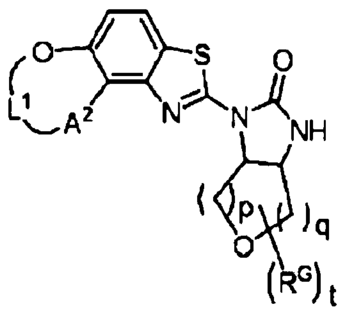
(1c)

dans laquelle

$A^2$ représente un méthylène éventuellement substitué ou un atome d'oxygène,
$L^1$ représente un méthylène éventuellement substitué ou un éthylène éventuellement substitué,
p et q représentent chacun indépendamment 1, 2, ou 3, dans lequel la somme de p et q est égale à 5 ou moins,
t représente 1, 2, 3, ou 4,
$R^G$ représente un atome d'hydrogène, un atome d'halogène, un alkyle en $C_{1-6}$ éventuellement substitué, un alcoxy en $C_{1-6}$ éventuellement substitué, ou un groupe CN,
lorsque t est égal à 2, 3, ou 4, chaque $R^G$ peut être identique ou différent, et deux $R^G$ sur le même atome de carbone, conjointement avec l'atome de carbone auquel ils sont chacun liés, peuvent former un cycle spirannique consistant en un hétérocycle saturé de 4 à 8 chaînons ou un cycle carboné saturé de 3 à 8 chaînons, ou deux $R^G$ sur différents atomes de carbone peuvent se lier ensemble pour former une liaison de réticulation.

**17.** Composé selon l'une quelconque des revendications 1 à 16 ou un sel pharmaceutiquement acceptable de celui-ci, dans lequel le composé est choisi dans le groupe des composés suivants :

cis-3-(7,8-dihydrofuro[3,2-e][1,3]benzothiazol-2-yl)-5-méthyloctahydro-2H-imidazo[4,5-c]pyridin-2-one (Exemple 7) ;
cis-3-(7,8-dihydrofuro[3,2-e][1,3]benzothiazol-2-yl)-5-(2-méthoxyéthyl)octahydro-2H-imidazo[4,5-c]pyridin-2-one (Exemple 8) ;
cis-1-(7,8-dihydrofuro[3,2-e][1,3]benzothiazol-2-yl)-5-méthyloctahydro-2H-imidazo[4,5-c]pyridin-2-one (Exemple 9) ;
cis-3-(7,8-dihydrofuro[3,2-e][1,3]benzothiazol-2-yl)-5-éthyloctahydro-2H-imidazo[4,5-c]pyridin-2-one (Exemple 11) ;
cis-1-(7,8-dihydrofuro[3,2-e][1,3]benzothiazol-2-yl)-5-méthylhexahydropyrrolo[3,4-d]imidazol-2(1H)-one (Exemple 14) ;
cis-3-(7,8-dihydrofuro[3,2-e][1,3]benzothiazol-2-yl)-5-(propan-2-yl)octahydro-2H-imidazo[4,5-c]pyridin-2-one (Exemple 15) ;
cis-3-(7,8-dihydrofuro[3,2-e][1,3]benzothiazol-2-yl)-5-méthyloctahydroimidazo[4,5-c]azépin-2(1H)-one (Exemple 17) ;
(3aS,7aS)-3-(7,8-dihydrofuro[3,2-e][1,3]benzothiazol-2-yl)-5-(oxétan-3-yl)octahydro-2H-imidazo[4,5-c]pyridin-2-one (Exemple 19) ;
(3aR,7aR)-1-(2H-[1,3]dioxolo[4,5-e][1,3]benzothiazol-7-yl)hexahydropyrano[3,4-d]imidazol-2(3H)-one (Exemple 21) ;
(4S,5R)-1-(7,8-dihydrofuro[3,2-e][1,3]benzothiazol-2-yl)-4-méthyl-5-[(morpholin-4-yl)méthyl]imidazolidin-2-one (Exemple 23) ;
(4R,5R)-1-(7,8-dihydrofuro[3,2-e][1,3]benzothiazol-2-yl)-4-méthyl-5-[(morpholin-4-yl)méthyl]imidazolidin-2-one (Exemple 24) ;
(3aR,7aS)-3-(7,8-dihydrofuro[3,2-e][1,3]benzothiazol-2-yl)-5-méthyloctahydro-2H-imidazo[4,5-c]pyridin-2-one (Exemple 28) ;

(3aR,7aS)-5-cyclopropyl-3-(7,8-dihydro[1,4]dioxino[2,3-e][1,3]benzothiazol-2-yl)octahydro-2H-imidazo[4,5-c]pyridin-2-one (Exemple 34) ;
(3aR,7aS)-5-cyclopropyl-3-(2H-[1,3]dioxolo[4,5-e][1,3]benzothiazol-7-yl)octahydro-2H-imidazo[4,5-c]pyridin-2-one (Exemple 36) ;
(3aR,7aR)-1-(7,8-dihydro[1,4]dioxino[2,3-e][1,3]benzothiazol-2-yl)hexahydropyrano[3,4-d]imidazol-2(3H)-one (Exemple 37) ;
(3aR,6S,7aR)-1-(7,8-dihydrofuro[3,2-e][1,3]benzothiazol-2-yl)-6-méthylhexahydropyrano[3,4-d]imidazol-2(3H)-one (Exemple 38) ;
(3aR,6S,7aR)-1-(7,8-dihydro[1,4]dioxino[2,3-e][1,3]benzothiazol-2-yl)-6-méthylhexahydropyrano[3,4-d]imidazol-2(3H)-one (Exemple 39) ;
(3aR,6R,7aR)-1-(7,8-dihydrofuro[3,2-e][1,3]benzothiazol-2-yl)-6-(fluorométhyl)hexahydropyrano[3,4-d]imidazol-2(3H)-one (Exemple 40) ;
(3aR,6R,7aR)-1-(7,8-dihydro[1,4]dioxino[2,3-e][1,3]benzothiazol-2-yl)-6-(fluorométhyl)hexahydropyrano[3,4-d]imidazol-2(3H)-one (Exemple 41) ;
(3aR,6S,7aR)-1-(7,8-dihydrofuro[3,2-e][1,3]benzothiazol-2-yl)-6-éthylhexahydropyrano[3,4-d]imidazol-2(3H)-one (Exemple 42) ;
(3aR,6S,7aR)-1-(7,8-dihydro[1,4]dioxino[2,3-e][1,3]benzothiazol-2-yl)-6-éthylhexahydropyrano[3,4-d]imidazol-2(3H)-one (Exemple 43) ;
(4S,5S)-1-(7,8-dihydrofuro[3,2-e][1,3]benzothiazol-2-yl)-5-(méthoxyméthyl)-4-méthylimidazolidin-2-one (Exemple 44) ;
(3aR,6aS)-1-(7,8-dihydrofuro[3,2-e][1,3]benzothiazol-2-yl)tétrahydro-1H-furo[3,4-d]imidazol-2(3H)-one (Exemple 65) ;
rac-(3aR,7aS)-3-(7,8-dihydrofuro[3,2-e][1,3]benzothiazol-2-yl)-5-(oxétan-3-yl)octahydro-2H-imidazo[4,5-c]pyridin-2-one (Exemple 71) ;
rac-(3aR,7aS)-5-cyclobutyl-3-(7,8-dihydrofuro[3,2-e][1,3]benzothiazol-2-yl)octahydro-2H-imidazo[4,5-c]pyridin-2-one (Exemple 72) ;
rac-(3aR,8aS)-3-(7,8-dihydrofuro[3,2-e][1,3]benzothiazol-2-yl)-5-(oxétan-3-yl)octahydroimidazo[4,5-c]azépin-2(1H)-one (Exemple 73) ;
rac-(3aR,7aS)-3-(7,8-dihydro[1,4]dioxino[2,3-e][1,3]benzothiazol-2-yl)-5-méthyloctahydro-2H-imidazo[4,5-c]pyridin-2-one (Exemple 76) ;
rac-(3aR,7aS)-1-(7,8-dihydro[1,4]dioxino[2,3-e][1,3]benzothiazol-2-yl)-5-méthyloctahydro-2H-imidazo[4,5-c]pyridin-2-one (Exemple 77) ;
rac-(3aR,7aS)-1-(2H-[1,3]dioxolo[4,5-e][1,3]benzothiazol-7-yl)-5-méthyloctahydro-2H-imidazo[4,5-c]pyridin-2-one (Exemple 78) ;
rac-(3aR,7aS)-3-(2H-[1,3]dioxolo[4,5-e][1,3]benzothiazol-7-yl)-5-méthyloctahydro-2H-imidazo[4,5-c]pyridin-2-one (Exemple 79) ;
rac-(3aR,7aS)-3-(7,8-dihydro[1,4]dioxino[2,3-e][1,3]benzothiazol-2-yl)-5-(oxétan-3-yl)octahydro-2H-imidazo[4,5-c]pyridin-2-one (Exemple 80) ;
rac-(3aR,7aS)-3-(2H-[1,3]dioxolo[4,5-e][1,3]benzothiazol-7-yl)-5-(oxétan-3-yl)octahydro-2H-imidazo[4,5-c]pyridin-2-one (Exemple 81) ;
rac-(3aR,8aS)-3-(2H-[1,3]dioxolo[4,5-e][1,3]benzothiazol-7-yl)-5-méthyloctahydroimidazo[4,5-c]azépin-2(1H)-one (Exemple 85) ;
rac-(3aR,8aS)-3-(2H-[1,3]dioxolo[4,5-e][1,3]benzothiazol-7-yl)-5-(oxétan-3-yl)octahydroimidazo[4,5-c]azépin-2(1H)-one (Exemple 86) ;
(3aS,6S,7aR)-1-(7,8-dihydrofuro[3,2-e][1,3]benzothiazol-2-yl)-5,6-diméthyloctahydro-2H-imidazo[4,5-c]pyridin-2-one (Exemple 93) ;
rac-(3aR,7aS)-5-cyclopropyl-3-(7,8-dihydrofuro[3,2-e][1,3]benzothiazol-2-yl)octahydro-2H-imidazo[4,5-c]pyridin-2-one (Exemple 98) ;
rac-(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Exemple 99) ;
rac-[(3aR,7aS)-3-(7,8-dihydrofuro[3,2-e][1,3]benzothiazol-2-yl)-2-oxooctahydro-5H-imidazo[4,5-c]pyridin-5-yl]acétonitrile (Exemple 100) ;
rac-(3aR,6aS)-1-(7,8-dihydrofuro[3,2-e][1,3]benzothiazol-2-yl)-5-(2-propyn-1-yl)hexahydropyrrolo[3,4-d]imidazol-2(1H)-one (Exemple 102) ;
rac-[(3aR,6aS)-1-(7,8-dihydrofuro[3,2-e][1,3]benzothiazol-2-yl)-2-oxohexahydropyrrolo[3,4-d]imidazol-5(1H)-yl]acétonitrile (Exemple 103) ;
rac-(3aR,8aS)-5-cyclopropyl-3-(7,8-dihydrofuro[3,2-e][1,3]benzothiazol-2-yl)octahydroimidazo[4,5-c]azépin-2(1H)-one (Exemple 104) ;

rac-(3aR,8aS)-3-(7,8-dihydrofuro[3,2-e][1,3]benzothiazol-2-yl)-5-(2-propyn-1-yl)octahydroimidazo[4,5-c]azépin-2(1H)-one (Exemple 105) ;
rac-[(3aR,8aS)-3-(7,8-dihydrofuro[3,2-e][1,3]benzothiazol-2-yl)-2-oxooctahydroimidazo[4,5-c]azépin-5(1H)-yl] acétonitrile (Exemple 106) ;
rac-(3aR,7aS)-3-(7,8-dihydrofuro[3,2-e][1,3]benzothiazol-2-yl)-5-(2,2,2-trifluoroéthyl)octahydro-2H-imidazo [4,5-c]pyridin-2-one (Exemple 107) ;
rac-(3aR,7aS)-5-(2,2-difluoroéthyl)-3-(7,8-dihydrofuro[3,2-e][1,3]benzothiazol-2-yl)octahydro-2H-imidazo[4,5-c]pyridin-2-one (Exemple 108) ;
(3aR,7aS)-5-cyclopropyl-3-(7,8-dihydrofuro[3,2-e][1,3]benzothiazol-2-yl)octahydro-2H-imidazo[4,5-c]pyridin-2-one (Exemple 110) ;
[(3aR,7aS)-3-(7,8-dihydrofuro[3,2-e][1,3]benzothiazol-2-yl)-2-oxooctahydro-5H-imidazo[4,5-c]pyridin-5-yl] acétonitrile (Exemple 112) ;
(3aR,7aS)-3-(7,8-dihydrofuro[3,2-e][1,3]benzothiazol-2-yl)-5-(2-propyn-1-yl)octahydro-2H-imidazo[4,5-c]pyridin-2-one (Exemple 114) ;
(4S,5R)-1-(7,8-dihydrofuro[3,2-e][1,3]benzothiazol-2-yl)-5-{[(3S)-3-fluoropyrrolidin-1-yl]méthyl}-4-méthylimidazolidin-2-one (Exemple 115) ;
(4S,5R)-5-[(3,3-difluoroazétidin-1-yl)méthyl]-1-(7,8-dihydrofuro[3,2-e][1,3]benzothiazol-2-yl)-4-méthylimidazolidin-2-one (Exemple 116) ;
(4S,5R)-5-[(3,3-difluoropyrrolidin-1-yl)méthyl]-1-(7,8-dihydrofuro[3,2-e][1,3]benzothiazol-2-yl)-4-méthylimidazolidin-2-one (Exemple 117) ;
(4S,5R)-1-(7,8-dihydrofuro[3,2-e][1,3]benzothiazol-2-yl)-5-[(3-fluoroazétidin-1-yl)méthyl]-4-méthylimidazolidin-2-one (Exemple 118) ;
rac-(3aR,7aR)-3-(7,8-dihydrofuro[3,2-e][1,3]benzothiazol-2-yl)hexahydropyrano[3,4-d]imidazol-2(3H)-one (Exemple 120) ;
rac-(3aR,7aR)-1-(7,8-dihydrofuro[3,2-e][1,3]benzothiazol-2-yl)hexahydropyrano[3,4-d]imidazol-2(3H)-one (Exemple 121) ;
(3aR,7aR)-1-(7,8-dihydrofuro[3,2-e][1,3]benzothiazol-2-yl)hexahydropyrano[3,4-d]imidazol-2(3H)-one (Exemple 122) ;
(3aR,7aR)-1-(7,8-dihydrofuro[3,2-e][1,3]benzothiazol-2-yl)-6,6-diméthylhexahydropyrano[3,4-d]imidazol-2(3H)-one (Exemple 124) ;
(3aR,6R,7aR)-1-(7,8-dihydrofuro[3,2-e][1,3]benzothiazol-2-yl)-2-oxooctahydropyrano[3,4-d]imidazole-6-carbonitrile (Exemple 127) ;
(3aR,6R,7aR)-1-(7,8-dihydrofuro[3,2-e][1,3]benzothiazol-2-yl)-6-méthoxyhexahydropyrano[3,4-d]imidazol-2(3H)-one (Exemple 129) ;
(3aR,6R,7aR)-1-(2H-[1,3]dioxolo[4,5-e][1,3]benzothiazol-7-yl)-2-oxooctahydropyrano[3,4-d]imidazole-6-carbonitrile (Exemple 131) ;
(3aR,6R,7aR)-1-(7,8-dihydro[1,4]dioxino[2,3-e][1,3]benzothiazol-2-yl)-2-oxooctahydropyrano[3,4-d]imidazole-6-carbonitrile (Exemple 133) ;
(3aR,8aR)-1-(7,8-dihydro[1,4]dioxino[2,3-e][1,3]benzothiazol-2-yl)hexahydro-1H-oxépino[3,4-d]imidazol-2(3H)-one (Exemple 135) ;
(3aR,6S,7aR)-1-(2H-[1,3]dioxolo[4,5-e][1,3]benzothiazol-7-yl)-6-éthylhexahydropyrano[3,4-d]imidazol-2(3H)-one (Exemple 138) ;
(3aR,6S,7aR)-1-(2H-[1,3]dioxolo[4,5-e][1,3]benzothiazol-7-yl]-6-méthylhexahydropyrano[3,4-d]imidazol-2(3H)-one (Exemple 139) ;
(3aR,6R,7aR)-1-(2H-[1,3]dioxolo[4,5-e][1,3]benzothiazol-7-yl]-6-(fluorométhyl)hexahydropyrano[3,4-d]imidazol-2(3H)-one (Exemple 140) ;
(3aR,6R,7aR)-1-(7,8-dihydrofuro[3,2-e][1,3]benzothiazol-2-yl)-6-(hydroxyméthyl)hexahydropyrano[3,4-d]imidazol-2(3H)-one (Exemple 144) ;
(3aR,6R,7aR)-1-(7,8-dihydro[1,4]dioxino[2,3-e][1,3]benzothiazol-2-yl)-6-(hydroxyméthyl)hexahydropyrano [3,4-d]imidazol-2(3H)-one (Exemple 146) ;
(3aR,6R,7aR)-1-(2H-[1,3]dioxolo[4,5-e][1,3]benzothiazol-7-yl)-6-(hydroxyméthyl)hexahydropyrano[3,4-d]imidazol-2(3H)-one (Exemple 147) ;
(3aR,6R,7aR)-1-(7,8-dihydrofuro[3,2-e][1,3]benzothiazol-2-yl)-6-éthynylhexahydropyrano[3,4-d]imidazol-2(3H)-one (Exemple 149) ;
(3aR,7aR)-1-(7,8-dihydrofuro[3,2-e][1,3]benzothiazol-2-yl)-6-fluorohexahydropyrano[3,4-d]imidazol-2(3H)-one (Exemple 151) ;
(3aR,6S,6aS)-1-(7,8-dihydrofuro[3,2-e][1,3]benzothiazol-2-yl)-6-(hydroxyméthyl)tétrahydro-1H-furo[3,4-d]imidazol-2(3H)-one (Exemple 157) ;

(3aR,6S,6aS)-1-(7,8-dihydrofuro[3,2-e][1,3]benzothiazol-2-yl)-6-(fluorométhyl)tétrahydro-1H-furo[3,4-d]imidazol-2(3H)-one (Exemple 159) ;
(3aR,6R,6aS)-1-(7,8-dihydrofuro[3,2-e][1,3]benzothiazol-2-yl)-6-méthyltétrahydro-1H-furo[3,4-d]imidazol-2(3H)-one (Exemple 161) ; et
[(3aS,4R,6aR)-3-(7,8-dihydrofuro[3,2-e][1,3]benzothiazol-2-yl)-2-oxohexahydro-1H-furo[3,4-d]imidazol-4-yl] acétonitrile (Exemple 162).

**18.** Médicament comprenant le composé selon l'une quelconque des revendications 1 à 17 ou un sel pharmaceutiquement acceptable de celui-ci en tant que principe actif.

**19.** Composition pharmaceutique comprenant le composé selon l'une quelconque des revendications 1 à 17 ou un sel pharmaceutiquement acceptable de celui-ci en tant que principe actif.

**20.** Agent thérapeutique et/ou agent prophylactique pour son utilisation dans la prévention ou le traitement d'une maladie impliquant DYRK, comprenant le composé selon l'une quelconque des revendications 1 à 17 ou un sel pharmaceutiquement acceptable de celui-ci en tant que principe actif.

**21.** Agent thérapeutique et/ou agent prophylactique selon la revendication 20, dans lequel la maladie impliquant DYRK est la démence fronto-temporale, la paralysie supranucléaire progressive, la dégénérescence cortico-basale, la maladie à corps de Lewy, la démence vasculaire, le traumatisme crânien, l'encéphalopathie traumatique chronique, l'accident vasculaire cérébral, la maladie d'Alzheimer, la maladie de Parkinson, le syndrome de Down, ou la dépression, et le retard mental, les troubles de la mémoire, la perte de mémoire, le trouble de l'apprentissage, le handicap mental, les dysfonctionnements cognitifs, les troubles cognitifs légers, ou le symptôme de la démence associé à ceux-ci, ou la tumeur cérébrale, le cancer du pancréas, le cancer des ovaires, l'ostéosarcome, le cancer du gros intestin, le cancer des poumons, les maladies à résorption osseuse, l'ostéoporose, la drépanocytose, les maladies rénales chroniques, ou les maladies à résorption osseuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201010797 A
- WO 201326806 A
- WO 2013043001 A
- US 200619965 B
- US 20131503254 A
- EP 1679308 A
- WO 2015061572 A
- WO 20073965 A
- US 2010216812 A
- WO 2008136457 A
- WO 2014015905 A
- WO 2015177326 A
- WO 2016044641 A
- WO 2012061418 A
- WO 2010097248 A


### Non-patent literature cited in the description


- **BECKER W. et al.** *J.Biol.Chem.*, 1998, vol. 273, 25893-25902
- **KIMURA R. et al.** *Hum.Mol.Genet.*, 2007, vol. 16, 15-23
- **RYOO SR. et al.** *J.Biol.Chem.*, 2007, vol. 282, 34850-34857
- **NARENDRA D. et al.** *J.Cell.Biol.*, 2008, vol. 183, 795-803
- **IM E**. *J.Neurochem.*, 2015, vol. 134, 756-768
- **BRANCHI I. et al.** *J. Neuropathol. Exp. Neurol.*, 2004, vol. 63, 429-440
- **DOWJAT WK. et al.** *Neurosci.Lett.*, 2007, vol. 413, 77-81
- **WEGIEL J. et al.** *FEBS J.*, 2011, vol. 278, 236-245
- **POZO N. et al.** *J.Clin.Invest.*, 2013, vol. 123, 2475-2487
- **DENG X. et al.** *Cancer Res.*, 2006, vol. 66, 4149-4158
- **EWTON DZ et al.** *Mol.Cancer Ther.*, 2011, vol. 10, 2104-2114
- **DENG X. et al.** *Genes Cancer.*, 2014, vol. 5, 201-211
- **YANG C. et al.** *Carcinogenesis.*, 2010, vol. 31, 522-528
- **JIN K. et al.** *J.Biol.Chem.*, 2009, vol. 284, 22916-22925
- **GAO J et al.** *Cancer Cell Int.*, 2013, vol. 13, 2
- **TAIRA N. et al.** *Mol.Cell.*, 2007, vol. 25, 725-738
- **BOGACHEVA O. et al.** *J.Biol.Chem.*, 2008, vol. 283, 36665-36675
- **T.W. GREENE** ; **P.G.M. WUTS**. Protective Groups in Organic Synthesis. John Wiley and Sons, inc., 1999
- *Chemical & Pharmaceutical Bulletin*, 2007, 1406
- *Advanced Synthesis & Catalysis*, 2005, vol. 1643
- *Bioorganic & Medicinal Chemistry Letters*, 2007, vol. 28
- *J. Org. Chem.*, 1986, vol. 2613
- *Tetrahedron Letters*, 2015, vol. 946
- *Synlett*, 1999, vol. 426
- *Journal of Organic Chemistry*, 2003, vol. 8693
- *Organic Letters*, 2015, vol. 5136
- *Bioorganic & Medicinal Chemistry*, 2008, vol. 822
- *Journal of Medicinal Chemistry*, 2003, vol. 3680
- *Journal of Organic Chemistry*, 1999, vol. 2564
- *Organic Letters*, 2004, vol. 4739
- *Chemical Communications*, 2015, vol. 7693
- *Journal of Medicinal Chemistry*, 1995, vol. 3918
- *Bioorganic & Medicinal Chemistry Letters*, 2007, vol. 5227
- *Journal of Organic Chemistry*, 1986, vol. 2613
- *Chemical Communications*, 2004, vol. 446
- *Journal of Organic Chemistry*, 2009, vol. 8719
- *Journal of the American Chemical Society*, 1996, vol. 12521
- *Green Chemistry*, 2005, vol. 451